# EUROPEAN PATENT APPLICATION

(11) **EP 2 175 005 A1**
(43) Date of publication of application: **14.04.2010**
(21) Application number: 09156611.7
(22) Date of filing: 30.03.2009
(51) Int. Cl.: C09K 11/06

(54) **Novel organic electroluminescent compounds and organic electroluminescent device using the same**

(30) Priority: 13.10.2008 KR 20080100027
(71) Applicant: Gracel Display Inc., Seoul 133-833 (KR)
(72) Inventor: KIM, Young Gil, 431-075, Gyeonggi-do (KR); CHO, Young Jun, 136-060, Seoul (KR); KIM, Chi-Sik, 156-825, Seoul (KR); EUM, Sung Jin, , Seoul (KR); KWON, Hyuck Joo, 130-100, Seoul (KR); KIM, Bong Ok, 135-090, Seoul (KR); KIM, Sung Min, 157-886, Seoul (KR); YOON, Seung Soo, 135-884, Seoul (KR)
(74) Representative: Kent, Venetia Katherine

(57) **Abstract**

Provided are novel organic electroluminescent compounds, and organic electroluminescent devices comprising the same as electroluminescent material. Specifically, the organic electroluminescent compounds according to the present invention are represented by Chemical Formula (1): 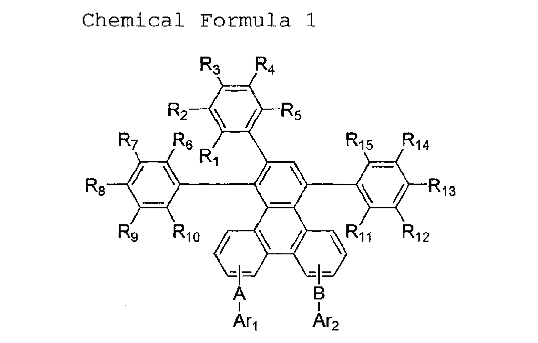 wherein, at least one substituent(s) among Ar₁, Ar₂, R₆, R₇, R₈, R₉, R₁₀, R₁₁, R₁₂, R₁₃, R₁₄ and R₁₅ represent (s)

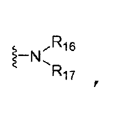

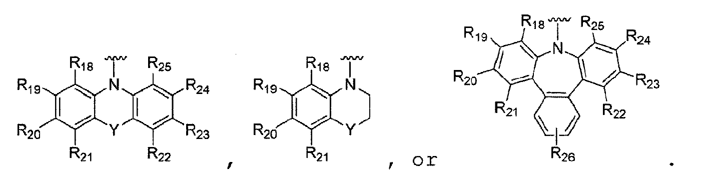

The organic electroluminescent compounds according to the present invention exhibit high luminous efficiency and excellent life property of material, so that an OLED having very good operation life can be manufactured therefrom.

Further, the organic electroluminescent compound according to the present invention, when it is contained in an electroluminescent layer or a hole transport layer, lowers the operation voltage to result in noticeable decrease in power consumption, with exhibiting at least comparable luminous efficiency as compared to conventional OLED's.

## Description

### FIELD OF THE INVENTION

The present invention relates to novel organic electroluminescent compounds and organic electroluminescent devices employing the same in an electroluminescent layer. More specifically, the invention relates to novel organic electroluminescent compounds to be employed as blue electroluminescent material, and organic electroluminescent devices comprising the same in an electroluminescent layer or a hole transport layer.

### BACKGROUND OF THE INVENTION

Among display devices, electroluminescence devices (EL devices) are self-luminescent display devices showing the advantage of wide angle of view, excellent contrast and rapid response rate. Eastman Kodak developed in 1987 an organic EL device which employs a low molecular weight aromatic diamine and an aluminum complex as material for forming an EL layer, for the first time [Appl. Phys. Lett. 51, 913, 1987].

An organic EL device is a device wherein, when charge is applied to an organic film formed between an electron injection electrode (cathode) and a hole injection electrode (anode), an electron and a hole form a pair and then become extinct with emitting light. A device can be formed on a transparent flexible substrate such as plastics. The device can be operated at a lower voltage (not more than 10 V) with relatively lower power consumption but excellent color purity, as compared to a plasma display panel or an inorganic EL display.

Since the organic electroluminescent (EL) devices can develop three colors (green, blue and red), they have been focused as full colored display devices for next generation.

The procedure for manufacturing an organic EL device comprises the following steps:
(1) First, anode material is coated on a transparent substrate. As the anode material, ITO (indium tin oxide) is usually employed.
(2) A hole injecting layer (HIL) is coated thereon. As the hole injecting layer, it is common to coat copper phthalocyanine (CuPc) with a thickness of 10 nm to 30 nm.
(3) Then, a hole transport layer (HTL) is introduced. As the hole transport layer, 4,4'-bis[N-(1-naphthyl)-N-phenylamino]biphenyl (NPB) is vapor-deposited with a thickness of about 30 nm to 60 nm.
(4) An organic emitting layer is formed thereon. If required, dopant is added. In case of green electroluminescence, tris(8-hydroxyquinolate)aluminium (Alq₃) is commonly vapor-deposited with a thickness of 30 to 60 nm as the organic emitting layer, and MQD(N-methylquinacridone) is usually employed as dopant.
(5) An electron transport layer (ETL) and an electron injecting layer (EIL) is then sequentially coated thereon, or an electron injecting/transport layer is formed. In case of green electroluminescence, since Alq₃ of (4) has good capability of electron transport, an electron injecting/transport layer may not be necessarily employed.
(6) Then, a cathode is coated, and finally passivation is carried out.

Depending upon how the emitting layer is formed in such a structure, a blue, green or red electroluminescent device can be realized. In the meanwhile, conventional substances used as green electroluminescent compound for realizing a green electroluminescent device had problems of insufficient life and poor luminous efficiency.

The most important factor to determine luminous efficiency, lifetime or the like in an organic EL device is electroluminescent material. Several properties required for such electroluminescent materials include that the material should have high fluorescent quantum yield in solid state and high mobility of electrons and holes, is not easily decomposed during vapor-deposition in vacuo, and forms uniform and stable thin film.

Organic electroluminescent materials can be generally classified into high-molecular materials and low-molecular materials. The low-molecular materials include metal complexes and thoroughly organic electroluminescent materials which do not contain metal, in view of molecular structure. Such electroluminescent materials include chelate complexes such as tris(8-quinolinolato)aluminum complexes, coumarin derivatives, tetraphenylbutadiene derivatives, bis(styrylarylene) derivatives and oxadiazole derivatives. From those materials, it is reported that light emission of visible region from blue to red can be obtained, so that realization of full-colored display devices is anticipated thereby.

In the meanwhile, for conventional blue materials, a number of materials have been developed and commercialized since the development of diphenylvinyl-biphenyl (DPVBi) (Compound a) by Idemitsu-Kosan. In addition to the blue material system from Idemitsu-Kosan, dinaphthylanthracene (DNA) (Compound b) of Kodac, tetra(t-butyl)perylene (Compound c) system or the like have been known. However, extensive research and development should be performed with respect to these materials. The distryl compound system of Idemitsu-Kosan, which is known to have highest efficiency up to now, has 6 lm/W of power efficiency and beneficial device lifetime of more than 30,000 hr. However, when it is applied to a full-colored display, the lifetime is merely several thousand hours, owing to decrease of color purity over operation time. In case of blue electroluminescence, it becomes advantageous from the aspect of the luminous efficiency, if the electroluminescent wavelength is shifted a little toward longer wavelength. However, it is not easy to apply the material to a display of high quality because of unsatisfactory color purity in blue. Furthermore, the research and development of such materials are urgent because of the problems in color purity, efficiency and thermal stability.

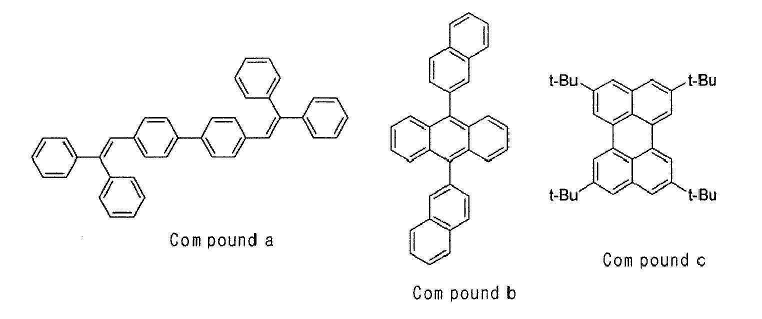

### SUMMARY OF THE INVENTION

With intensive efforts to overcome the problems of conventional techniques as described above, the present inventors have invented novel electroluminescent compounds to realize an organic electroluminescent device having excellent luminous efficiency and noticeably improved lifetime.

The object of the present invention is to provide organic electroluminescent compounds having the backbone to give more excellent electroluminescent property, longer device life and appropriate color coordinate, as compared to those of conventional dopant materials, with overcoming disadvantages of them.

Another object of the invention is to provide organic electroluminescent devices of high efficiency and long life, which employ said organic electroluminescent compounds as electroluminescent material. Still another object of the invention is to provide an organic electroluminescent device employing the novel organic electroluminescent in a hole injecting layer or an electroluminescent layer.

Yet still another object of the invention is to provide organic solar cells comprising the novel organic electroluminescent compounds.

The present invention relates to organic electroluminescent compounds represented by Chemical Formula (1), and organic electroluminescent devices comprising the same. Since the organic electroluminescent compounds according to the invention show good luminous efficiency and excellent color purity and life property of material, OLED's having very good operation life can be manufactured therefrom. 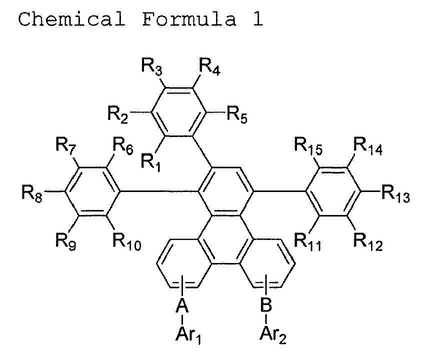 wherein, A and B independently represent a chemical bond, (C6-C60)arylene, (C3-C60)heteroarylene, (C6-C60)arylenoxy, (C1-C60)alkylenoxy, (C6-C60)arylenethio, (C1-C60)alkylenethio or (C1-C60)alkylene;
Ar₁ and Ar₂ independently represent hydrogen or deuterium, or a substituent selected from the following structures: 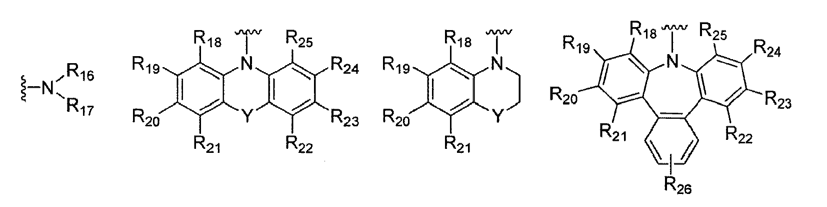 R₁ through R₅ independently represent hydrogen, deuterium, halogen, (C1-C60)alkyl, (C6-C60)aryl, (C3-C60)heteroaryl containing one or more heteroatom(s) selected from N, O and S, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkyl(C6-C60)aryl, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or each of them may be linked to an adjacent substituent via (C3-C60)alkylene or (C3-C60)alkenylene with or without a fused ring to form an alicyclic ring, or a monocyclic or polycyclic aromatic ring;
R₆ through R₁₅ independently represent hydrogen, deuterium, halogen, (C1-C60)alkyl, (C6-C60)aryl, (C3-C60)heteroaryl containing one or more heteroatom(s) selected from N, O and S, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkyl(C6-C60)aryl, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro, hydroxyl, 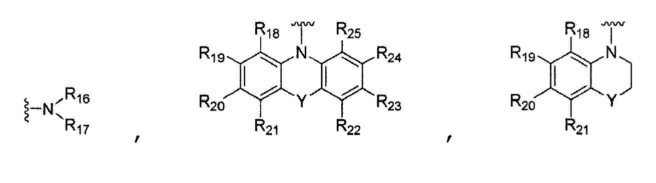 or 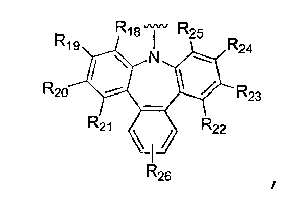 or each of them may be linked to an adjacent substituent via (C3-C60)alkylene or (C3-C60)alkenylene with or without a fused ring to form an alicyclic ring, or a monocyclic or polycyclic aromatic ring;
R₁₆ and R₁₇ independently represent (C6-C60)aryl, (C3-C60)heteroaryl containing one or more heteroatom(s) selected
from N, O and S, 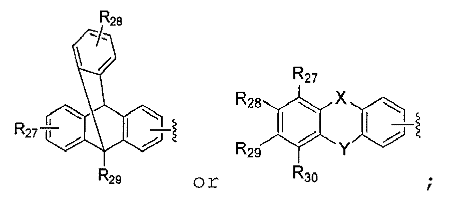 R₁₈ through R₂₆ and R₂₇ to R₃₀ independently represent hydrogen, deuterium, halogen, (C1-C60)alkyl, (C6-C60)aryl, (C3-C60)heteroaryl containing one or more heteroatom(s) selected from N, O and S, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkyl(C6-C60)aryl, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or each of them may be linked to an adjacent substituent via (C3-C60)alkylene or (C3-C60)alkenylene with or without a fused ring to form an alicyclic ring, or a monocyclic or polycyclic aromatic ring;
X and Y independently represent a chemical bond, - (CR₃₁R₃₂)ₘ-, -N(R₃₃)-, -S-, -O-, -Si(R₃₄)(R₃₅)-_{,} -P(R₃₆)-, -C(=O)-, -B(R₃₇)-, -In(R₃₈)-, -Se-, -Ge(R₃₉) (R₄₀)-, -Sn(R₄₁) (R₄₂)-, - Ga(R₄₃)- or -(R₄₄)C=C(R₄₅)-; excluding that both X and Y represent chemical bonds;
R₃₁ through R₄₅ independently represent hydrogen, deuterium, halogen, (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C1-C60)alkyl(C6-C60)aryl, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or R₃₁ and R₃₂, R₃₄ and R₃₅, R₃₉ and R₄₀, R₄₁ and R₄₂, or R₄₄ and R₄₅ may be linked via (C3-C60)alkylene or (C3-C60)alkenylene with or without a fused ring to form an alicyclic ring, or a monocyclic or polycyclic aromatic ring;
the arylene, heteroarylene, arylenethio, arylenoxy, alkylenoxy or alkylenethio of A and B; the alkyl, aryl, heteroaryl, heterocycloalkyl, cycloalkyl, alkylsilyl, arylsilyl, alkenyl, alkynyl, alkylamino or arylamino of R₁ through R₄₅ may be further substituted by one or more substituent(s) selected from a group consisting of halogen, (C1-C60)alkyl, halo(C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, carbazolyl, (C1-C60)alkylamino, (C6-C60)arylamino, (C1-C60)alkyl(C6-C60)aryl, (C6-C60)ar(C1-C60)alkyl, (C1-C60)alkyl(C6-C60)aryl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro and hydroxyl;
m is an integer from 1 to 4;
provided that, at least one substituent(s) among Ar₁, Ar₂, R₆, R₇, R₈, R₉, R₁₀, R₁₁, R₁₂, R₁₃, R₁₄ and R₁₅ represent (s)

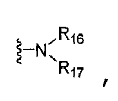

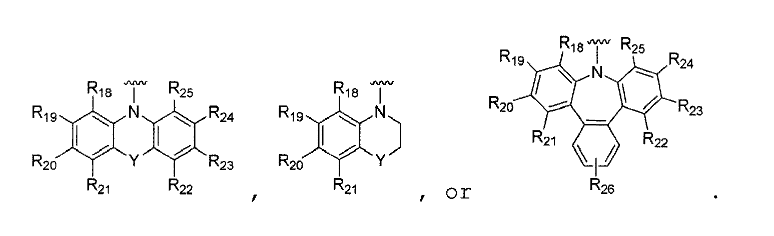

### BRIEF DESCRIPTION OF THE DRAWINGS

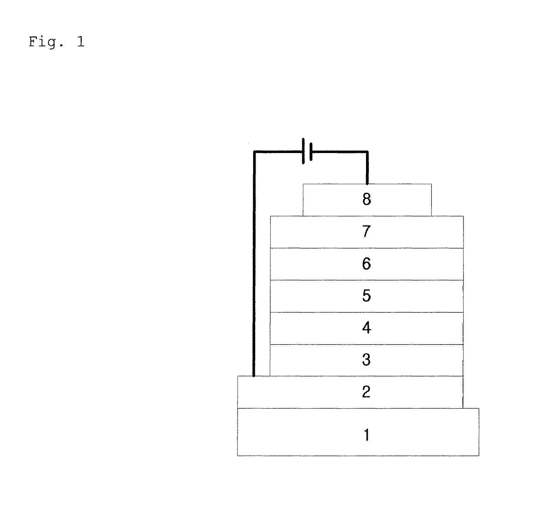

Fig. 1 is a cross-sectional view of an OLED.

### DETAILED DESCRIPTION OF THE INVENTION

Referring now to the Drawings, Fig. 1 illustrates a cross-sectional view of an OLED of the present invention comprising a Glass 1, Transparent electrode 2, Hole injecting layer 3, Hole transport layer 4, Electroluminescent layer 5, Electron transport layer 6, Electron injecting layer 7 and Al cathode 8.

The term "alkyl", "alkoxy" and any other substituents comprising "alkyl" moiety include linear and branched species.

The term "aryl" described herein means an organic radical derived from aromatic hydrocarbon via elimination of one hydrogen atom. Each ring suitably comprises a monocyclic or fused ring system containing from 4 to 7, preferably from 5 to 6 cyclic atoms. Specific examples include phenyl, naphthyl, biphenyl, anthryl, tetrahydronaphthyl, indenyl, fluorenyl, phenanthryl, triphenylenyl, pyrenyl, perylenyl, chrysenyl, naphthacenyl and fluoranthenyl, but they are not restricted thereto.

The term "heteroaryl" described herein means an aryl group containing from 1 to 4 heteroatom(s) selected from N, O and S for the aromatic cyclic backbone atoms, and carbon atom(s) for remaining aromatic cyclic backbone atoms. The heteroaryl may be 5- or 6-membered monocyclic heteroaryl or a polycyclic heteroaryl which is fused with one or more benzene ring(s), and may be partially saturated. The heteroaryl groups include bivalent aryl group of which the heteroatom in the ring is oxidized or quarternized to form an N-oxide or a quaternary salt. Specific examples include monocyclic heteroaryl groups such as furyl, thiophenyl, pyrrolyl, imidazolyl, pyrazolyl, thiazolyl, thiadiazolyl, isothiazolyl, isoxazolyl, oxazolyl, oxadiazolyl, triazinyl, tetrazinyl, triazinyl, tetrazinyl, triazolyl, tetrazolyl, furazanyl, pyridyl, pyrazinyl, pyrimidinyl, pyridazinyl; polycyclic heteroaryl groups such as benzofuranyl, benzothiophenyl, isobenzofuranyl, benzimidazolyl, benzothiazolyl, benzisothiazolyl, benzisoxazolyl, benzoxazolyl, isoindolyl, indolyl, indazolyl, benzothiadiazolyl, quinolyl, isoquinolyl, cinnolinyl, quinazolinyl, quinolizinyl, quinoxalinyl, carbazolyl, phenanthridinyl and benzodioxolyl; and corresponding N-oxides (e.g., pyridyl N-oxides, quinolyl N-oxides) and quaternary salts thereof; but they are not restricted thereto.

The substituents comprising "(C1-C60)alkyl" moiety described herein may contain 1 to 60 carbon atoms, 1 to 20 carbon atoms, or 1 to 10 carbon atoms. The substituents comprising "(C6-C60)aryl" moiety may contain 6 to 60 carbon atoms, 6 to 20 carbon atoms, or 6 to 12 carbon atoms. The substituents comprising "(C3-C60)heteroaryl" moiety may contain 3 to 60 carbon atoms, 4 to 20 carbon atoms, or 4 to 12 carbon atoms. The substituents comprising "(C3-C60)cycloalkyl" moiety may contain 3 to 60 carbon atoms, 3 to 20 carbon atoms, or 3 to 7 carbon atoms. The substituents comprising "(C2-C60)alkenyl or alkynyl" moiety may contain 2 to 60 carbon atoms, 2 to 20 carbon atoms, or 2 to 10 carbon atoms.

Preferably, A and B independently represent a chemical bond, or arylene represented by one of the following structural formulas: 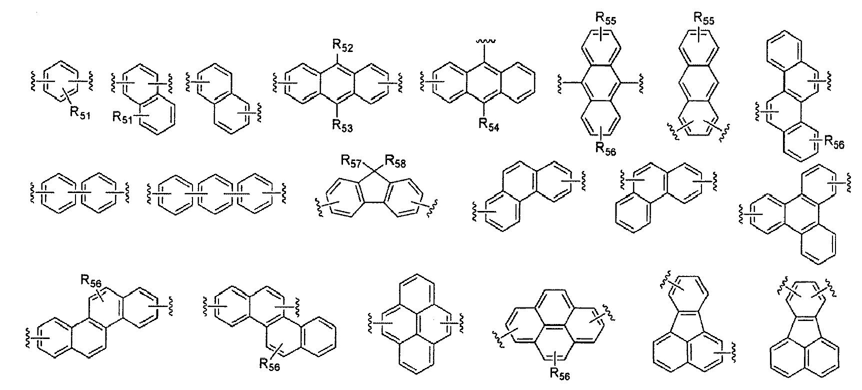 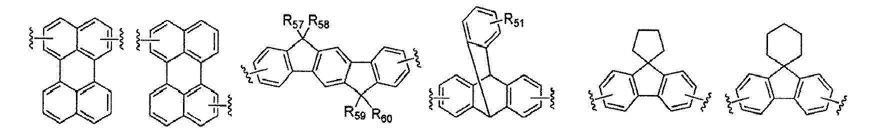 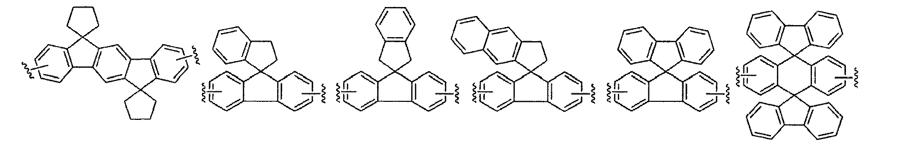 wherein, R₅₁ through R₆₀ independently represent hydrogen, deuterium, halogen, (C1-C60)alkyl, halo(C1-C60)alkyl, (C6-C60)aryl, (C3-C60)heteroaryl containing one or more heteroatom(s) selected from N, O and S, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl; and
the alkyl, aryl, heteroaryl, heterocycloalkyl, cycloalkyl, alkylsilyl, arylsilyl, alkenyl, aralkyl, alkylamino or arylamino of R₅₁ through R₆₀ may be further substituted by one or more substituent(s) selected from a group consisting of halogen, (C1-C60)alkyl, halo(C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, carbazolyl, (C1C60)alkylamino, (C6-C60)arylamino, (C1 C60)alkyl(C6-C60)aryl, (C6-C60)ar(C1C60)alkyl, (C1-C60)alkyl(C6-C60)aryl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro and hydroxyl.

In Chemical Formula (1), Ar₁ and Ar₂ independently represent hydrogen, deuterium, or a substituent represented by one of the following structural formulas, without restriction: 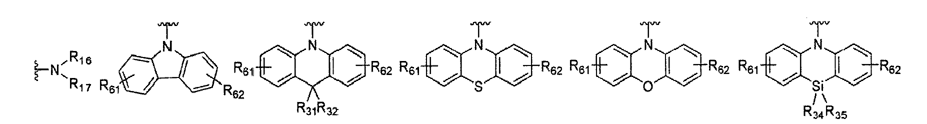 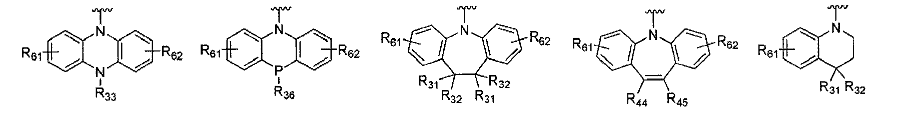 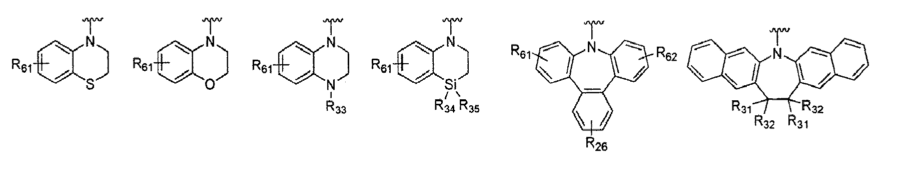 wherein, R₁₆ and R₁₇ are defined as in Chemical Formula (1);
R₂₆, R₃₁ through R₃₆, R₄₄ and R₄₅ independently represent hydrogen, deuterium, halogen, (C1-C60)alkyl, (C6-C60) aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C1-C60)alkyl(C6-C60)aryl, (C6-C60)ar(C1-C60)alkyl, (C1-C60)alkyloxy, (C1-C60)alkylthio, (C6-C60)aryloxy, (C1-C60)arylthio, (C1-C60)alkoxycarbonyl, (C1-C60)alkylcarbonyl, (C6-C60)arylcarbonyl, carboxyl, nitro or hydroxyl, or R₃₁ and R₃₂, or R₃₄ and R₃₅ may be linked via (C3-C60)alkylene or (C3-C60)alkenylene with or without a fused ring to form an alicyclic ring, or a monocyclic or polycyclic aromatic ring; and
R₆₁ and R₆₂ independently represent hydrogen, deuterium, (C1-C60)alkyl or (C6-C60)aryl.

In the chemical formula, R₁₆ and R₁₇ may be independently selected from the following structures, without restriction: 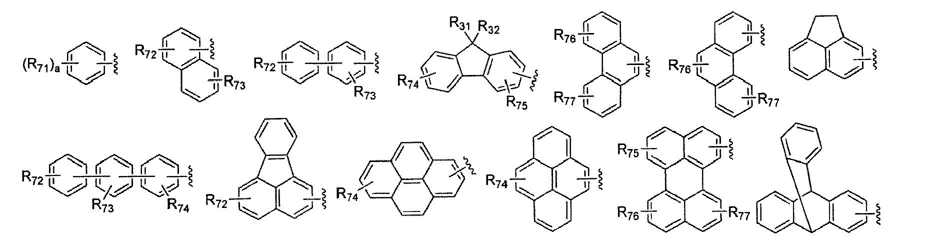 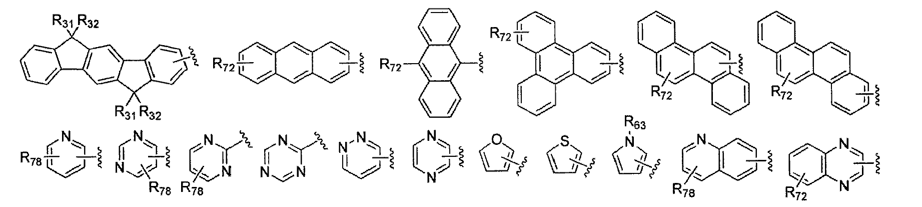 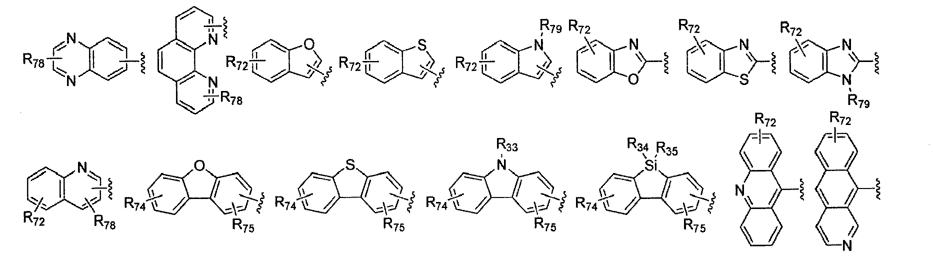 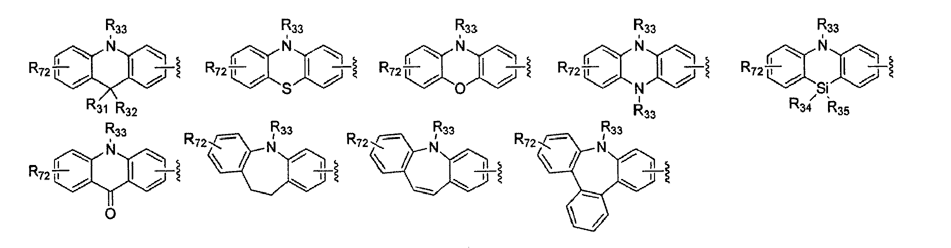 wherein, R₃₁ through R₃₅ and R₇₁ through R₇₉ independently represent hydrogen, deuterium, halogen, (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C1-C60)alkyl(C6-C60)aryl, (C6-C60)ar(C1-C60)alkyl, (C1-C60)alkyloxy, (C1-C60)alkylthio, (C6-C60)aryloxy, (C1-C60)arylthio, (C1-C60)alkoxycarbonyl, (C1-C60)alkylcarbonyl, (C6-C60)arylcarbonyl, carboxyl, nitro or hydroxyl, or R₃₁ and R₃₂, or R₃₄ and R₃₅ may be linked via (C3-C60)alkylene or (C3-C60)alkenylene with or without a fused ring to form an alicyclic ring, or a monocyclic or polycyclic aromatic ring;
the alkyl, aryl, heteroaryl, heterocycloalkyl, cycloalkyl, alkenyl or alkynyl of R₃₁ through R₃₅ and R₇₁ through R₇₉ may be further substituted by one or more substituent(s) selected from a group consisting of halogen, (C1-C60)alkyl with or without halogen substituent(s), (C6-C60)aryl, (C3-C60)heteroaryl containing one or more heteroatoms selected from N, O and S, without or without (C6-C60)aryl substituent(s), 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, carbazolyl, (C1-C60)alkylamino, (C6-C60)arylamino, (C6-C60)ar(C1-C60)alkyl, (C1-C60)alkyl(C6-C60)aryl, (C1-C60)alkyloxy, (C1-C60)alkylthio, (C6-C60)aryloxy, (C1-C60)arylthio, (C1-C60)alkoxycarbonyl, (C1-C60)alkylcarbonyl, (C6-C60)arylcarbonyl, carboxyl, nitro and hydroxyl; and
a is an integer from 1 to 5.

More specifically, R₁₆ and R₁₇ may be independently selected from the following groups, without restriction.

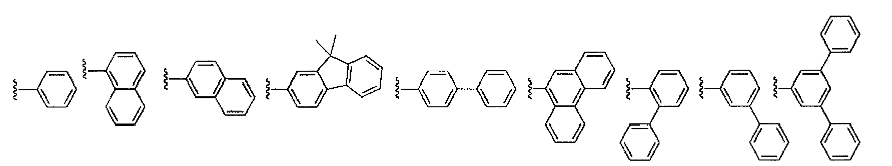

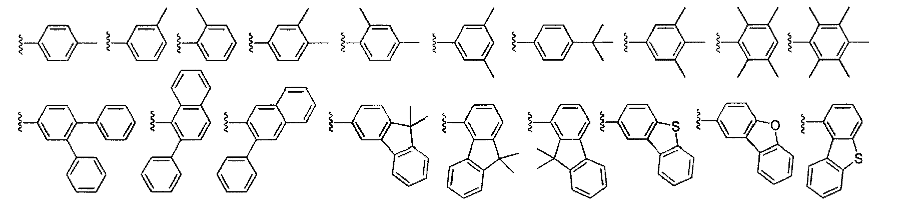

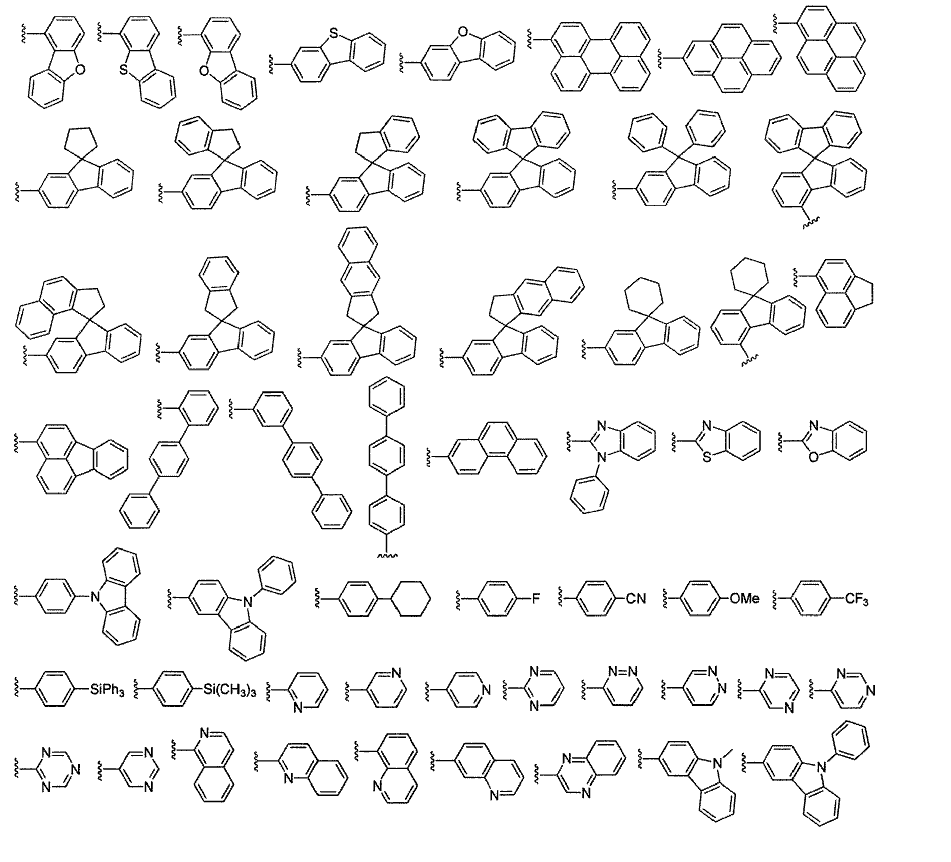

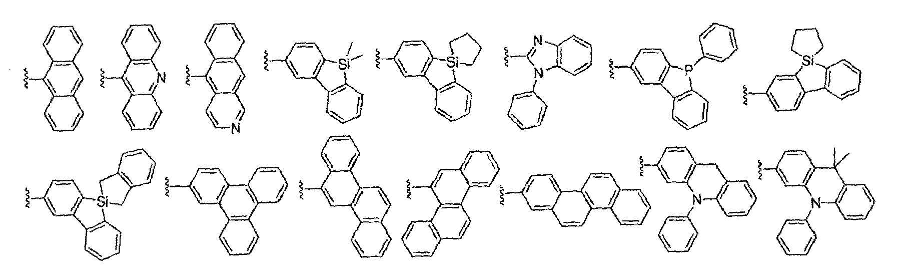

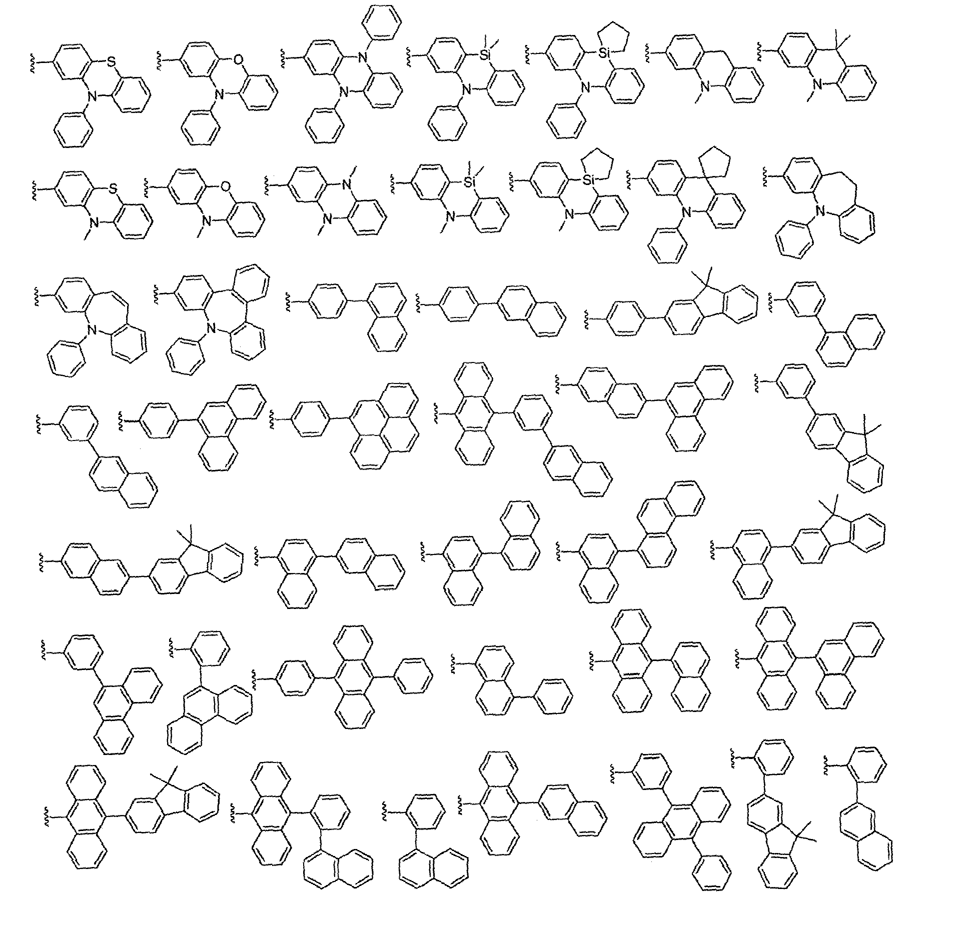

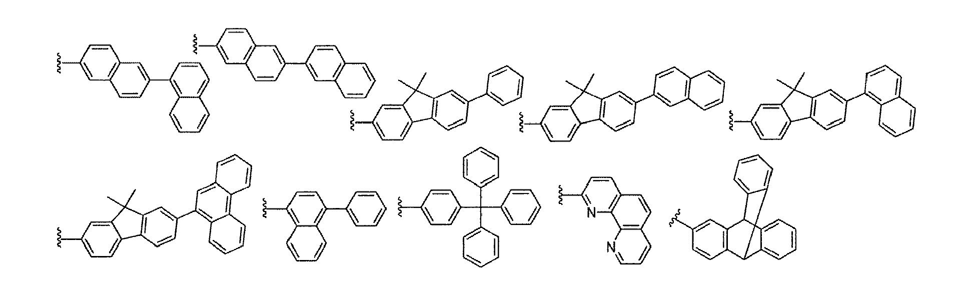

Specifically, 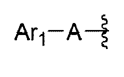 and 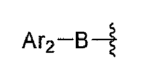 independently
represent hydrogen, or a group represented by one of the following structural formulas, but they are not restricted thereto: 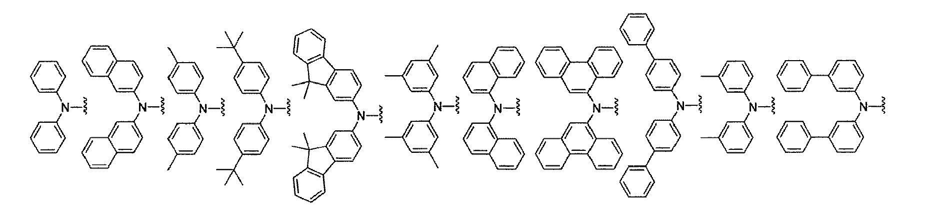 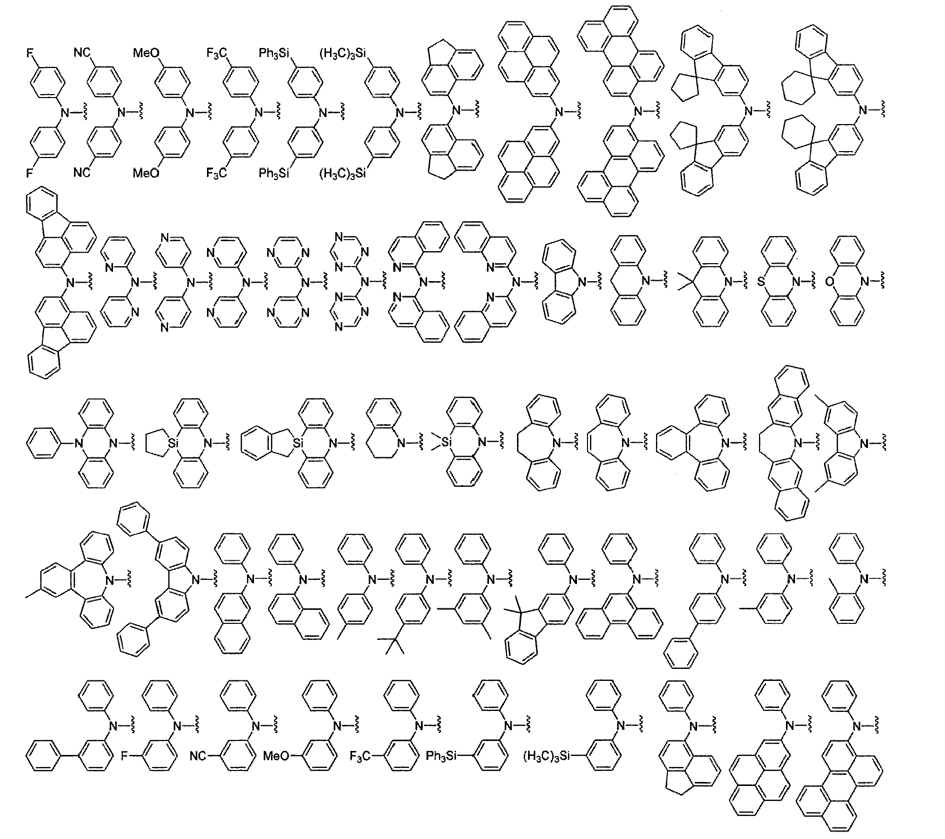 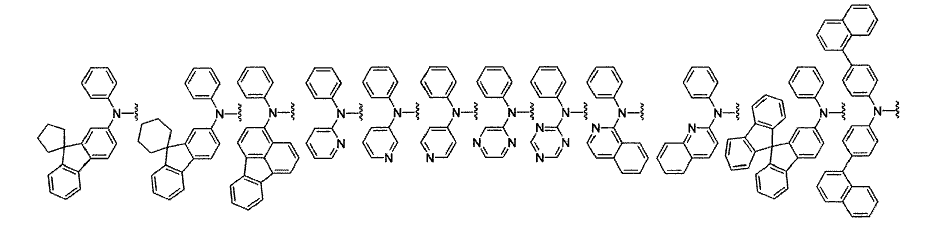 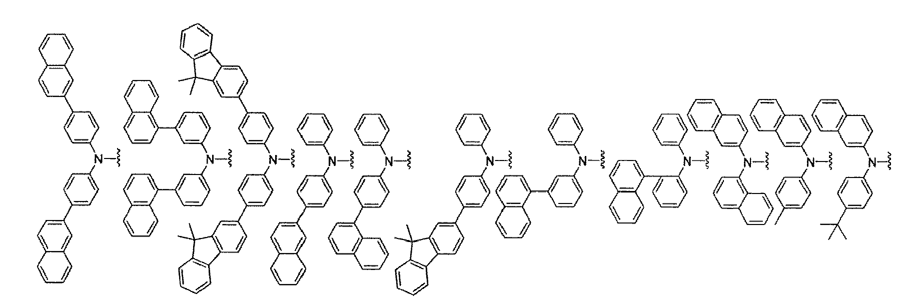 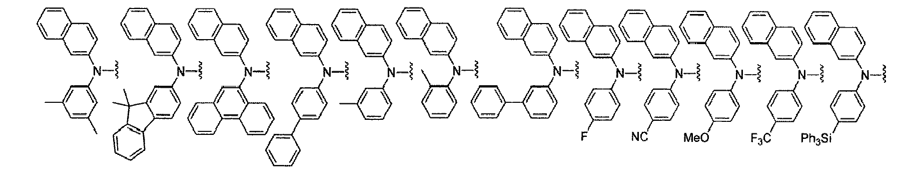 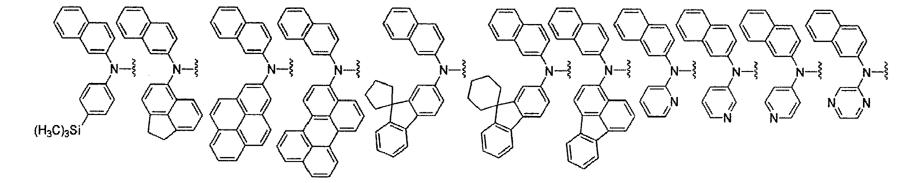 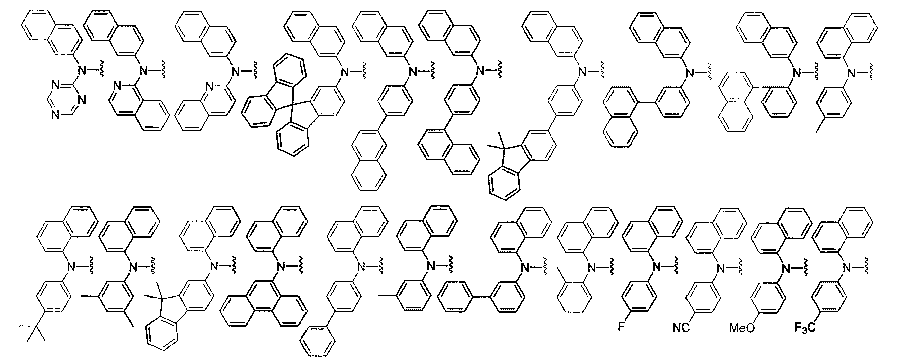 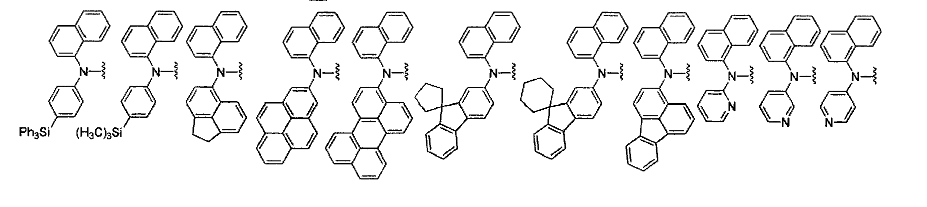 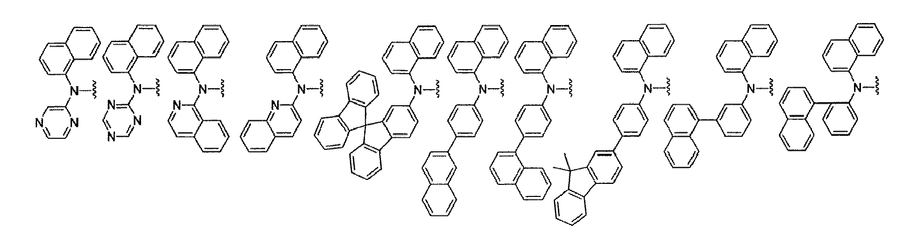 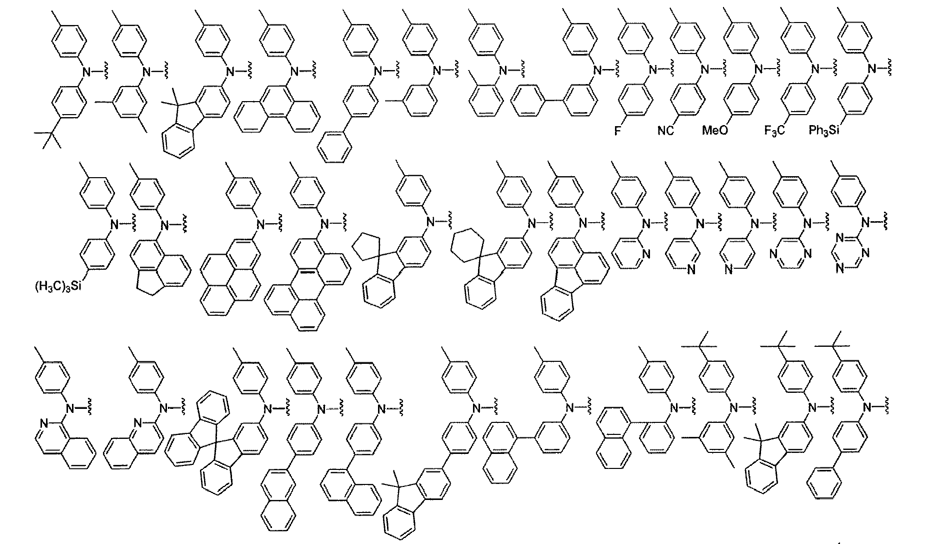 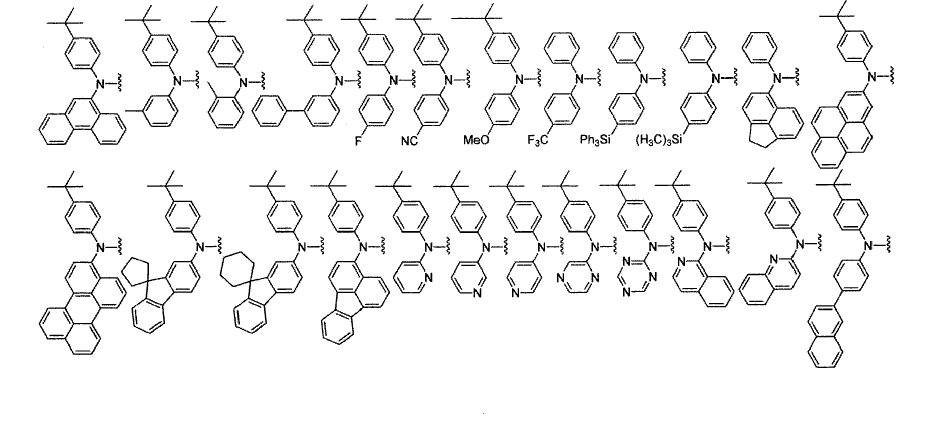 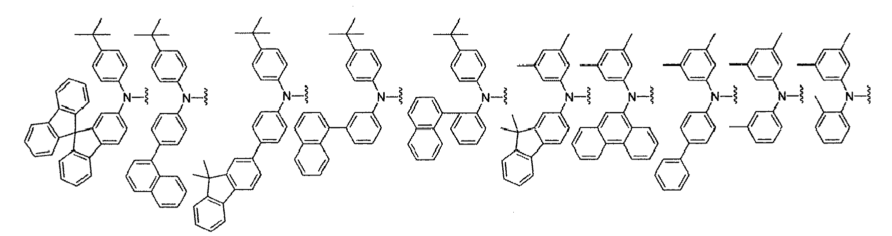 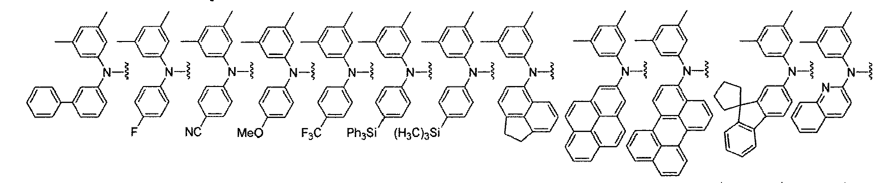 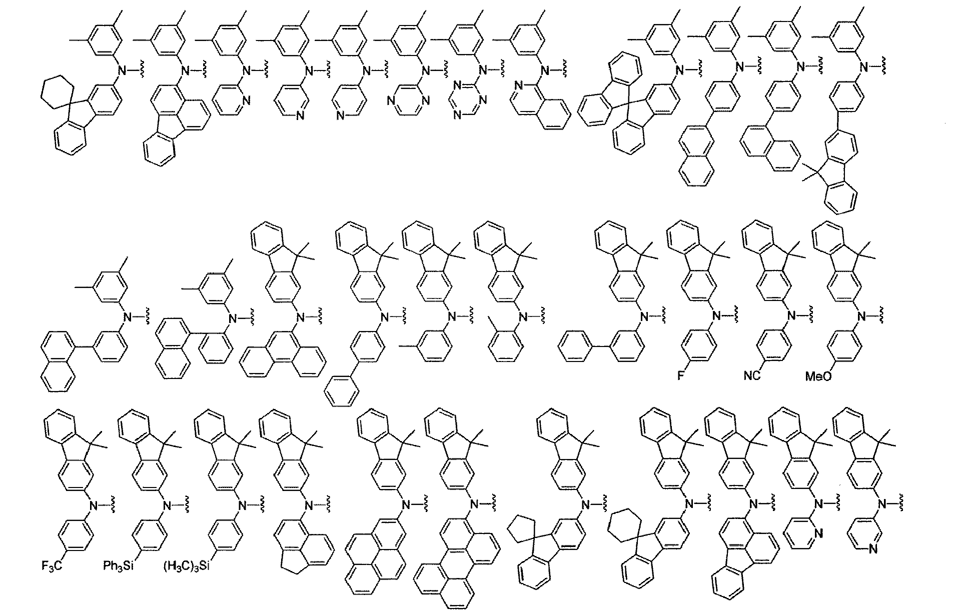 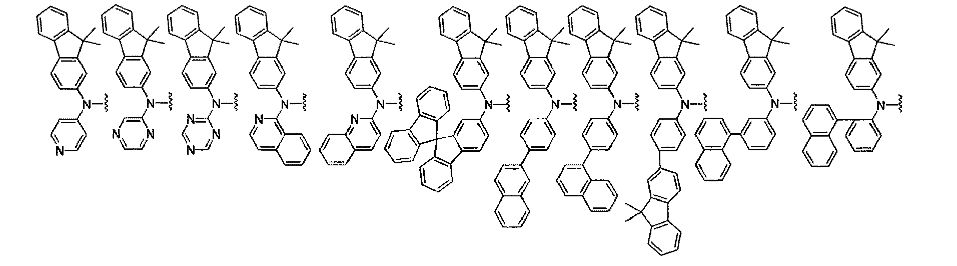 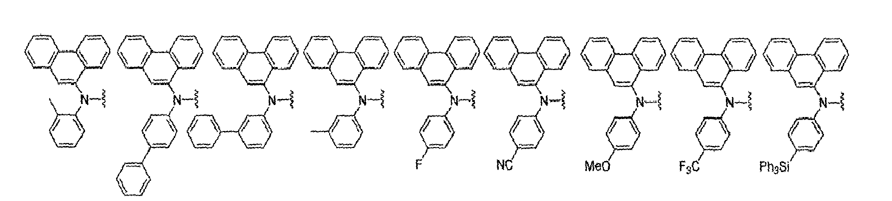 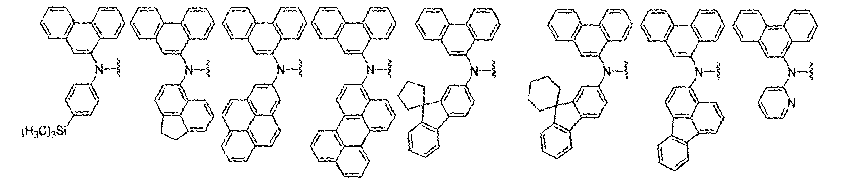 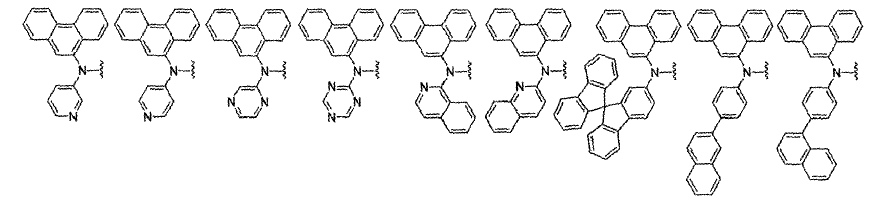 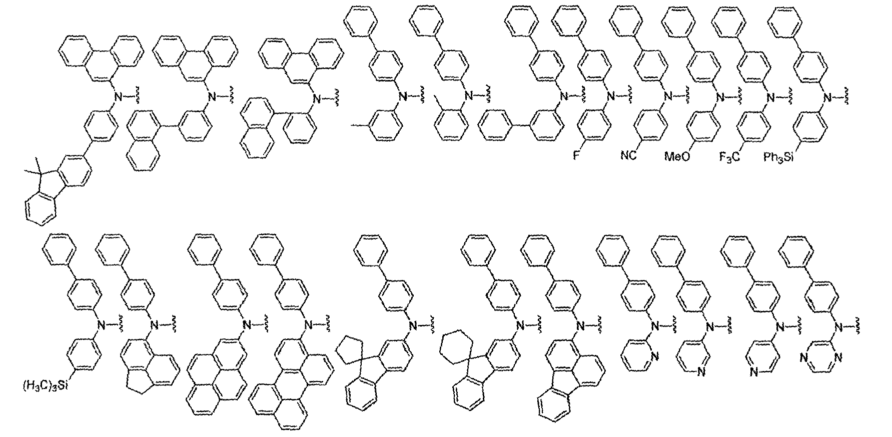 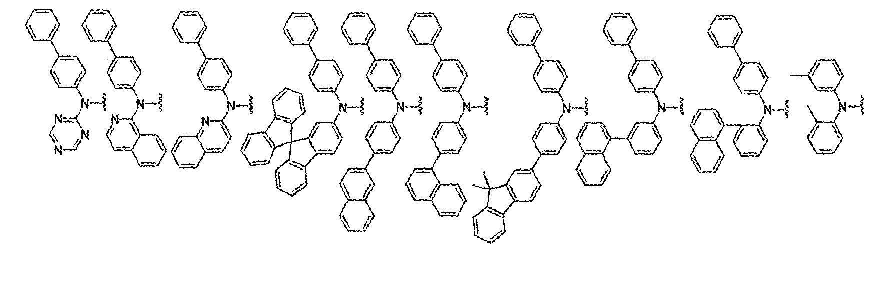 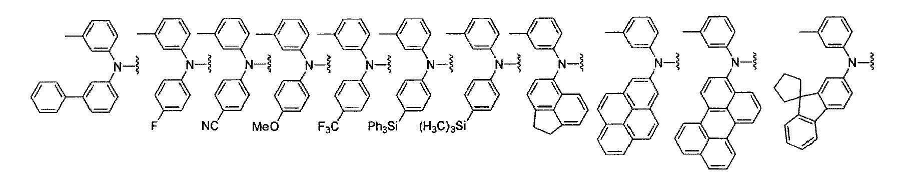 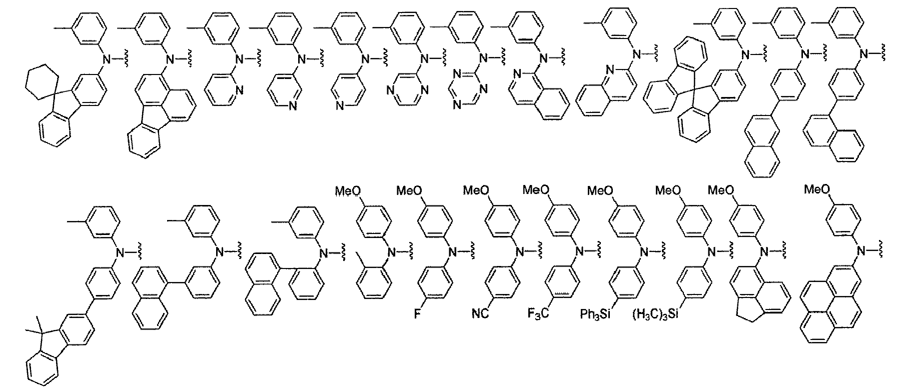 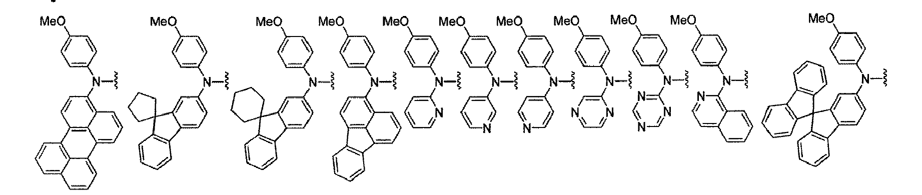 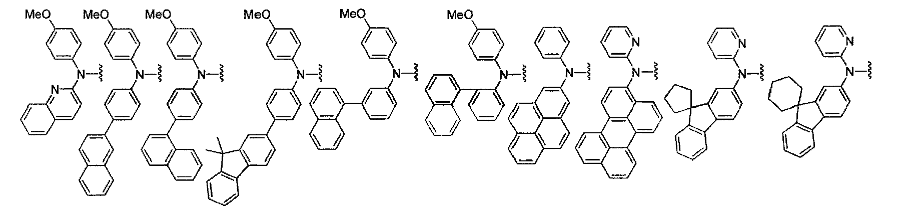 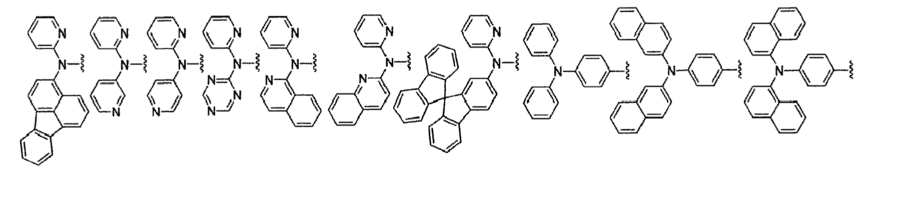 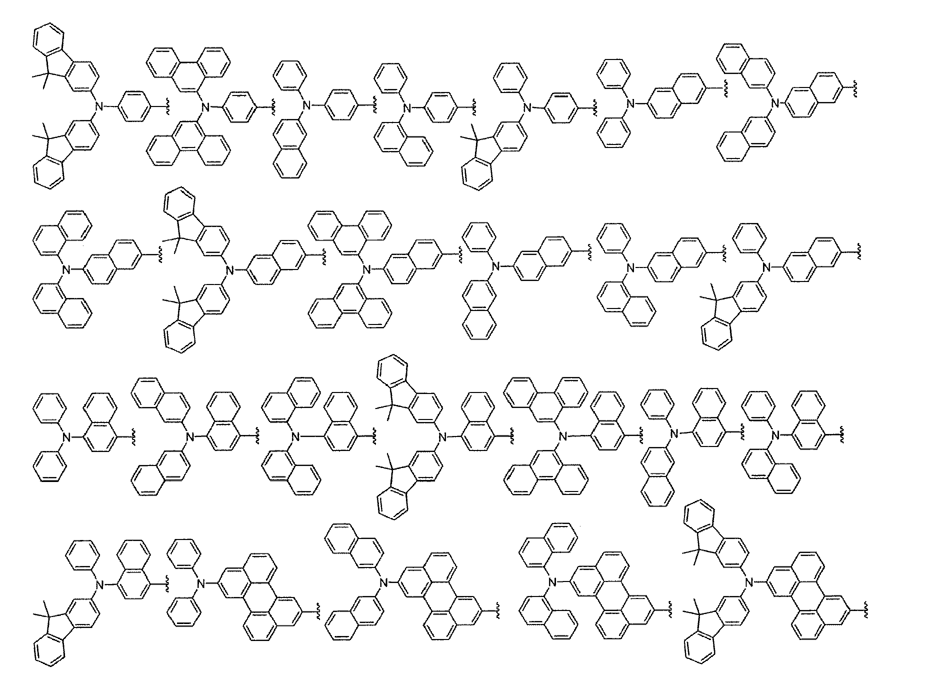 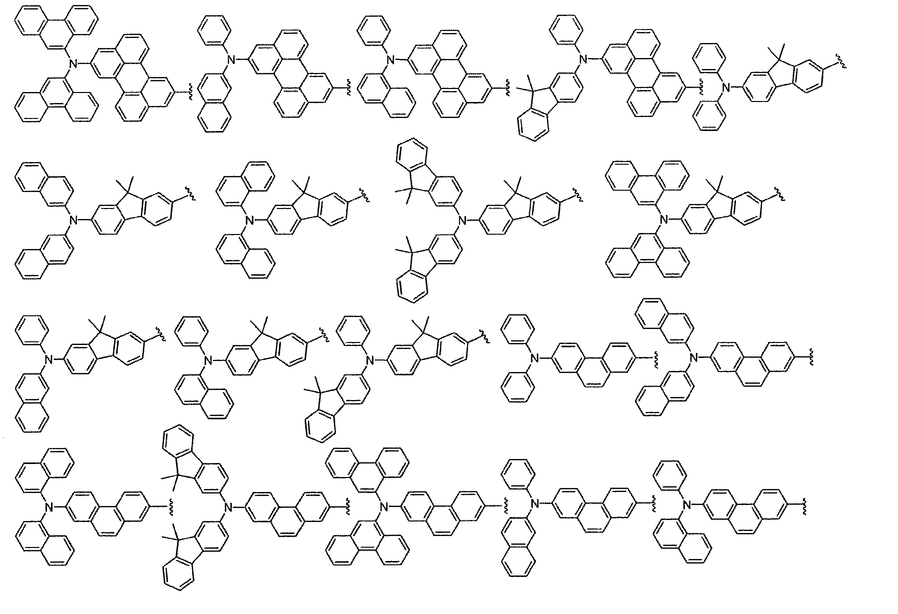 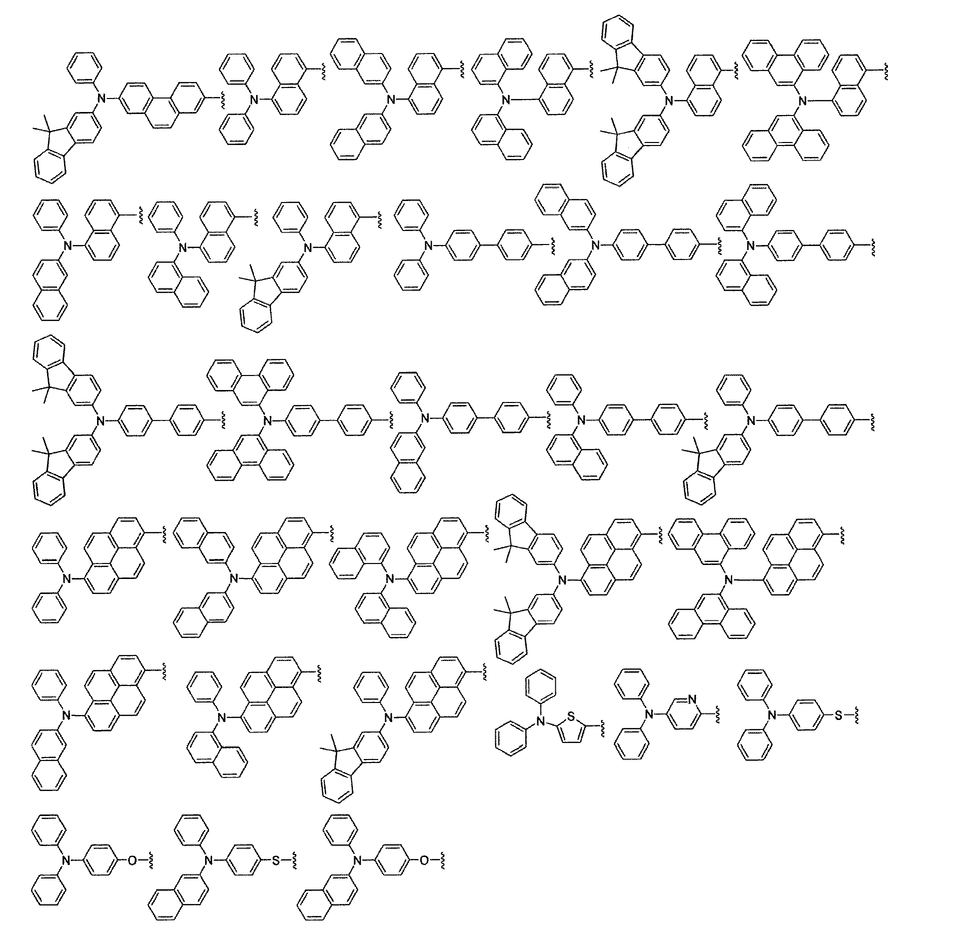 wherein, R₁ through R₅ independently represent hydrogen, deuterium, chloro, fluoro, methyl, ethyl, n-propyl, i-propyl, n-butyl, i-butyl, t-butyl, n-pentyl, i-pentyl, n-hexyl, n-heptyl, n-octyl, 2-ethylhexyl, n-nonyl, decyl, dodecyl, hexadecyl, benzyl, trifluoromethyl, perfluorethyl, trifluoroethyl, perfluoropropyl, perfluorobutyl, methoxy, ethoxy, n-propoxy, i-propoxy, n-butoxy, i-butoxy, t-butoxy, n-pentoxy, i-pentoxy, n-hexyloxy, n-heptoxy, cyclopropyl, cyclobutyl, cyclopentyl, cyclohexyl, cycloheptyl, cyclooctyl, cyclononyl, cyclodecyl, phenyl, naphthyl, biphenyl, 9,9-dimethylfluorenyl, 9,9-diphenylfluorenyl, phenanthryl, anthryl, fluoranthenyl, triphenylenyl, pyrenyl, chrysenyl, naphthacenyl, perylenyl, spirobifluorenyl, pyridyl, pyrrolyl, furanyl, thiophenyl, imidazolyl, benzimidazolyl, pyrazinyl, pyrimidinyl, pyridazinyl, quinolyl, triazinyl, benzofuranyl, benzothiophenyl, pyrazolyl, indolyl, carbazolyl, thiazolyl, oxazolyl, benzothiazolyl, benzoxazolyl, phenanthrolinyl, trimethylsilyl, triethylsilyl, tripropylsilyl, tri(t-butyl)silyl, t-butyldimethylsilyl, dimethylphenylsilyl, triphenylsilyl, adamantyl, bicyclo[2.2.1]heptyl, bicyclo[2.2.2]octyl, bicyclo[3.2.1]octyl, bicyclo[5.2.0]nonyl, bicyclo[4.2.2]decyl, bicyclo[2.2.2]octyl, 4-pentylbicyclo[2.2.2]octyl, ethenyl, phenylethenyl, ethynyl, phenylethynyl, cyano, dimethylamino, diphenylamino, monomethylamino, monophenylamino, phenyloxy, phenylthio, methoxycarbonyl, ethoxycarbonyl, t-butoxycarbonyl, methylcarbonyl, ethylcarbonyl, benzylcarbonyl, phenylcarbonyl, carboxyl, nitro or hydroxyl.

In group 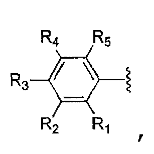 the alicyclic ring, or the monocyclic or polycyclic aromatic ring formed from each of R₁ through R₅ by linkage to an adjacent substituent via alkylene or alkenylene may be naphthyl, fluorenyl, phenanthryl, anthryl, fluoranthenyl, triphenylenyl, pyrenyl, chrysenyl, naphthacenyl, perylenyl or spirobifluorenyl.

Specifically, R₆ through R₁₅ independently represent hydrogen, deuterium, chloro, fluoro, methyl, ethyl, n-propyl, i-propyl, n-butyl, i-butyl, t-butyl, n-pentyl, i-pentyl, n-hexyl, n-heptyl, n-octyl, 2-ethylhexyl, n-nonyl, decyl, dodecyl, hexadecyl, benzyl, trifluoromethyl, perfluorethyl, trifluoroethyl, perfluoropropyl, perfluorobutyl, methoxy, ethoxy, n-propoxy, i-propoxy, n-butoxy, i-butoxy, t-butoxy, n-pentoxy, i-pentoxy, n-hexyloxy, n-heptoxy, cyclopropyl, cyclobutyl, cyclopentyl, cyclohexyl, cycloheptyl, cyclooctyl, cyclononyl, cyclodecyl, phenyl, naphthyl, biphenyl, 9,9-dimethylfluorenyl, 9,9-diphenylfluorenyl, phenanthryl, anthryl, fluoranthenyl, triphenylenyl, pyrenyl, chrysenyl, naphthacenyl, perylenyl, spirobifluorenyl, pyridyl, pyrrolyl, furanyl, thiophenyl, imidazolyl, benzimidazolyl, pyrazinyl, pyrimidinyl, pyridazinyl, quinolyl, triazinyl, benzofuranyl, benzothiophenyl, pyrazolyl, indolyl, carbazolyl, thiazolyl, oxazolyl, benzothiazolyl, benzoxazolyl, phenanthrolinyl, trimethylsilyl, triethylsilyl, tripropylsilyl, tri(t-butyl)silyl, t-butyldimethylsilyl, dimethylphenylsilyl, triphenylsilyl, adamantyl, bicyclo[2.2.1]heptyl, bicyclo[2.2.2]octyl, bicyclo[3.2.1]octyl, bicyclo[5.2.0]nonyl, bicyclo[4.2.2]decyl, bicyclo[2.2.2]octyl, 4-pentylbicyclo[2.2.2]octyl, ethenyl, phenylethenyl, ethynyl, phenylethynyl, cyano, dimethylamino, diphenylamino, monomethylamino, monophenylamino, phenyloxy, phenylthio, methoxycarbonyl, ethoxycarbonyl, t-butoxycarbonyl, methylcarbonyl, ethylcarbonyl, benzylcarbonyl, phenylcarbonyl, carboxyl, nitro or hydroxyl, or a substituent selected from the following structures, without restriction.

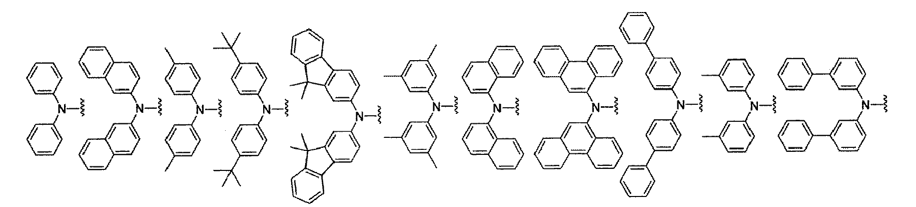

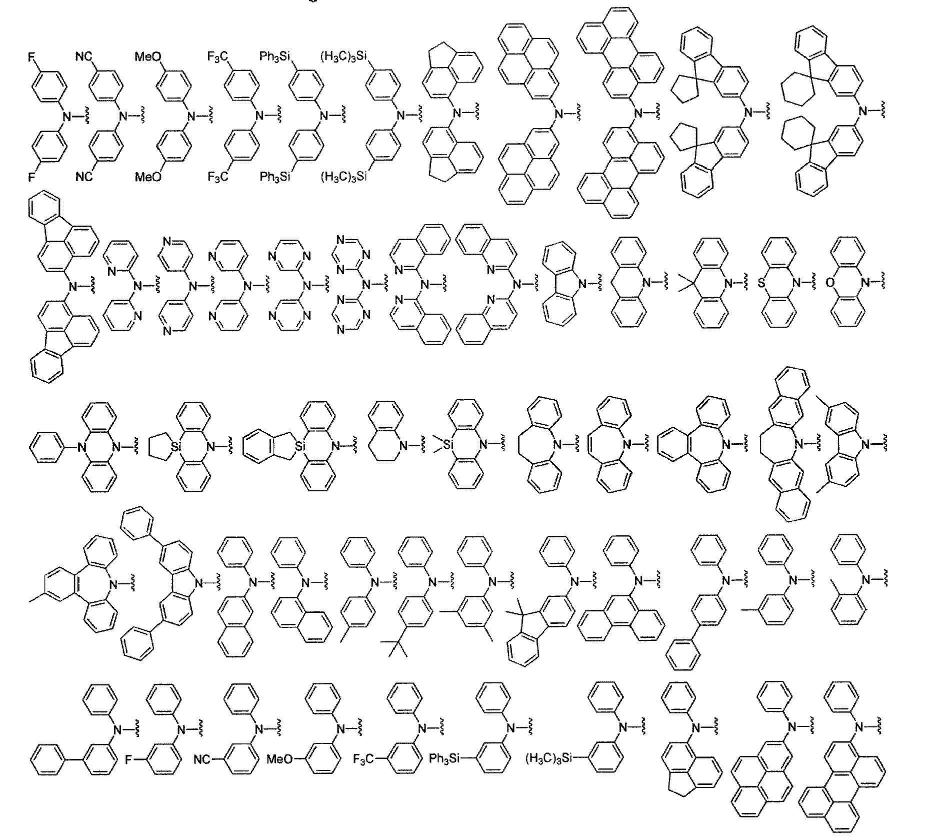

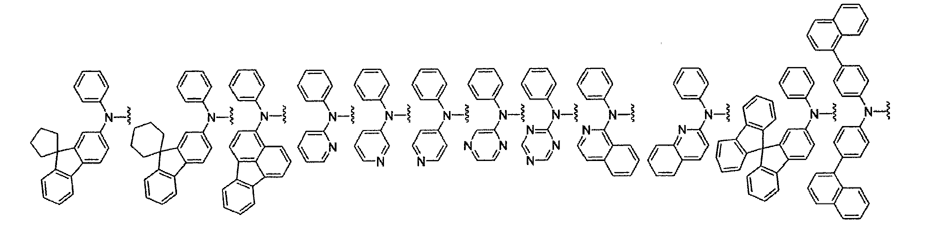

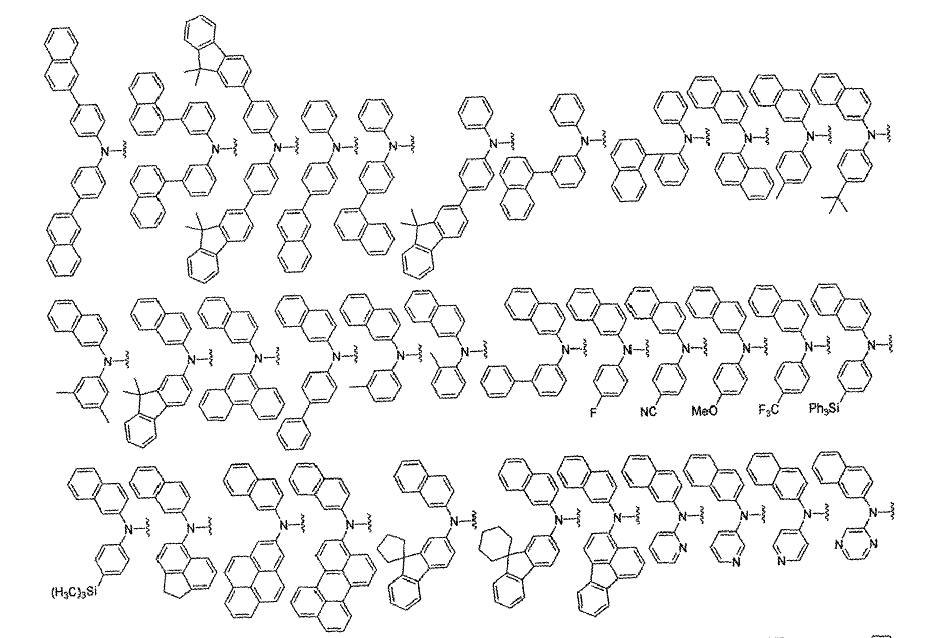

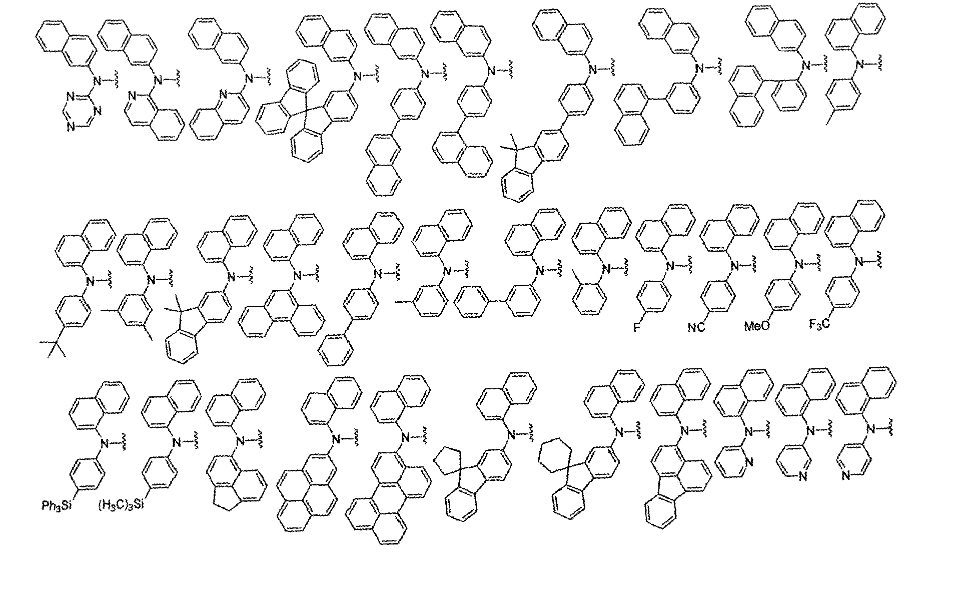

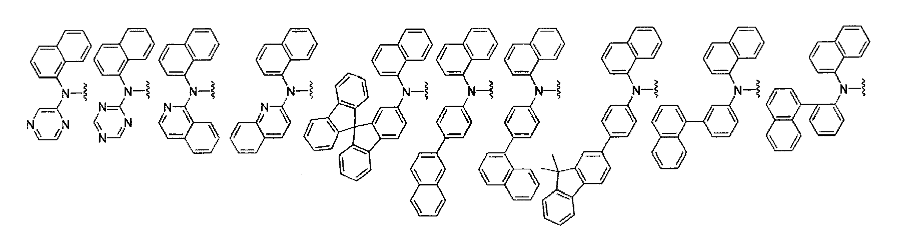

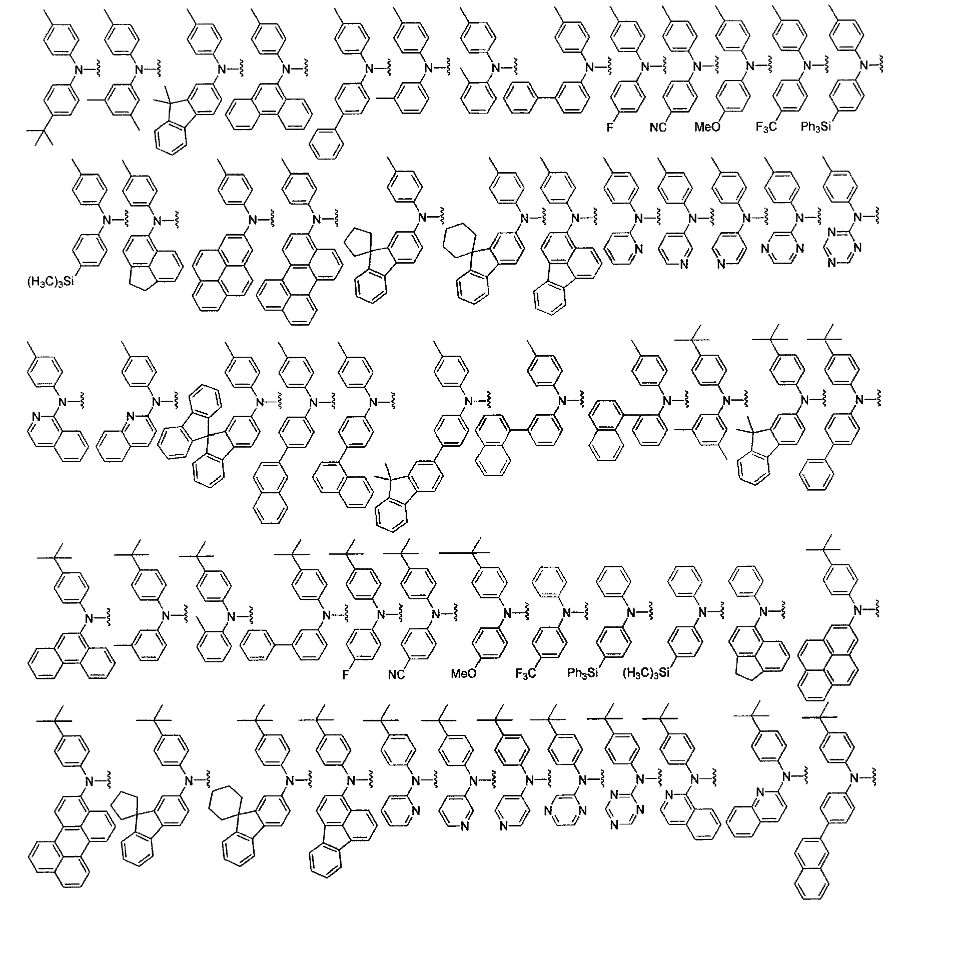

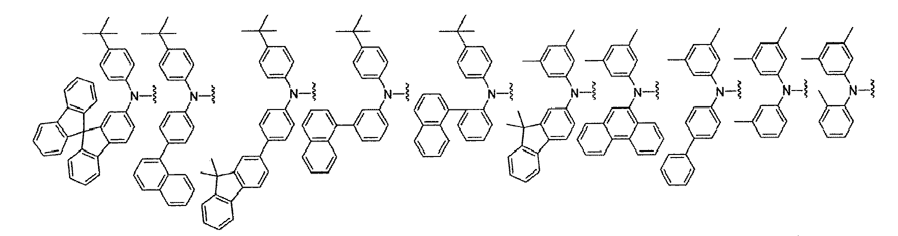

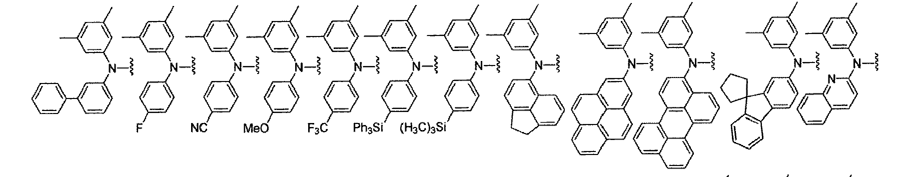

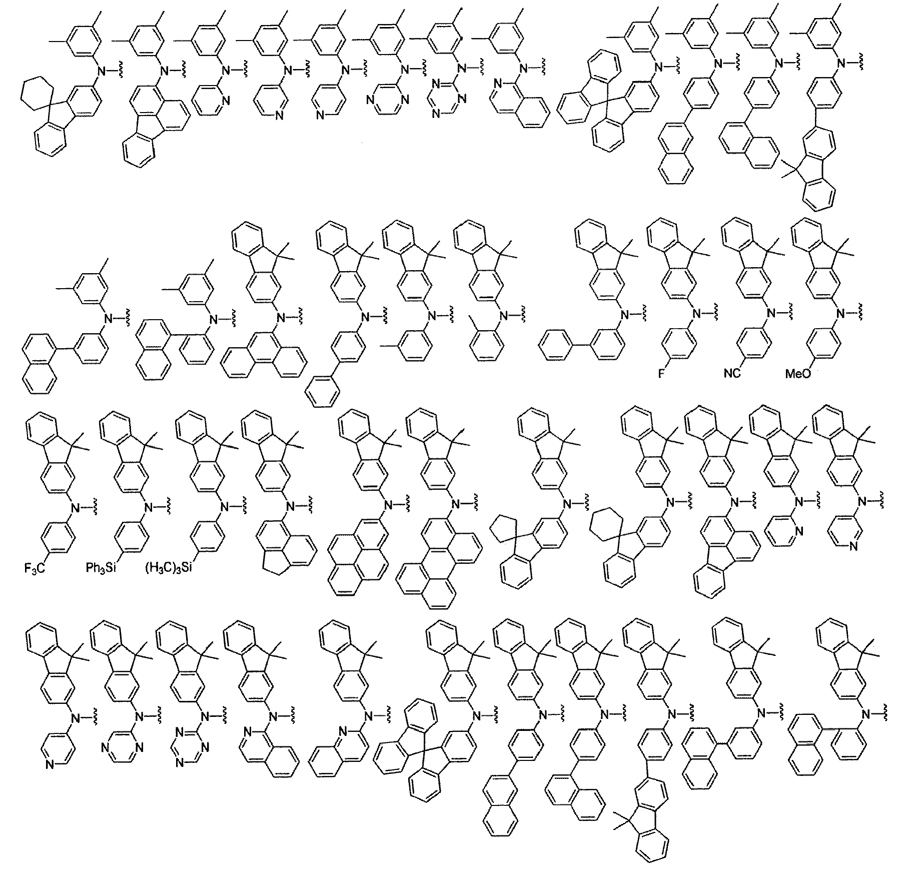

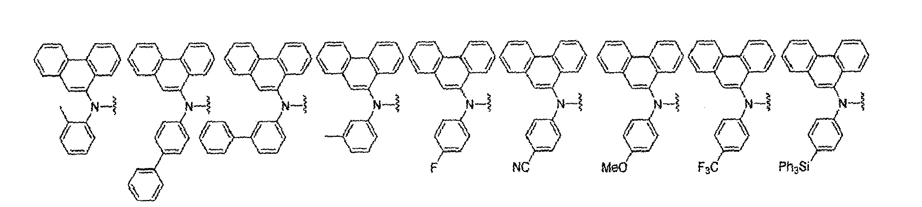

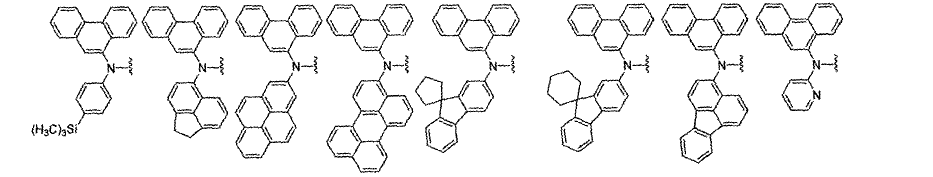

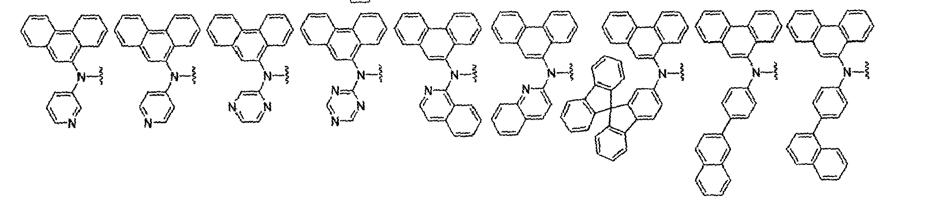

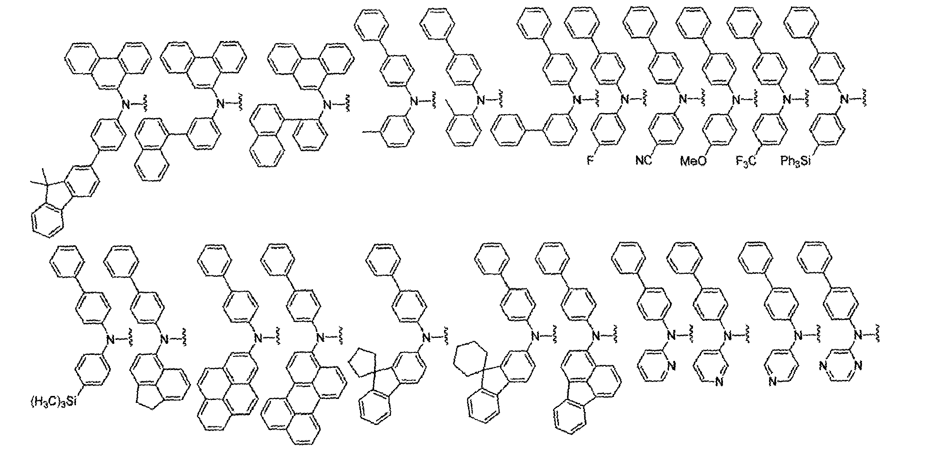

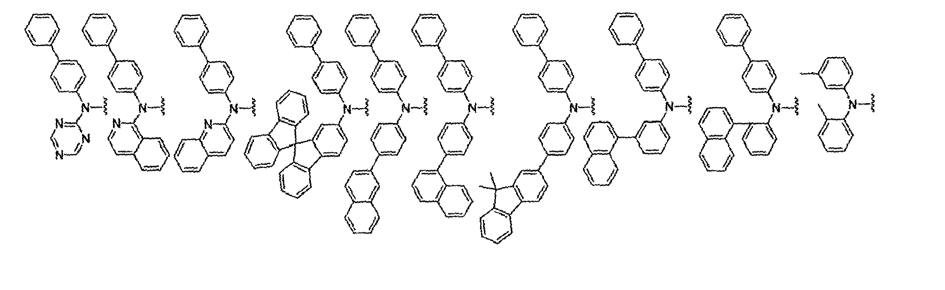

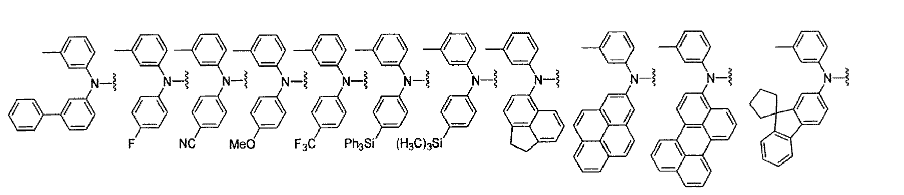

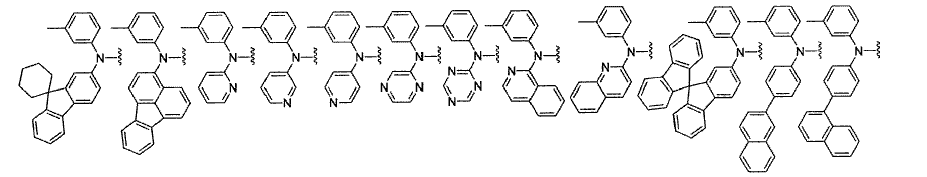

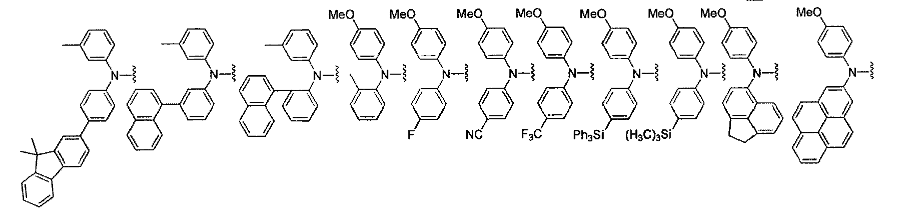

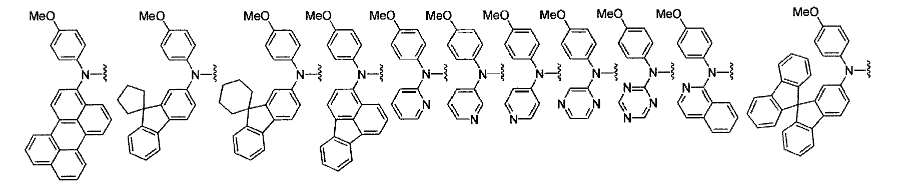

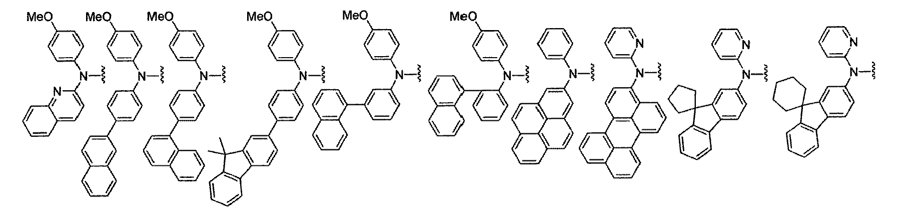

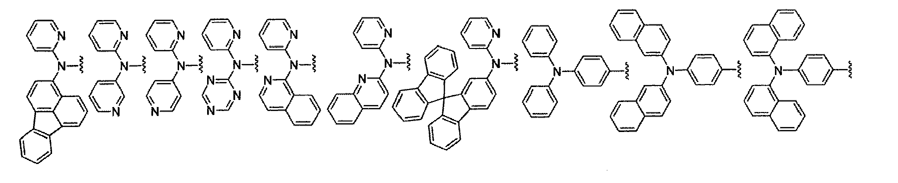

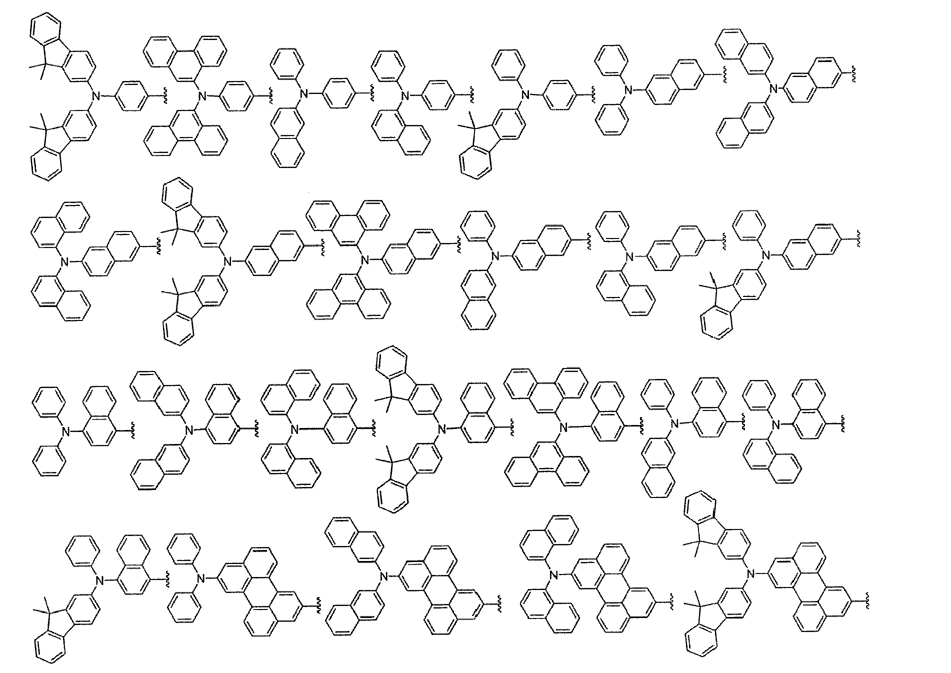

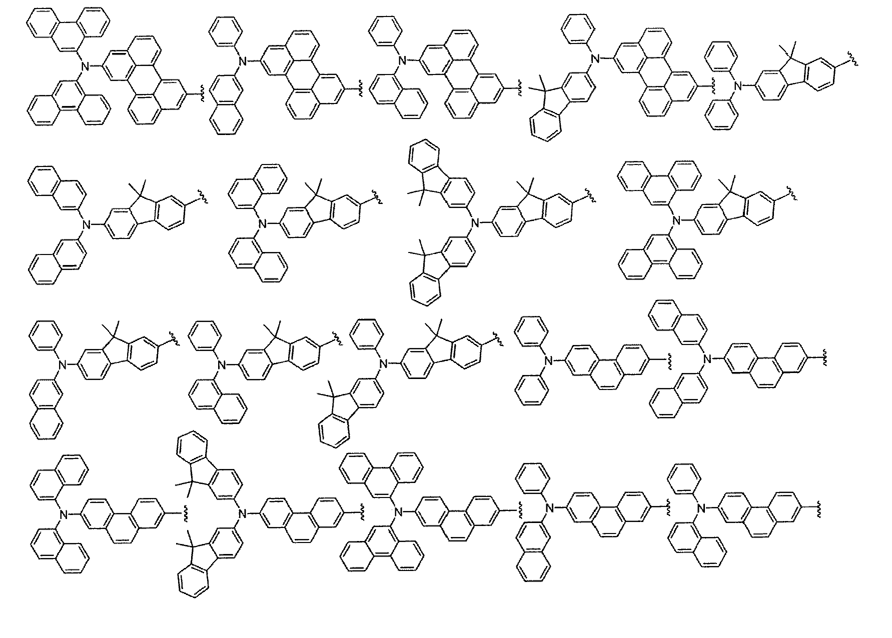

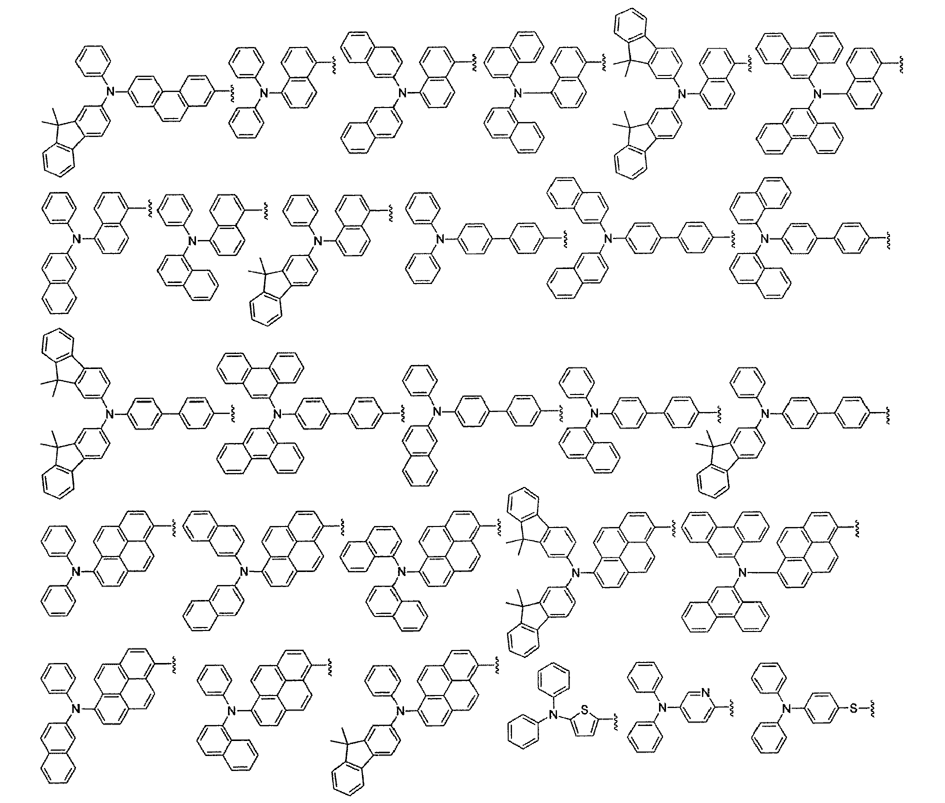

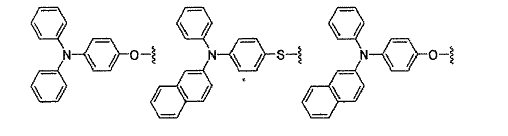

In groups 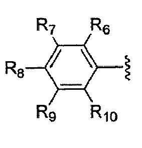 and 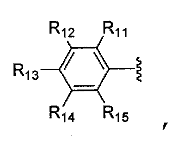 the alicyclic ring, or the monocyclic or polycyclic aromatic ring formed from each of R₆ through R₁₅ by linkage to an adjacent substituent via alkylene or alkenylene with or without a fused ring may be naphthyl, fluorenyl, phenanthryl, anthryl, fluoranthenyl, triphenylenyl, pyrenyl, chrysenyl, naphthacenyl, perylenyl or spirobifluorenyl.

The organic electroluminescent compounds according to the present invention can be more specifically exemplified by the following compounds, without restriction:

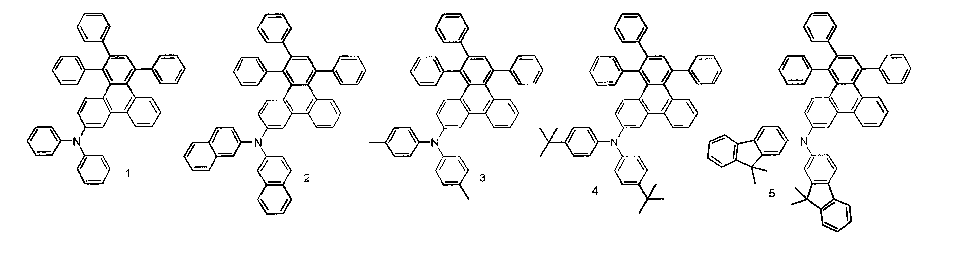

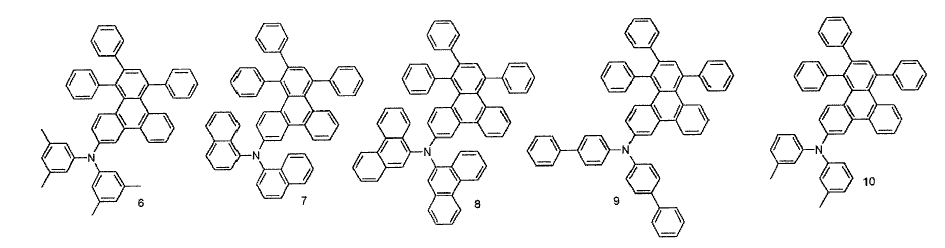

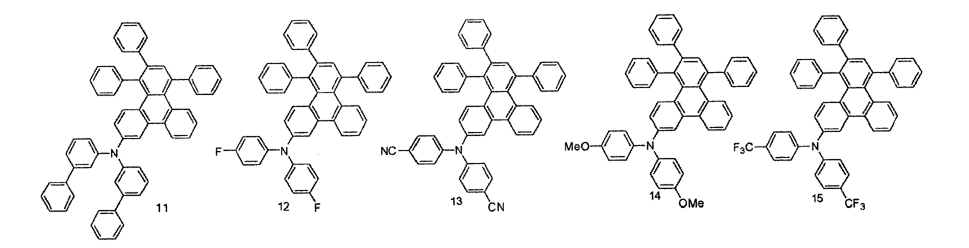

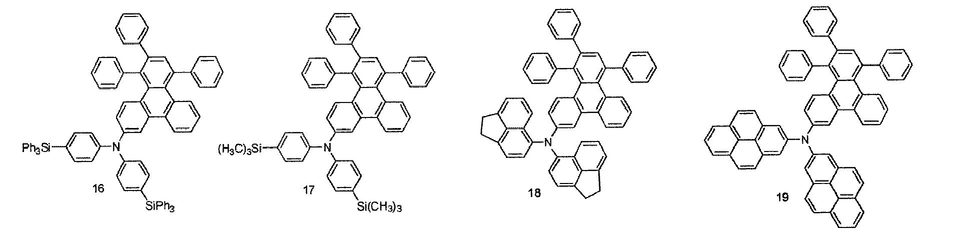

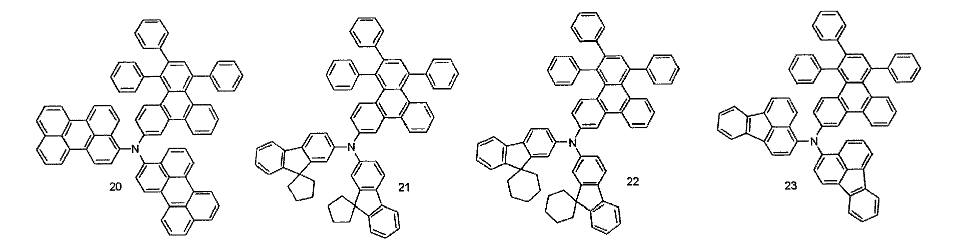

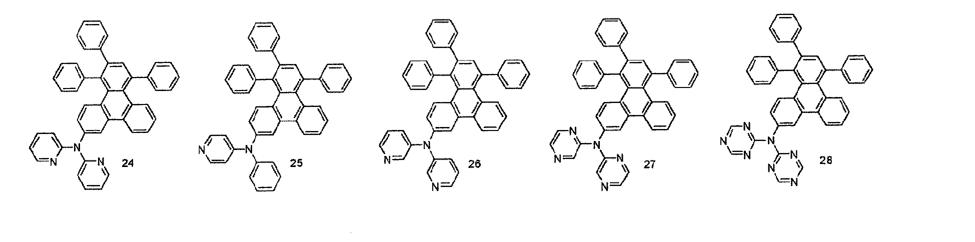

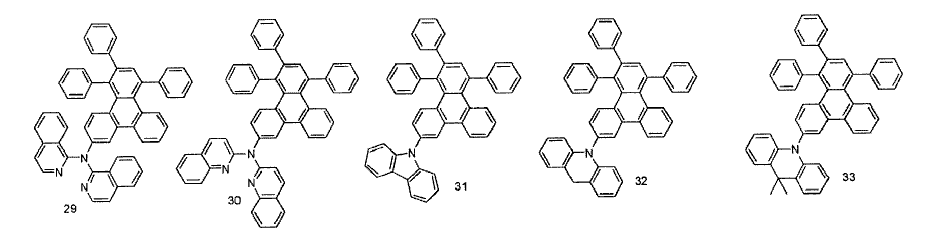

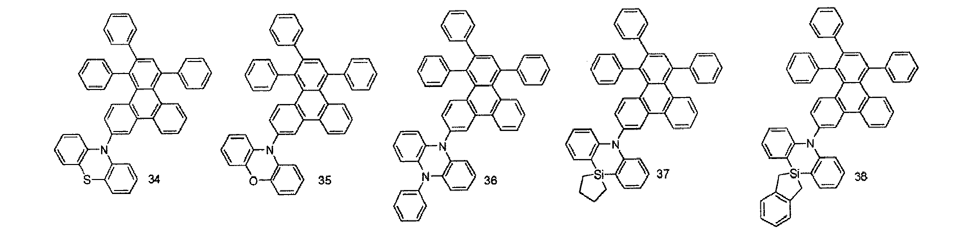

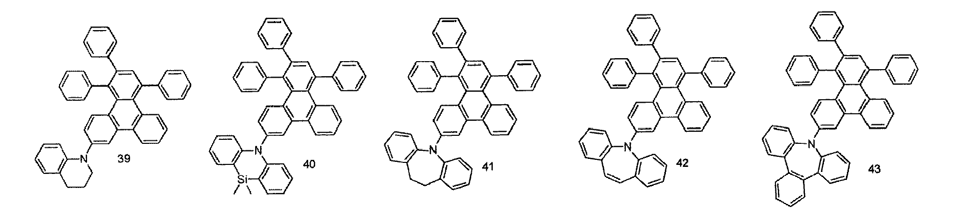

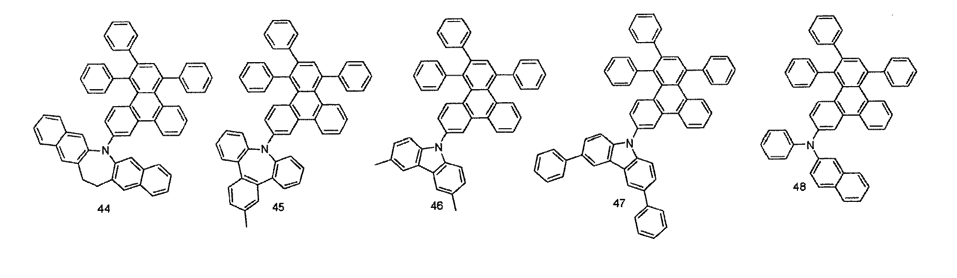

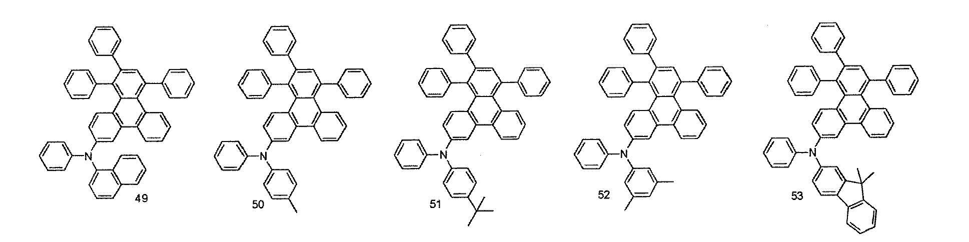

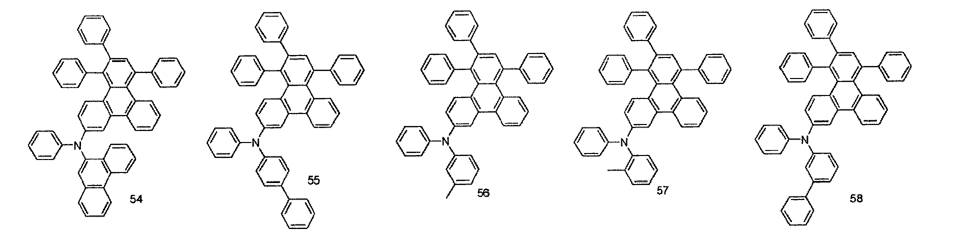

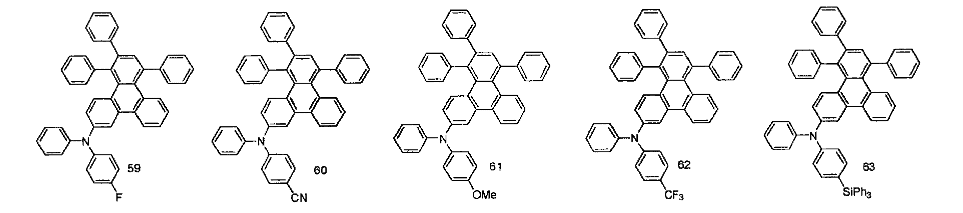

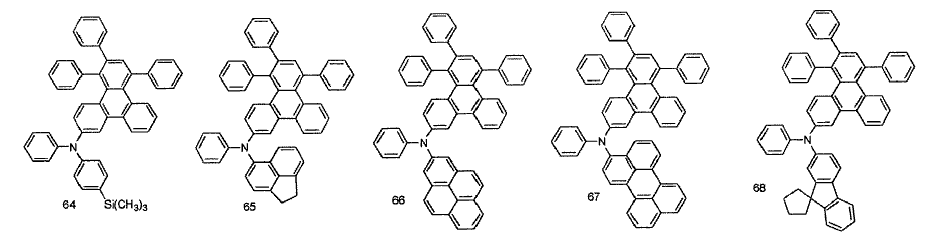

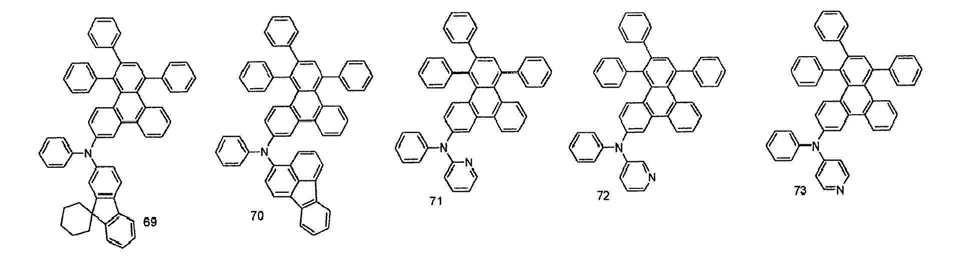

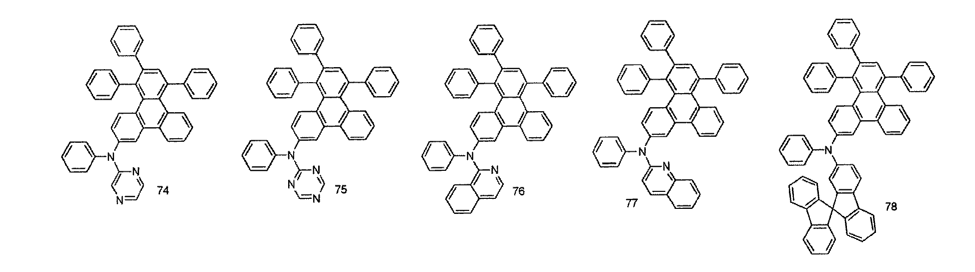

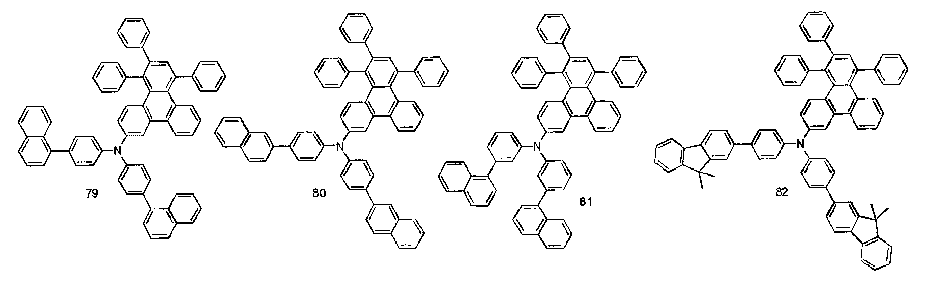

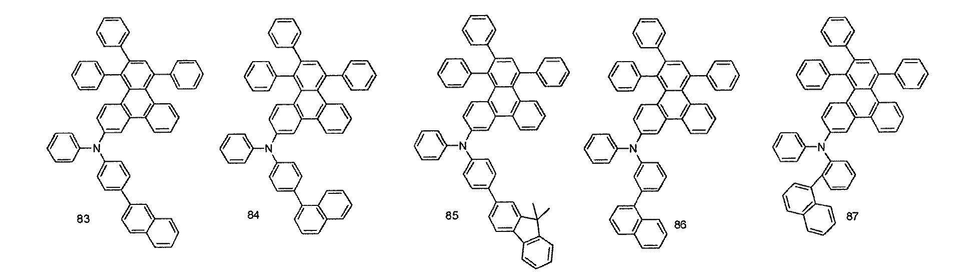

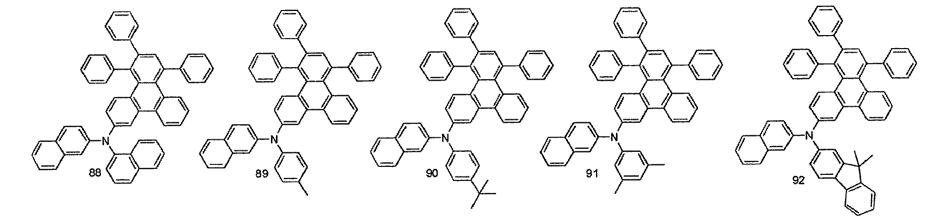

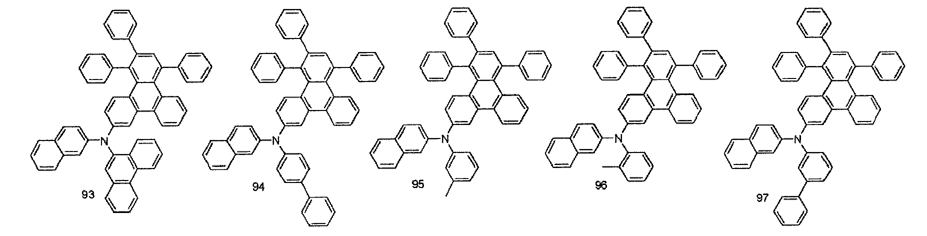

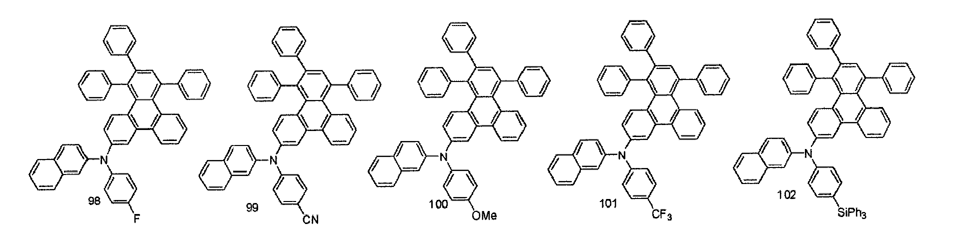

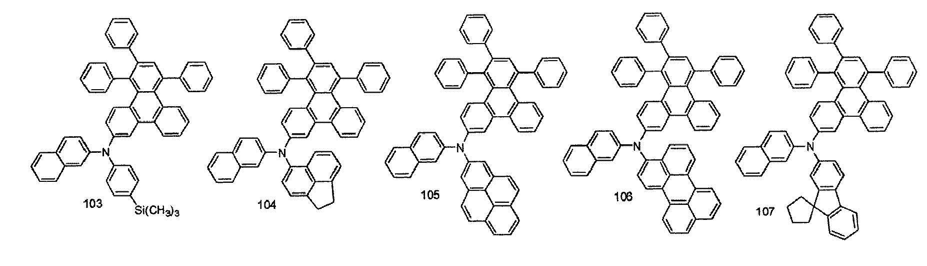

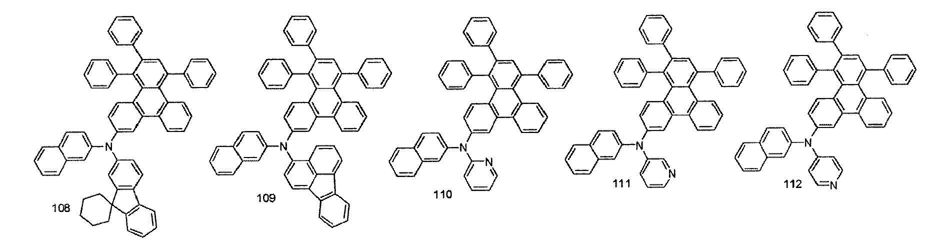

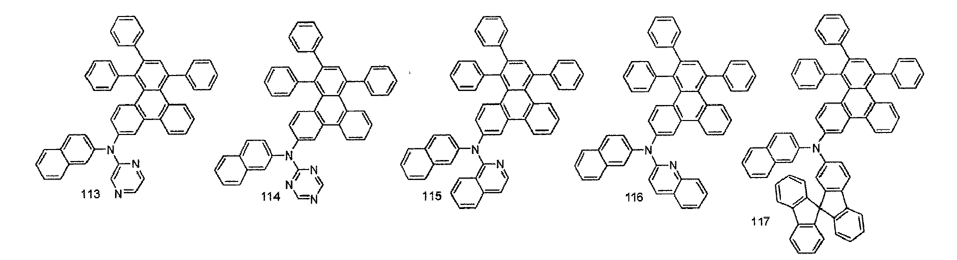

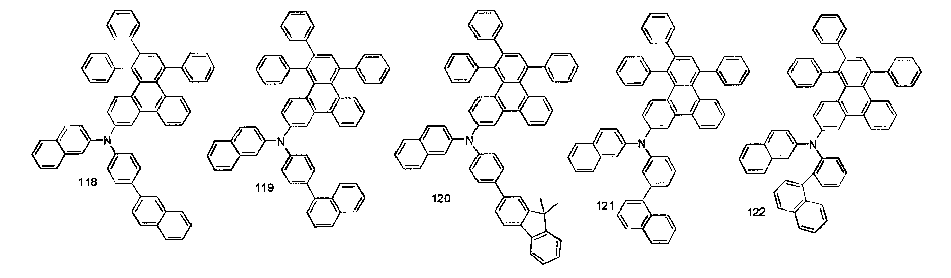

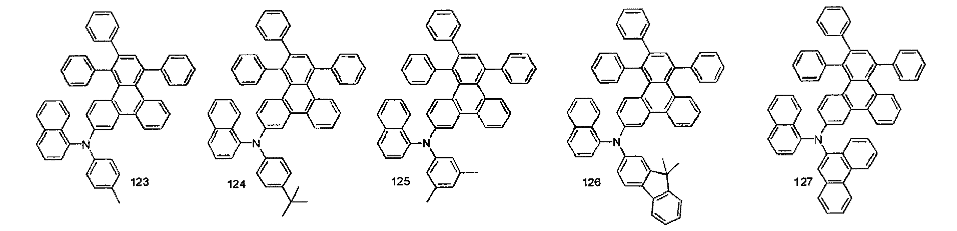

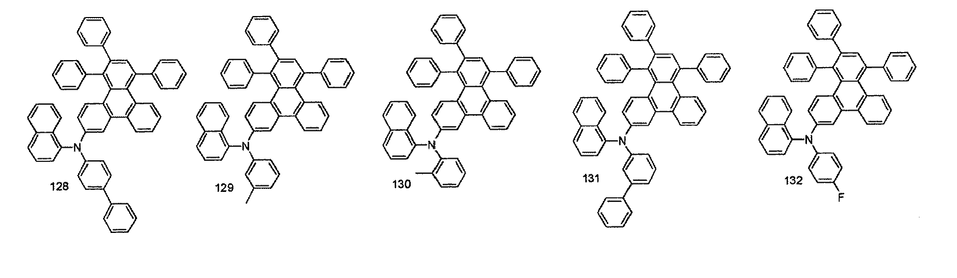

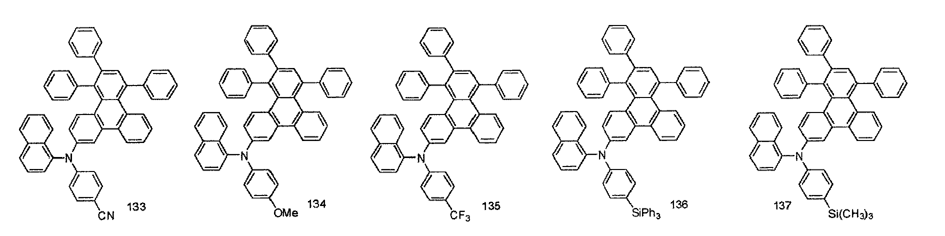

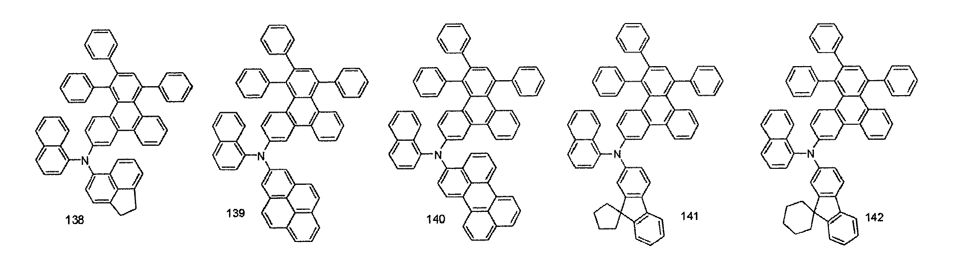

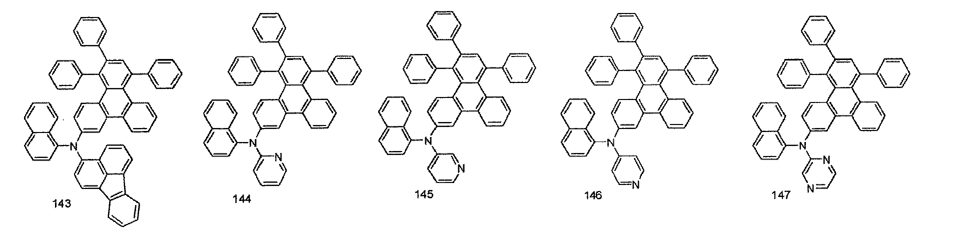

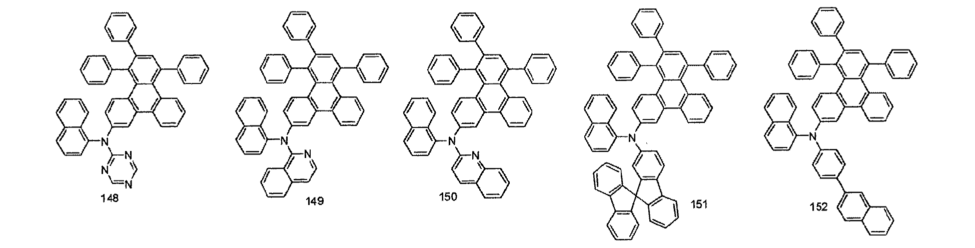

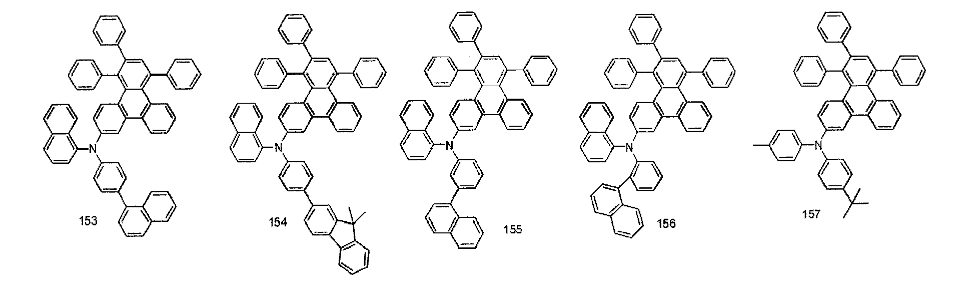

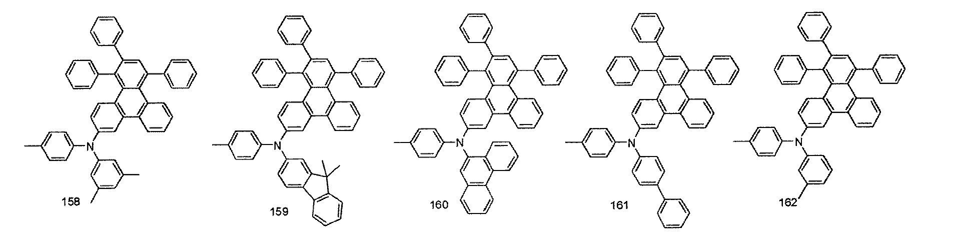

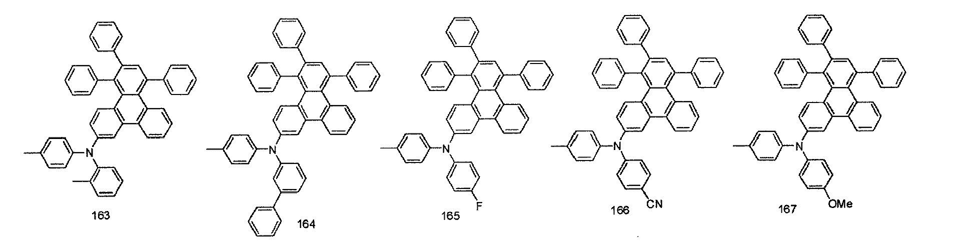

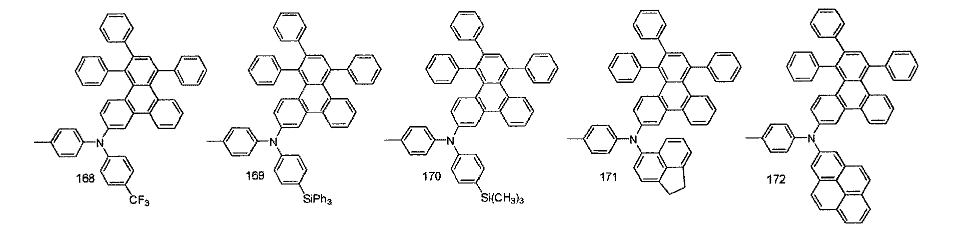

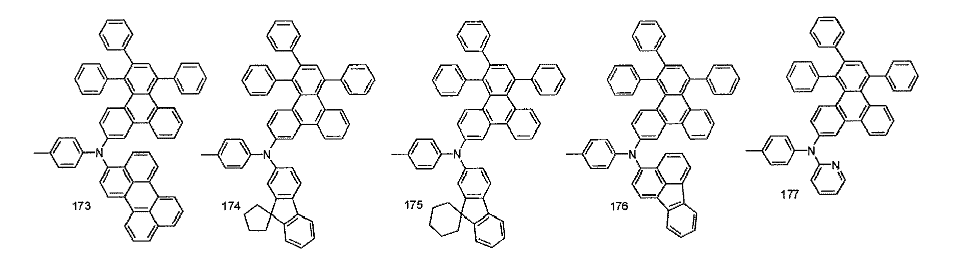

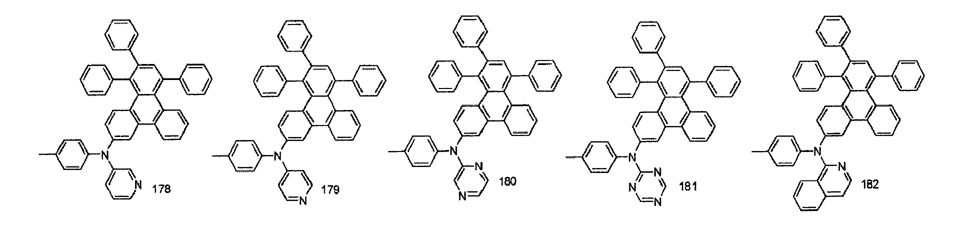

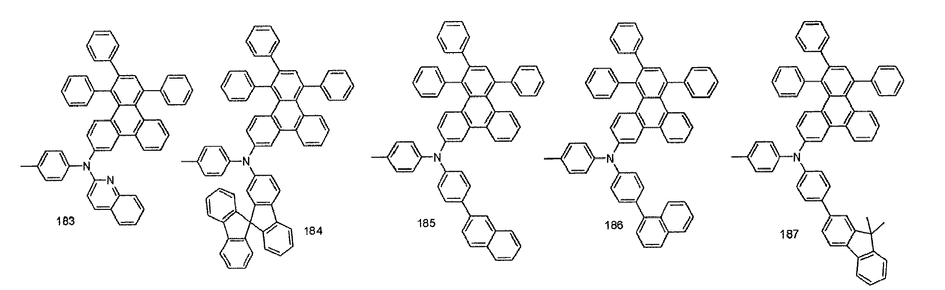

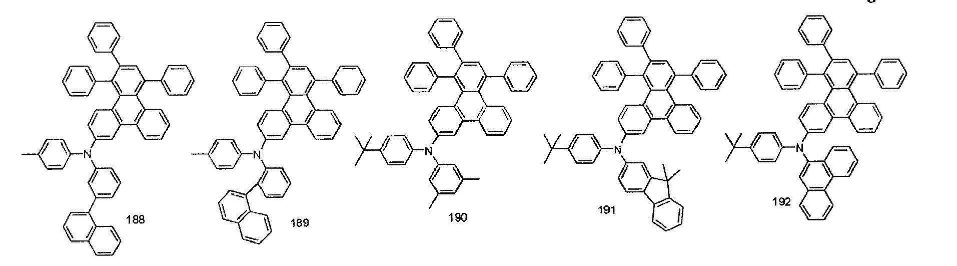

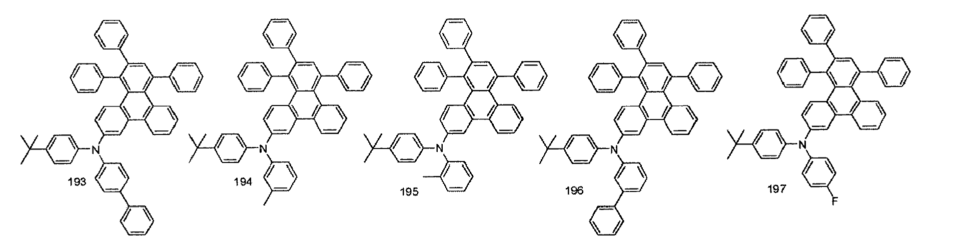

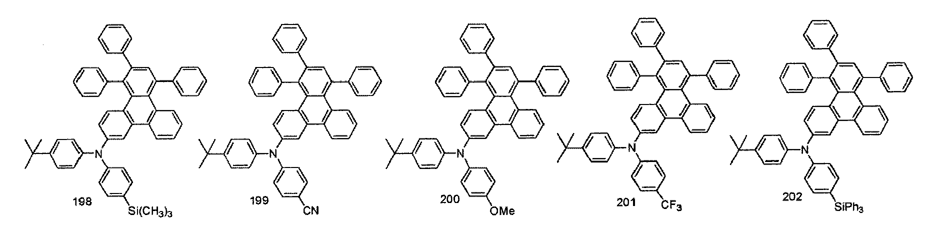

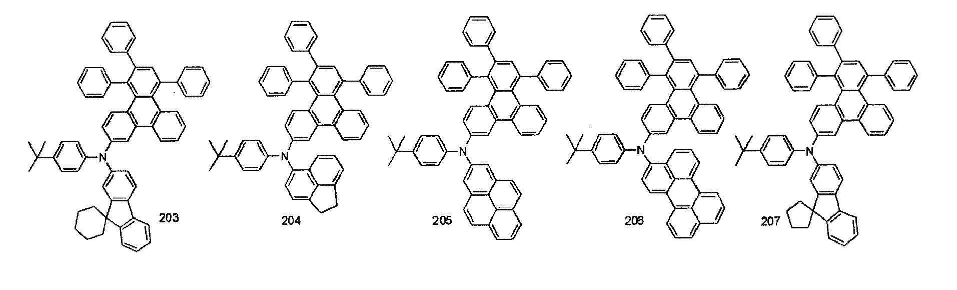

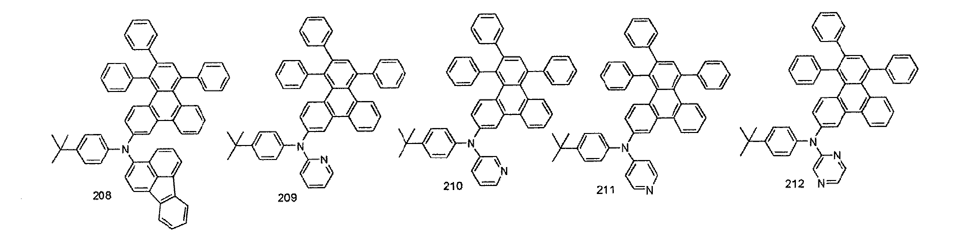

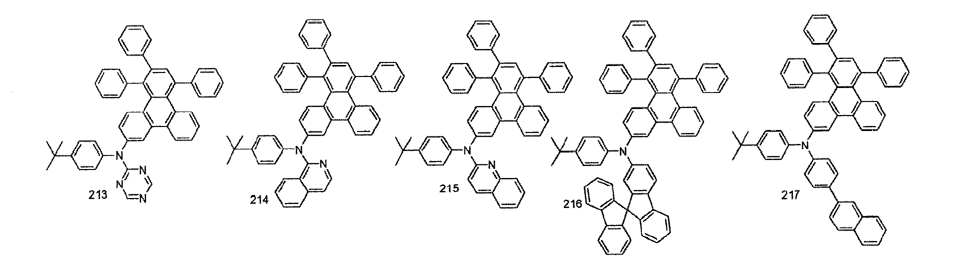

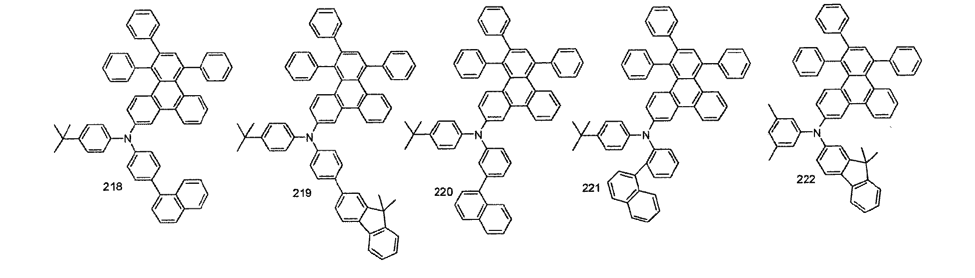

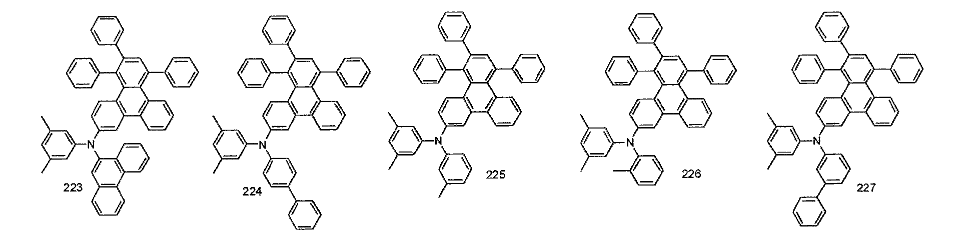

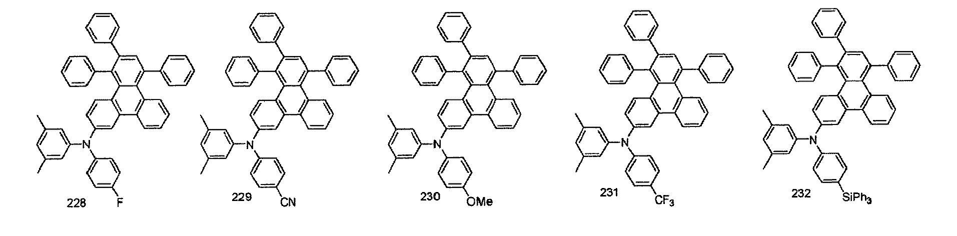

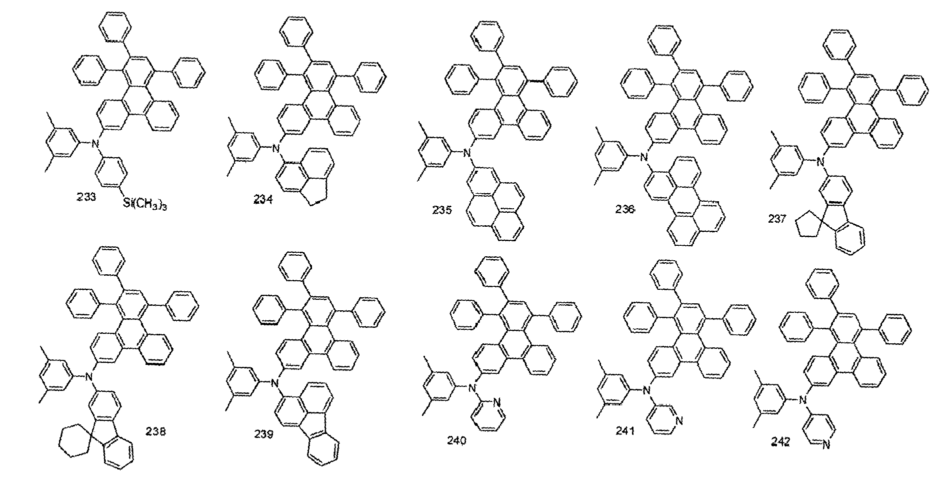

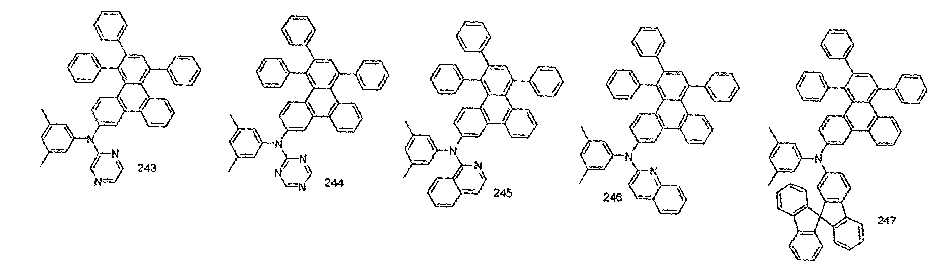

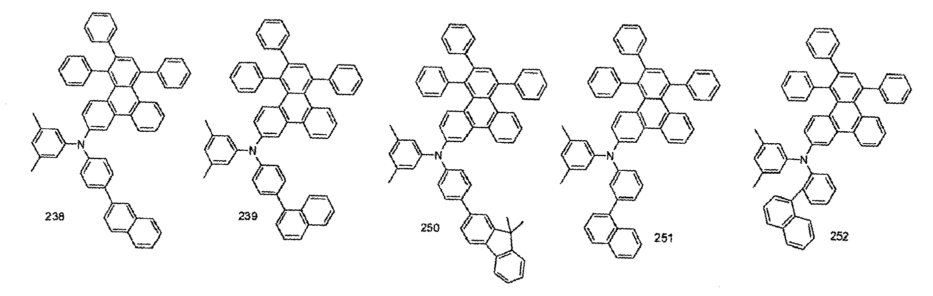

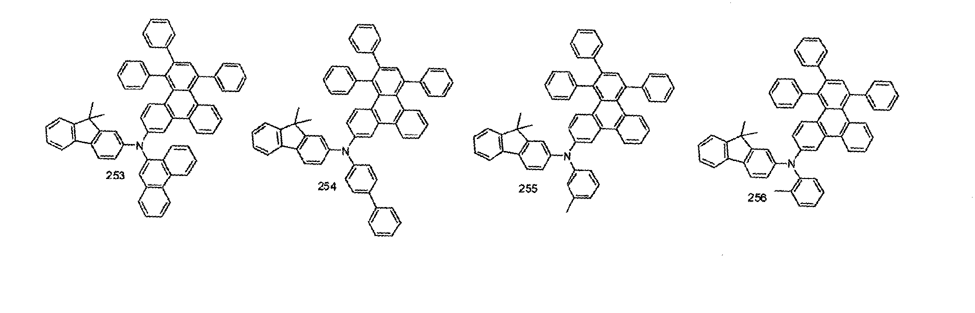

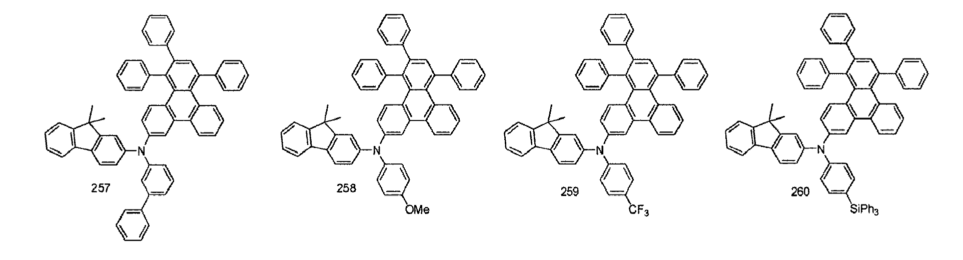

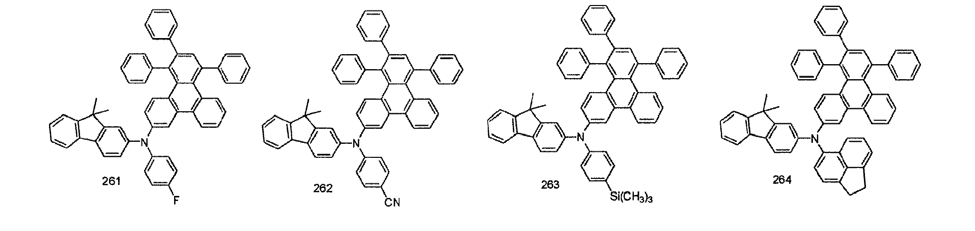

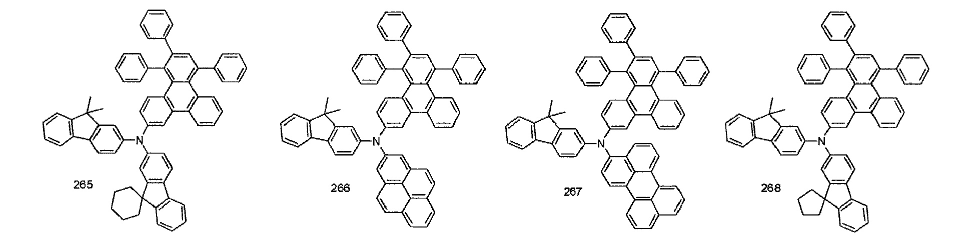

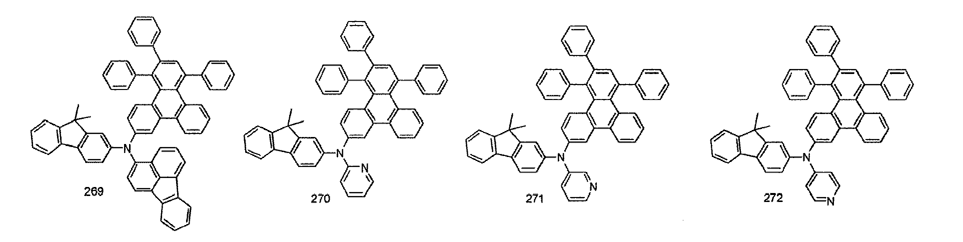

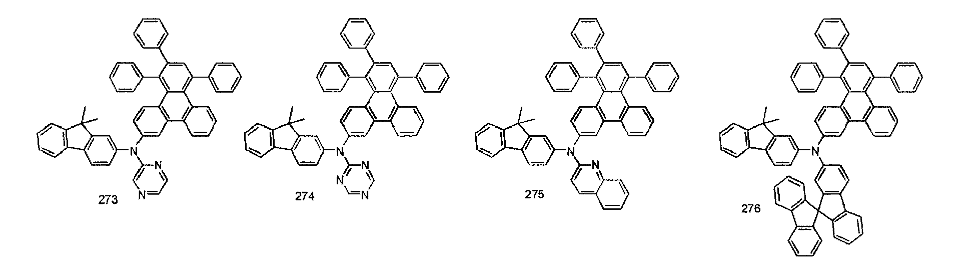

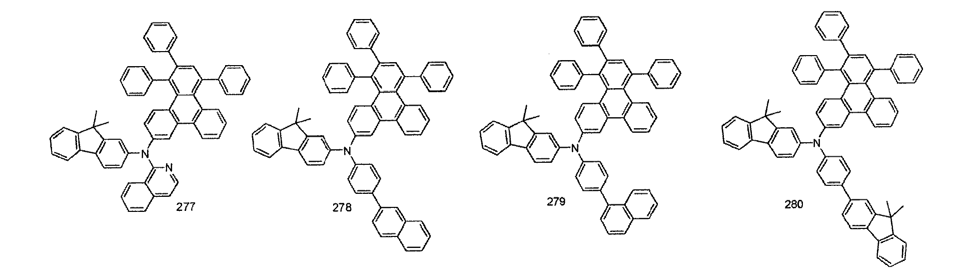

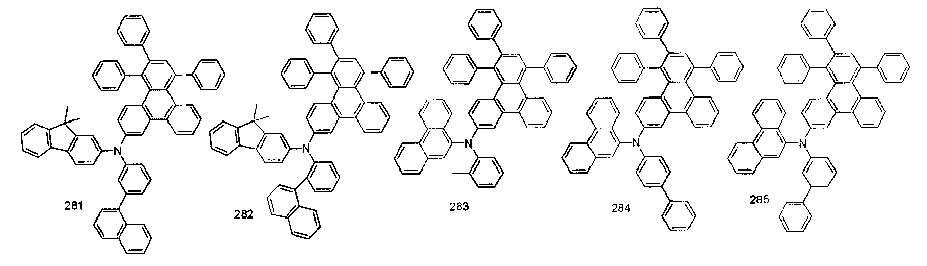

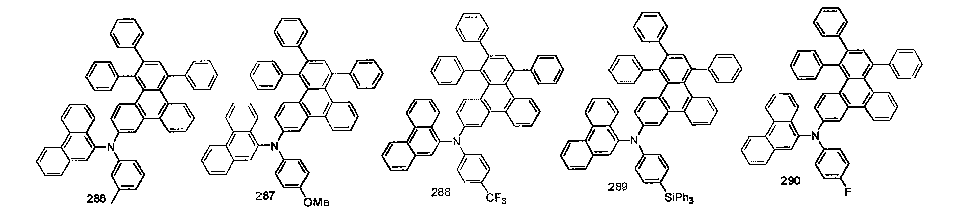

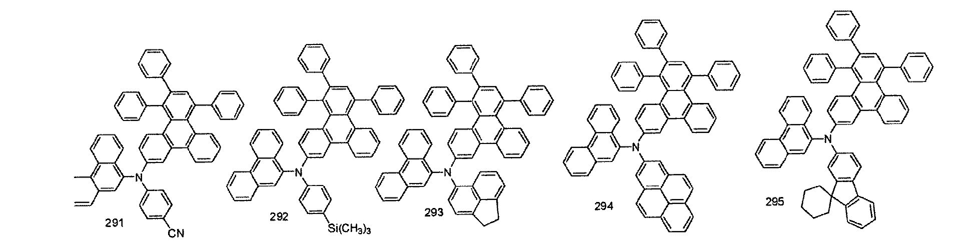

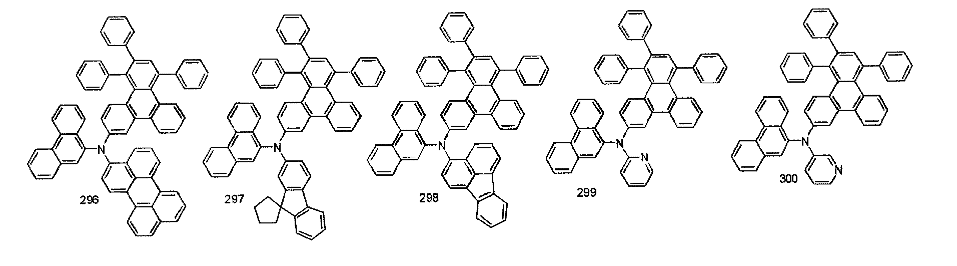

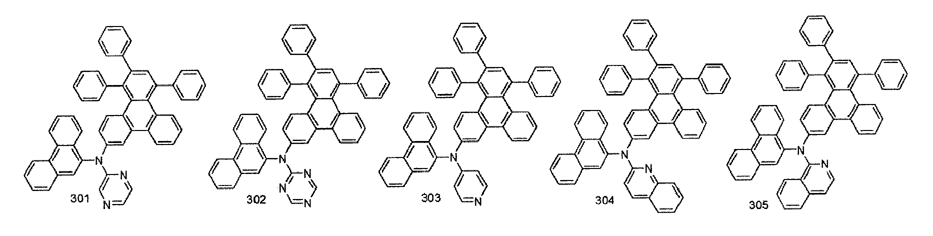

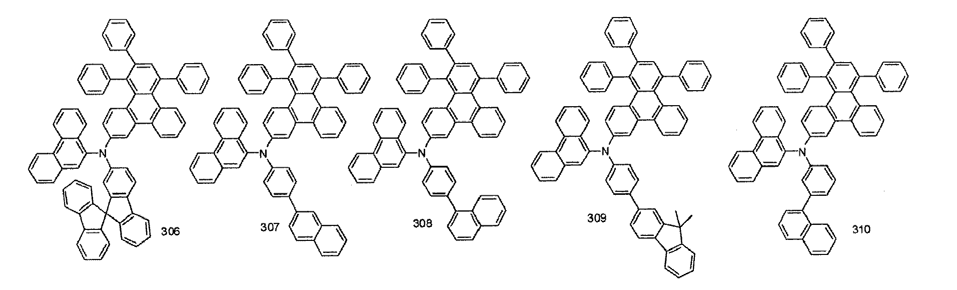

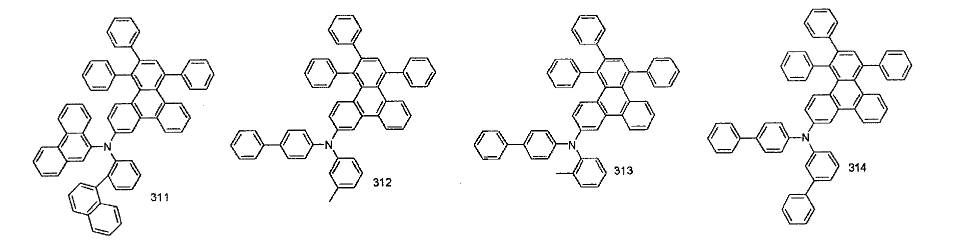

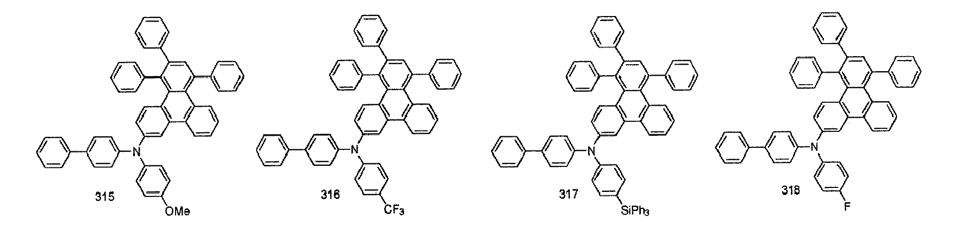

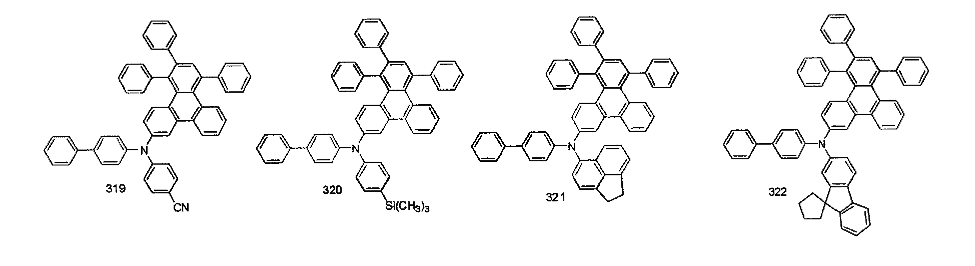

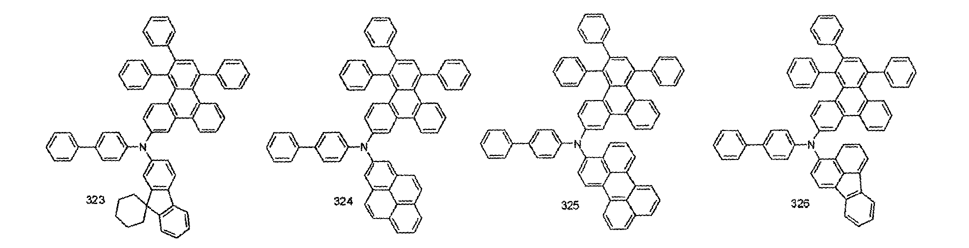

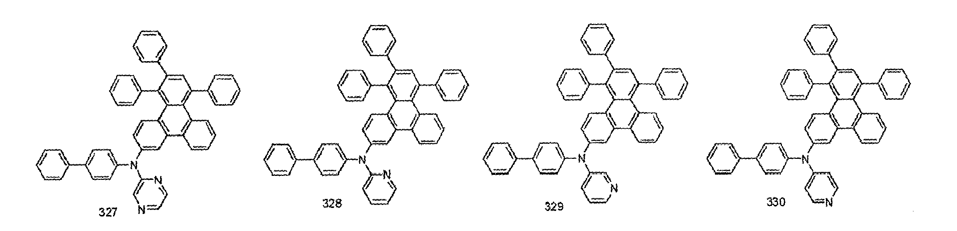

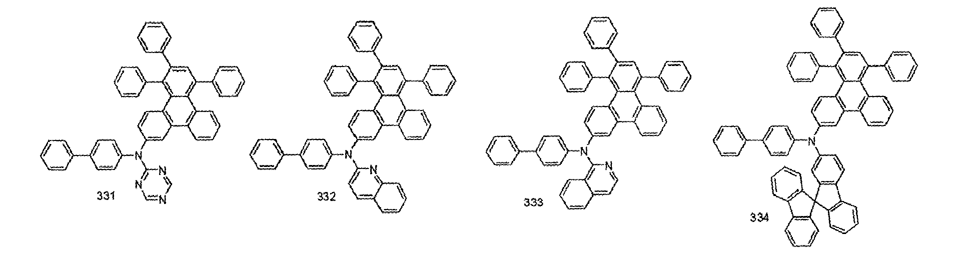

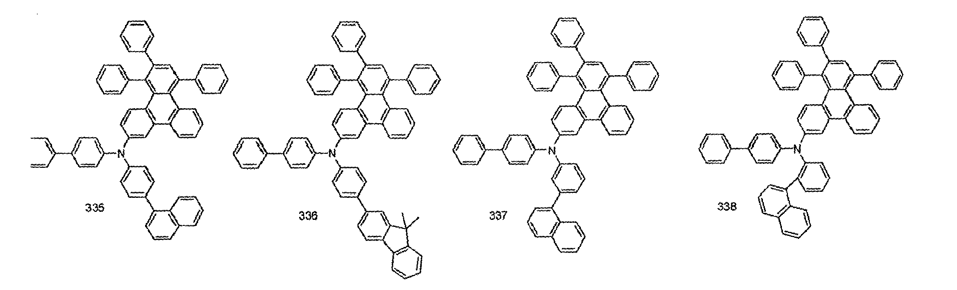

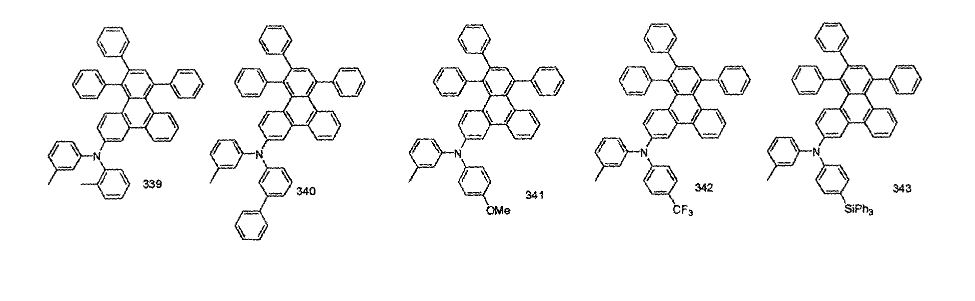

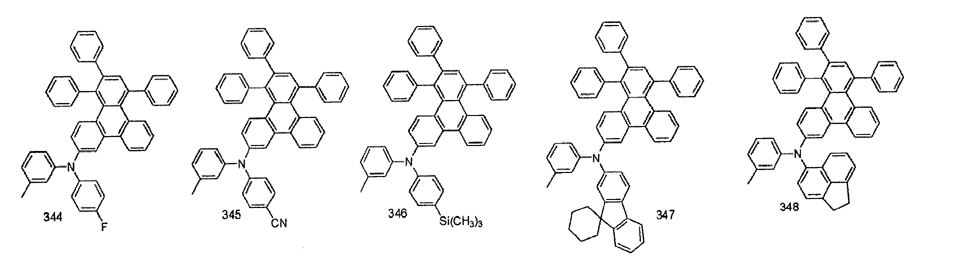

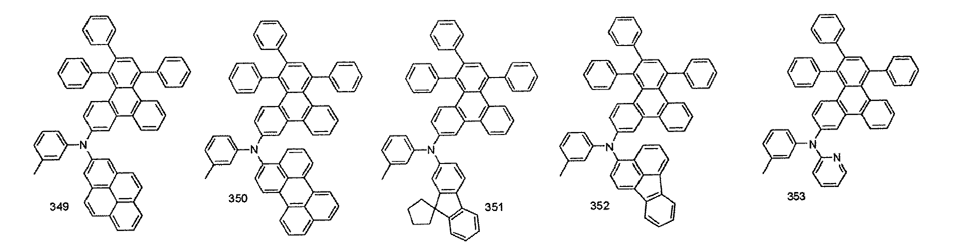

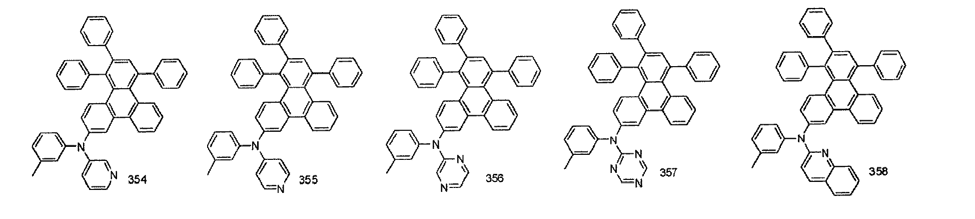

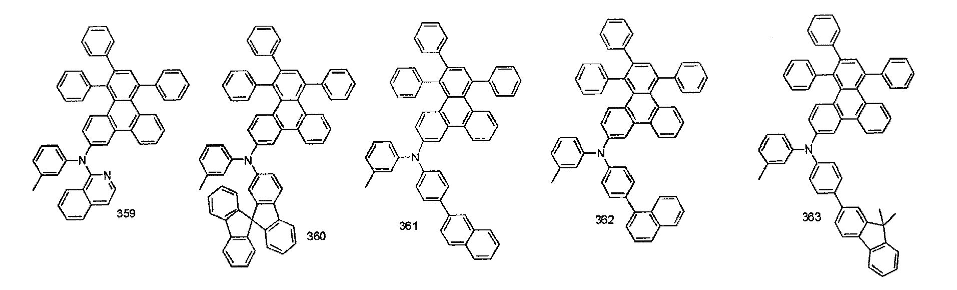

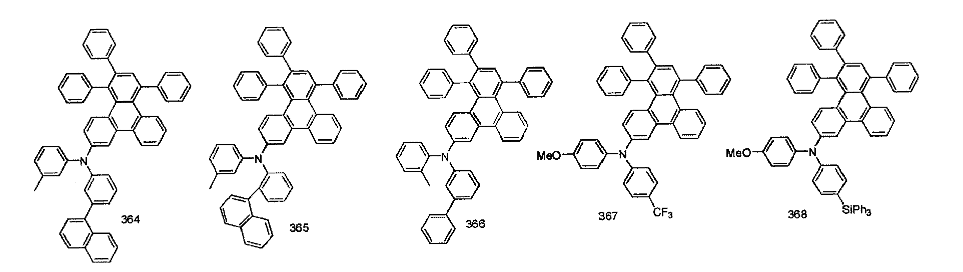

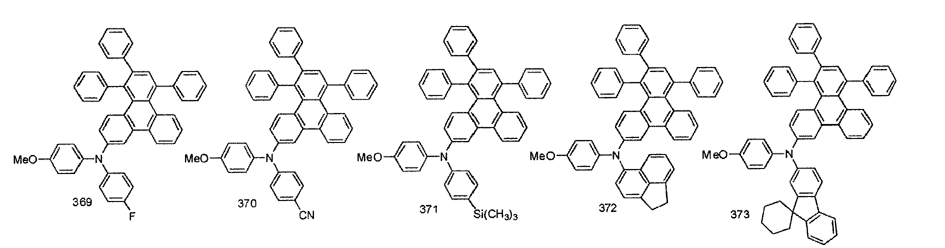

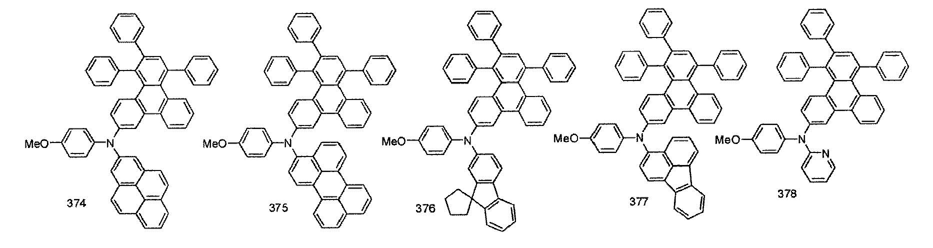

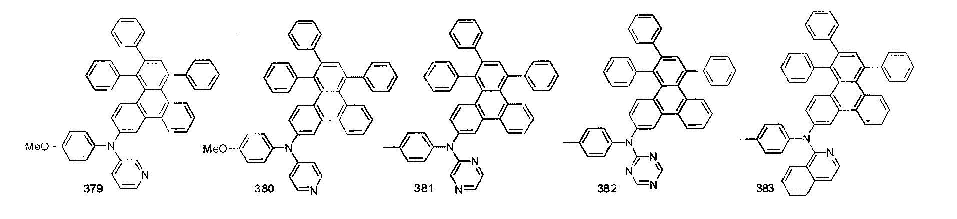

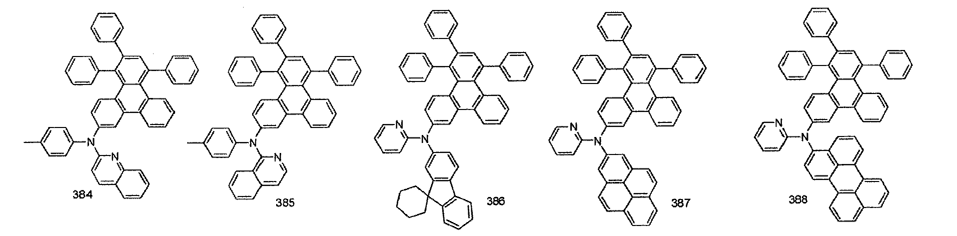

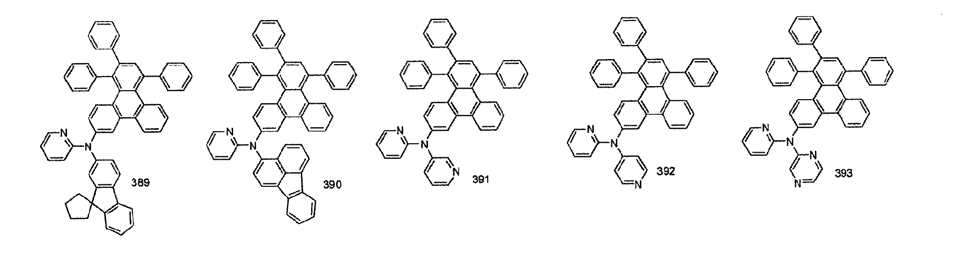

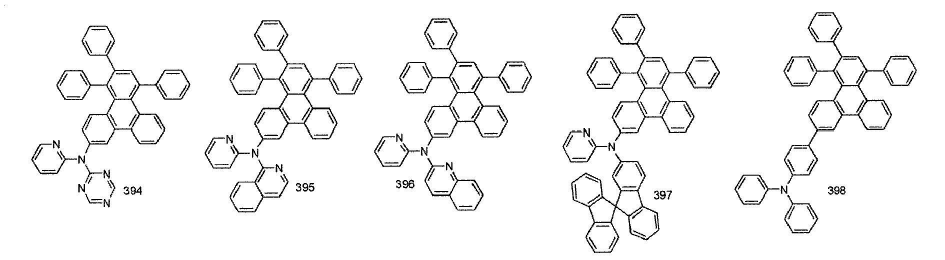

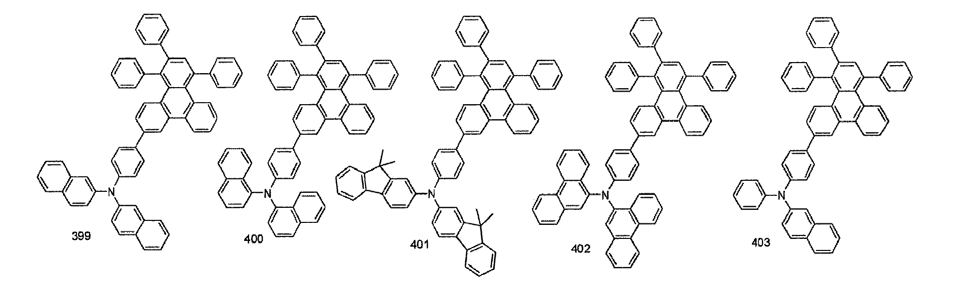

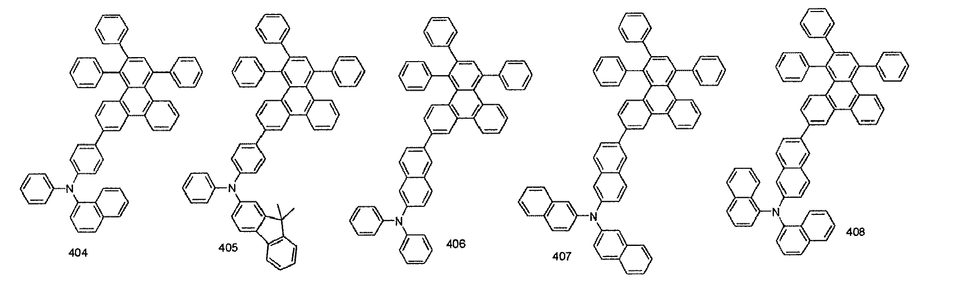

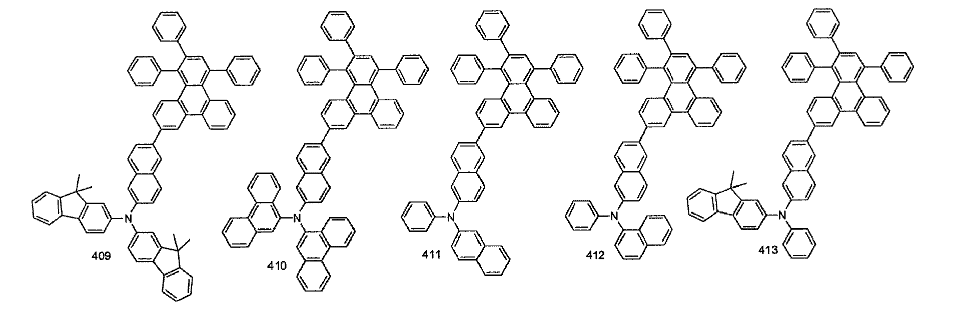

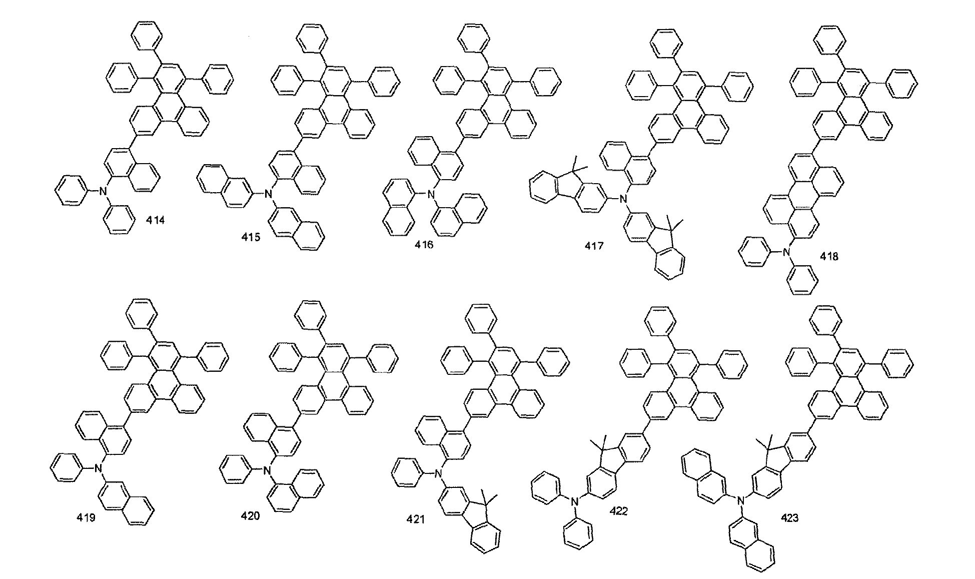

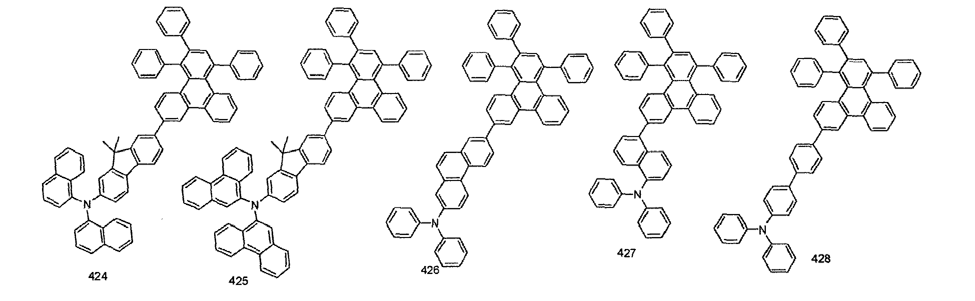

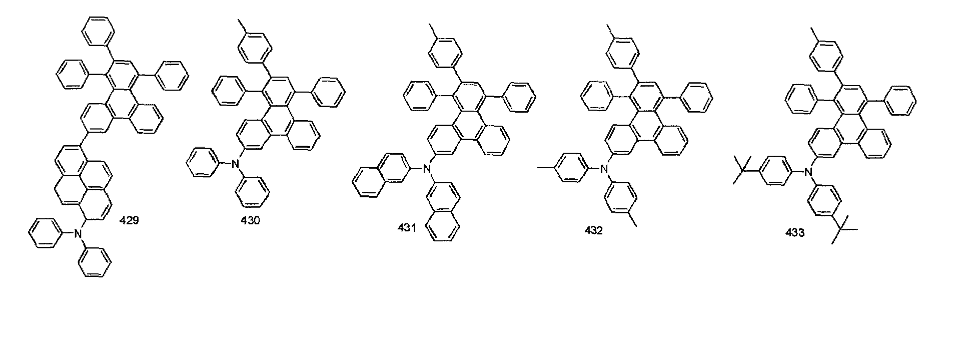

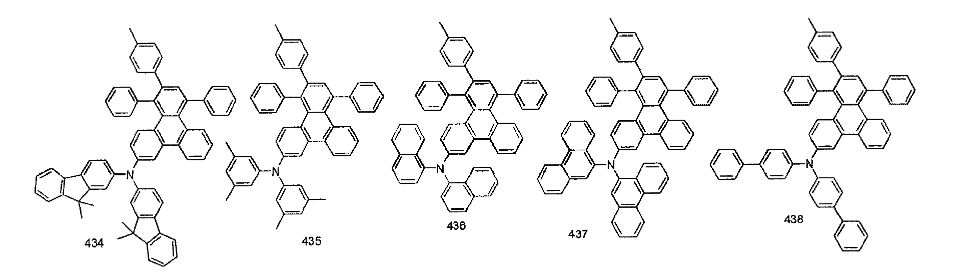

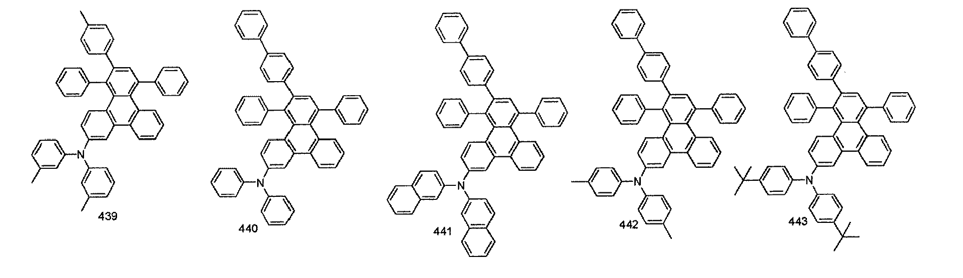

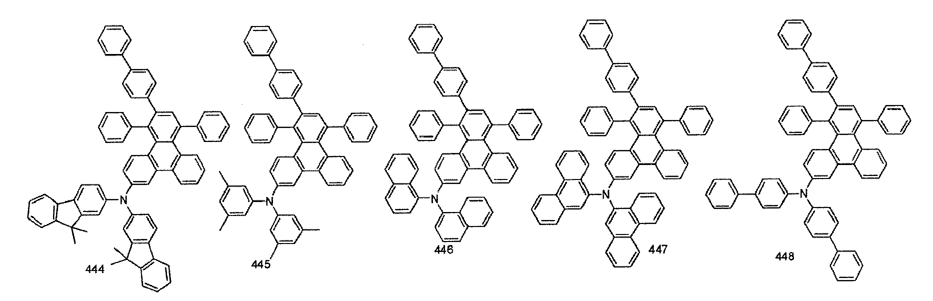

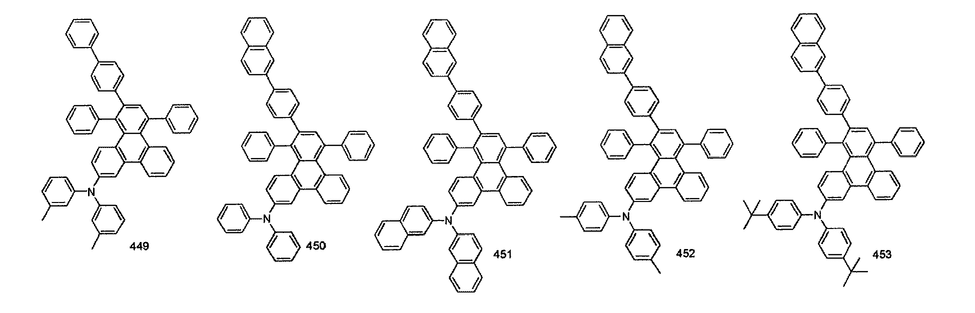

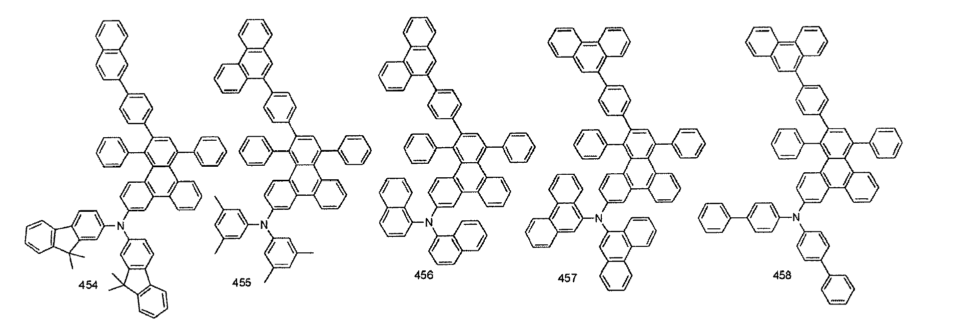

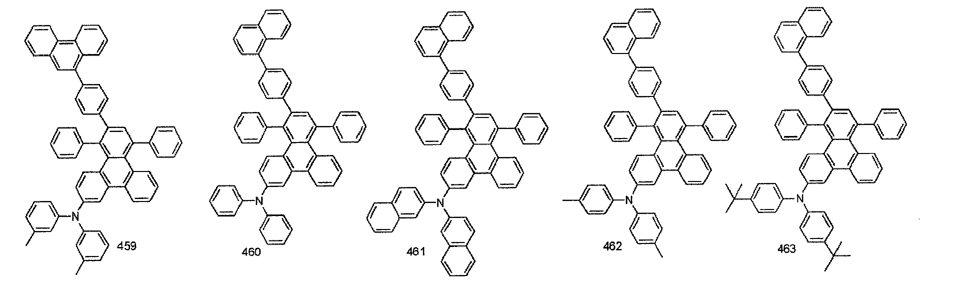

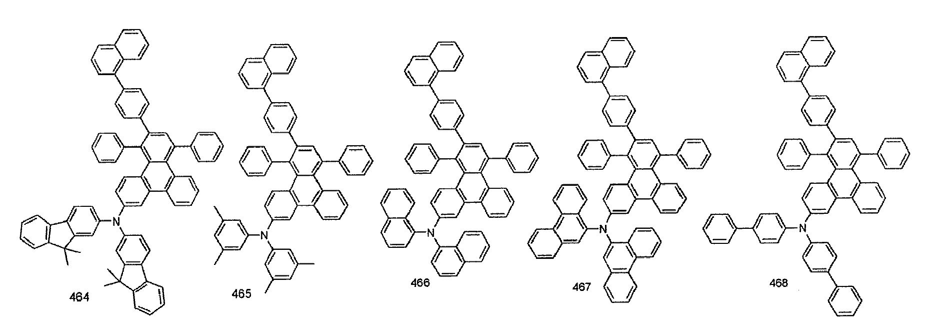

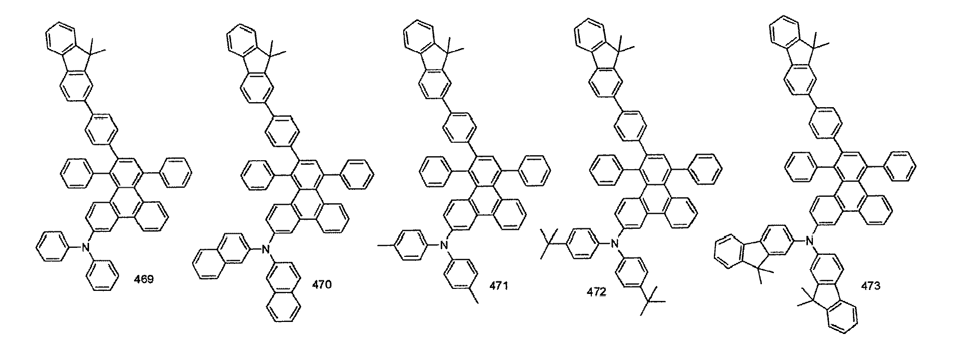

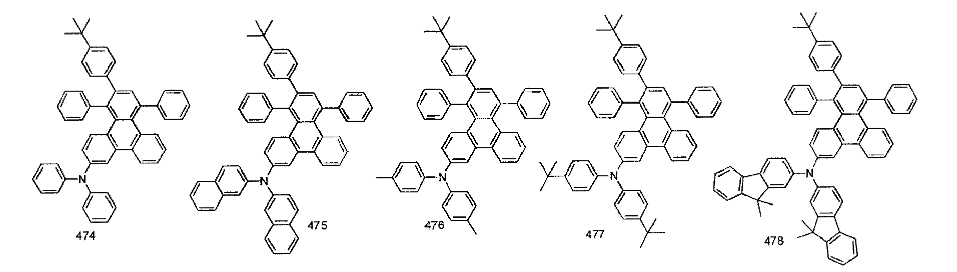

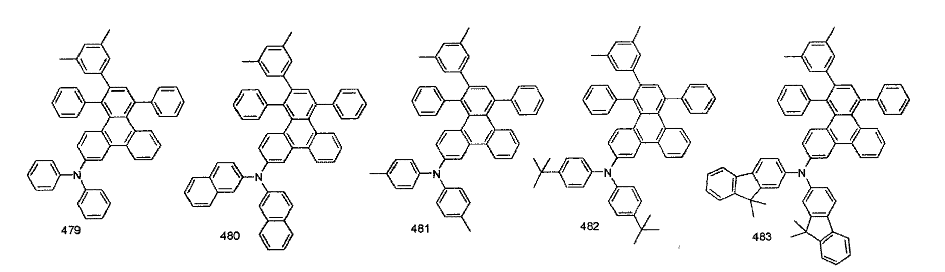

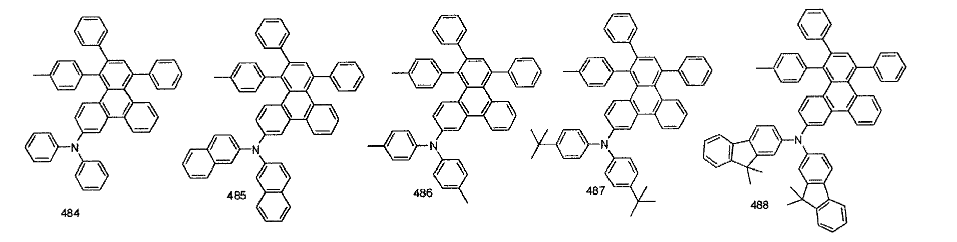

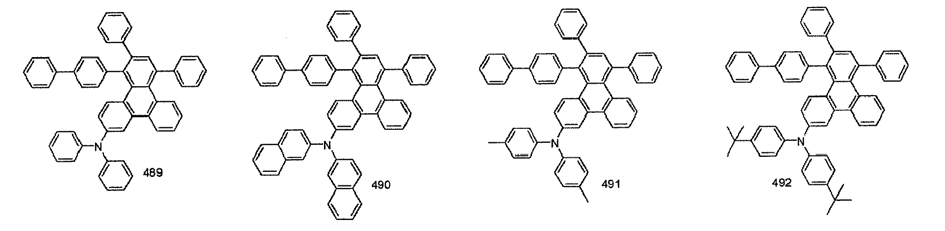

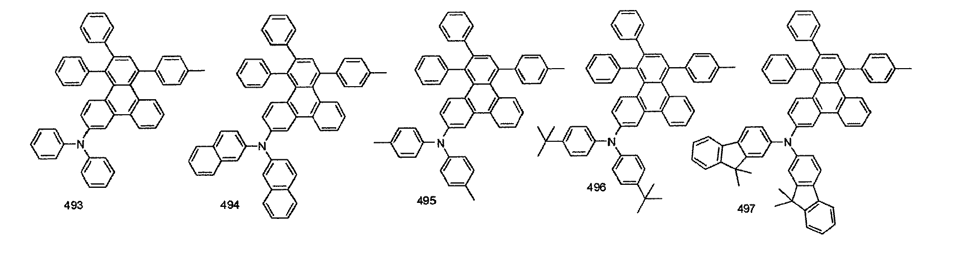

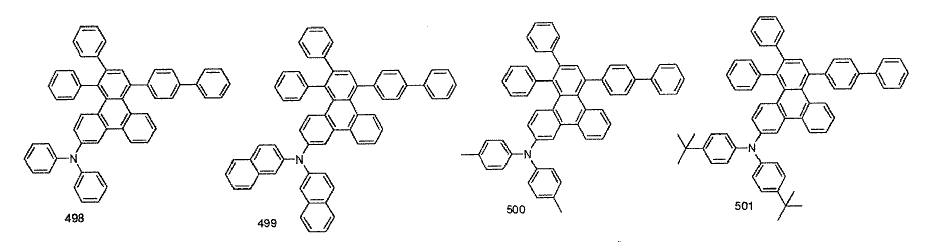

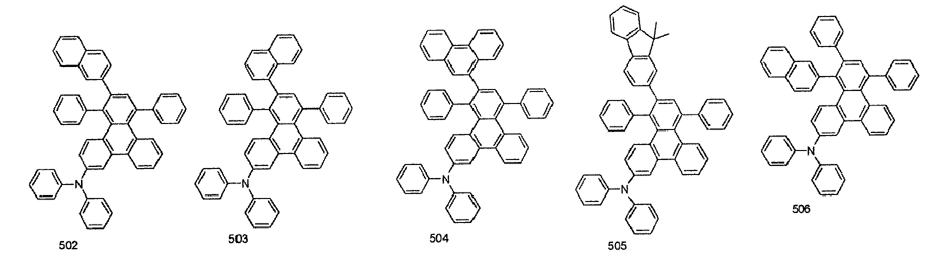

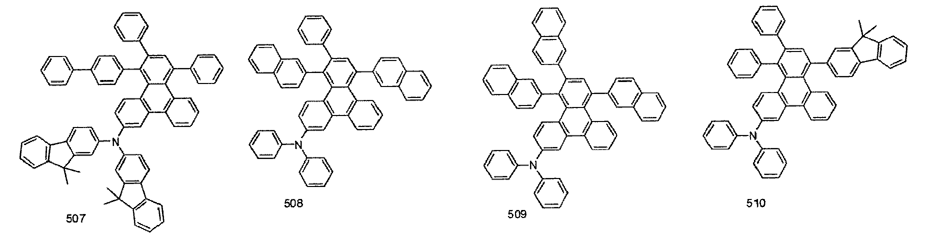

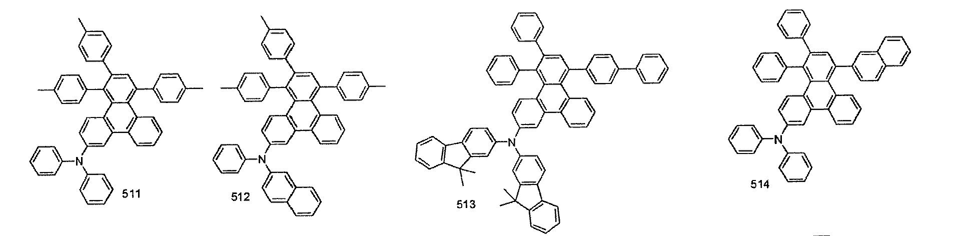

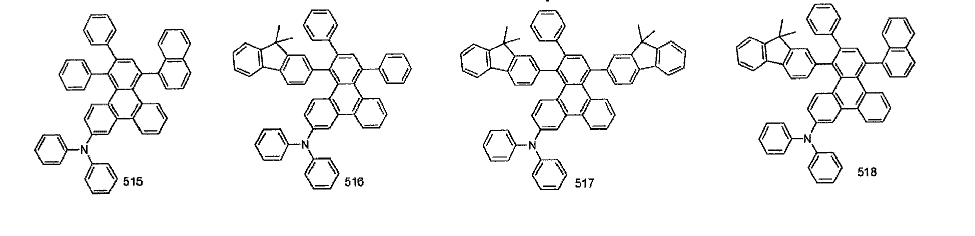

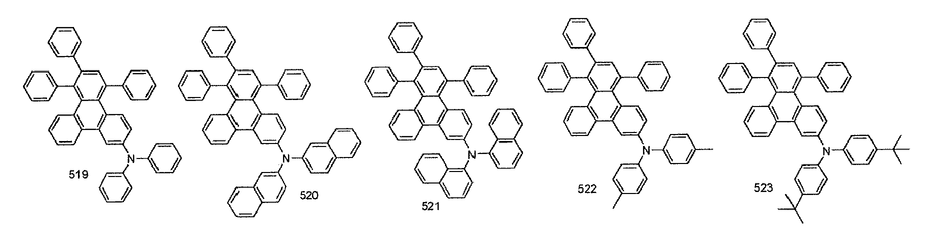

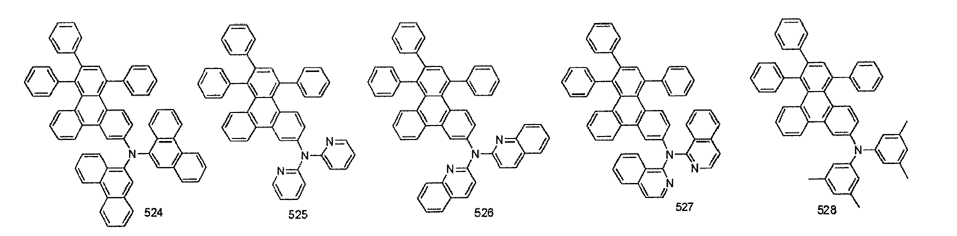

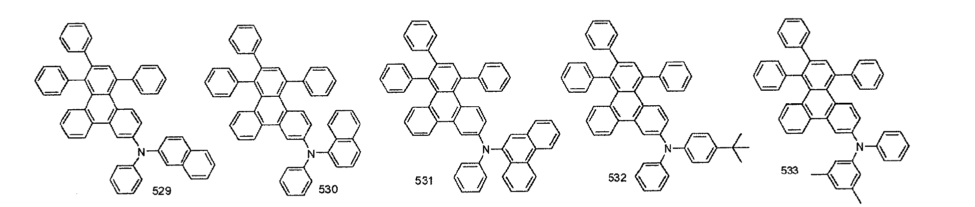

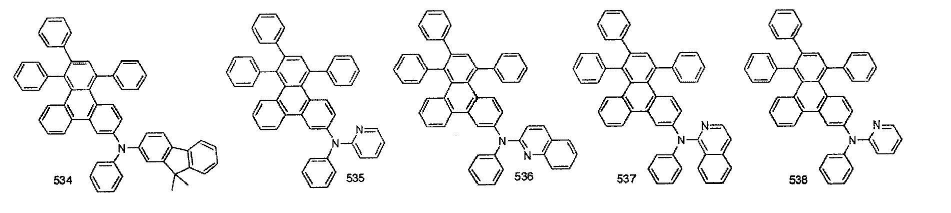

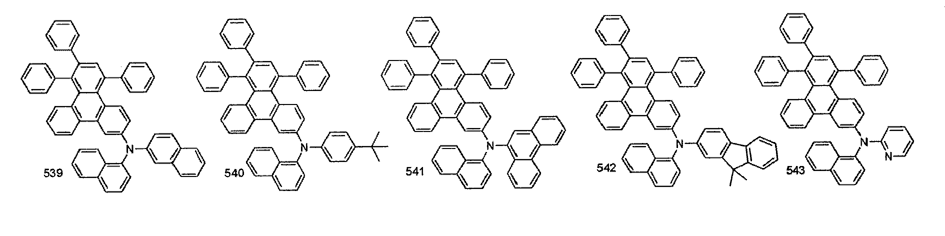

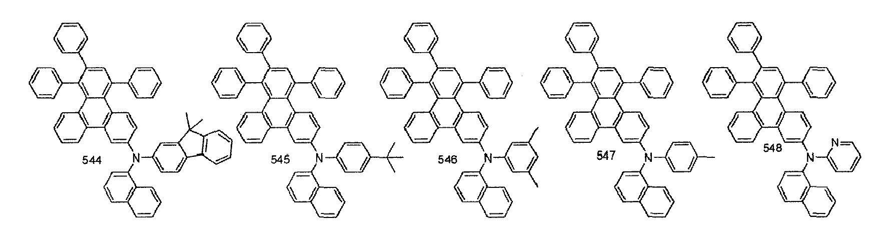

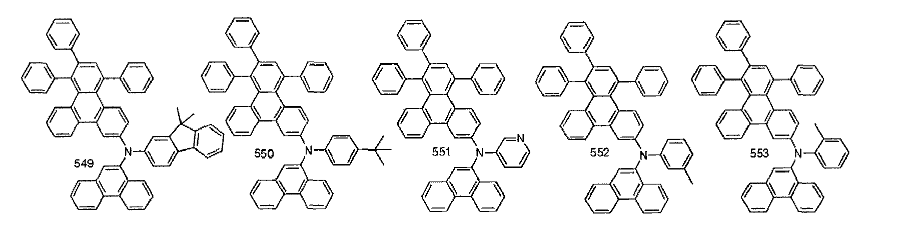

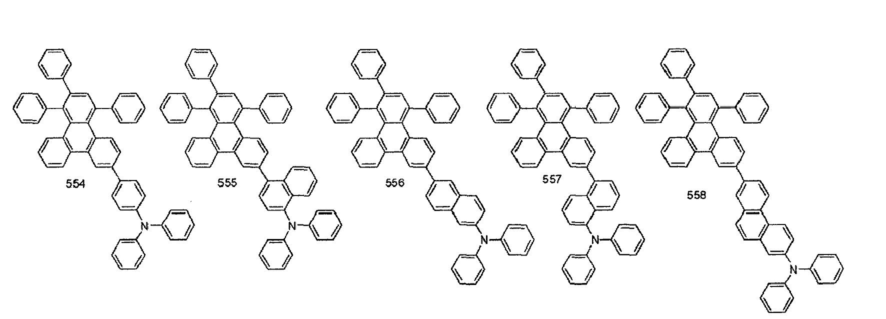

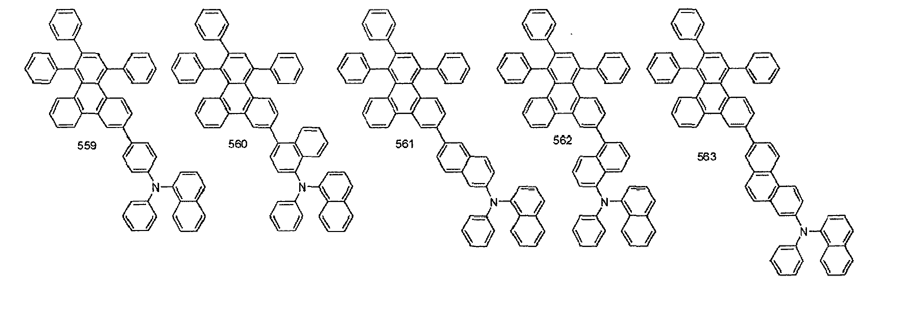

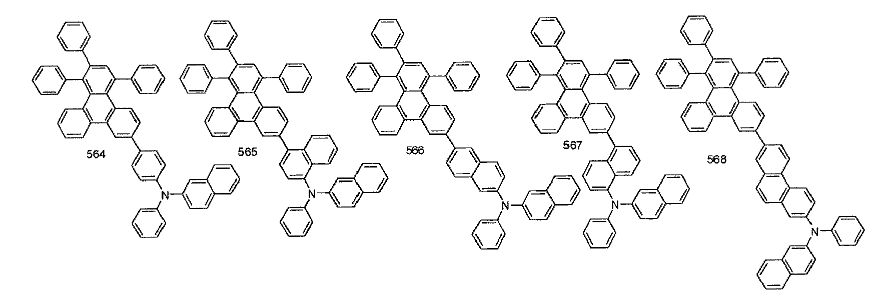

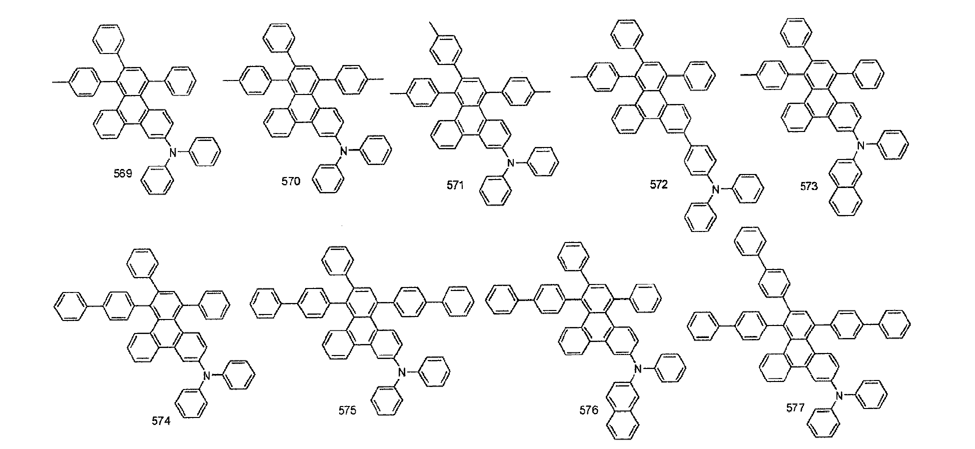

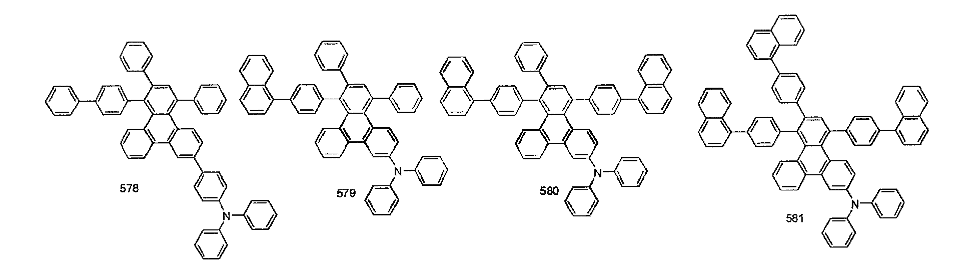

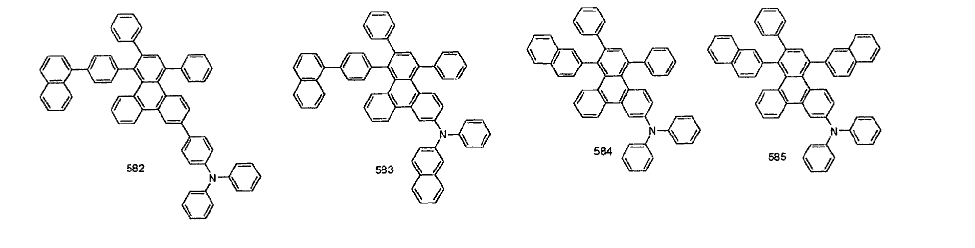

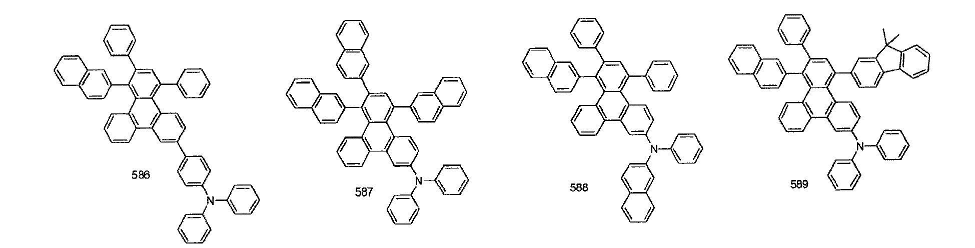

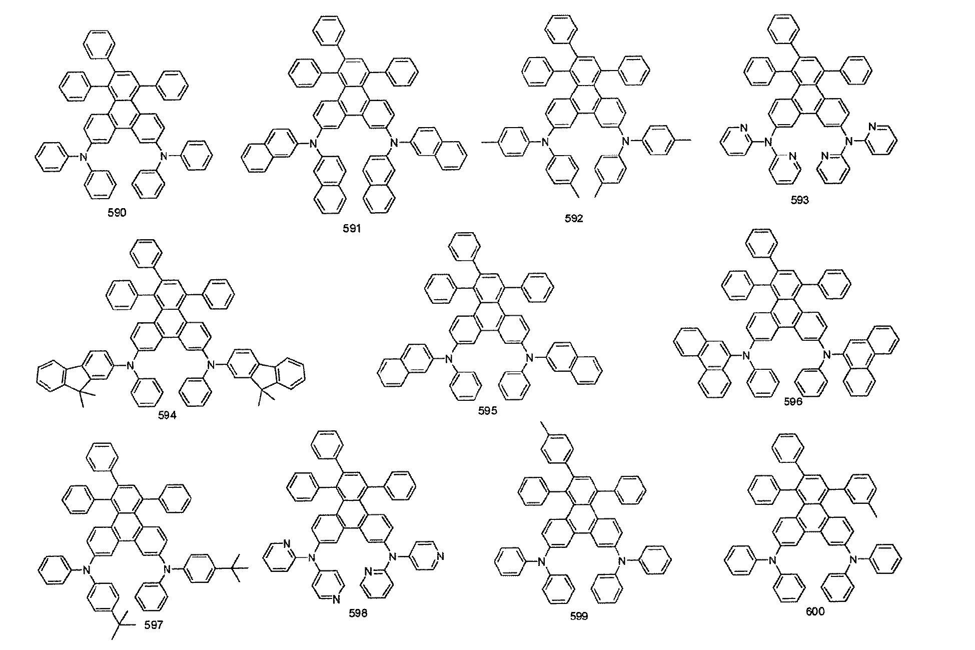

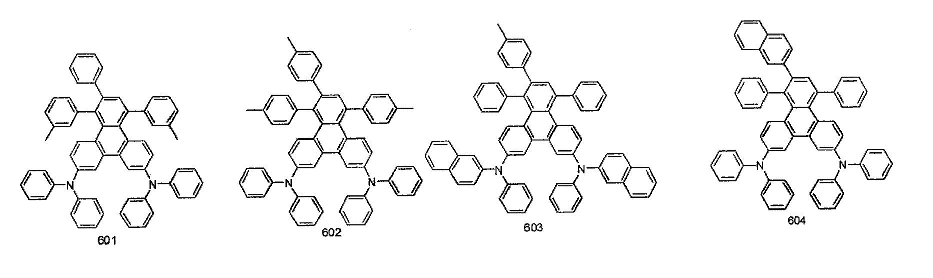

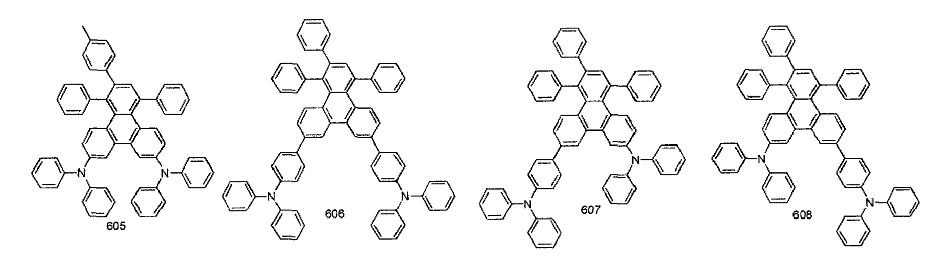

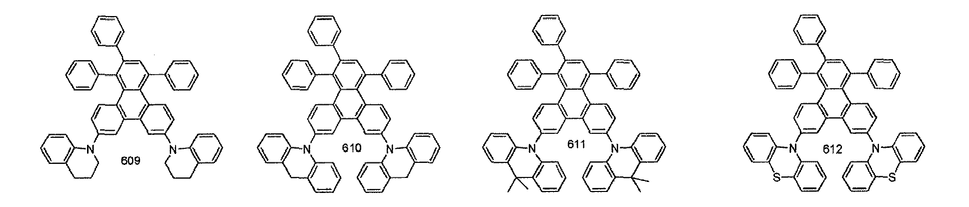

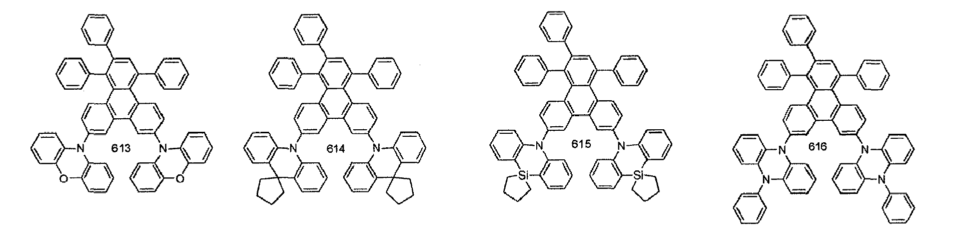

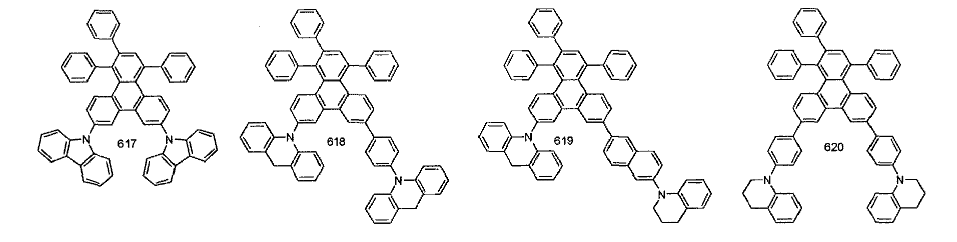

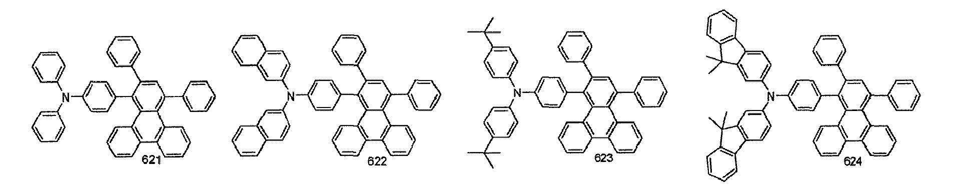

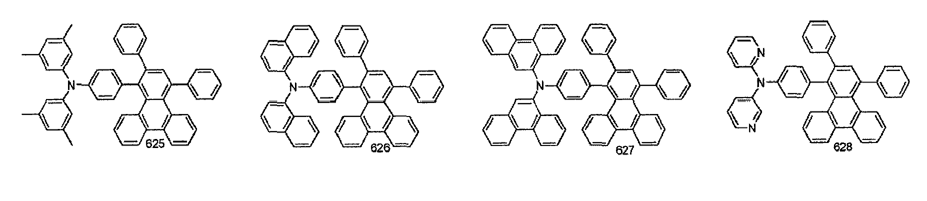

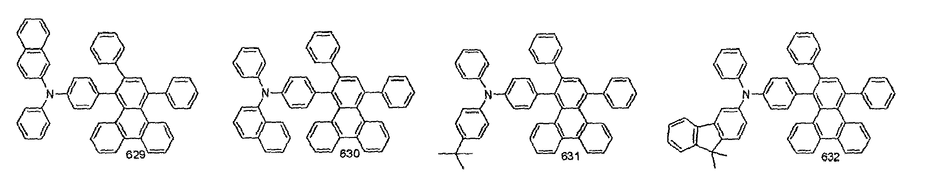

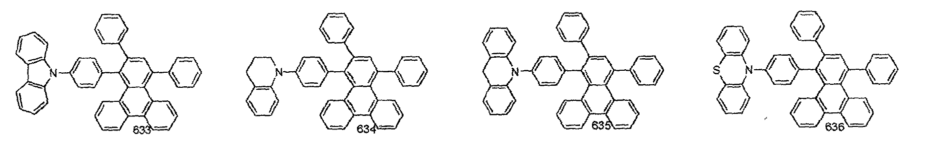

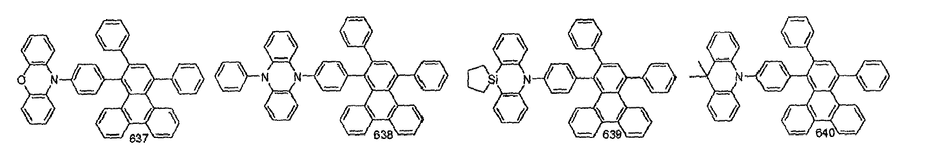

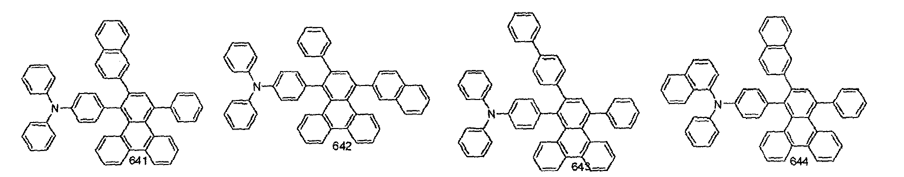

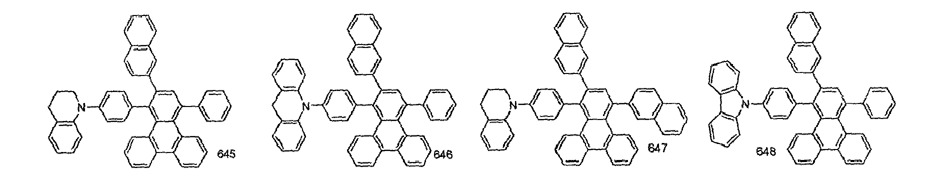

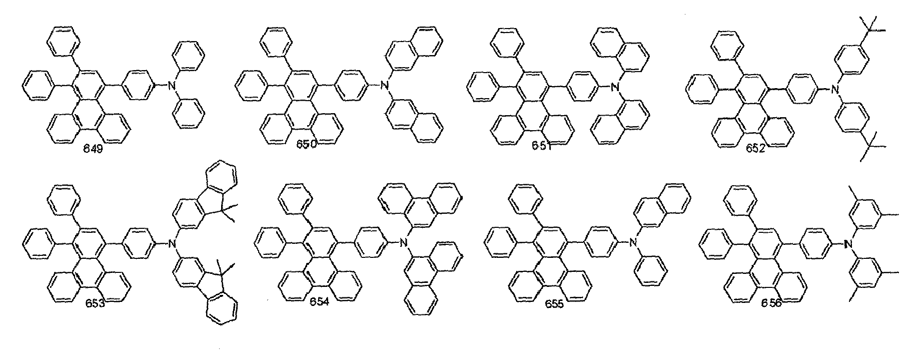

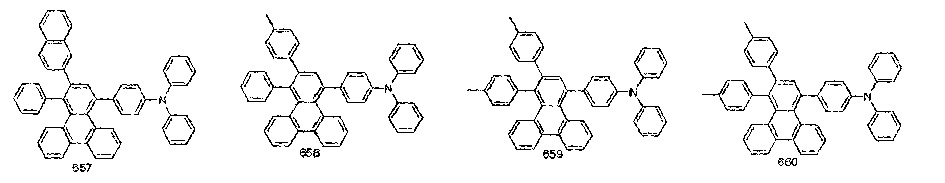

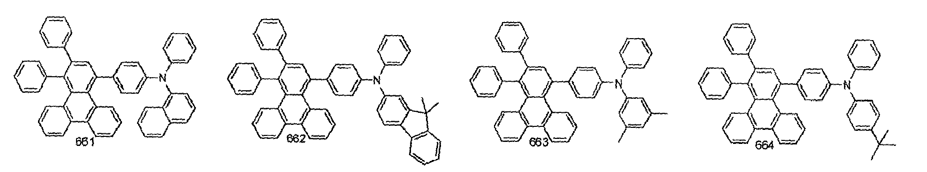

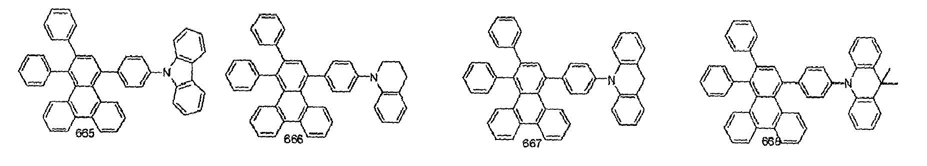

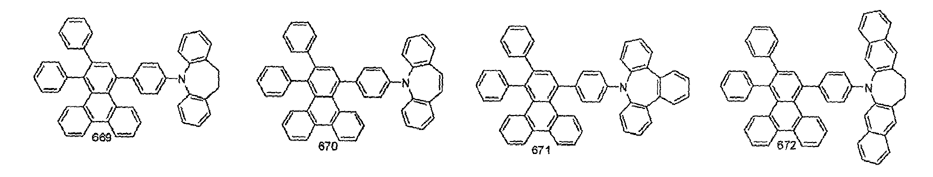

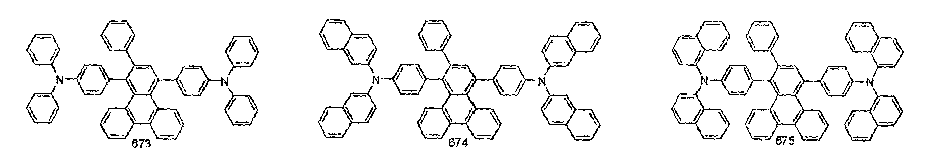

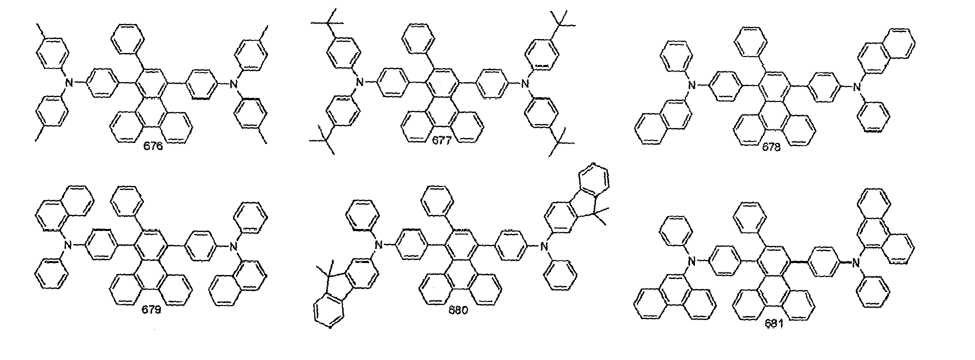

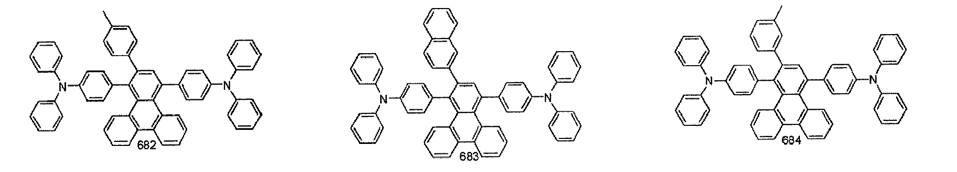

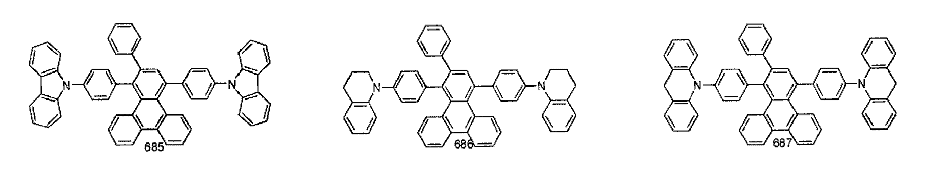

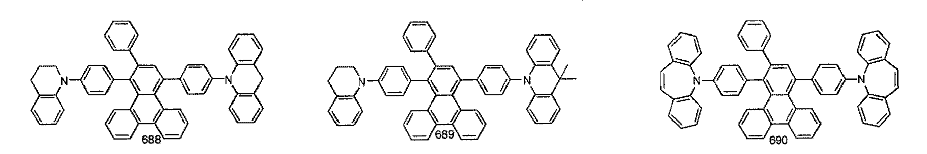

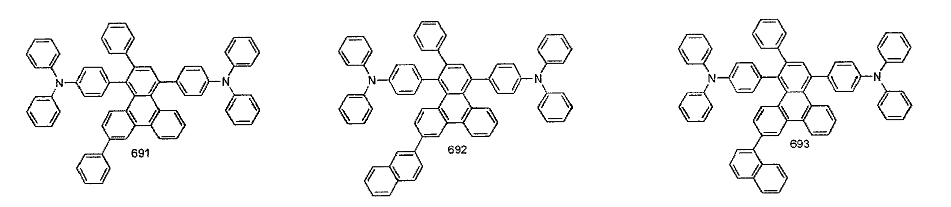

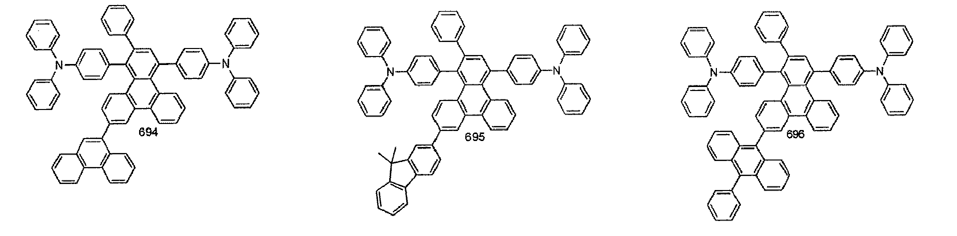

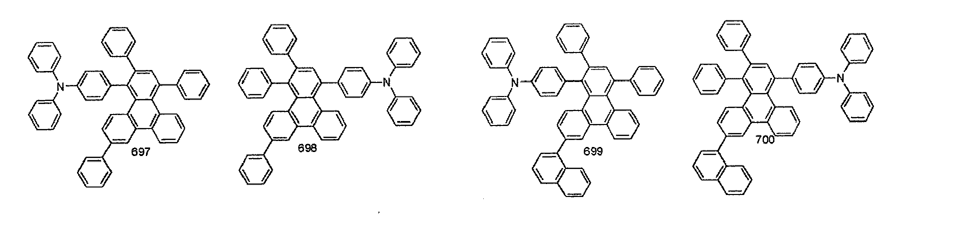

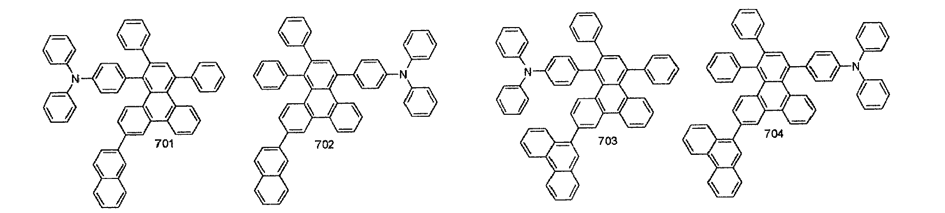

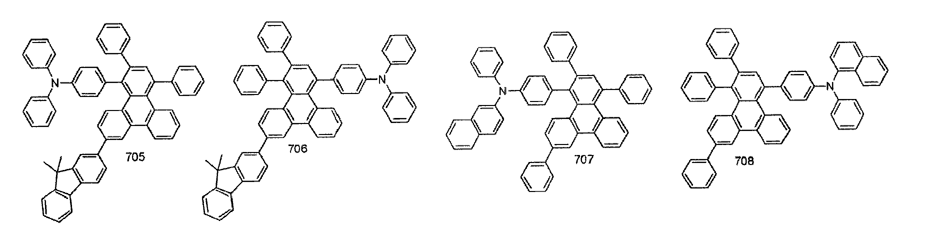

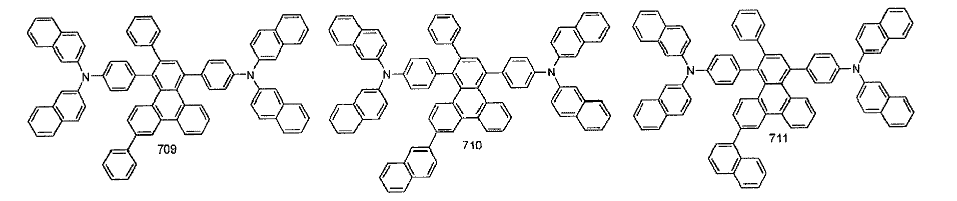

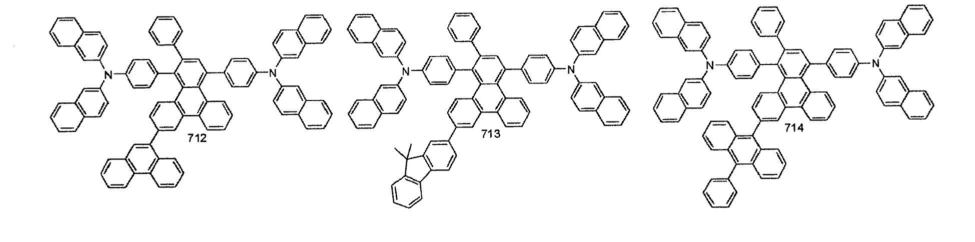

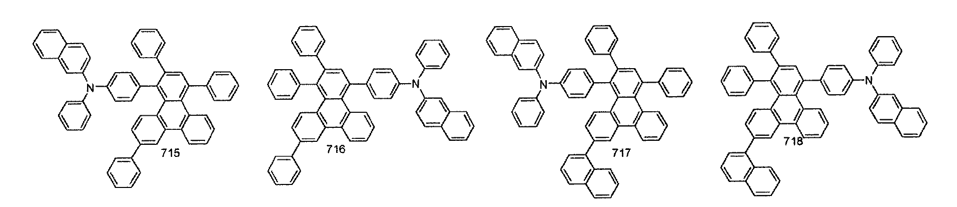

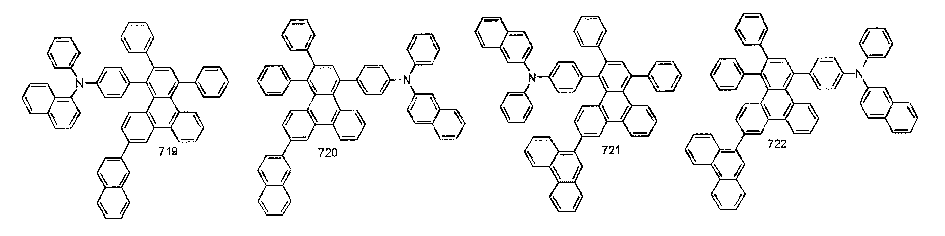

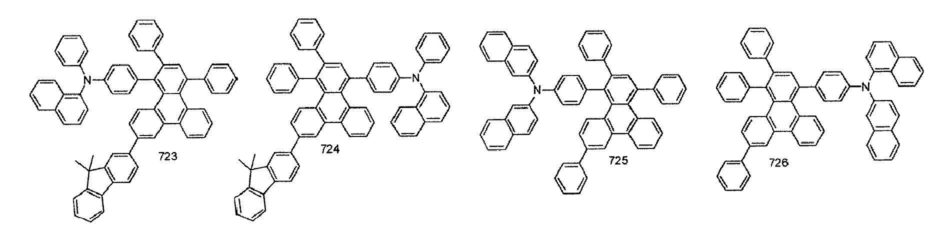

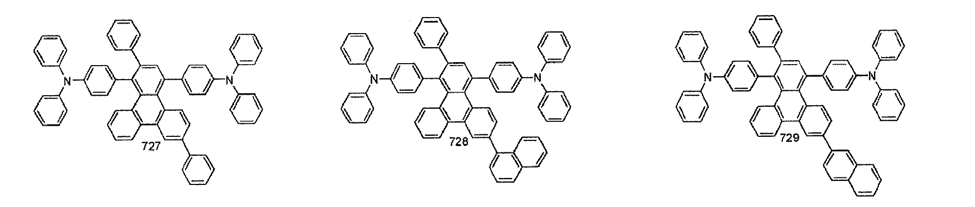

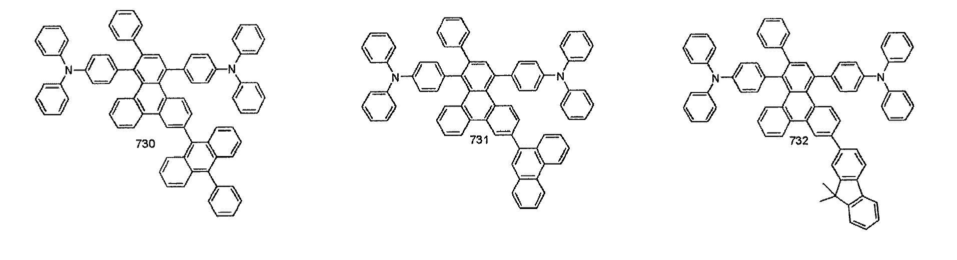

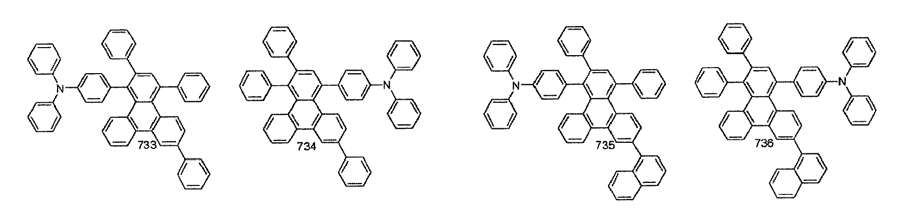

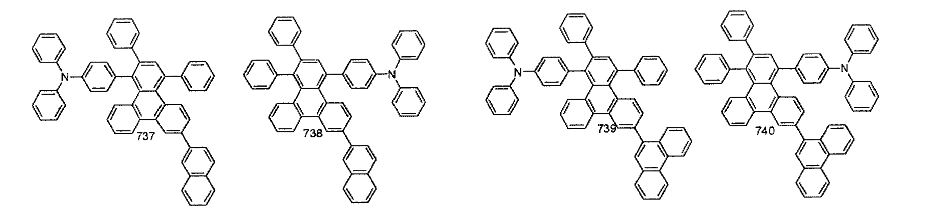

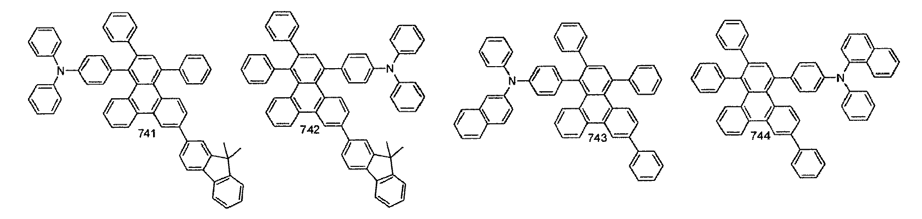

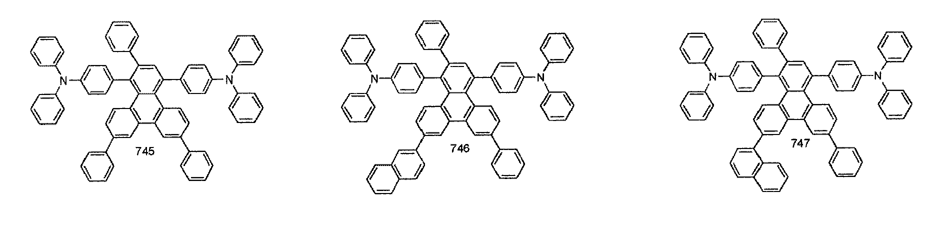

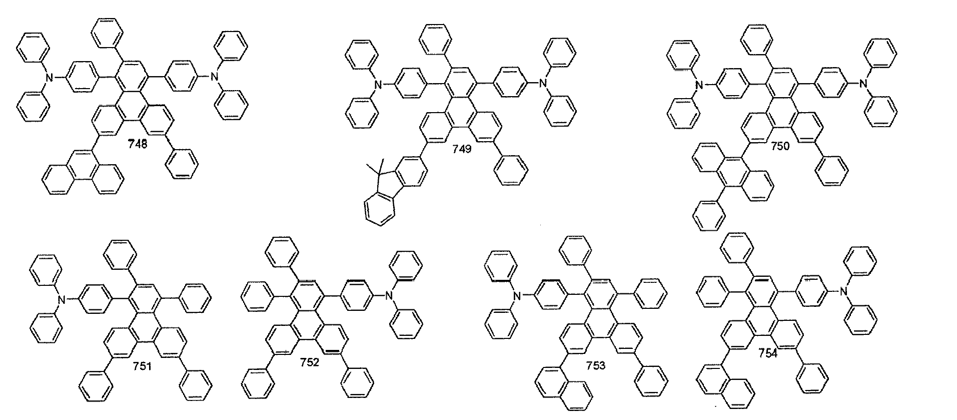

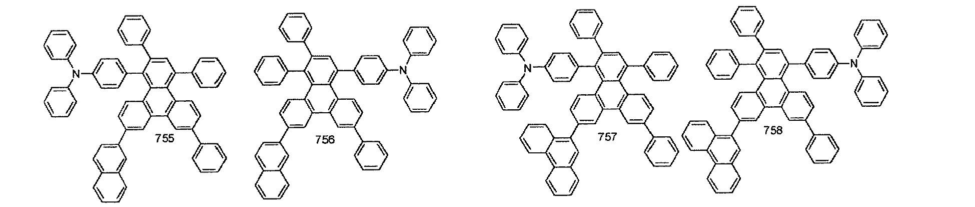

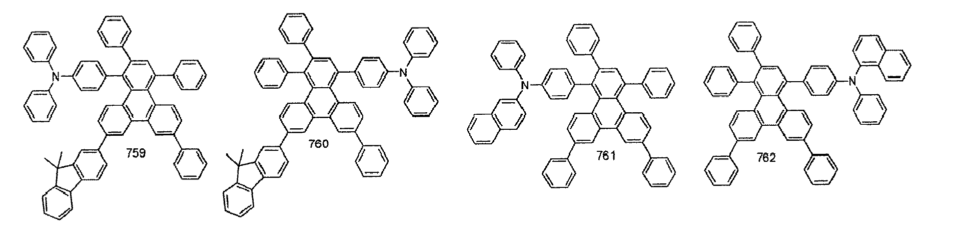

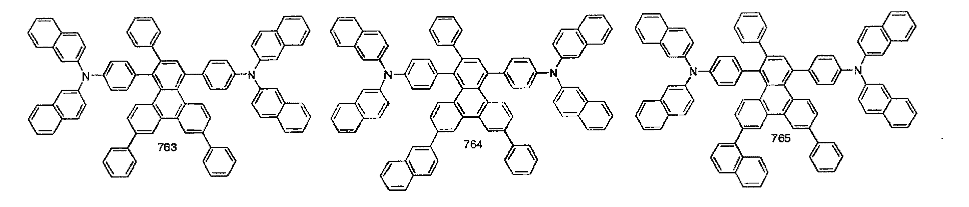

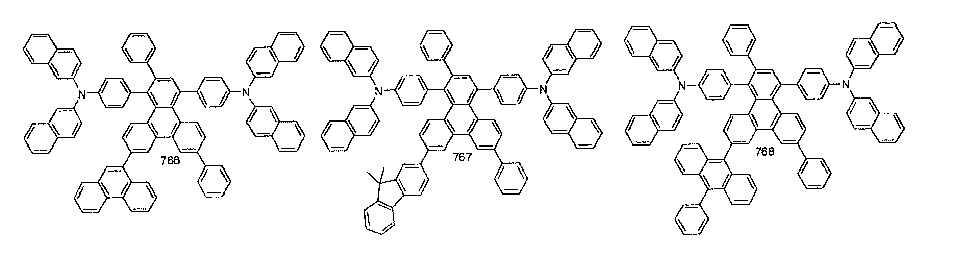

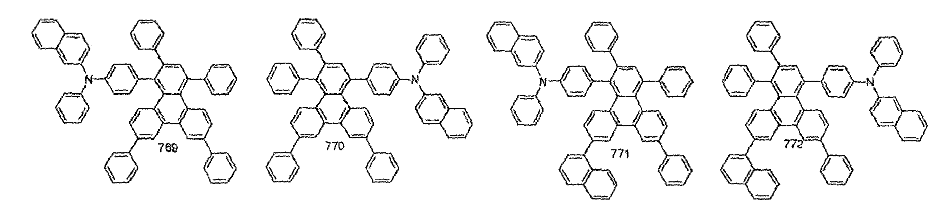

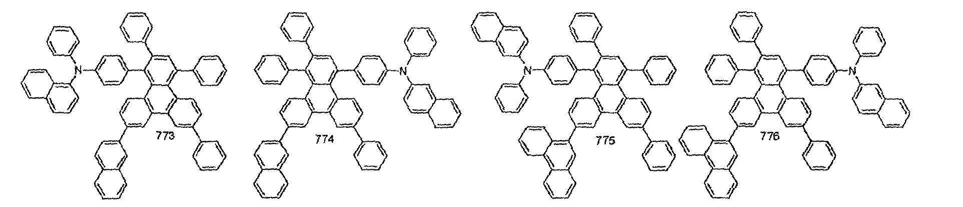

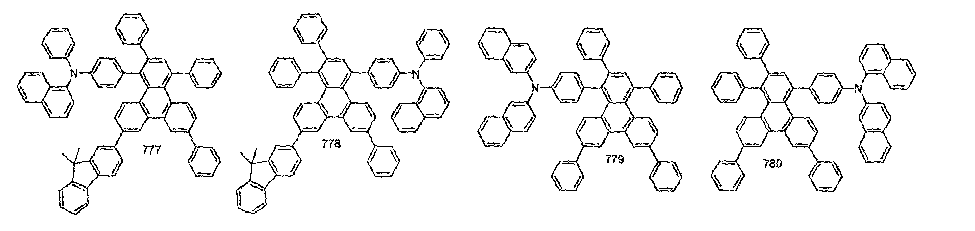

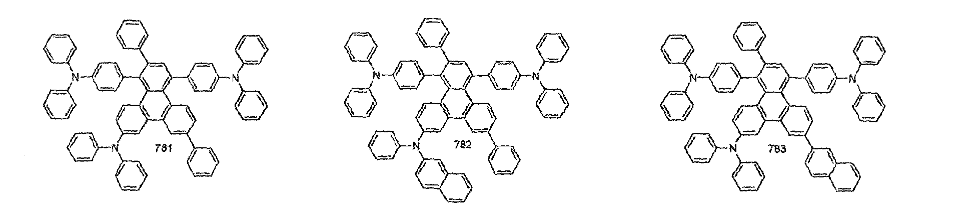

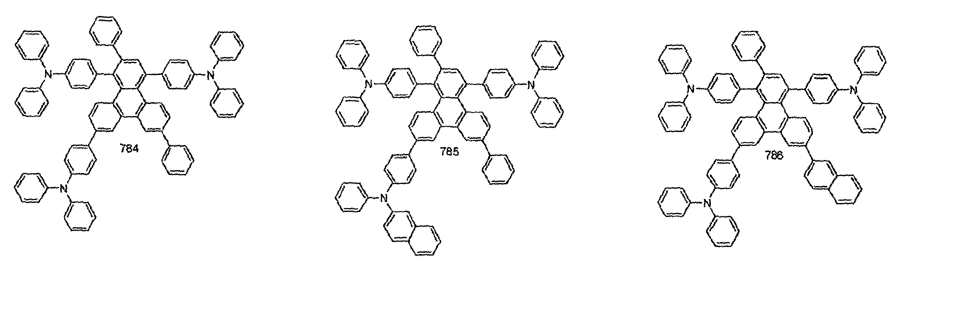

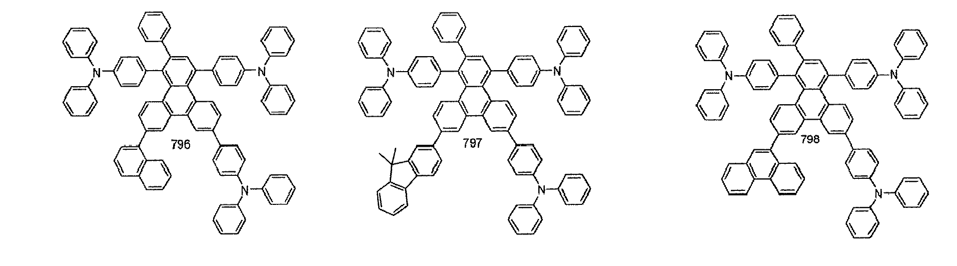

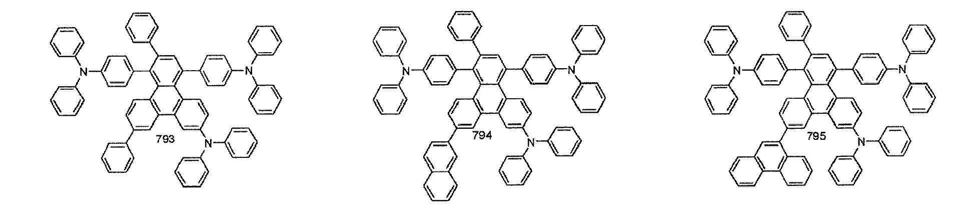

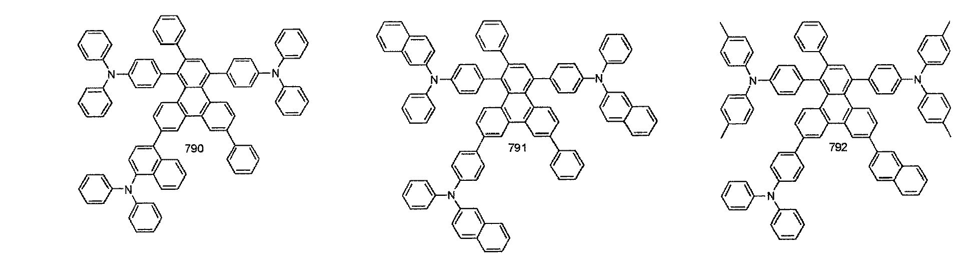

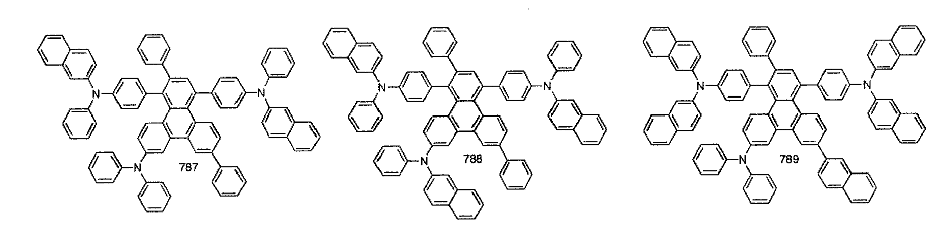

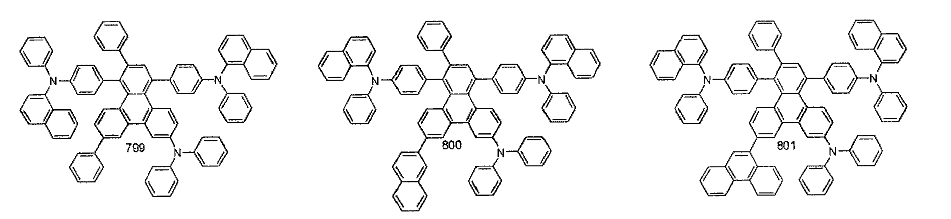

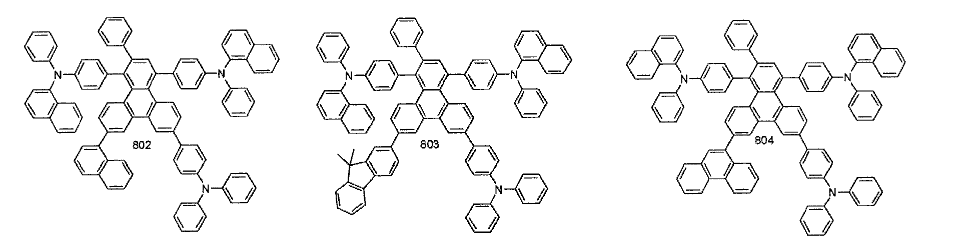

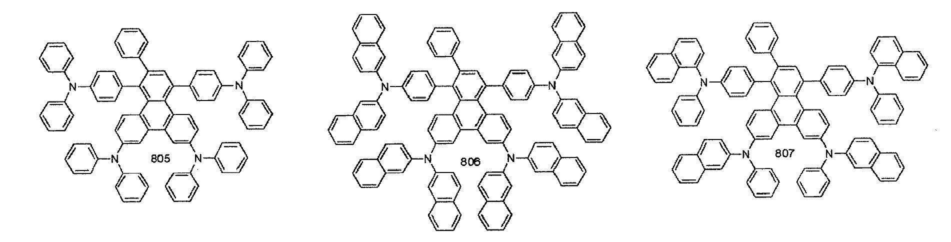

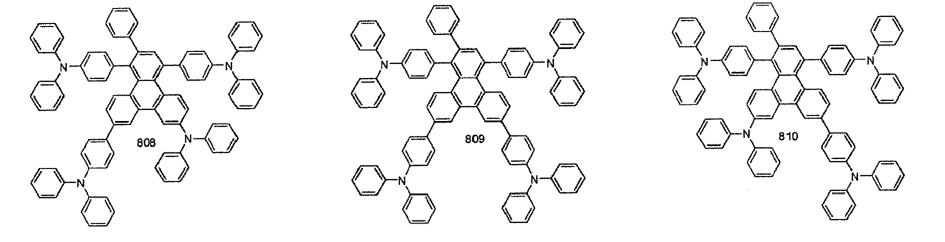

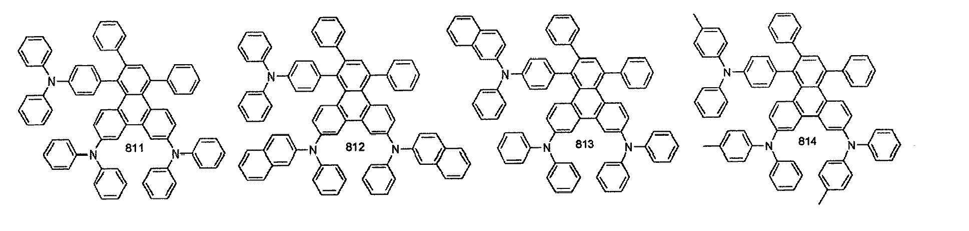

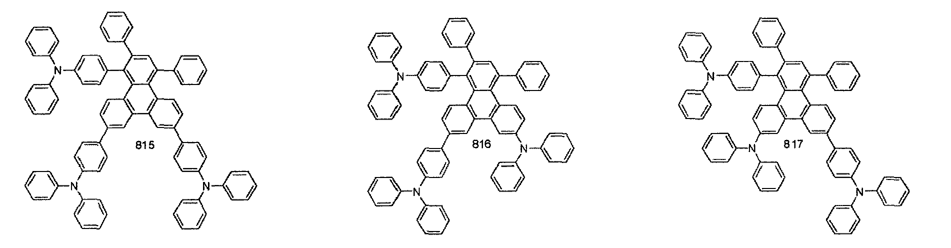

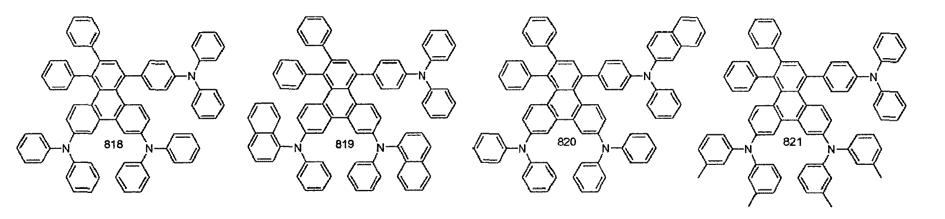

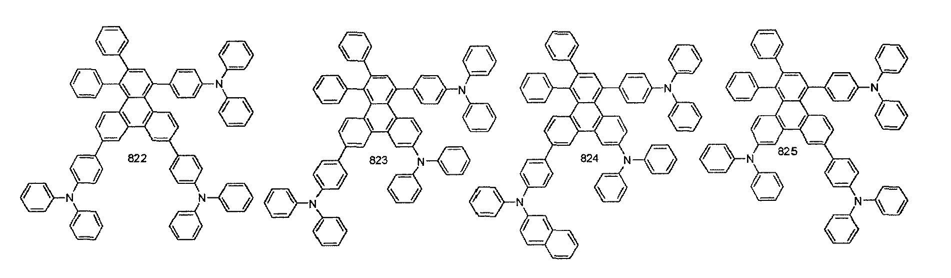

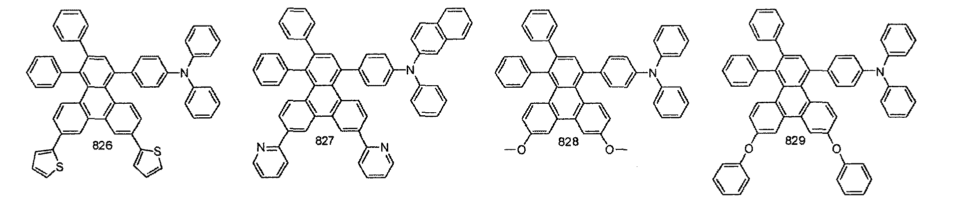

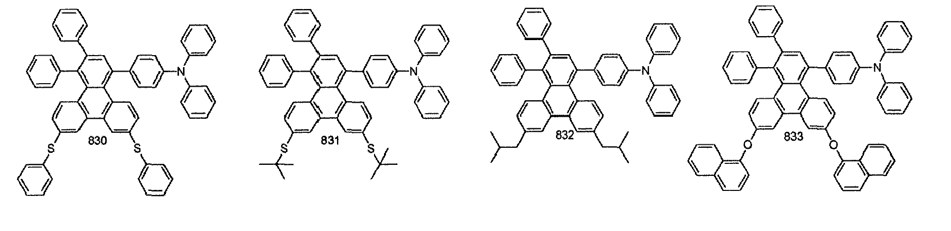

The organic electroluminescent compounds according to the present invention can be prepared according to the procedure illustrated by Reaction Scheme (1) or (2):

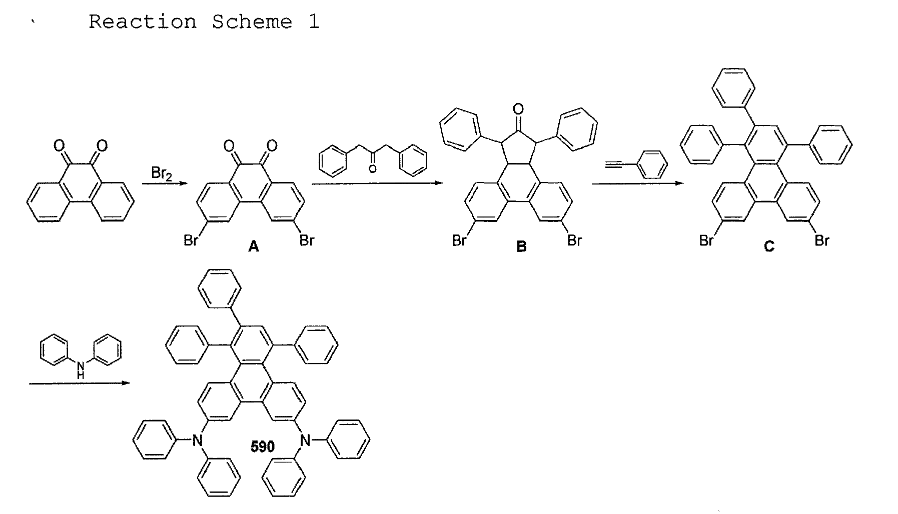

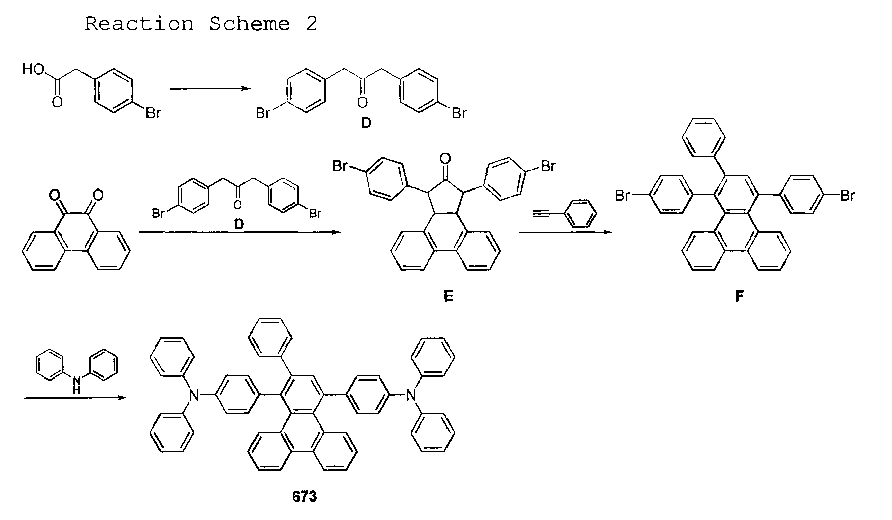

Further, the present invention provides organic solar cells, which comprise one or more organic electroluminescent compound(s) represented by Chemical Formula (1).

The present invention also provides an organic electroluminescent device which is comprised of a first electrode; a second electrode; and at least one organic layer(s) interposed between the first electrode and the second electrode; wherein the organic layer comprises one or more organic electroluminescent compound(s) represented by Chemical Formula (1). The organic electroluminescent compounds may be employed as dopant material for an electroluminescent layer, or material for a hole injecting layer.

The organic electroluminescent device according to the present invention is **characterized in that** the organic layer comprises an electroluminescent layer, which contains one or more host(s) in addition to one or more organic electroluminescent compound(s) represented by Chemical Formula (1) as electroluminescent dopant. The host to be applied to an organic electroluminescent device according to the present invention is not particularly restrictive, but preferably selected from the compounds represented by Chemical Formula (2) or (3):
Chemical Formula 2

   (Ar₁₁)_{b}-L₁-(Ar₁₂)_{c}
Chemical Formula 3

   (Ar₁₃)_{d}-L₂-(Ar₁₄)ₑ

   wherein, L₁ represents (C6-C60)arylene or (C4-C60)heteroarylene;
   L₂ represents anthracenylene;
   Ar₁₁ through Ar₁₄ are independently selected from hydrogen, deuterium, (C1-C60)alkyl, (C1-C60)alkoxy, halogen, (C4-C60)heteroaryl, (C5-C60)cycloalkyl or (C6-C60)aryl; the cycloalkyl, aryl or heteroaryl of Ar₁₁ through Ar₁₄ may be further substituted by one or more substituent(s) selected from a group consisting of (C6-C60)aryl or (C4-C60)heteroaryl with or without one or more substituent(s) selected from a group consisting of (C1-C60)alkyl with or without halogen substituent(s), (C1-C60)alkoxy, (C3-C60)cycloalkyl, halogen, cyano, tri(C1-C60)alkylsilyl, di(C1-C60)alkyl(C6-C60)arylsilyl and tri(C6-C60)arylsilyl; (C1-C60)alkyl with or without halogen substituent(s), (C1-C60)alkoxy, (C3-C60)cycloalkyl, halogen, cyano, tri(C1-C60)alkylsilyl, di(C1-C60)alkyl(C6-C60)arylsilyl and tri(C6-C60)arylsilyl, and
   b, c, d and e independently represent an integer from 0 to 4.

The host represented by Chemical Formulas (2) and (3) can be exemplified by anthracene derivatives or benz[a]anthracene derivatives represented by one of Chemical Formulas (4) to (7): 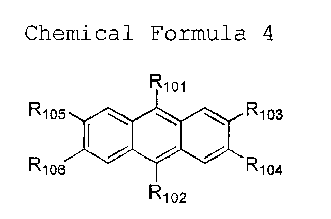 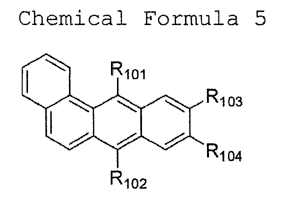 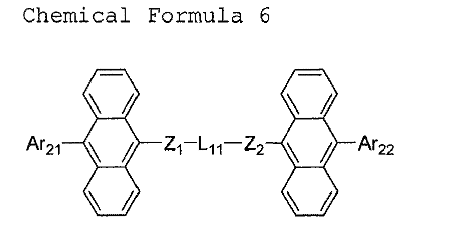 wherein, R₁₀₁ and R₁₀₂ independently represent hydrogen, deuterium, (C1-C60)alkyl, halogen, (C6-C60)aryl, (C3-C60)heteroaryl, 5- or 6-membered heterocycloalkyl containing one or more heteroatom(s) selected from N, O and S, or (C3-C60)cycloalkyl; the aryl or heteroaryl of R₁₀₁ and R₁₀₂ may be further substituted by one or more substituent(s) selected from a group consisting of (C1-C60)alkyl, halo(C1-C60)alkyl, (C1-C60)alkyloxy, (C3-C60)cycloalkyl, (C6-C60)aryl, (C4-C60)heteroaryl, halogen, cyano, tri(C1-C60)alkylsilyl, di(C1-C60)alkyl(C6-C60)arylsilyl and tri(C6-C60)arylsilyl;
R₁₀₃ through R₁₀₆ independently represent hydrogen, deuterium, (C1-C60)alkyl, (C1-C60)alkyloxy, halogen, (C4-C60)heteroaryl, (C5-C60)cycloalkyl or (C6-C60)aryl; the heteroaryl, cycloalkyl or aryl of R₁₀₃ through R₁₀₆ may be further substituted by one or more substituent(s) selected from a group consisting of (C1-C60)alkyl with or without halogen substituent(s), (C1-C60)alkyloxy, (C3-C60)cycloalkyl, halogen, cyano, tri(C1-C60)alkylsilyl, di(C1-C60)alkyl(C6-C60)arylsilyl and tri(C6-C60)arylsilyl;
Z₁ and Z₂ independently represent a chemical bond or (C6-C60)arylene with or without one or more substituent(s) selected from (C1-C60)alkyl, (C1-C60)alkyloxy, (C6-C60)aryl, (C4-C60)heteroaryl and halogen;
Ar₂₁ and Ar₂₂ independently represent aryl selected from the following structures, or (C4-C60)heteroaryl: 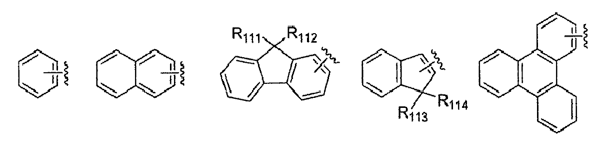 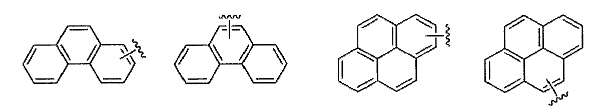 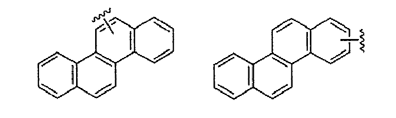 the aryl or heteroaryl of Ar₂₁ and Ar₂₂ may be further substituted by one or more substituent(s) selected from (C1-C60)alkyl, (C1-C60)alkoxy, (C6-C60)aryl and (C4-C60)heteroaryl;
L₁₁ represents (C6-C60)arylene, (C4-C60)heteroarylene or a group having the following structure: 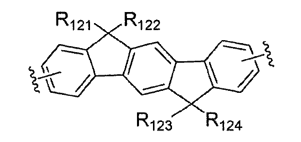 the arylene or heteroarylene of L₁₁ may be further substituted by one or more substituent(s) selected from (C1-C60)alkyl, (C1-C60)alkyloxy, (C6-C60)aryl, (C4-C60)heteroaryl and halogen;
R₁₁₁ through R₁₁₄ independently represent hydrogen, deuterium, (C1-C60)alkyl or (C6-C60)aryl, or each of them may be linked to an adjacent substituent via (C3-C60)alkylene or (C3-C60)alkenylene with or without a fused ring to form an alicyclic ring, or a monocyclic or polycyclic aromatic ring;
R₁₂₁ through R₁₂₄ independently represent hydrogen, deuterium, (C1-C60)alkyl, (C1-C60)alkyloxy, (C6-C60)aryl, (C4-C60)heteroaryl or halogen, or each of them may be linked to an adjacent substituent via (C3-C60)alkylene or (C3-C60)alkenylene with or without a fused ring to form an alicyclic ring, or a monocyclic or polycyclic aromatic ring; 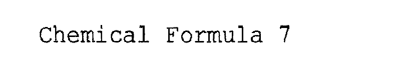 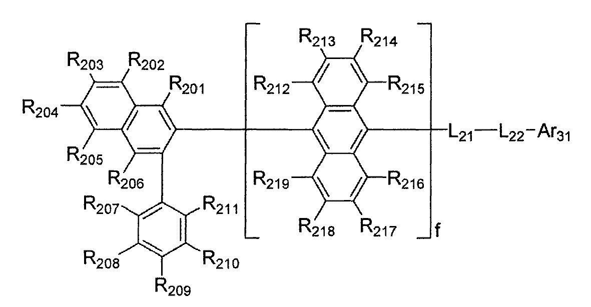 wherein, L₂₁ and L₂₂ independently represent a chemical bond, (C6-C60)arylene or (C3-C60)heteroarylene; the arylene or heteroarylene of L₂₁ and L₂₂ may be further substituted by one or more substituent(s) selected from (C1-C60)alkyl, halogen, cyano, (Cl-C60)alkoxy, (C3-C60)cycloalkyl, (C6-C60) aryl, (C3-C60)heteroaryl, tri(C1-C60)alkylsilyl, di(C1-C60)alkyl(C6-C60)arylsilyl or tri(C6-C60)arylsilyl; R₂₀₁ through R₂₁₉ independently represent hydrogen, deuterium, halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkyloxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of R₂₀₁ through R₂₁₉ may be linked to an adjacent substituent via (C3-C60)alkylene or (C3-C60)alkenylene with or without a fused ring to form an alicyclic ring, or a monocyclic or polycyclic aromatic ring;
Ar₃₁ represents (C6-C60)aryl, (C4-C60)heteroaryl, 5- or 6-membered heterocycloalkyl containing one or more heteroatom(s) selected from N, O and S, (C3-C60)cycloalkyl, adamantyl, (C7-C60)bicycloalkyl, or a substituent selected from the following structures: 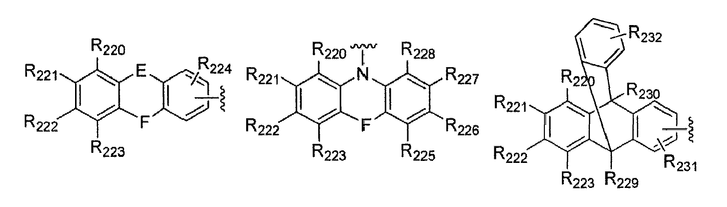 wherein, R₂₂₀ through R₂₃₂ independently represent hydrogen, deuterium, halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl;
E and F independently represent a chemical bond, - (CR₂₃₃R₂₃₄)_{g}-, -N(R₂₃₅)-, -S-, -O-, -Si(R₂₃₆) (R₂₃₇)-, -P(R₂₃₈)-, - C(=O)-, -B(R₂₃₉)-, -In(R₂₄₀)-, -Se-, -Ge(R₂₄₁)(R₂₄₂)-, -Sn(R₂₄₃)(R₂₄₄)-, -Ga(R₂₄₅)- or -(R₂₄₆)C=C(R₂₄₇)-;
R₂₃₃ through R₂₄₇ independently represent hydrogen, deuterium, halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkyloxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of R₂₃₃ through R₂₄₇ may be linked to an adjacent substituent via (C3-C60)alkylene or (C3-C60)alkenylene with or without a fused ring to form an alicyclic ring, or a monocyclic or polycyclic aromatic ring;
the aryl, heteroaryl, heterocycloalkyl, adamantyl or bicycloalkyl of Ar₃₁; the alkyl, alkenyl, alkynyl, cycloalkyl, heterocycloalkyl, aryl, heteroaryl, arylsilyl, alkylsilyl, alkylamino or arylamino of R₂₀₁ though R₂₃₂ may be further substituted by one or more substituent(s) selected from halogen, (C1-C60)alkyl, (C6-C60)aryl, (C4-C60)heteroaryl, 5-or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkyloxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl;
f is an integer from 1 to 4; and
g is an integer from 0 to 4.

The electroluminescent layer means the layer where electroluminescence occurs, and it may be a single layer or a multi-layer consisting of two or more layers laminated. When a mixture of host-dopant is used according to the constitution of the present invention, noticeable improvement in luminous efficiency by the electroluminescent host according to the invention could be confirmed. Those results can be achieved by doping concentration of 0.5 to 10% by weight. The host according to the present invention exhibits higher hole and electron conductivity, and excellent stability of the material as compared to other conventional host materials, and provides improved device life as well as luminous efficiency.

Thus, it can be described that use of the compound represented by one of Chemical Formulas (4) to (7) as electroluminescent host significantly supplements electronic drawback of the organic electroluminescent compounds of Chemical Formula (1) according to the present invention.

The host compounds represented by one of Chemical Formulas (4) to (7) can be exemplified by the following compounds, but are not restricted thereto.

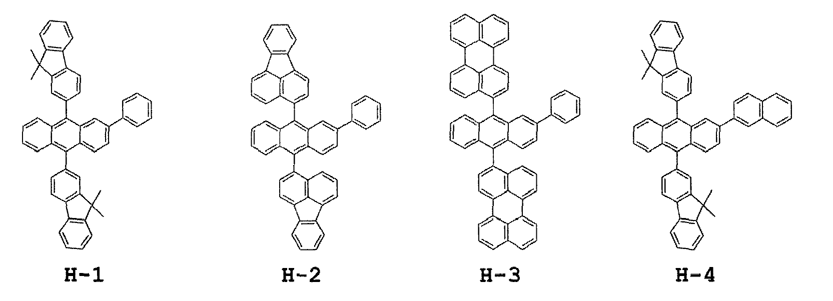

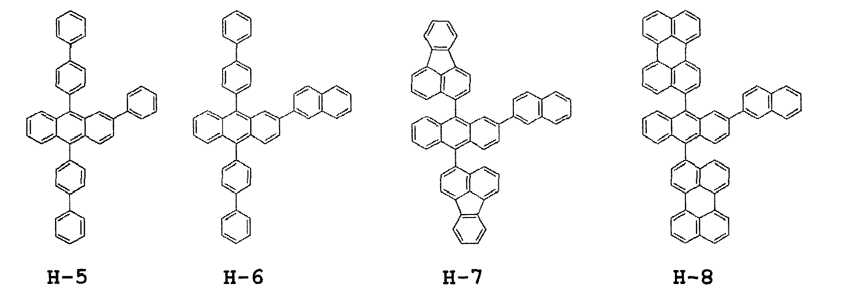

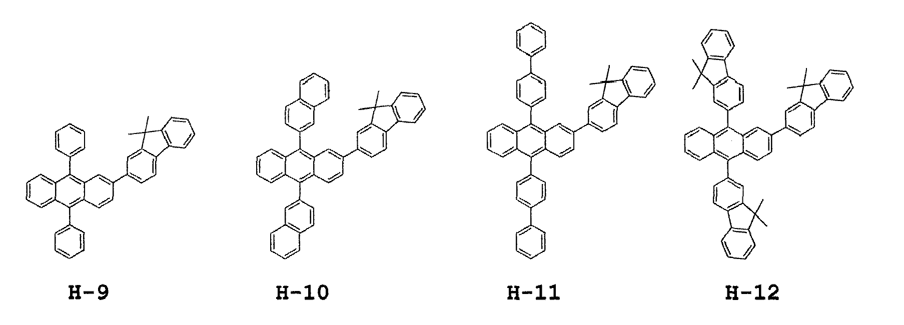

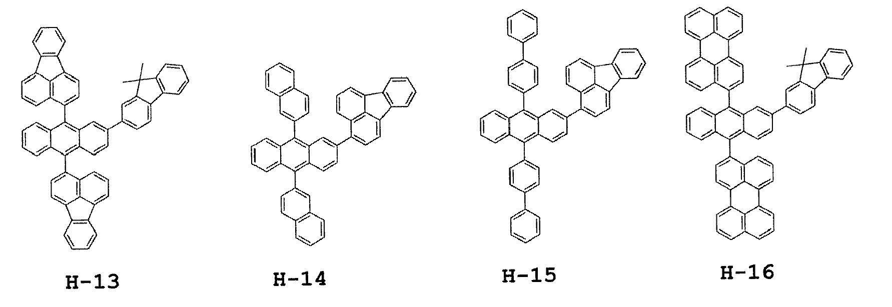

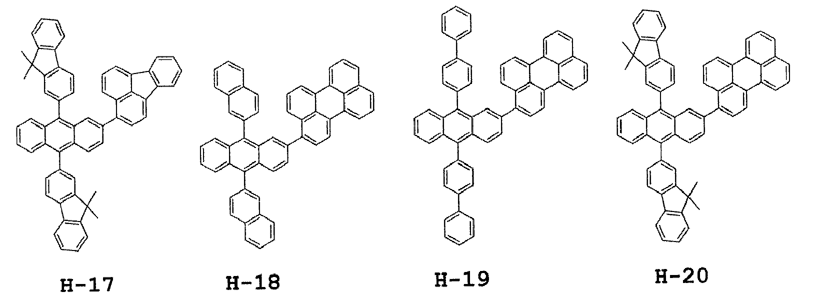

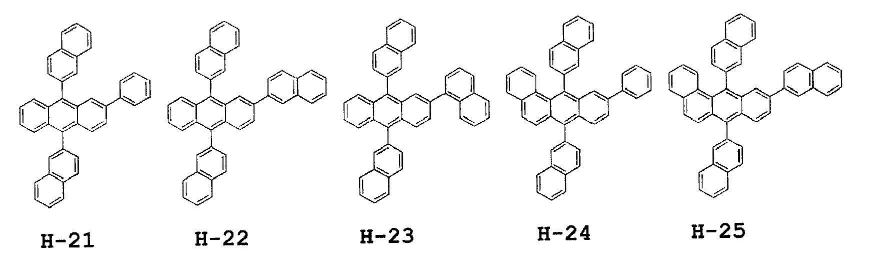

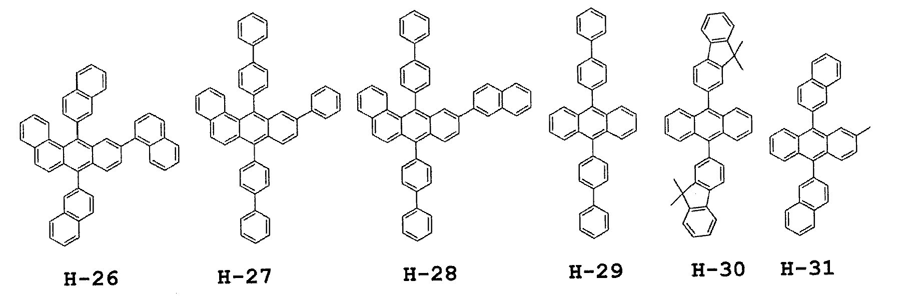

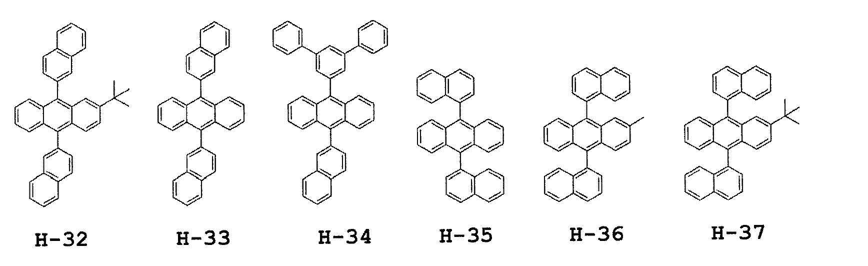

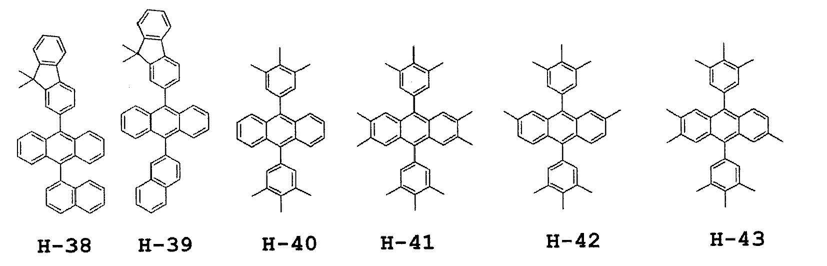

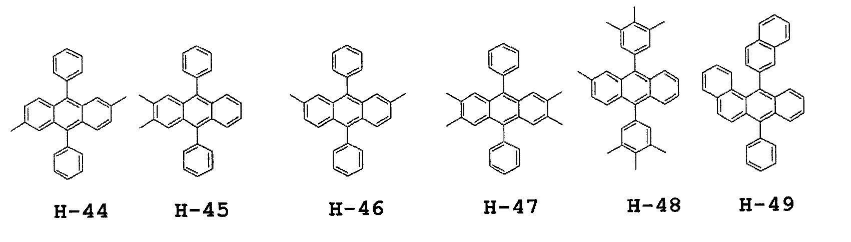

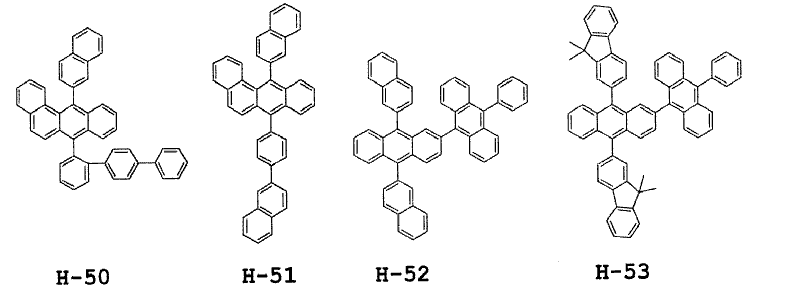

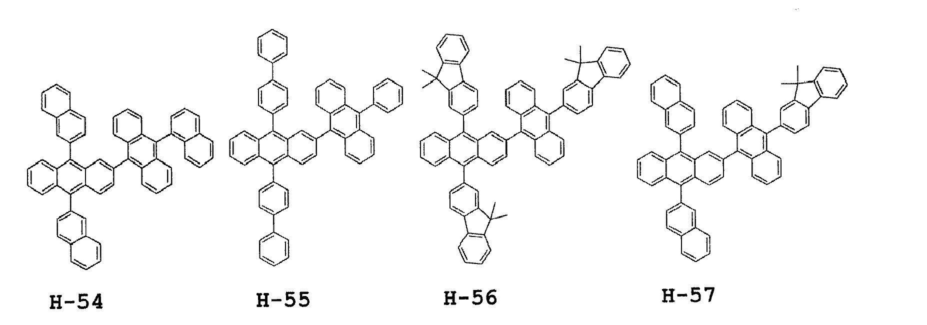

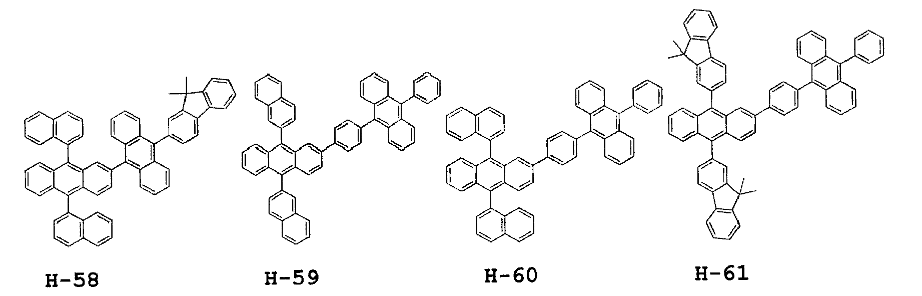

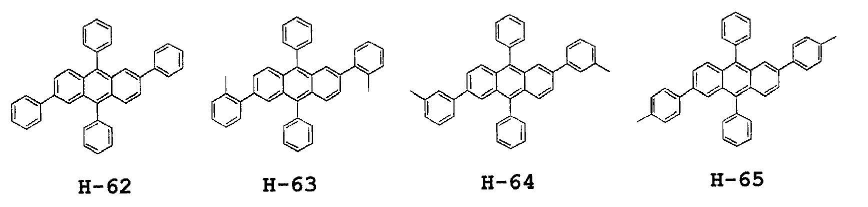

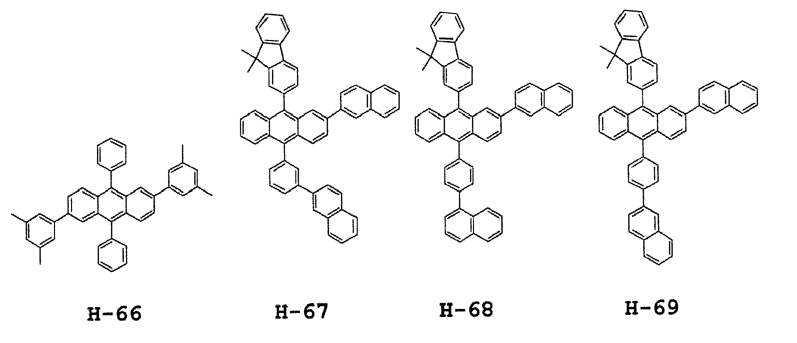

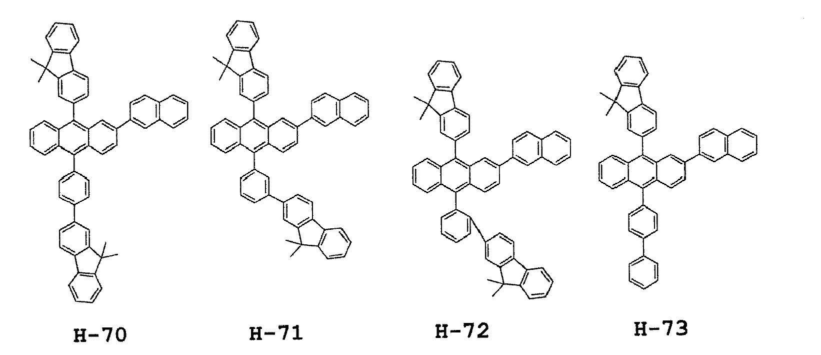

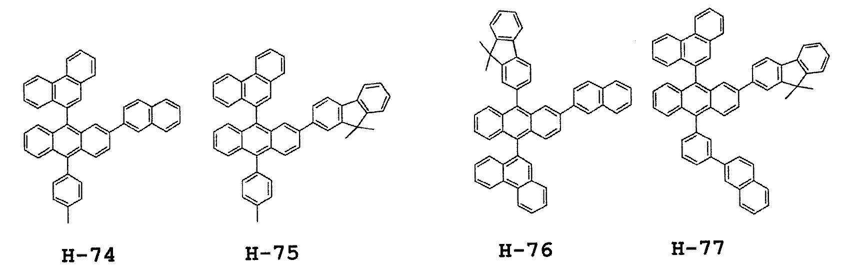

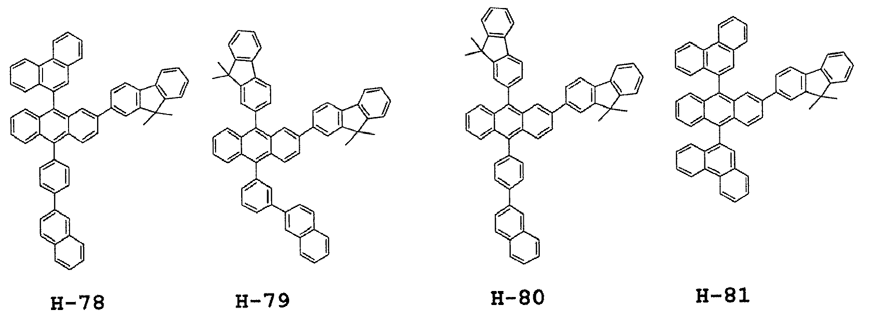

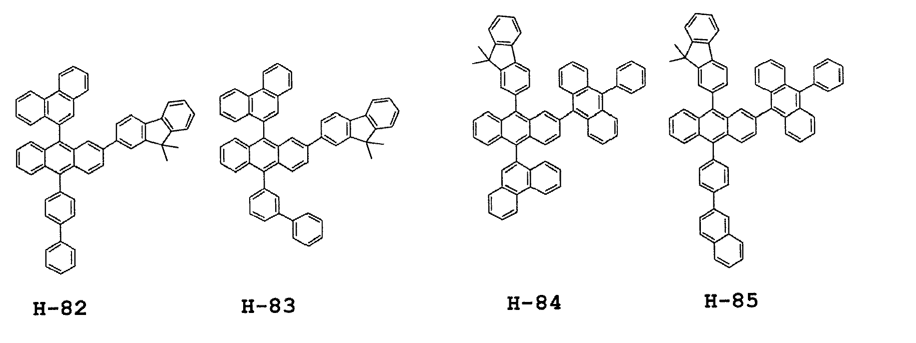

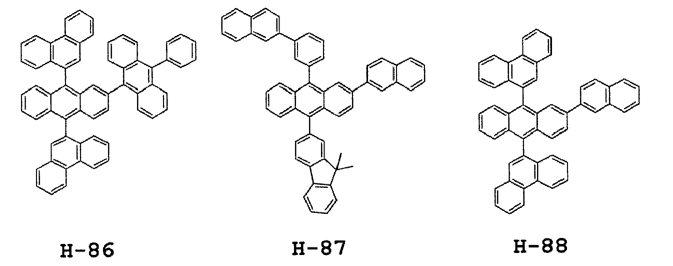

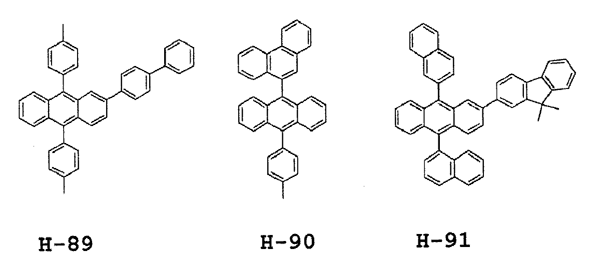

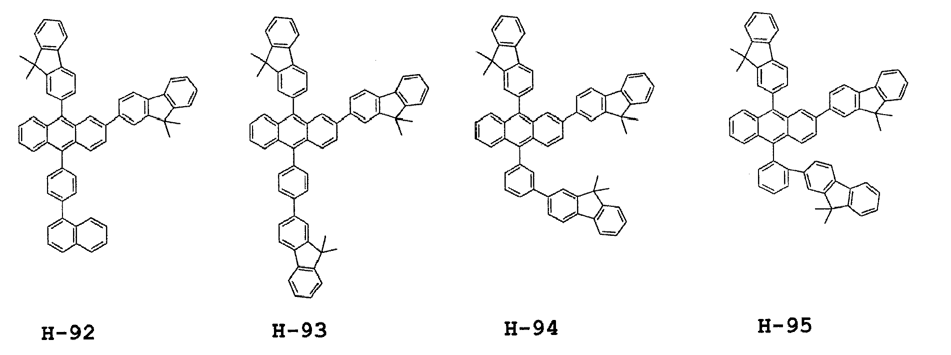

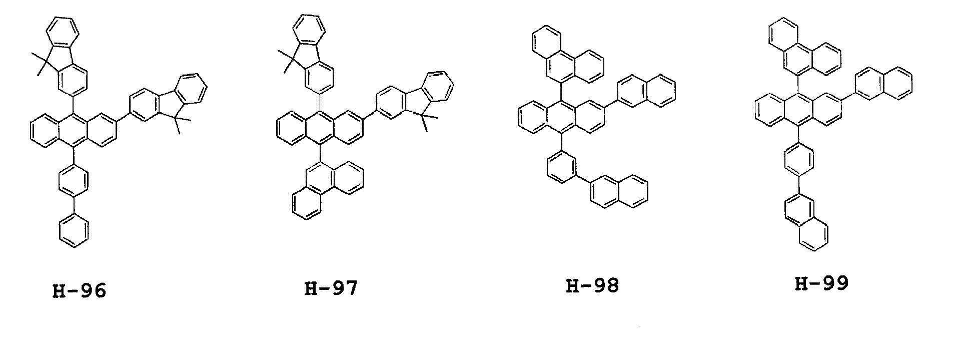

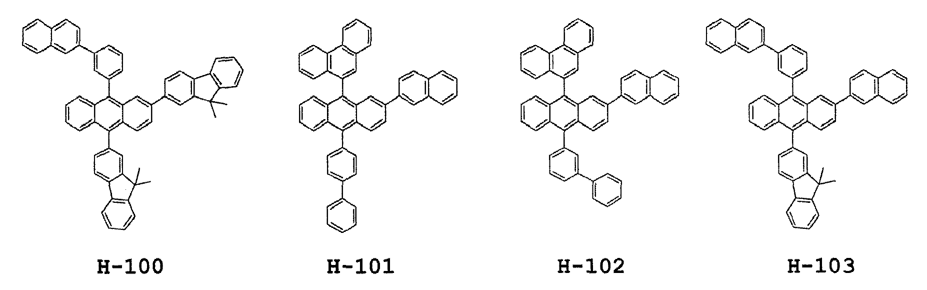

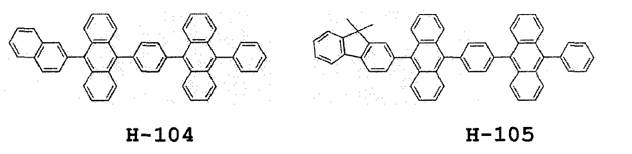

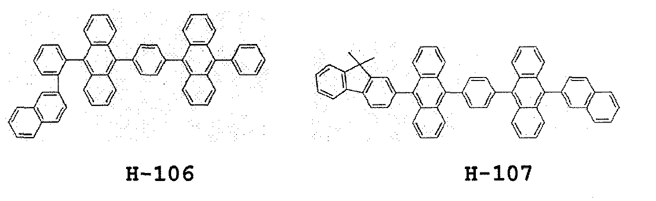

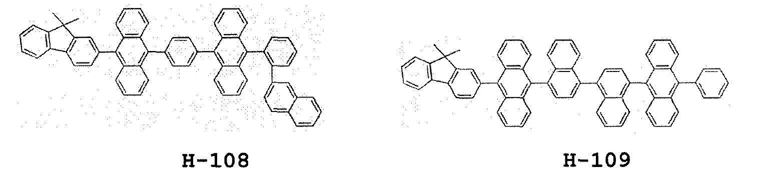

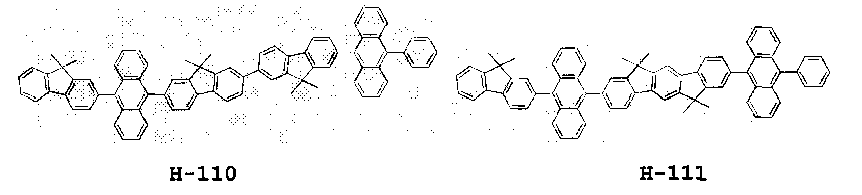

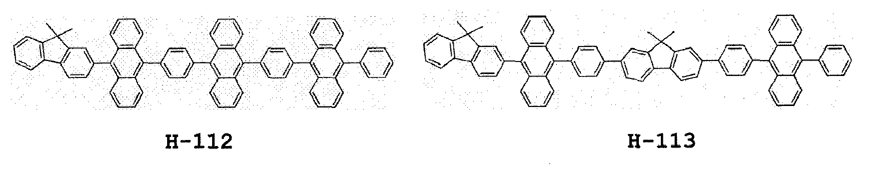

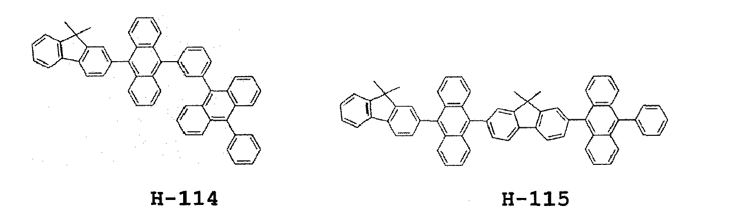

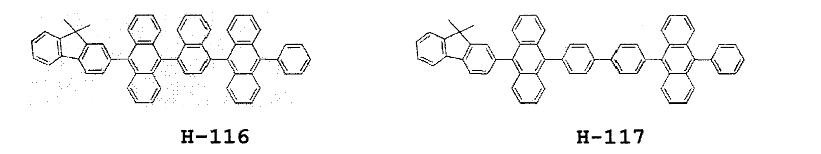

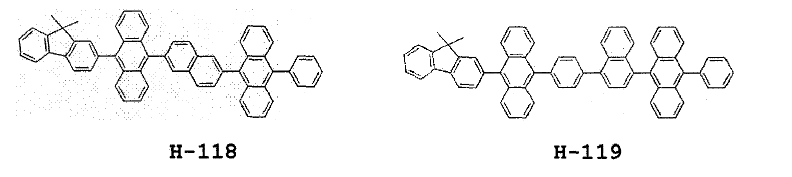

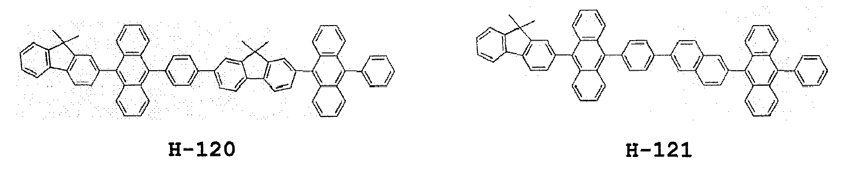

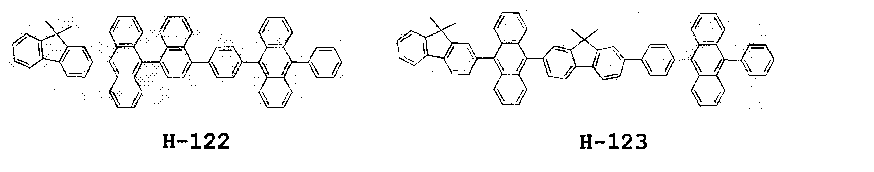

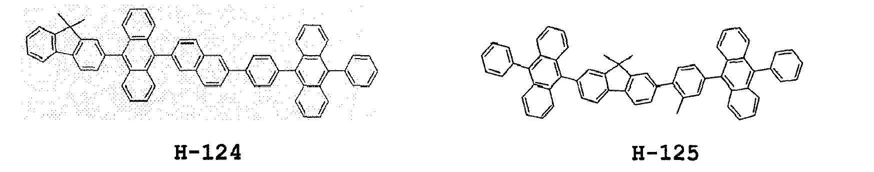

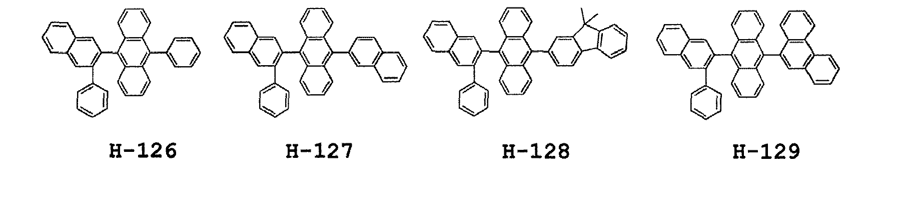

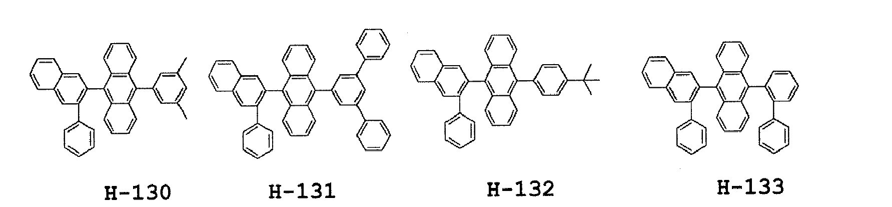

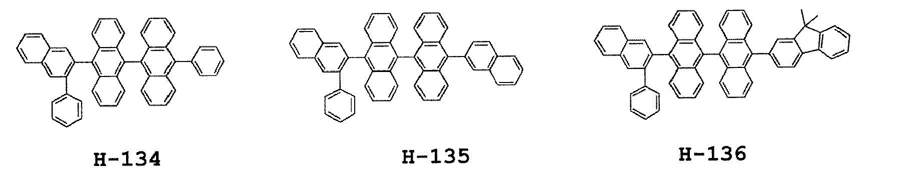

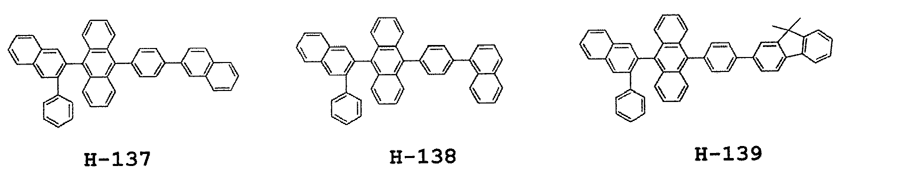

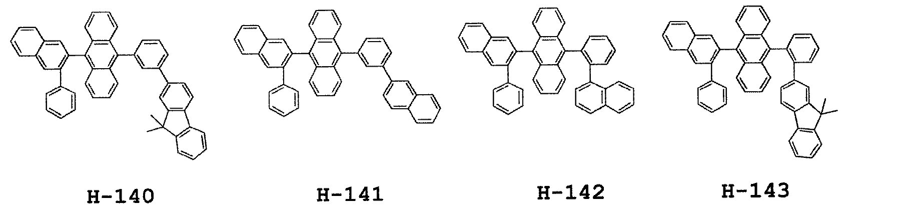

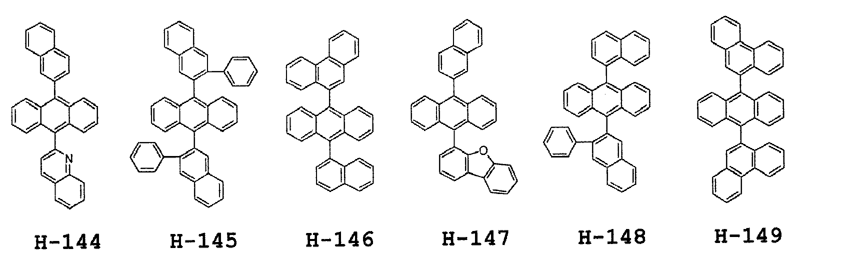

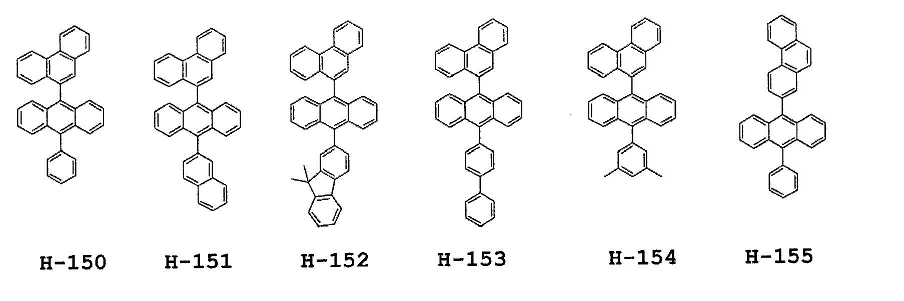

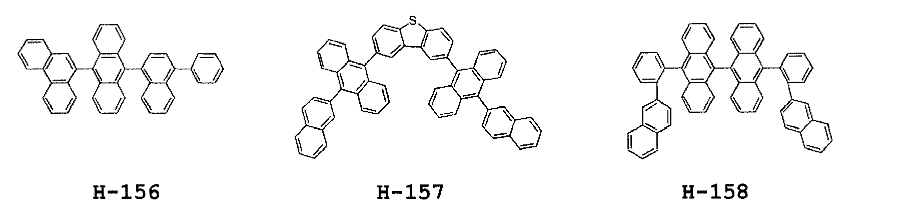

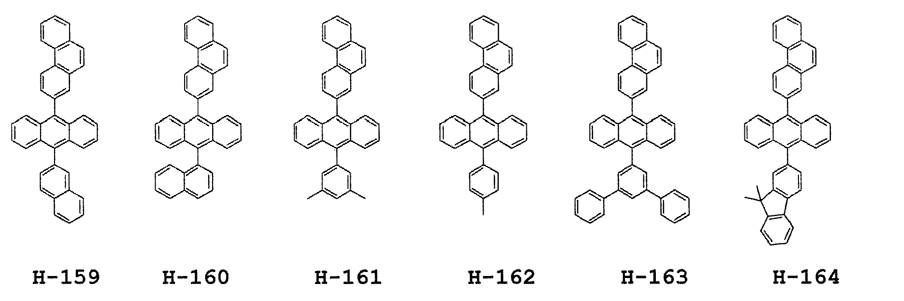

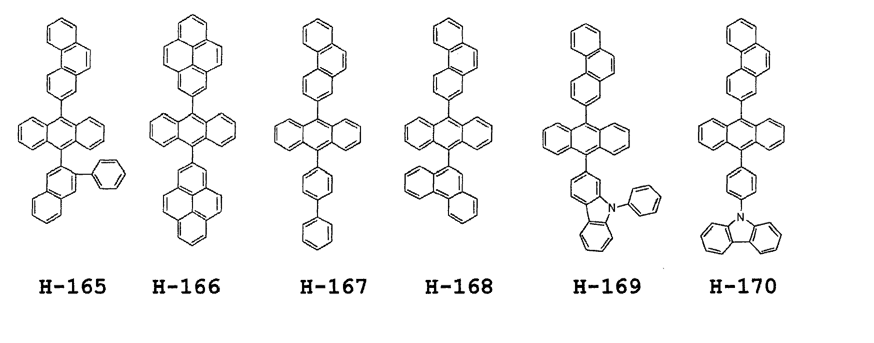

The organic electroluminescent device according to the present invention may further comprise one or more compound(s) selected from a group consisting of arylamine compounds and styrylarylamine compounds, in addition to the organic electroluminescent compound represented by Chemical Formula (1). Examples of the arylamine or styrylarylamine compounds include the compounds represented by Chemical Formula (8), but they are not restricted thereto: 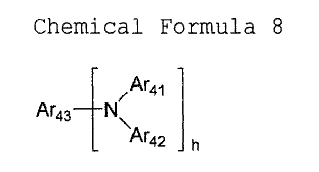 wherein, Ar₄₁ and Ar₄₂ independently represent (C1-C60)alkyl, (C6-C60)aryl, (C4-C60)heteroaryl, (C6-C60)arylamino, (C1-C60)alkylamino, 5- or 6-membered heterocycloalkyl containing one or more heteroatom(s) selected from N, O and S, or (C3-C60)cycloalkyl, or Ar₄₁ and Ar₄₂ may be linked via (C3-C60)alkylene or (C3-C60)alkenylene with or without a fused ring to form an alicyclic ring, or a monocyclic or polycyclic aromatic ring;
when h is 1, Ar₄₃ represents (C6-C60)aryl, (C4-C60)heteroaryl or aryl having one of the following structures: 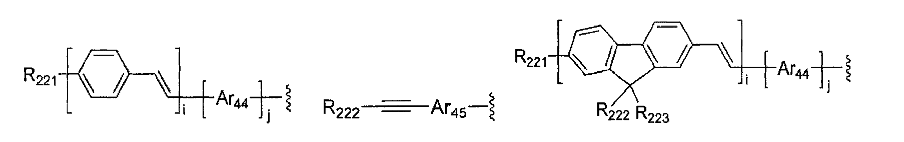 when h is 2, Ar₄₃ represents (C6-C60)arylene, (C4-C60)heteroarylene or arylene having one of the following structures: 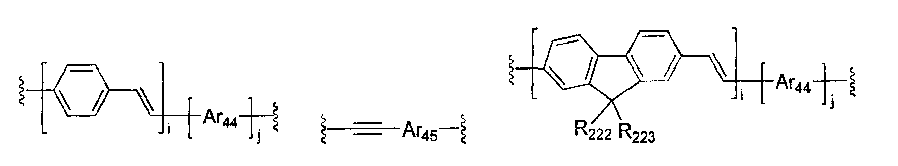 wherein, Ar₄₄ and Ar₄₅ independently represent (C6-C60)arylene or (C4-C60)heteroarylene;
R₂₂₁, R₂₂₂ and R₂₂₃ independently represent hydrogen, deuterium, (C1-C60)alkyl or (C6-C60)aryl;
i is an integer from 1 to 4; j is an integer of 0 or 1; and
the alkyl, aryl, heteroaryl, arylamino, alkylamino, cycloalkyl or heterocycloalkyl of Ar₄₁ and Ar₄₂; the aryl, heteroaryl, arylene or heteroarylene of Ar₄₃; the arylene or heteroarylene of Ar₄₄ and Ar₄₅; or the alkyl or aryl of R₂₂₁ through R₂₂₃ may be further substituted by one or more substituent(s) selected from a group consisting of halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C6-C60)aryloxy, (C1-C60)alkyloxy, (C6-C60)arylthio, (C1-C60)alkylthio, (C1-C60)alkoxycarbonyl, (C1-C60)alkylcarbonyl, (C6-C60)arylcarbonyl, carboxyl, nitro and hydroxyl.

The arylamine compounds or styrylarylamine compounds can be more specifically exemplified by the following compounds, but they are not restricted thereto.

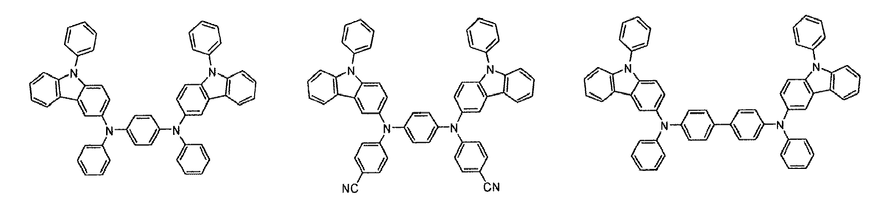

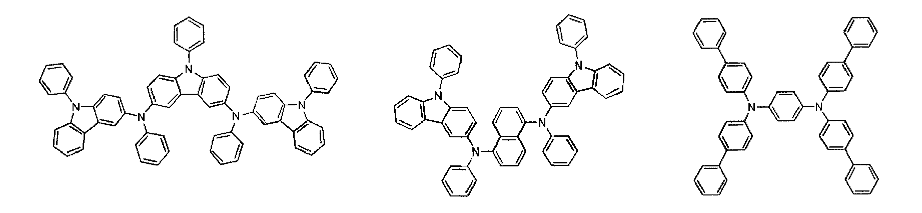

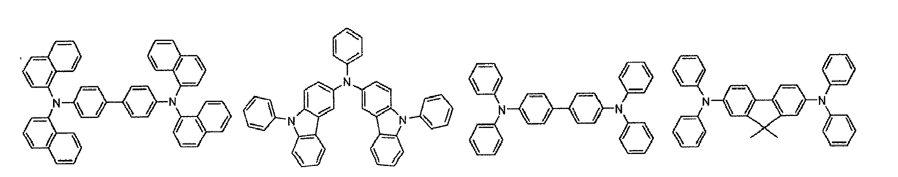

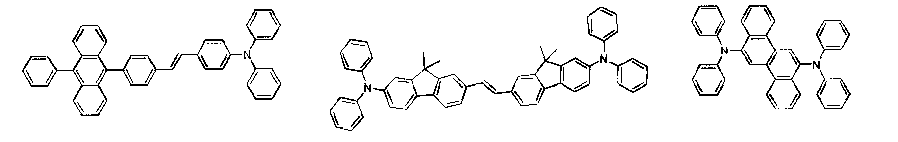

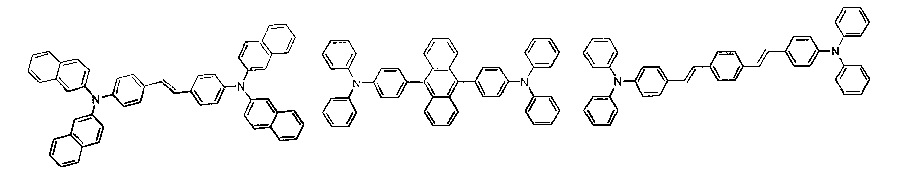

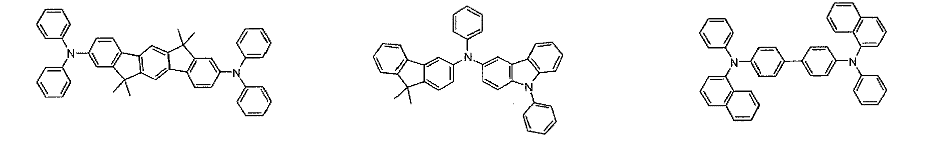

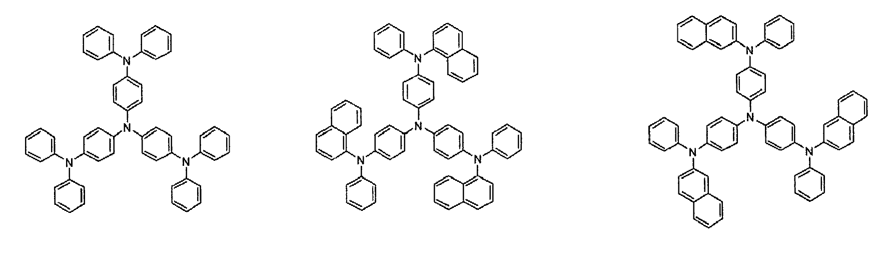

In an organic electroluminescent device according to the present invention, the organic layer may further comprise one or more metal(s) selected from a group consisting of organometals of Group 1, Group 2, 4^{th} period and 5^{th} period transition metals, lanthanide metals and d-transition elements in the Periodic Table of Elements, in addition to the compound for electronic material represented by Chemical Formula (1). The organic layer may comprise a charge generating layer in addition to an electroluminescent layer at the same time.

The present invention can realize an organic electroluminescent device having a pixel structure of independent light-emitting mode, which comprises an organic electroluminescent device containing the organic electroluminescent compound represented by Chemical Formula (1) as a sub-pixel, and one or more sub-pixel(s) comprising one or more metallic compound(s) selected from a group consisting of Ir, Pt, Pd, Rh, Re, Os, Tl, Pb, Bi, In, Sn, Sb, Te, Au and Ag, patterned in parallel at the same time.

Further, the organic layer (particularly, the electroluminescent layer) of the organic electroluminescent device may comprise, in addition to the organic electroluminescent compound according to the invention, one or more compound(s) having the electroluminescent peak of wavelength of not less than 560 nm, at the same time, to form a white electroluminescent device. Those compounds can be exemplified by the compounds represented by one of Chemical Formulas (9) to (13), without restriction.

Chemical Formula 9 **M¹L¹⁰¹L¹⁰²L¹⁰³**

In Chemical Formula (9), M¹ is selected from metals of Group 7, 8, 9, 10, 11, 13, 14, 15 and 16 in the Periodic Table of Elements, and ligands L¹⁰¹, L¹⁰² and L¹⁰³ are independently selected from the following structures: 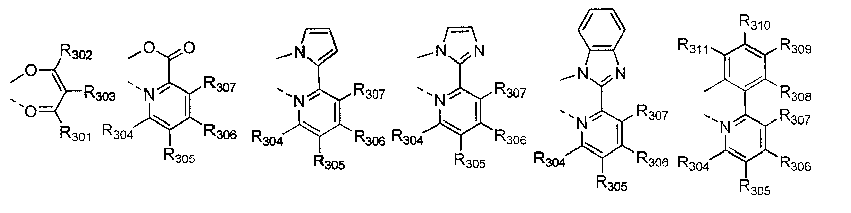 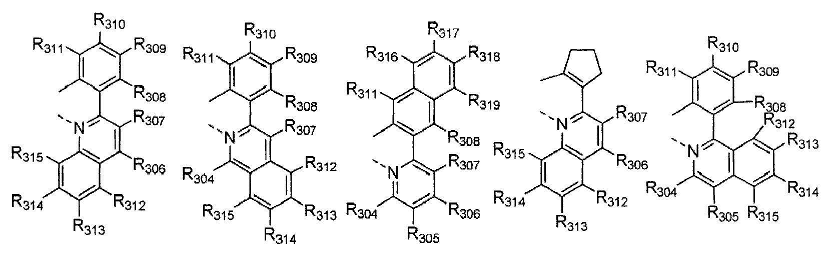 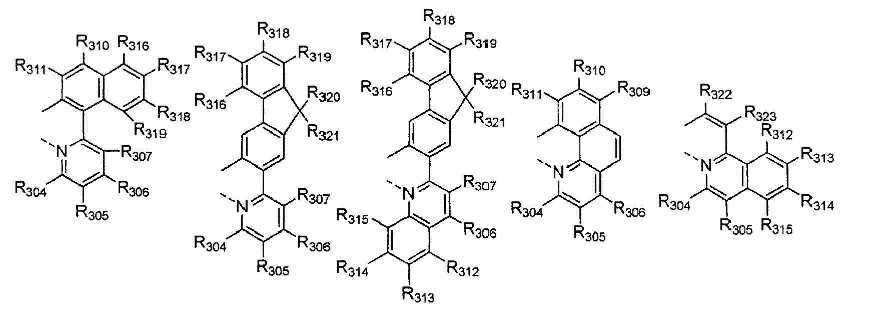 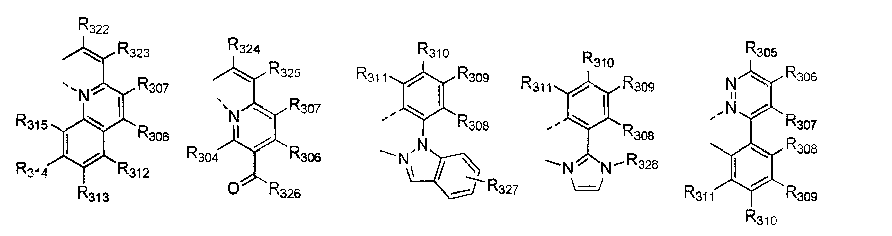 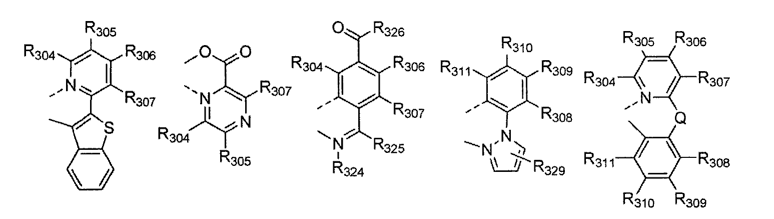 wherein, R₃₀₁ through R₃₀₃ independently represent hydrogen, deuterium, (C1-C60)alkyl with or without halogen substituent(s), (C6-C60)aryl with or without (C1-C60)alkyl substituent(s), or halogen;
R₃₀₄ through R₃₁₉ independently represent hydrogen, deuterium, (C1-C60)alkyl, (C1-C30)alkoxy, (C3-C60)cycloalkyl, (C2-C30)alkenyl, (C6-C60)aryl, mono or di(C1-C30)alkylamino, mono or di(C6-30)arylamino, SF₅, tri(C1-C30)alkylsilyl, di(C1-C30)alkyl(C6-C30)arylsilyl, tri(C6-C30)arylsilyl, cyano or halogen; the alkyl, cycloalkyl, alkenyl or aryl of R₃₀₄ through R₃₁₉ may be further substituted by one or more substituent(s) selected from (C1-C60)alkyl, (C6-C60)aryl and halogen;
R₃₂₀ through R₃₂₃ independently represent hydrogen, deuterium, (C1-C60)alkyl with or without halogen substituent(s), (C6-C60)aryl with or without (C1-C60)alkyl substituent(s);
R₃₂₄ and R₃₂₅ independently represent hydrogen, deuterium, (C1-C60)alkyl, (C6-C60) aryl or halogen, or R₃₂₄ and R₃₂₅ may be linked via (C3-C12)alkylene or (C3-C12)alkenylene with or without a fused ring to form an alicyclic ring, or a monocyclic or polycyclic aromatic ring; the alkyl or aryl of R₃₂₄ and R₃₂₅, or the alicyclic ring, or the monocyclic or polycyclic aromatic ring formed therefrom via (C3-C12)alkylene or (C3-C12)alkenylene with or without a fused ring may be further substituted by one or more substituent(s) selected from (C1-C60)alkyl with or without halogen substituent(s), (C1-C30)alkoxy, halogen, tri(C1-C30)alkylsilyl, tri(C6-C30)arylsilyl and (C6-C60)aryl;
R₃₂₆ represents (C1-C60)alkyl, (C6-C60)aryl, (C5-C60)heteroaryl or halogen;
R₃₂₇ through R₃₂₉ independently represent hydrogen, deuterium, (C1-C60)alkyl, (C6-C60)aryl or halogen; the alkyl or aryl of R₃₂₆ through R₃₂₉ may be further substituted by halogen or (C1-C60)alkyl;
Q represents 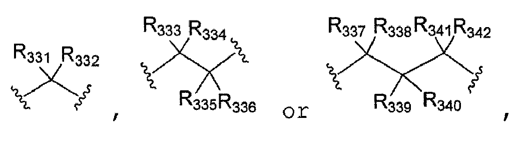 and R₃₃₁ through R₃₄₂ independently represent hydrogen, deuterium, (C1-C60)alkyl with or without halogen substituent(s), (C1-C30)alkoxy, halogen, (C6-C60)aryl, cyano or (C5-C60)cycloalkyl, or each of R₃₃₁ through R₃₄₂ may be linked to an adjacent substituent via alkylene or alkenylene to form a (C5-C7) spiro-ring or a (C5-C9) fused ring, or each of them may be linked to R₃₀₇ or R₃₀₈ via alkylene or alkenylene to form a (C5-C7) fused ring.

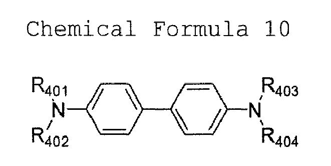

In Chemical Formula (10), R₄₀₁ through R₄₀₄ independently represent (C1-C60)alkyl or (C6-C60)aryl, or each of them may be linked to an adjacent substituent via (C3-C60)alkylene or (C3-C60)alkenylene with or without a fused ring to form an alicyclic ring, or a monocyclic or polycyclic aromatic ring; and the alkyl or aryl of R₄₀₁ through R₄₀₄, or the alicyclic ring, or the monocyclic or polycyclic aromatic ring formed therefrom by linkage via (C3-C60)alkylene or (C3-C60)alkenylene with or without a fused ring may be further substituted by one or more substituent(s) selected from (C1-C60)alkyl with or without halogen substituent(s), (C1-C60)alkoxy, halogen, tri(C1-C60)alkylsilyl, tri(C6-C60)arylsilyl and (C6-C60)aryl.

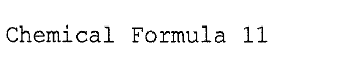

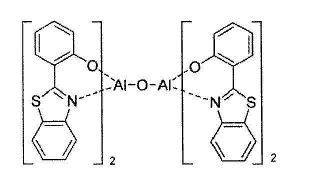

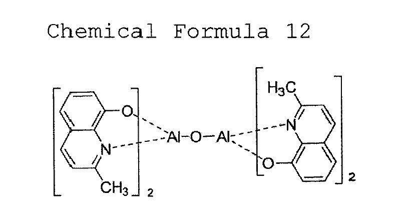

Chemical Formula 13 **L²⁰¹L²⁰²M²(T)ₖ**

In Chemical Formula (13), the ligands, L²⁰¹ and L²⁰² are independently selected from the following structures: 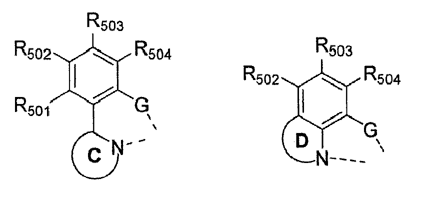 M² is a bivalent or trivalent metal;
k is 0 when M² is a bivalent metal, while k is 1 when M² is a trivalent metal;
T represents (C6-C60)aryloxy or tri(C6-C60)arylsilyl, and the aryloxy and triarylsilyl of T may be further substituted by (C1-C60)alkyl or (C6-C60)aryl;
G represents O, S or Se;
ring C represents oxazole, thiazole, imidazole, oxadiazole, thiadiazole, benzoxazole, benzothiazole, benzimidazole, pyridine or quinoline;
ring D represents pyridine or quinoline, and ring D may be further substituted by (C1-C60)alkyl, or phenyl or naphthyl with or without (C1-C60)alkyl substituent(s);
R₅₀₁ through R₅₀₄ independently represent hydrogen, deuterium, (C1-C60)alkyl, halogen, tri(C1-C60)alkylsilyl, tri(C6-C60)arylsilyl or (C6-C60)aryl, or each of them may be linked to an adjacent substituent via (C3-C60)alkylene or (C3-C60)alkenylene to form a fused ring; the pyridine or quinoline may form a chemical bond with R₅₀₁ to provide a fused ring; and
ring C or the aryl group of R₅₀₁ through R₅₀₄ may be further substituted by (C1-C60)alkyl, halogen, (C1-C60)alkyl with halogen substituent(s), phenyl, naphthyl, tri(C1-C60)alkylsilyl, tri(C6-C60)arylsilyl or amino group.

The compounds having the electroluminescent peak of wavelength of not less than 560 nm, in the electroluminescent layer, can be exemplified by the following compounds, but they are not restricted thereto.

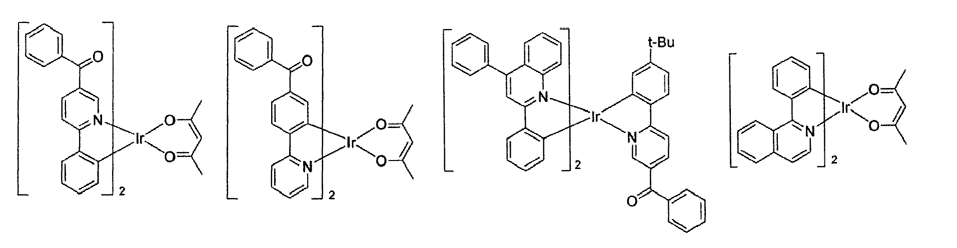

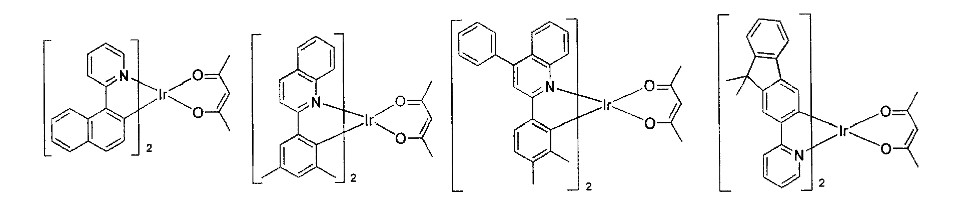

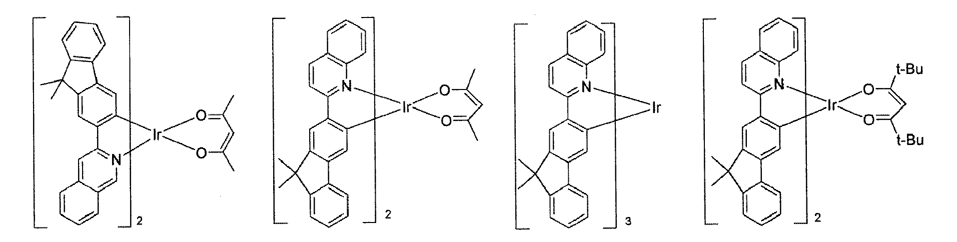

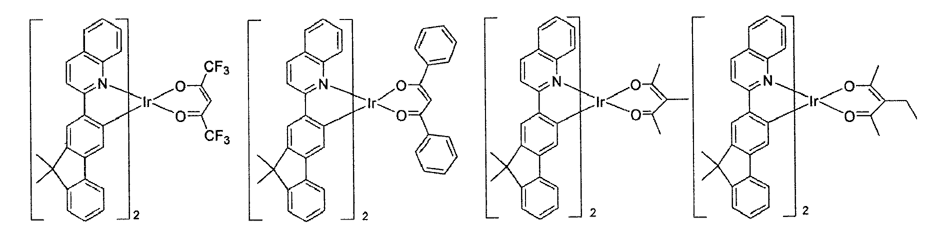

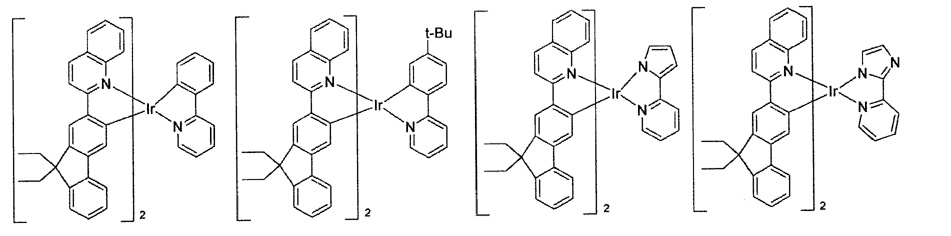

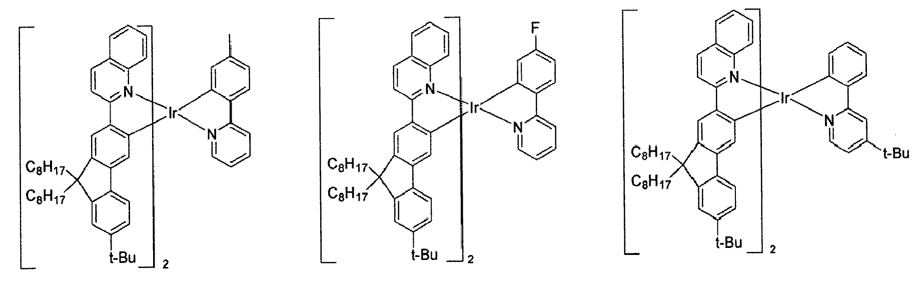

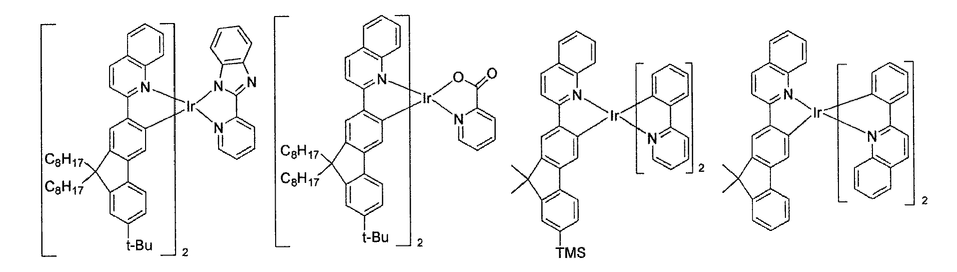

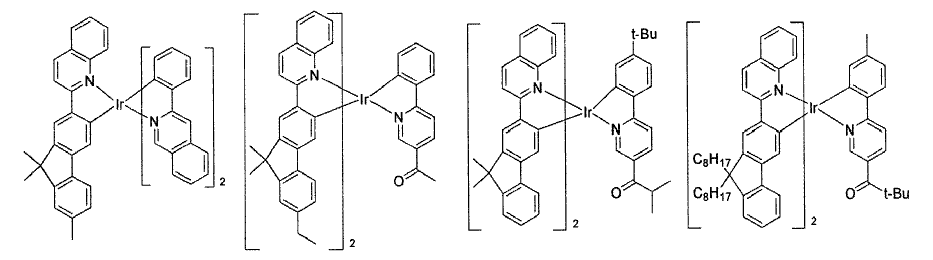

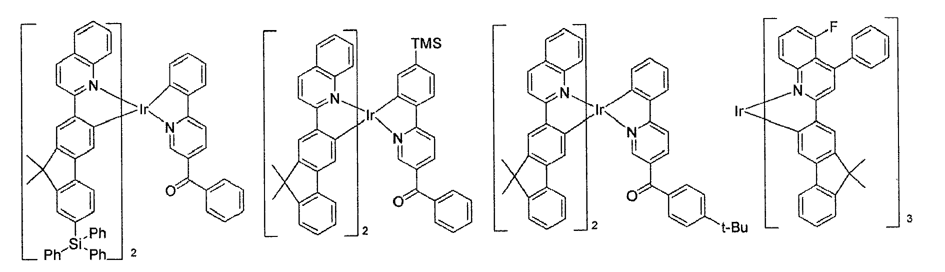

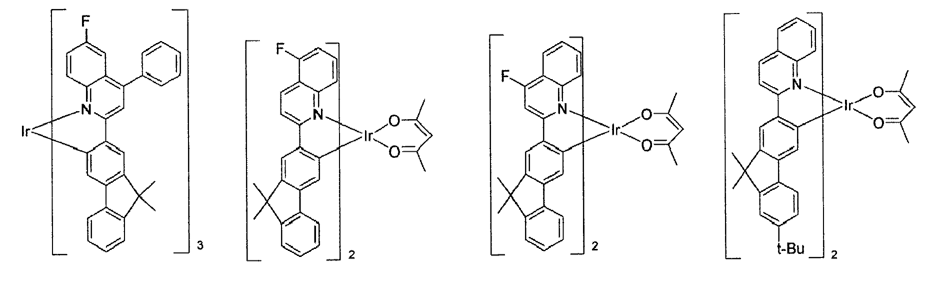

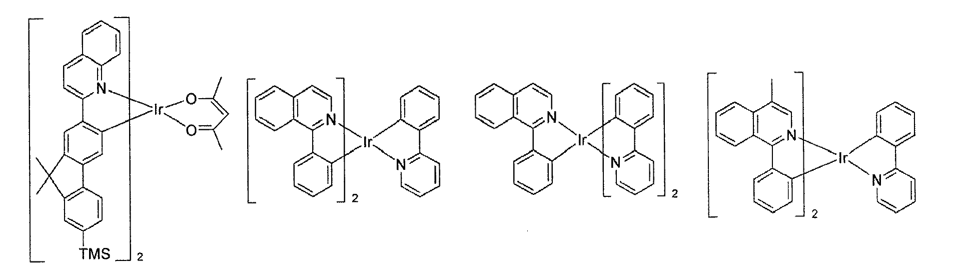

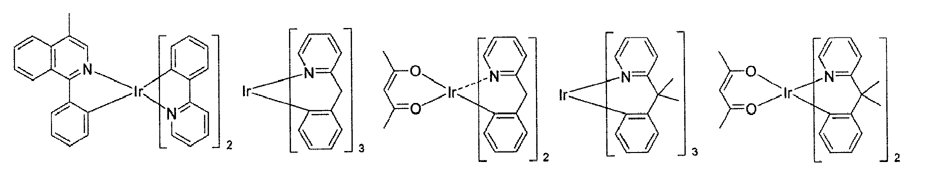

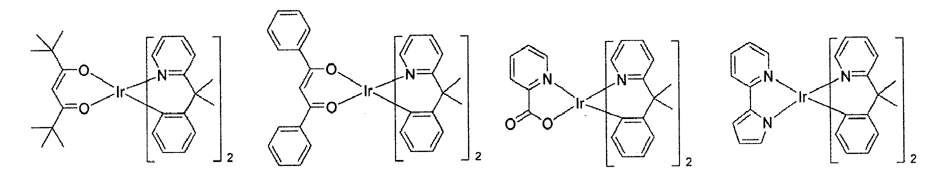

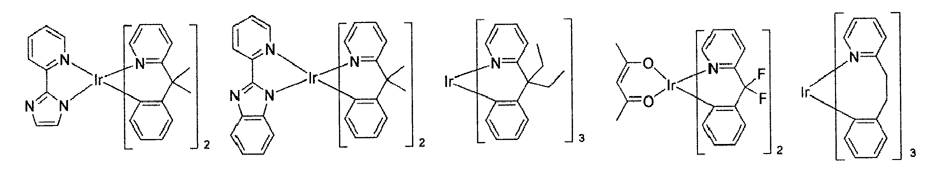

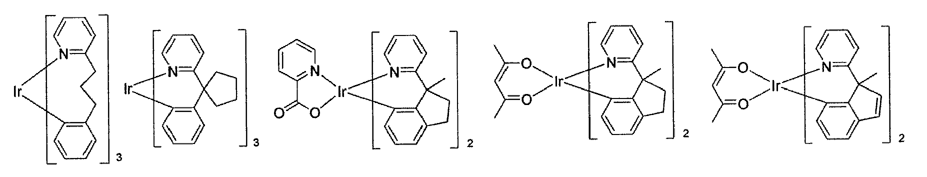

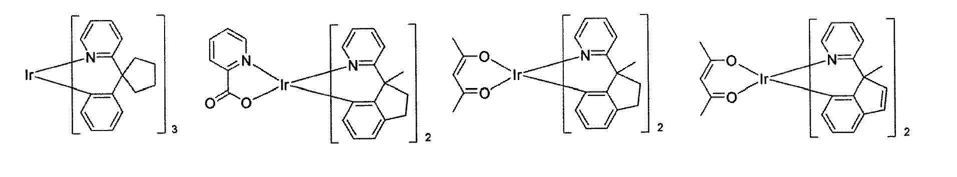

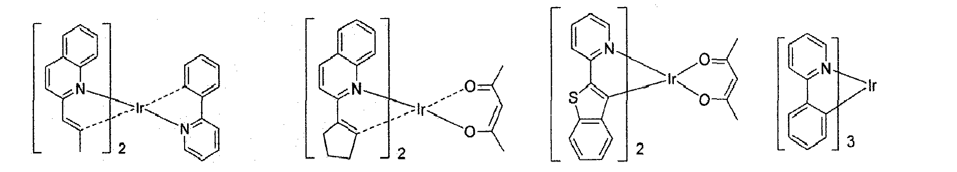

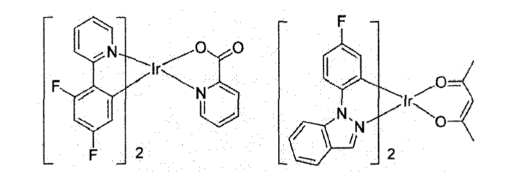

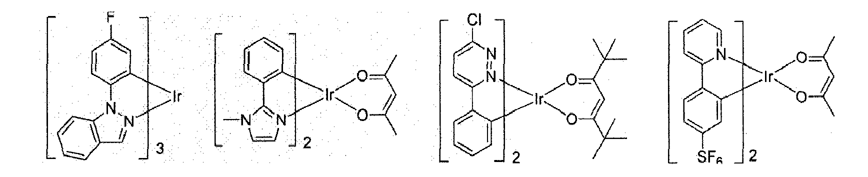

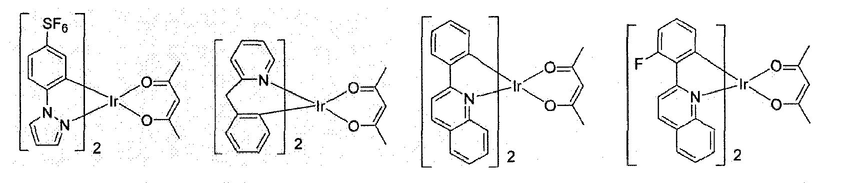

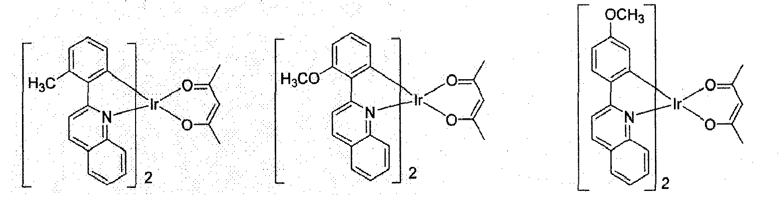

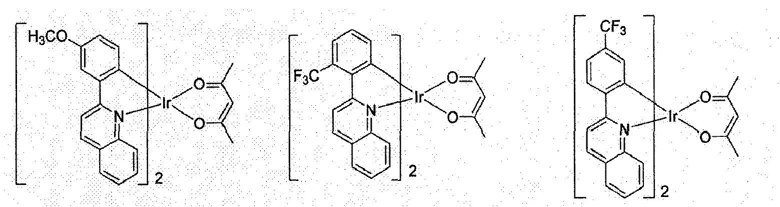

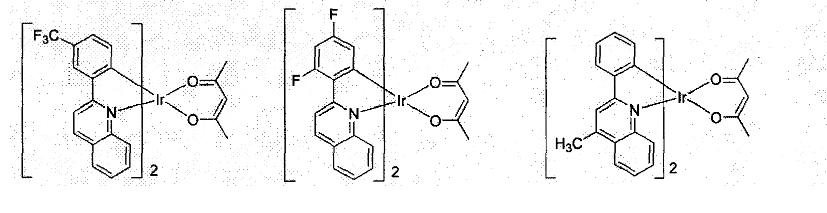

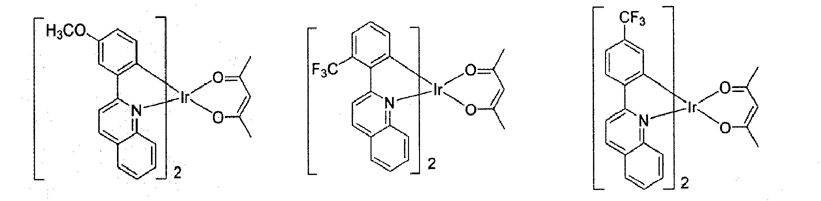

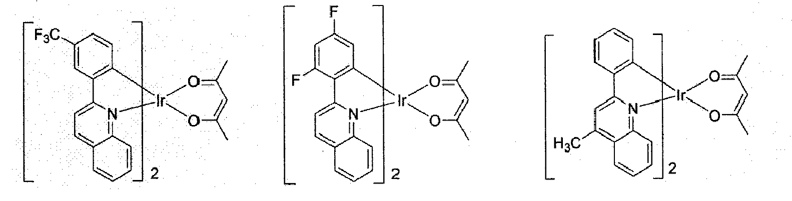

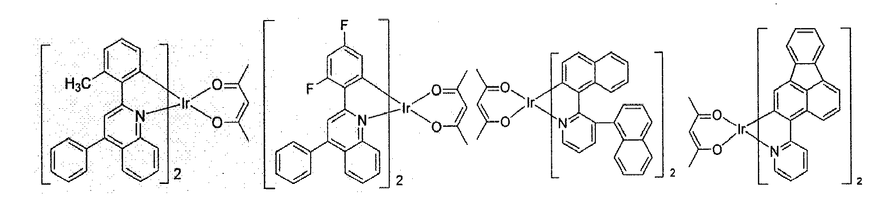

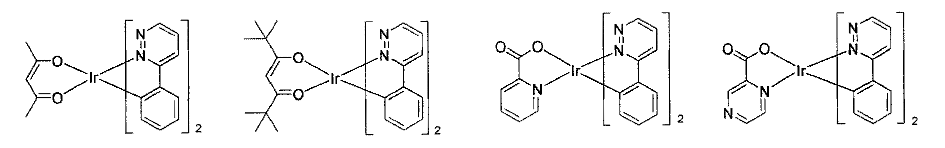

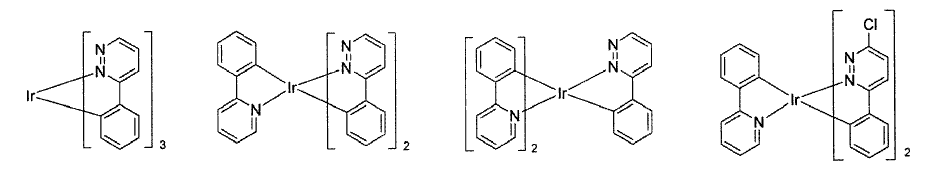

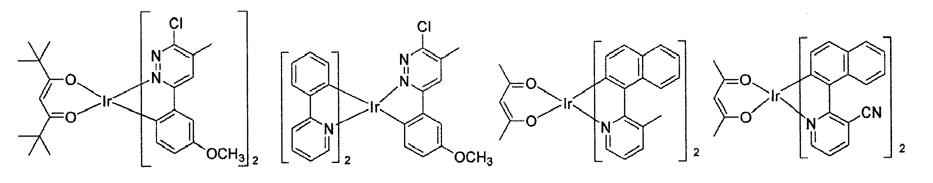

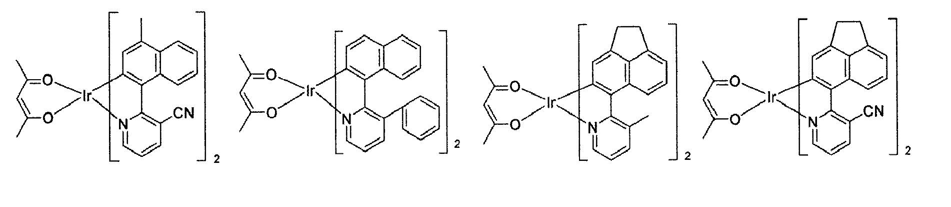

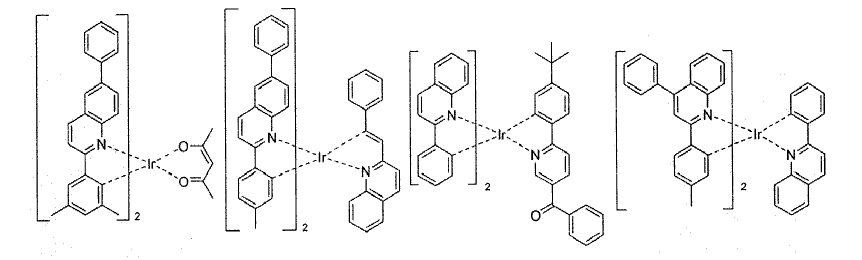

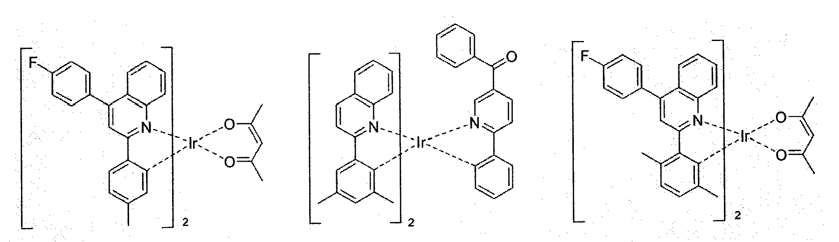

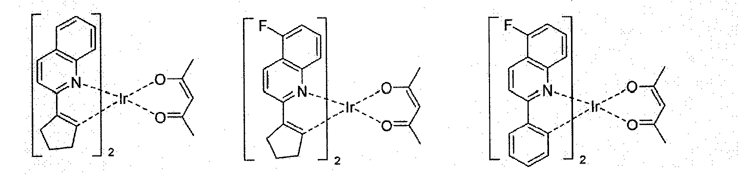

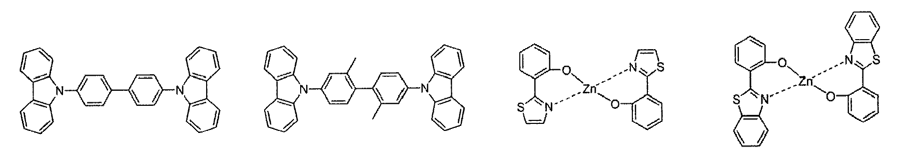

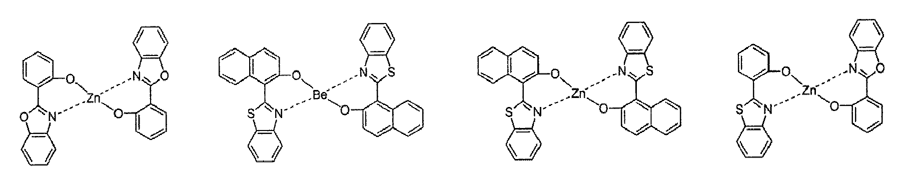

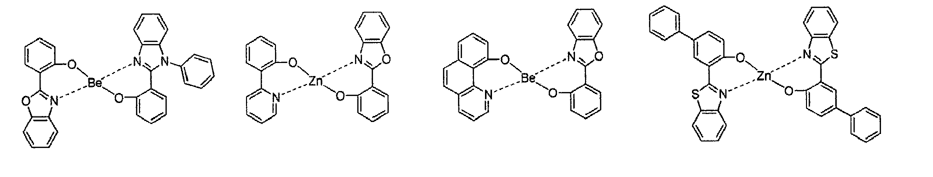

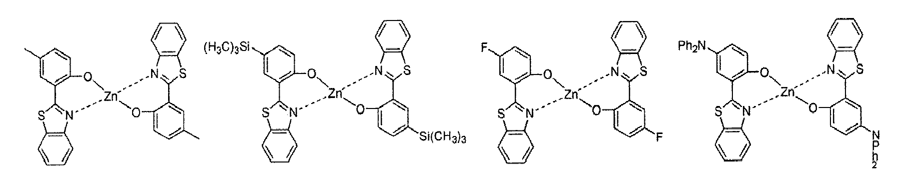

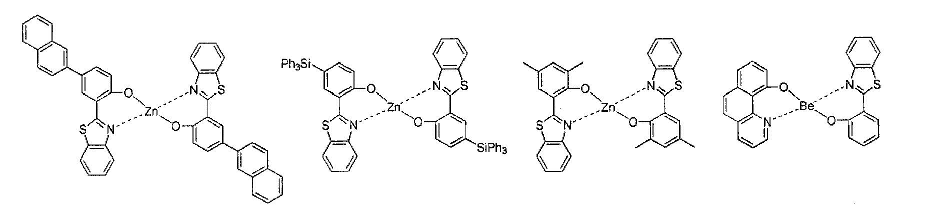

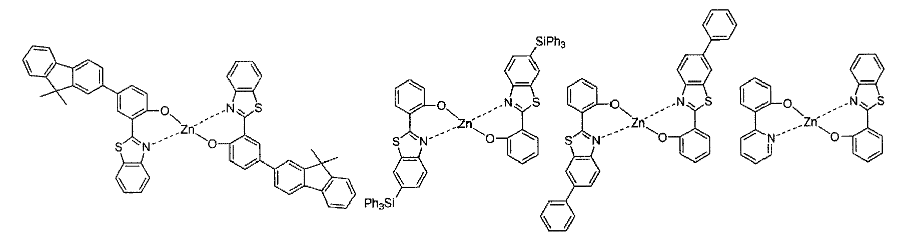

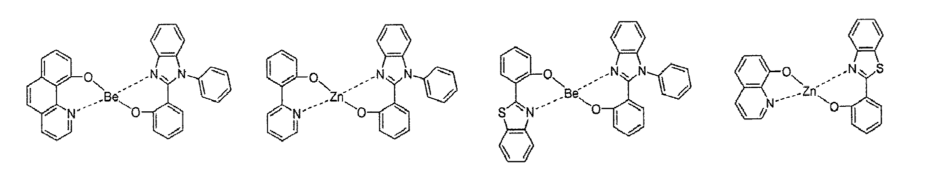

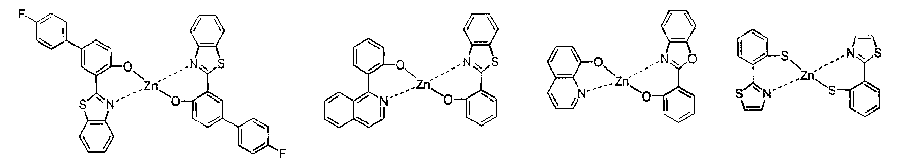

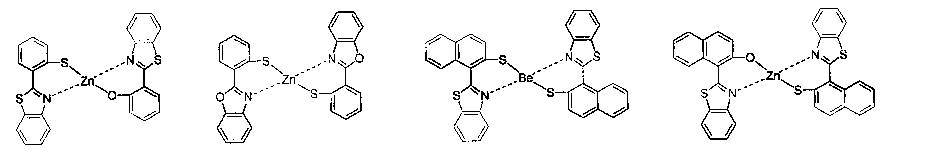

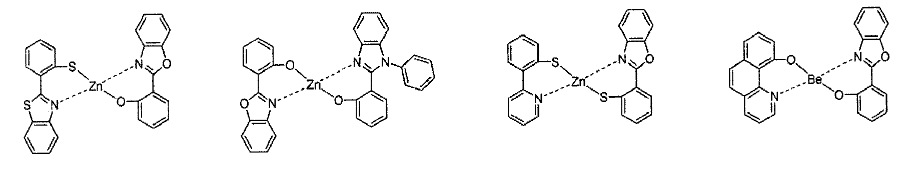

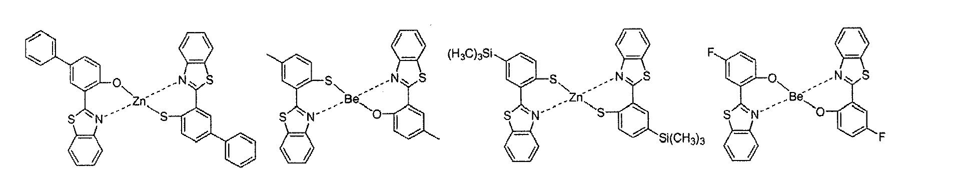

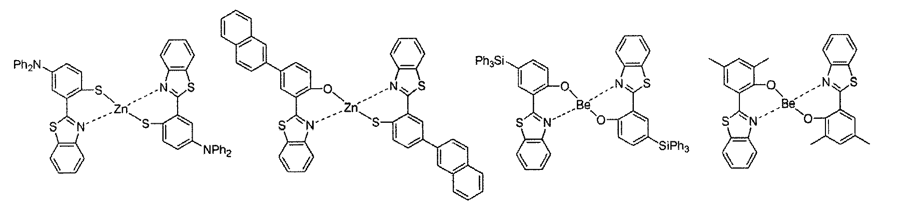

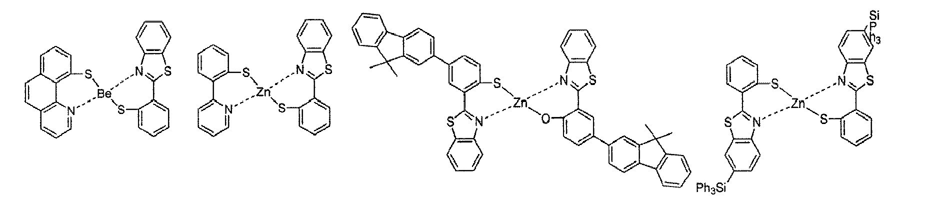

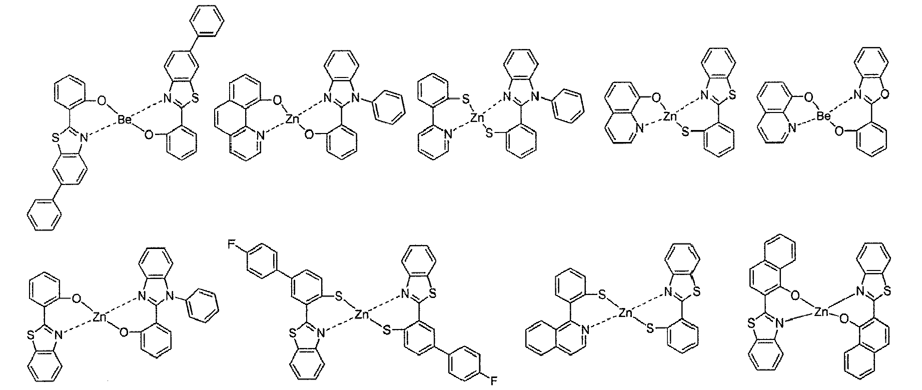

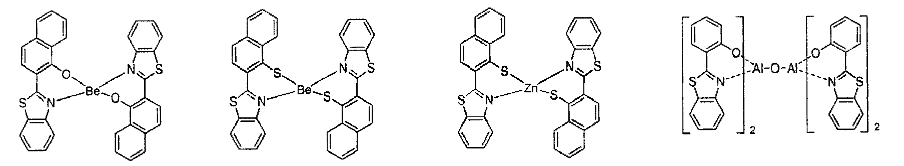

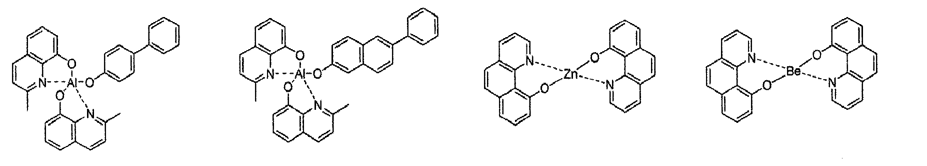

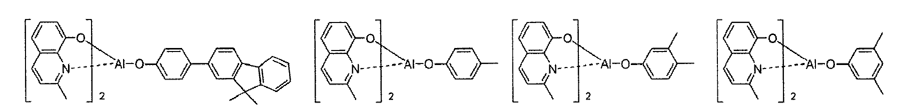

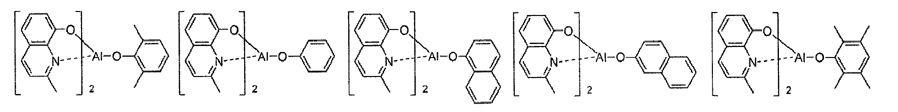

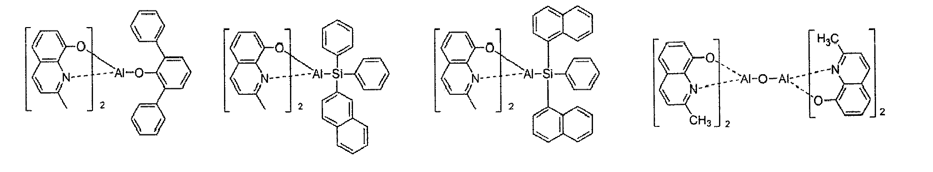

In an organic electroluminescent device according to the present invention, it is preferable to place one or more layer(s) (here-in-below, referred to as the "surface layer") selected from chalcogenide layers, metal halide layers and metal oxide layers, on the inner surface of at least one side of the pair of electrodes. Specifically, it is preferable to arrange a chalcogenide layer of silicon and aluminum metal (including oxides) on the anode surface of the EL medium layer, and a metal halide layer or a metal oxide layer on the cathode surface of the EL medium layer. As the result, stability in operation can be obtained.

Examples of chalcogenides preferably include SiOₓ (1≤X≤2), AlOₓ (1≤X≤1.5), SiON, SiAlON, or the like. Examples of metal halides preferably include LiF, MgF₂, CaF₂, fluorides of rare earth metal, or the like. Examples of metal oxides preferably include Cs₂O, Li₂O, MgO, SrO, BaO, CaO, or the like.

In an organic electroluminescent device according to the present invention, it is also preferable to arrange, on at least one surface of the pair of electrodes thus manufactured, a mixed region of electron transport compound and a reductive dopant, or a mixed region of a hole transport compound with an oxidative dopant. Accordingly, the electron transport compound is reduced to an anion, so that injection and transportation of electrons from the mixed region to an EL medium are facilitated. In addition, since the hole transport compound is oxidized to form a cation, injection and transportation of holes from the mixed region to an EL medium are facilitated. Preferable oxidative dopants include various Lewis acids and acceptor compounds. Preferable reductive dopants include alkali metals, alkali metal compounds, alkaline earth metals, rare-earth metals, and mixtures thereof.

The organic electroluminescent compounds according to the invention exhibit high luminous efficiency and excellent life property of material, so that OLED's having very good operation life can be manufactured therefrom.

### Best Mode

The present invention is further described by referring to representative compounds with regard to the organic electroluminescent compounds according to the invention, preparation thereof and electroluminescent properties of the devices manufactured therefrom, but those examples are provided for illustration of the embodiments only, not being intended to limit the scope of the invention by any means.

### Preparation Examples

### [Preparation Example 1] Preparation of Compound (590)

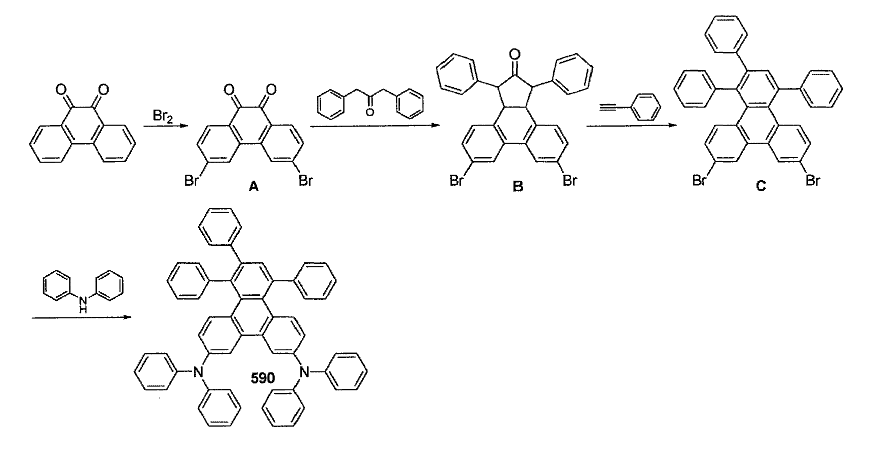

### Preparation of Compound (A)

A two-necked flask was charged with 9,10-phenanthraquinone (30 g, 0.14 mol) and benzoylperoxide (2.8 g, 11.52 mmol), and the flask was evacuated to form a vacuum, and then filled with argon (Ar) gas. Nitrobenzene (240 mL) was added to the flask, and cooled to 0°C. After stirring the mixture for 10 minutes, bromine (14.7 mL, 0.28 mmol) was slowly added thereto. The reaction was continued for 16 hours, while heating at 110°C. When the reaction was completed, the solid obtained was washed with n-hexane to obtain the target compound (A) (45 g, 86%) as orange solid.

### Preparation of Compound (B)

A one-necked flask was charged with Compound (A) (20 g, 0.05 mol) and 1,3-diphenyl-propan-2-one (11.5 g, 0.05 mol). After adding methanol (900 mL), the mixture was heated at 80°C. Potassium hydroxide (KOH) (3.06 g, 0.05 mol) dissolved in methanol (20 mL) was slowly added to the flask. After heating at 80°C for 30 minutes, the mixture was cooled at 0°C for 15 minutes. The solid produced was washed with methanol to obtain the target compound (B) (24.4 g, 82%) as dark solid.

### Preparation of Compound (C)

Compound (B) (15 g, 0.027 mol) and ethynylbenzene (2.97 g, 0.029 mol) were charged to a two-necked flask, and the flask was evacuated to form a vacuum, and then filled with argon (Ar) gas. After adding xylene (700 mL), the mixture was stirred under reflux for 12 hours. When the reaction was completed, the mixture was cooled to room temperature, and extracted with distilled water and ethyl acetate. The organic layer was dried over magnesium sulfate (MgSO₄), and the solvent was removed by using a rotary evaporator. Purification via column chromatography by using hexane and dichloromethane as eluent gave the target compound (Compound C) (14 g, 82%).

### Preparation of Compound (590)

A two-necked flask was charged with Compound (C) (6 g, 9.76 mmol), diphenylamine (4.1 g, 24.4 mmol), tris(dibenzylideneacetone)dipalladium (0) (178 mg, 0.19 mmol), tricyclohexylphosphine (109 mg, 90.39 mmol), sodium tert-butoxide (3.75 g, 3.90 mmol), and it was evacuated to form a vacuum, and then filled with argon gas. Toluene (180 mL) was added thereto, and the mixture was stirred under reflux for 5 hours. When the reaction was completed, the reaction mixture was cooled to room temperature, and extracted with distilled water and ethyl acetate. The organic layer was dried over magnesium sulfate (MgSO₄), the solvent was removed by using a rotary evaporator. Purification via column chromatography by using hexane and dichloromethane as eluent gave the target compound (Compound 590) (4.6 g, 60%).

### [Preparation Example 2] Preparation of Compound (673)

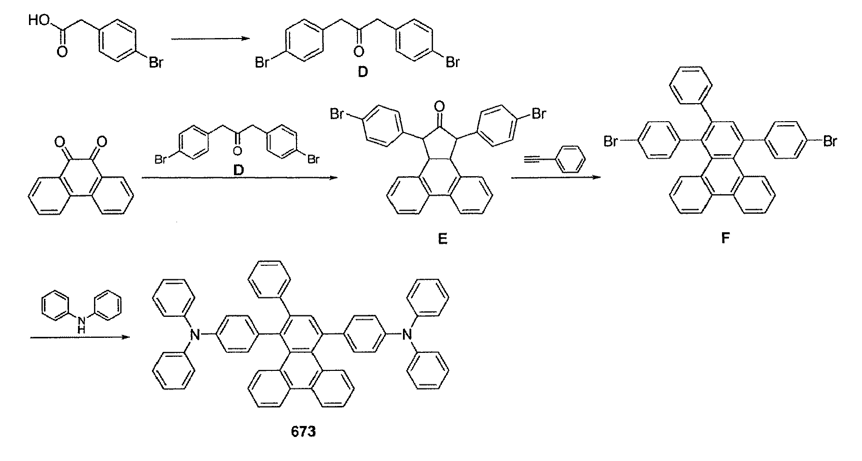

### Preparation of Compound (D)

Dicyclohexylcarbodiimide (47.9 g, 0.23 mol) and 4-(dimethylamino)pyridine (7.1 g, 0.058 mol) were charged to a one-necked flask, and the flask was evacuated to form a vacuum, and then filled with argon gas. After adding dichloromethane (500 mL) thereto, the mixture was stirred at 25°C for 20 minutes. Solution of (4-bromophenyl)acetic acid (50 g, 0.23 mol) dissolved in dichloromethane (500 mL) was slowly added to the flask. After stirring at 25°C for 24 hours, the solid produced was filtered off. The filtrate was purified via column chromatography by using hexane and dichloromethane as eluent to obtain Compound (D) (31 g, 68%).

### Preparation of Compound (E)

A one-necked flask was charged with 9,10-phenanthraquinone (15.2 g" 0.073 mol) and Compound (D) (27 g, 0.073 mol). After adding methanol (900 mL), the mixture was heated at 80°C. Potassium hydroxide (KOH) (4.1 g, 0.073 mol) dissolved in methanol (20 mL) was slowly added to the flask. After heating at 80°C for 30 minutes, the mixture was cooled at 0°C for 15 minutes. The solid produced was washed with methanol to obtain Compound (E) (36.6 g, 92%) as dark solid.

### Preparation of Compound (F)

Compound (E) (15 g, 0.027 mol) and ethynylbenzene (2.97 g, 0.029 mol) were charged to a two-necked flask, and the flask was evacuated to form a vacuum, and then filled with argon gas. After adding xylene (700 mL), the mixture was stirred under reflux for 12 hours. When the reaction was completed, the mixture was cooled to room temperature, and extracted with distilled water and ethyl acetate. The organic layer was dried over magnesium sulfate (MgSO₄), and the solvent was removed by using a rotary evaporator. Purification via column chromatography by using hexane and dichloromethane as eluent gave the Compound F (13 g, 76%).

### Preparation of Compound (673)

A two-necked flask was charged with Compound (F) (6 g, 9.76 mmol), diphenylamine (4.1 g, 24.4 mmol), tris(dibenzylideneacetone)dipalladium (0) (178 mg, 0.19 mmol), tricyclohexylphosphine (109 mg, 90.39 mmol), sodium tert-butoxide (3.75 g, 3.90 mmol), and it was evacuated to form a vacuum, and then filled with argon gas. Toluene (180 mL) was added thereto, and the mixture was stirred under reflux for 5 hours. When the reaction was completed, the reaction mixture was cooled to room temperature, and extracted with distilled water and ethyl acetate. The organic layer was dried over magnesium sulfate (MgSO₄), the solvent was removed by using a rotary evaporator. Purification via column chromatography by using hexane and dichloromethane as eluent gave Compound (673) (6.5 g, 84%).

Organic electroluminescent compounds (Compounds 1 to 825) were prepared according to the same procedure as in Preparation Examples 1 and 2, and the ¹H NMR and MS/FAB data of the organic electroluminescent compounds prepared are listed in Table 1.

**Table 1**

| Compound | ¹H NMR(CDCl₃, 200 MHz) | MS/FAB | |
|---|---|---|---|
| | | found | calculated |
| **1** | δ = 6.63(4H, m), 6.81(2H, m), 7.08(1H, m), 7.2(4H, m), 7.32(1H, m), 7.41(3H, m), 7.51-7.52(8H, m), 7.79-7.88(6H, m), 8.12(1H, m), 8.32(1H, s), 8.68(1H, m), 8.93(1H, m) | 623.78 | 623.26 |
| **2** | δ = 7.08(1H, m), 7.32∼7.41(6H, m), 7.49∼7.52(12H, m), 7.74∼7.88(14H, m), 8.12(1H, m), 8.32(1H, s), 8.68(1H, m), 8.93(1H, m) | 273.90 | 723.29 |
| **3** | δ = 2.34(6H, s), 6.51(4H, m), 6.98(4H, m), 7.08(1H, m), 7.32(1H, m), 7.41(3H, m), 7.51∼7.52(8H, m), 7.79∼7.88(6H, m), 8.12(1H, m), 8.32(1H, s), 8.68(1H, m), 8.93(1H, m) | 651.84 | 651.29 |
| **4** | δ = 1.35(18H, s), 6.55(4H, m), 7.01(4H, m), 7.08(1H, m), 7.32(1H, m), 7.41(3H, m), 7.51∼7.52(8H, m), 7.79∼7.88(6H, m), 8.12(1H, m), 8.32(1H, s), 8.68(1H, m), 8.93(1H, m) | 735.99 | 735.39 |
| **5** | δ = 1.72(12H, s), 6.58(2H, m), 6.75(2H, m), 7.08(1H, m), 7.28∼7.41(8H, m), 7.51∼7.55(10H, m), 7.62(2H, m), 7.79∼7.88(8H, m), 8.12(1H, m), 8.32(1H, s), 8.68(1H, m), 8.93(1H, m) | 856.10 | 55.39 |
| **6** | δ = 2.34(12H, s), 6.36(4H, m), 6.71(2H, m), 7.08(1H, m), 7.32(1H, m), 7.41(3H, m), 7.51∼7.52(8H, m), 7.79∼7.88(6H, m), 8.12(1H, m), 8.32(1H, s), 8.68(1H, m), 8.93(1H, m) | 679.89 | 679.32 |
| **7** | δ = 6.98(2H, m), 7.08(1H, m), 7.32∼7.41(6H, m), 7.51∼7.57(14H, m), 7.79∼7.88(6H, m). 8.02∼8.12(5H, m), 8.32(1H, s), 8.68(1H, m), 8.93(1H, m) | 723.90 | 723.29 |
| **8** | δ = 6.91(2H, m), 7.08(1H, m), 7.32(1H, m), 7.41(3H, m), 7.51∼7.52(8H, m), 7.79∼7.88(14H, m), 8.12(5H, m), 8.32(1H, s), 8.68(1H, m), 8.93(5H, m) | 824.02 | 823.32 |
| **10** | δ = 2.34(6H, s), 6.44(2H, m), 6.55∼6.59(4H, m), 7.08(3H, m), 7.32(1H, m), 7.41(3H, m), 7.51∼7.52(8H, m), 7.79∼7.88(6H, m), 8.12(1H, m), 8.32(1H, s), 8.68(1H, m). 8.93(1H, m) | 651.84 | 651.29 |
| **24** | δ = 6.62(2H, m), 6.7(2H, m), 7.08(1H, m), 7.32(1H, m), 7.41(3H, m), 7.51∼7.55(10H, m), 7.79∼7.88(6H, m), 8.07∼8.12(3H, m), 8.32(1H, s), 8.68(1H, m), 8.93(1H, m) | 625.76 | 625.25 |
| **31** | δ = 7.25∼7.33(3H, m), 7.41(3H, m), 7.5∼7.52(9H, m), 7.63(1H, m), 7.79∼7.94(8H, m), 8.1∼8.12(3H, m), 8.3(1H, s), 8.55(1H, m), 8.9∼8.93(2H, m) | 621.77 | 621.25 |
| **34** | δ = 6.97(2H, m), 7.08(1H, m), 7.16∼7.21(6H, m), 7.32(1H, m), 7.41(3H, m), 7.51∼7.52(8H, m), 7.79∼7.88(6H, m), 8.12(1H, m), 8.32(1H, s), 8.68(1H, m), 8.93(1H, m) | 653.83 | 653.22 |
| **36** | δ = 6.38(4H, m), 6.56(4H, m), 6.63(2H, m), 6.81(1H, m), 7.08(1H, m), 7.2(2H, m), 7.32(1H, m), 7.41(3H, m), 7.51∼7.52(8H, m), 7.79∼7.88(6H, m), 8.12(1H, m), 8.32(1H, s), 8.68(1H, m), 8.93(1H, m) | 712.88 | 712.29 |
| **42** | δ = 6.63(2H, m), 6.81(2H, m), 6.99∼7.08(5H, m), 7.25(2H, m), 7.32(1H, m), 7.41(3H, m), 7.51∼7.52(8H, m), 7.79∼7.88(6H, m), 8.12(1H, m), 8.32(1H, s), 8.68(1H, m), 8.93(1H, m) | 647.80 | 647.26 |
| **49** | δ = 6.63(2H, m), 6.81(1H, m), 6.98(1H, m), 7.08(1H, m), 7.2(2H, m), 7.32∼7.41(5H, m), 7.51∼7.57(11H, m), 7.79∼7.88(6H, m), 8.02∼8.12(3H, m), 8.32(1H, s), 8.68(1H, m), 8.93(1H, m) | 673.84 | 673.28 |
| **53** | δ = 1.72(6H, s), 6.58∼6.63(3H, m), 6.75∼6.81(2H, m), 7.08(1H, m), 7.2(2H, m), 7.28∼7.41(6H, m), 7.51∼7.55(9H, m), 7.62(1H, m), 7.79∼7.88(7H, m), 8.12(1H, m), 8.32(1H, s), 8.68(1H, m), 8.93(1H, m) | 739.94 | 739.32 |
| **60** | δ = 6.63(2H, m), 6.81(3H, m), 7.08(1H, m), 7.2(2H, m), 7.32(1H, m), 7.39∼7.41(5H, m), 7.51∼7.52(8H, m), 7.79∼7.88(6H, m), 8.12(1H, m), 8.32(1H, s), 8.68(1H, m), 8.93(1H, m) | 648.79 | 648.26 |
| **72** | δ = 6.63(2H, m), 6.81(1H, m), 7.08(1H, m), 7.2(2H, m), 7.27∼7.41(6H, m), 7.51∼7.52(8H, m), 7.79∼7.88(6H, m), 8.04∼8.12(3H, m), 8.32(1H, s), 8.68(1H, m), 8.93(1H, m) | 624.77 | 624.36 |
| **82** | δ = 1.72(12H, s), 6.69(4H, m), 7.08(1H, m), 7.28∼7.41(8H, m), 7.51∼7.55(14H, m), 7.63(2H, m), 7.77∼7.93(12H, m), 8.12(1H, m), 8.32(1H, s), 8.68(1H, m), 8.93(1H, m) | 1008.29 | 1007.45 |
| **84** | δ = 6.63(2H, m), 6.69(2H, m), 6.81(1H, m), 7.08(1H, m), 7.2(2H, m), 7.32(1H, m), 7.41(3H, m), 7.51∼7.55(12H, m), 7.61(1H, m), 7.79∼7.88(6H, m), 8.04∼8.12(3H, m), 8.32(1H, s), 8.42(1H, m), 8.55(1H, m), 8.68(1H, m), 8.93(1H, m) | 749.94 | 749.31 |
| **85** | δ = 1.72(6H, s), 6.63(2H, m), 6.69(2H, m), 6.81(1H, m), 7.08(1H, m), 7.2(2H, m), 7.28∼7.41(6H, m), 7.51∼7.55(11H, m), 7.63(1H, m), 7.77∼7.93(9H, m), 8.12(1H, m), 8.32(1H, s), 8.68(1H, m), 8.93(1H, m) | 816.04 | 815.36 |
| **90** | δ = 1.35(9H, s), 6.55(2H, m), 7.01(2H, m), 7.08(1H, m), 7.32∼7.41(5H, m), 7.49-7.52(10H, m), 7.74∼7.88(10H, m), 8.12(1H, m), 8.32(1H, s), 8.68(1H, m), 8.93(1H, m) | 729.95 | 729.34 |
| **92** | δ = 1.72(6H, s), 6.58(1H, m), 6.75(1H, m), 7.08(1H, m), 7.28∼7.41(7H, m), 7.49∼7.55(11H, m), 7.62(1H, m), 7.74∼7.88(11H, m), 8.12(1H, m), 8.32(1H, s), 8.68(1H, m), 8.93(1H, m) | 790.00 | 789.34 |
| **93** | δ = 6.91(1H, m), 7.08(1H, m), 7.32∼7.41(5H, m), 7.49∼7.52(10H, m), 7.74∼7.88(14H, m), 8.12(3H, m), 8.32(1H, s), 8.68(1H, m), 8.93(3H, m) | 773.96 | 773.31 |
| **120** | δ = 1.72(6H, s), 6.69(2H, m), 7.08(1H, m), 7.28∼7.41(7H, m), 7.49∼7.55(13H, m), 7.63(1H, m), 7.74∼7.82(13H, m), 8.12(1H, m), 8.32(1H, s), 8.68(1H, m), 8.93(1H, m) | 866.10 | 865.37 |
| **125** | δ = 2.34(6H, s), 6.36(2H, m), 6.71(1H, m), 6.98(1H, m), 7.08(1H, m), 7.32∼7.41(5H, m), 7.51∼7.57(11H, m), 7.79∼7.88(6H, m), 8.02∼8.12(3H, m), 8.32(1H, s), 8.68(1H, m), 8.93(1H, m) | 701.89 | 701.31 |
| **126** | δ = 1.72(6H, s), 6.58(1H, m), 6.75(1H, m), 6.98(1H, m), 7.08(1H, m), 7.28∼7.41(7H, m), 7.51∼7.62(13H, m), 7.79∼7.88(7H, m), 8.02∼8.12(3H, m), 8.32(1H, s), 8.68(1H, m), 8.93(1H, m) | 790.00 | 789.34 |
| **127** | δ = 6.91(1H, m), 6.98(1H, m), 7.08(1H, m), 7.32∼7.41(5H, m), 7.51∼7.57(11H, m), 7.79∼7.88(10H, m), 8.02∼8.12(5H, m), 8.32(1H, s), 8.68(1H, m), 8.93(3H, m) | 773.96 | 773.31 |
| **129** | δ = 2.34(3H, s), 6.44(1H, m), 6.55∼6.59(2H, m), 6.98(1H, m), 7.08(2H, m), 7.32∼7.41(5H, m), 7.51∼7.57(11H, m), 7.79∼7.88(6H, m), 8.02∼8.12(3H, m), 8.32(1H, s), 8.68(1H, m), 8.93(1H, m) | 687.87 | 687.29 |
| **136** | δ = 6.73(2H, m), 6.98(1H, m), 7.08(1H, m), 7.21(2H, m), 7.32(1H, m), 7.37(6H, m), 7.38(1H, m), 7.41∼7.52(23H, m), 7.79∼7.88(6H, m), 8.02∼8.12(3H, m), 8.32(1H, s), 8.68(1H, m), 8.93(1H, m) | 932.23 | 931.36 |
| **146** | δ = 6.98∼6.99(3H, m), 7.08(1H, m), 7-32∼7.41(5H, m), 7.51∼7.57(11H, m), 7.79∼7.88(6H, m), 8.02∼8.12(3H, m), 8.32(1H, s), 8.46(2H, m), 8.68(1H, m), 8.93(1H, m) | 674.83 | 673.27 |
| **152** | δ = 6.69(2H, m), 6.98(1H, m), 7.08(1H, m), 7.32∼7.41(5H, m), 7.51∼7.59(16H, m), 7.73∼7.92(8H, m), 8-8.12(5H, m), 8.32(1H, s), 8.68(1H, m), 8.93(1H, m) | 800.00 | 799.32 |
| **157** | δ = 1.35(9H, s), 2.34(3H, s), 6.51∼6.55(4H, m), 6.98∼7.01(4H, m), 7.08(1H, m), 7.32(1H, m), 7.41(3H, m), 7.51∼7.52(8H, m), 7.79∼7.88(6H, m), 8.12(1H, m), 8.32(1H, s), 8.68(1H, m), 8.93(1H, m) | 693.92 | 693.34 |
| **158** | δ = 2.34(9H, s), 6.36(2H, m), 6.51(2H, m), 6.71(1H, m), 6.98(2H, m), 7.08(1H, m), 7.32(1H, m), 7.41(3H, m), 7.51∼7.52(8H, m), 7.79∼7.88(6H, m), 8.12(1H, m), 8.32(1H, s), 8.68(1H, m), 8.93(1H, m) | 665.86 | 665.31 |
| **190** | δ = 1.35(9H, s), 2.34(6H, s), 6.36(2H, m), 6.55(2H, m), 6.71(1H, m), 7.01(2H, m), 7.08(1H, m), 7.32(1H, m), 7.41(3H, m), 7.51∼7.52(8H, m), 7.79∼7.88(6H, m), 8.12(1H, m), 8.32(1H, s), 8.68(1H, m), 8.93(1H, m) | 707.94 | 707.36 |
| **191** | δ = 1.35(9H, s), 1.72(6H, s), 6.55∼6.58(3H, m), 6.75(1H, m), 7.01(2H, m), 7.08(1H, m), 7.28∼7.41(6H, m), 7.51∼7.55(9H, m), 7.62(1H, m), 7.79∼7.88(7H, m), 8.12(1H, m), 8.32(1H, s), 8.68(1H, m), 8.93(1H, m) | 796.05 | 795.39 |
| **192** | δ = 1.35(9H, s), 6.55(2H, m), 6.91(1H, m), 7.01(2H, m), 7.08(1H, m), 7.32(1H, m), 7.41(3H, m), 7.51~7.52(8H, m), 7.79~7.88(10H, m), 8.12(3H, m), 8.32(1H, s), 8.68(1H, m), 8.93(3H, m) | 780.01 | 779.36 |
| **199** | δ = 1.35(9H, s), 6.55(2H, m), 6.81(2H, m), 7.01(2H, m), 7.08(1H, m), 7.32(1H, m), 7.39~7.41(5H, m), 7.51~7.52(8H, m), 7-79~7.88(6H, m), 8.12(1H, m), 8.32(1H, s), 8.68(1H, m), 8.93(1H, m) | 704.90 | 704.32 |
| **208** | δ = 1.35(9H, s), 6.55(2H, m), 6.81(1H, m), 7.01(2H, m), 7.08(1H, m), 7.32(1H, m), 7.41(3H, m), 7.51~7.57(9H, m), 7.73~7.88(9H, m), 8.1~8.12(3H, m), 8.32(1H, s), 8.42(2H, m), 8.68(1H, m), 8.93(1H, m) | 804.03 | 803.36 |
| **220** | δ = 1.35(9H, s), 6.55~6.59(3H, m), 6.88~6.89(2H, m), 7.01(2H, m), 7.08(1H, m), 7.32(1H, m), 7.41∼7.44(4H, m), 7.51~7.55(10H, m), 7.61(1H, m), 7.79~7.88(6H, m), 8.04~8.12(3H, m), 8.32(1H, s), 8.42(1H, m), 8.55(1H, m), 8.68(1H, m), 8.93(1H, m) | 806.04 | 805.37 |
| **224** | δ = 2.34(6H, s), 6.36(2H, m), 6.69~6.71(3H, m), 7.08(1H, m), 7.32(1H, m), 7.41(4H, m), 7.51~7.54(14H, m), 7.79∼7.88(6H, m), 8.12(1H, m), 8.32(1H, s), 8.68(1H, m), 8.93(1H, m) | 727.93 | 727.32 |
| **253** | δ = 1.72(6H, s), 6.58(1H, m), 6.75(1H, m), 6.91(1H, m), 7.08(1H, m), 7.28~7.41(6H, m), 7.51~7.55(9H, m), 7.62(1H, m), 7.79~7.88(11H, m), 8.12(3H, m), 8.32(1H, s), 8.68(1H, m), 8.93(3H, m) | 840.06 | 839.36 |
| **271** | δ = 1.72(6H, s), 6.58(1H, m), 6.75(1H, m), 7.08(1H, m), 7.28~7.41(6H, m), 7.51~7.55(9H, m), 7.62(1H, m), 7.79∼7.88(7H, m), 8.12(1H, m), 8.32(1H, s), 8.35~8.4(3H, m), 8.68(1H, m), 8.93(1H, m) | 741.92 | 741.31 |
| **278** | δ = 1.72(6H, s), 6.58(1H, m), 6.69~6.75(3H, m), 7.08(1H, m), 7.28~7.41(6H, m), 7.51~7.62(15H, m), 7.73∼7.92(9H, m), 8(2H, m), 8.12(1H, m), 8.32(1H, s), 8.68(1H, m), 8.93(1H, m) | 866.10 | 865.37 |
| **284** | δ = 6.69(2H, m), 6.91(1H, m), 7.08(1H, m), 7.32(1H, m), 7.41(4H, m), 7.51~7.54(14H, m), 7.79∼7.88(10H, m), 8.12(3H, m), 8.32(1H, s), 8.68(1H, m), 8.93(3H, m) | 800.00 | 799.32 |
| **307** | δ = 6.69(2H, m), 6.91(1H, m), 7.08(1H, m), 7.32(1H, m), 7.41(3H, m), 7.51∼7.59(13H, m), 7.73∼7.92(12H, m), 8(2H, m), 8.12(3H, m), 8.32(1H, s), 8.68(1H, m), 8.93(3H, m) | 850.05 | 849.34 |
| **314** | δ = 6.59(1H, m), 6.69(2H, m), 6.88∼6.89(2H, m), 7.08(1H, m), 7.32(1H, m), 7.41~7.44(6H, m), 7.51~7.54(18H, m), 7.79~7.88(6H, m), 8.12(1H, m), 8.32(1H, s), 8.68(1H, m), 8.93(1H, m) | 775.97 | 775.32 |
| **328** | δ = 6.62(1H, m), 6.69∼6.7(3H, m), 7.08(1H, m), 7.32(1H, m), 7.41(4H, m), 7.51∼7.55(15H, m), 7.79∼7.88(6H, m), 8.07∼8.12(2H, m), 8.32(1H, s), 8.68(1H, m), 8.93(1H, m) | 700.87 | 700.29 |
| **339** | δ = 2.12(3H, s), 2.34(3H, s), 6.44(1H, m), 6.51∼6.59(3H, m), 6.69(1H, m), 7.01(1H, m), 7.08(2H, m), 7.15(1H, m), 7.32(1H, m). 7.41(3H, m), 7.51∼7.52(8H, m), 7.79∼7.88(6H, m), 8.12(1H, m), 8.32(1H, s), 8.68(1H, m), 8.93(1H, m) | 651.84 | 651.29 |
| **364** | δ = 2.34(3H, m), 6.44(1H, m), 6.55∼6.59(3H, m), 6.88∼6.89(2H, m), 7.08(2H, m), 7.32(1H, m), 7.41∼7.44(4H, m). 7.51∼7.55(10H, m), 7.61(1H, m), 7.79∼7.88(6H, m), 8.04∼8.12(3H, m), 8.32(1H, s), 8.42(1H, m), 8.55(1H, m), 8.68(1H, m), 8.93(1H, m) | 763.96 | 763.32 |
| **377** | δ = 3.83(3H, m), 6.52(2H, m), 6.74(2H, m), 6.81(1H, m), 7.08(1H, m), 7.32(1H, m), 7.41(3H, m), 7.51∼7.57(9H, m), 7.73∼7.88(9H, m), 8.1∼8.12(3H, m), 8.32(1H, s), 8.42(2H, m), 8.68(1H, m), 8.93(1H, m) | 777.95 | 777.30 |
| **391** | δ = 6.62(1H, m), 6.7(1H, m), 7.08(1H, m), 7.27∼7.41(6H, m), 7.51∼7.55(9H, m), 7.79∼7.88(6H, m), 8.04∼8.12(4H, m), 8.32(1H, s), 8.68(1H, m), 8.93(1H, m) | 625.76 | 625.25 |
| **398** | δ = 6.63(4H, m), 6.69(2H, m), 6.81(2H, m), 7.2(4H, m), 7.41(3H, m), 7.51∼7.54(10H, m), 7.79∼7.88(6H, m), 8.1∼8.12(2H, m), 8.32(1H, s), 8.34(1H, m), 8.93(1H, m), 8.99(1H, m) | 699.88 | 699.29 |
| **403** | δ = 6.63(2H, m), 6.69(2H, m), 6.81(1H, m), 7.2(2H, m), 7.36∼7.41(4H, m), 7.49∼7.54(12H, m), 7.74∼7.88(10H, m), 8.1∼8.12(2H, m), 8.32(1H, s), 8.34(1H, m), 8.93(1H, m), 8.99(1H, m) | 749.94 | 749.31 |
| **406** | δ = 6.63(4H, m), 6.81(2H, m), 7.2(4H, m), 7.41∼7.52(13H, m), 7.64∼7.88(10H, m), 8.1∼8.12(2H, m), 8.32(1H, s), 8.34(1H, m), 8.93(1H, m), 8.99(1H, m) | 749.94 | 749.31 |
| **417** | δ = 1.72(12H, s), 6.58(2H, m), 6.75(2H, m), 7.04(1H, m), 7.28(2H, m), 7.38∼7.41(5H, m), 7.51∼7.55(12H, m), 7.62(2H, m), 7.78∼7.88(9H, m), 8.07∼8.12(3H, m), 8.32(1H, s), 8.34(1H, m), 8.49(1H, m), 8.93(1H, m), 8.99(1H, m) | 982.26 | 981.43 |
| **422** | δ = 1.72(6H, s), 6.58∼6.63(5H, m), 6.75∼6.81(3H, m), 7.2(4H, m), 7.41(3H, m), 7.51∼7.52(8H, m), 7.62∼7.63(2H, m), 7.77∼7.93(8H, m). 8.1∼8.12(2H, m), 8.32(1H, s), 8.34(1H, m), 8.93(1H, m), 8.99(1H, m) | 816.04 | 815.36 |
| **426** | δ = 6.63(4H, m), 6.81(2H, m), 7.08(1H, m), 7.2(4H, m), 7.32(1H, m), 7.41(3H, m), 7.51∼7.52(8H, m), 7.71(2H, m), 7.79∼7.88(6H, m), 8.1∼8.12(3H, m), 8.32(1H, s), 8.34(2H, m), 8.68(1H, m), 8.93(1H, m), 8.99(2H, m) | 800.00 | 799.32 |
| **427** | δ = 6.63(4H, m), 6.81(2H, m), 6.98(1H, m), 7.2(4H, m), 7.38∼7.41(4H, m), 7.51∼7.52(8H, m), 7.6(1H, m), 7.79∼7.88(6H, m), 8.03∼8.04(2H, m), 8.1∼8.12(2H, m), 8.32(1H, s), 8.34(1H, m), 8.4(1H, m), 8.93(1H, m), 8.99(1H, m) | 749.94 | 749.31 |
| **430** | δ = 2.34(3H, m), 6.63(4H, m), 6.81(2H, m), 7.08(1H, m), 7.2(4H, m), 7.29∼7.33(5H, m), 7.41(2H, m), 7.51∼7.52(6H, m), 7.79∼7.88(4H, m), 8.12(1H, m), 8.32(1H, s), 8.68(1H, m), 8.93(1H, m) | 637.81 | 637.28 |
| **435** | δ = 2.34(15H, s), 6.36(4H, m), 6.71(2H, m), 7.08(1H, m), 7.29∼7.33(5H, m), 7.41(2H, m), 7.51∼7.52(6H, m), 7.79∼7.88(4H, m), 8.12(1H, m), 8.32(1H, s), 8.68(1H, m), 8.93(1H, m) | 693.92 | 693.34 |
| **440** | δ = 6.63(4H, m), 6.81(2H, m), 7.08(1H, m), 7.2∼7.25(8H, m), 7.32(1H, m), 7.41(3H, m), 7.51∼7.52(10H, m), 7.79∼7.88(4H, m), 8.12(1H, m), 8.32(1H, s), 8.68(1H, m), 8.93(1H, m) | 699.88 | 699.29 |
| **452** | δ = 2.34(6H, s), 6.51(4H, m), 6.98(4H, m), 7.08(1H, m), 7.25(4H, m), 7.32(1H, m), 7.41(2H, m), 7.51∼7.52(6H, m), 7.58∼7.59(3H, m), 7.73∼7.92(6H, m), 8(2H, m), 8.12(1H, m), 8.32(1H, s), 8.68(1H, m), 8.93(1H, m) | 777.99 | 777.34 |
| **460** | δ = 6.63(4H, m), 6.81(2H, m), 7.08(1H, m), 7.2∼7.25(8H, m), 7.32(1H, m), 7.41(2H, m), 7.51∼7.55(8H, m), 7.61(1H, m), 7.79∼7.88(4H, m), 8.04∼8.12(3H, m), 8.32(1H, s), 8.42(1H, m), 8.55(1H, m), 8.68(1H, m), 8.93(1H, m) | 749.94 | 749.31 |
| **469** | δ = 1.72(6H, s), 6.63(4H, m), 6.81(2H, m), 7.08(1H, m), 7.2∼7.41(13H, m), 7.51∼7.55(7H, m), 7.63(1H, m), 7.77∼7.93(7H, m), 8.12(1H, m), 8.32(1H, s), 8.68(1H, m), 8.93(1H, m) | 816.04 | 815.36 |
| **476** | δ = 1.35(9H, s), 2.34(6H, s), 6.51(4H, m), 6.98(4H, m), 7.08(1H, m), 7.32∼7.41(7H, m), 7.51∼7.52(6H, m), 7.79∼7.88(4H, m), 8.12(1H, m), 8.32(1H, s), 8.68(1H, m), 8.93(1H, m) | 707.94 | 707.36 |
| **479** | δ = 2.34(6H, s), 6.63(4H, m), 6.81(2H, m), 7.08(1H, m), 7.2(4H, m), 7.31∼7.32(2H, m), 7.41(2H, m), 7.51∼7.52(6H, m), 7.6(2H, m), 7.79∼7.88(4H, m), 8.12(1H, m), 8.32(1H, s), 8.68(1H, m), 8.93(1H, m) | 651.84 | 651.29 |
| **484** | δ = 2.34(3H, m), 6.63(4H, m), 6.81(2H, m), 7.08(1H, m), 7.2(4H, m), 7.29∼7.33(5H, m), 7.41(2H, m), 7.51(4H, m), 7.79∼7.88(6H, m), 8.12(1H, m), 8.32(1H, s), 8.68(1H, m), 8.93(1H, m) | 637.81 | 637.28 |
| **493** | δ = 2.34(3H, m), 6.63(4H, m), 6.81(2H, m), 7.08(1H, m), 7.2(4H, m), 7.29∼7.33(5H, m), 7.41(2H, m), 7.51∼7.52(6H, m), 7.79∼7.88(4H, m), 8.12(1H, m), 8.32(1H, s), 8.68(1H, m), 8.93(1H, m) | 637.81 | 637.28 |
| **500** | δ = 2.34(6H, s), 6.51(4H, m), 6.98(4H, m), 7.08(1H, m), 7.25(4H, m), 7.32(1H, m), 7.41(3H, m), 7.51∼7.52(10H, m), 7.79∼7.88(4H, m), 8.12(1H, m), 8.32(1H, s), 8.68(1H, m), 8.93(1H, m) | 727.93 | 727.32 |
| **502** | δ = 6.63(4H, m), 6.81(2H, m), 7.08(1H, m), 7.2(4H, m), 7.32(1H, m), 7.41(2H, m), 7.51∼7.52(6H, m), 7.58∼7.59(3H, m), 7.73∼7.92(6H, m), 8(2H, m), 8.12(1H, m), 8.32(1H, s), 8.68(1H, m), 8.93(1H, m) | 673.84 | 673.28 |
| **509** | δ = 6.63(4H, m), 6.81(2H, m), 7.08(1H, m), 7.2(4H, m), 7.32(1H, m), 7.58∼7.59(9H, m), 7.73(3H, m), 7.82∼7.92(5H, m), 8(6H, m), 8.12(1H, m), 8.32(1H, s), 8.68(1H, m), 8.93(1H, m) | 773.96 | 773.31 |
| **514** | δ = 6.63(4H, m), 6.81(2H, m), 7.08(1H, m), 7.2(4H, m), 7.32(1H, m), 7.41(2H, m), 7.51∼7.52(6H, m), 7.58∼7.59(3H, m), 7.73∼7.92(6H, m), 8(2H, m), 8.12(1H, m), 8.32(1H, s), 8.68(1H, m), 8.93(1H, m) | 673.84 | 673.28 |
| **519** | δ = 6.63(4H, m), 6.81(2H, m), 7.08(1H, m), 7.2(4H, m), 7.32(1H, m), 7.41(3H, m), 7.51∼7.52(8H, m), 7.79∼7.88(6H, m), 8.12(1H, m), 8.32(1H, s), 8.68(1H, m), 8.93(1H, m) | 623.78 | 623.26 |
| **531** | δ = 6.63(2H, m), 6.81(1H, m), 6.91(1H, m), 7.08(1H, m), 7.2(2H, m), 7.32(1H, m), 7.41(3H, m), 7.51∼7.52(8H, m), 7.79∼7.88(10H, m), 8.12(3H, m), 8.32(1H, s), 8.68(1H, m), 8.93(3H, m) | 723.90 | 723.29 |
| **542** | δ = 1.72(6H, s), 6.58(1H, m), 6.75(1H, m), 6.98(1H, m), 7.08(1H, m), 7.28∼7.41(7H, m), 7.51∼7.62(13H, m), 7.79∼7.88(7H, m), 8.02∼8.12(3H, m), 8.32(1H, s), 8.68(1H, m), 8.93(1H, m) | 790.00 | 789.34 |
| **554** | δ = 6.63(4H, m), 6.69(2H, m), 6.81(2H, m), 7.2(4H, m), 7.41(3H, m), 7.51∼7.54(10H, m), 7.79∼7.88(6H, m), 8.1∼8.12(2H, m), 8.32(1H, s), 8.34(1H, m), 8.93(1H, m), 8.99(1H, m) | 699.88 | 699.29 |
| **565** | δ = 6.63(2H, m), 6.81(1H, m), 7.04(1H, m), 7.2(2H, m), 7.36∼7.41(4H, m), 7.49∼7.54(12H, m), 7.74∼7.88(11H, m), 8.07∼8.12(3H, m), 8.32(1H, s), 8.34(1H, m), 8.49(1H, m), 8.93(1H, m), 8.99(1H, m) | 800.00 | 799.32 |
| **571** | δ = 2.34(9H, s), 6.63(4H, m), 6.81(2H, m), 7.08(1H, m), 7.2(4H, m), 7.29∼7.33(13H, m), 7.82∼7.88(2H, m), 8.12(1H, m), 8.32(1H, s), 8.68(1H, m), 8.93(1H, m) | 665.86 | 665.31 |
| **590** | δ = 6.63(8H, m), 6.81(4H, m), 7.08(2H, m), 7.2(8H_{,} m), 7.32(2H_{,} m), 7.41(3H, m), 7.51∼7.52(8H, m), 7.79(4H, m), 8.32(1H, s), 8.68(2H, m) | 790.99 | 790.33 |
| **606** | δ = 6.63(8H, m), 6.69(4H, m), 6.81(4H, m), 7.2(8H, m), 7.41(3H, m), 7.51∼7.54(12H, m), 7.79(4H, m), 8.1(2H, m), 8.32(1H, s), 8.34(2H, m), 8.99(2H, m) | 943.18 | 942.40 |
| **607** | δ = 6.63(8H, m), 6.69(2H, m), 6.81(4H, m), 7.08(1H, m), 7.2(8H, m), 7.32(1H, m), 7.41(3H, m), 7.51∼7.54(10H, m), 7.79(4H, m), 8.1(1H, m), 8.32(1H, s), 8.34(1H, m), 8.68(1H, m), 8.99(1H, m) | 867.08 | 866.37 |
| **608** | δ = 6.63(8H, m), 6.69(2H, m), 6.81(4H, m), 7.08(1H, m), 7.2(8H, m), 7.32(1H, m), 7.41(3H, m), 7.51∼7.54(10H, m), 7.79(4H, m), 8.1(1H, m), 8.32(1H, s), 8.34(1H, m), 8.68(1H, m), 8.99(1H, m) | 867.08 | 866.37 |
| **617** | δ = 7.25∼7.33(6H, m), 7.41(3H, m), 7.5∼7.52(10H, m), 7.63(2H, m), 7.79(4H, m), 7.9∼7.94(4H, m), 8.1∼8.12(4H, m), 8.3(1H, s), 8.55(2H, m), 8.9(2H, m) | 786.96 | 786.30 |
| **621** | δ = 6.63(4H, m), 6.69(2H, m), 6.81(2H, m), 7.2(4H, m), 7.41(2H, m), 7.51∼7.54 (6H, m), 7.79∼7.88(8H, m), 8.12(2H, m), 8.32(1H, s), 8.93(2H, m) | 623.78 | 623.26 |
| **629** | δ = 6.63(2H, m), 6.69(2H, m), 6.81(1H, m), 7.2(2H, m), 7.36∼7.41(3H, m), 7.49∼7.54(8H, m), 7.74∼7.88(12H, m), 8.12(2H, m), 8.32(1H, s), 8.93(2H, m) | 673.84 | 673.28 |
| **642** | δ = 6.63(4H, m), 6.69(2H, m), 6.81(2H, m), 7.2(4H, m), 7.41(1H, m), 7.51∼7.59(7H, m), 7.73∼7.92(8H, m), 8(2H, m), 8.12(2H, m), 8.32(1H, s), 8.93(2H, m) | 673.84 | 673.28 |
| **659** | δ = 2.34(6H, s), 6.63(4H, m), 6.69(2H, m), 6.81(2H, m), 7.2(4H, m), 7.29∼7.33(8H, m), 7.54(2H, m), 7.82∼7.88(4H, m), 8.12(2H, m), 8.32(1H, s), 8.93(2H, m) | 651.84 | 651.29 |
| **673** | δ = 6.63(8H, m), 6.69(4H, m), 6.81(4H, m), 7.2(8H, m), 7.41(1H, m), 7.51∼7.54(6H, m), 7.79∼7.88(6H, m), 8.12(2H, m), 8.32(1H, s), 8.93(2H, m) | 790.99 | 790.33 |
| **680** | δ = 1.72(12H, s), 6.58∼6.63(6H, m), 6.69∼6.81(8H, m), 7.2(4H. m), 7.28(2H, m), 7.38∼7.41(3H, m), 7.51∼7.55(8H, m), 7.62(2H, m), 7.79∼7.88(8H, m), 8.12(2H, m), 8.32(1H, s), 8.93(2H, m) | 1023.31 | 1022.46 |
| **685** | δ = 7.25∼7.33(6H, m), 7.41(1H, m), 7.5∼7.51(4H, m), 7.63∼7.68(6H, m), 7.79∼.88(10H, m), 7.94(2H, m), 8.12(4H, m), 8.32(1H, s), 8.55(2H, m), 8.93(2H, m) | 786.96 | 786.30 |
| **691** | δ = 6.63(8H, m), 6.69(4H, m), 6.81(4H, m), 7.2(8H, m), 7.41(2H, m), 7.51∼.54(10H, m), 7.79∼7.88(4H, m), 8.1∼8.12(2H, m), 8.32(1H, s), 8.34(1H, m), 8.93(1H, m), 8.99(1H, m) | 867.08 | 866.37 |
| **705** | δ = 1.72(6H, s), 6.63(4H, m), 6.69(2H, m), 6.81(2H, m), 7.2(4H, m), 7.28(1H, m), 7.38∼7.41(3H, m), 7.51∼7.55(7H, m), 7.63(1H, m), 7.77∼7.93(9H, m), 8.1∼8.12(2H, m), 8.32(1H, s), 8.34(1H, m), 8.93(1H, m), 8.99(1H, m) | 816.04 | 815.36 |
| **716** | δ = 6.63(2H, m), 6.69(2H, m), 6.81(1H, m), 7.2(2H, m), 7.36∼7.41(4H, m), 7.49∼7.54(14H, m), 7.74∼7.88(8H, m), 8.1∼8.12(2H, m), 8.32(1H, s*),* 8.34(1H, m), 8.93(1H, m), 8.99(1H, m) | 749.94 | 749.31 |
| **728** | δ = 6.63(8H, m), 6.69(4H, m), 6.81(4H, m), 7.2(8H, m), 7.41(1H, m), 7.51∼7.55(8H, m), 7.61(1H, m), 7.79∼7.88(4H, m), 8.04∼8.12(4H, m), 8.32(1H, s), 8.34(1H, m), 8.42(1H, m), 8.55(1H, m), 8.93(1H, m), 8.99(1H, m) | 917.14 | 916.38 |
| **745** | δ = 6.63(8H, m), 6.69(4H, m), 6.81(4H, m), 7.2(8H, m), 7.41(3H, m), 7.51∼7.54(14H, m), 7.79(2H, m), 8.1(2H, m), 8.32(1H, s), 8.34(2H, m), 8.99(2H, m) | 943.18 | 942.40 |
| **749** | δ = 1.72(6H, s), 6.63(8H, m), 6.69(4H, m), 6.81(4H, m), 7.2(8H, m), 7.28(1H, m), 7.38∼7.41(3H, m), 7.51∼7.55(11H, m), 7.63(1H, m), 7.77∼7.79(3H, m), 7.87∼7.93(2H, m), 8.1(2H, m), 8.32(1H, s), 8.34(2H, m), 8.99(2H, m) | 1056.34 | 1055.46 |
| **756** | δ = 6.63(4H, m), 6.69(2H, m), 6.81(2H, m), 7.2(4H, m), 7.41(3H, m), 7.51∼7.59(15H, m), 7.73∼7.79(3H, m), 7.92(1H, m), 8(2H, m), 8.1(2H, m), 8.32(1H, s), 8.34(2H, m), 8.99(2H, m) | 826.03 | 825.34 |
| **764** | δ = 6.69(4H, m), 7.36∼7.41(6H, m), 7.49∼7.59(21H, m), 7.73∼7.92(20H, m), 8(2H, m), 8.1(2H, m), 8.32(1H, s), 8.34(2H, m), 8.99(2H, m) | 1193.47 | 1192.48 |
| **781** | δ = 6.63(12H, m), 6.69(4H, m), 6.81(6H, m), 7.08(1H, m), 7.2(12H, m), 7.32(1H, m), 7.41(2H, m), 7.51∼7.54(10H, m), 7.79(2H, m), 8.1(1H, m), 8.32(1H, s), 8.34(1H, m), 8.68(1H, m), 8.99(1H, m) | 1034.29 | 1033.44 |
| **784** | δ = 6.63(12H, m), 6.69(6H, m), 6.81(6H, m), 7.2(12H, m), 7.41(2H, m), 7.51∼7.54(12H, m), 7.79(2H, m), 8.1(2H, m), 8.32(1H, s), 8.34(2H, m), 8.99(2H, m) | 1110.39 | 1109.47 |
| **785** | δ = 6.63(10H, m), 6.69(6H, m), 6.81(5H, m), 7.2(10H, m), 7.36∼7.41(3H, m), 7.49∼7.54(14H, m), 7.74∼7.88(6H, m), 8.1(2H, m), 8.32(1H, s), 8.34(2H, m), 8.99(2H, m) | 1160.45 | 1159.49 |
| **793** | δ = 6.63(12H, m), 6.69(4H, m), 6.81(6H, m), 7.08(1H, m), 7.2(12H, m), 7.32(1H, m), 7.41(2H, m), 7.51∼7.54(10H, m), 7.79(2H, m), 8.1(1H, m), 8.32(1H, s), 8.34(1H, m), 8.68(1H, m), 8.99(1H, m) | 1034.29 | 1033.44 |
| **802** | δ = 6.63(8H, m), 6.69(6H, m), 6.81(4H, m), 6.98(2H, m), 7.2(8H, m), 7.38∼7.41(3H, m), 7.51∼7.61(17H, m), 7.79(2H, m), 8.02∼8.1(8H, m), 8.32(1H, s), 8.34(2H, m), 8.92(1H, m), 8.55(1H, m), 8.99(2H, m) | 1260.56 | 1259.52 |
| **805** | δ = 6.63(16H, m), 6.69(4H, m), 6.81(8H, m), 7.08(2H, m), 7.2(16H, m), 7.32(2H, m), 7.41(1H, m), 7.51∼7.54(6H, m), 7.79(2H, m), 8.32(1H, s), 8.68(2H, m) | 1125.40 | 1124.48 |
| **811** | δ = 6.63(12H, m), 6.69(2H, m), 6.81(6H, m), 7.08(2H, m), 7.2(12H, m), 7.32(2H, m), 7.41(2H, m), 7.51∼7.54(6H, m), 7.79(4H, m), 8.32(1H, s), 8.68(2H, m) | 958.20 | 957.41 |
| **815** | δ = 6.63(12H, m), 6.69(6H, m), 6.81(6H, m), 7.2(12H, m), 7.41(2H, m), 7.51∼7.54(10H, m), 7.79(4H, m), 8.1(2H, m), 8.32(1H, s), 8.34(2H, m), 8.99(2H, m) | 1110.39 | 1109.47 |
| **816** | δ = 6.63(12H, m), 6.69(4H, m), 6.81(6H, m), 7.08(1H, m), 7.2(12H, m), 7.32(1H, m), 7.41(2H, m), 7.51∼7.54(8H, m), 7.79(4H, m), 8.1(1H, m), 8.32(1H, s), 8.34(1H, m), 8.68(1H, m), 8.99(1H, m) | 1034.29 | 1033.44 |
| **820** | δ = 6.63(10H, m), 6.69(2H, m), 6.81(5H, m), 7.08(2H, m), 7.2(10H, m), 7.32∼7.41(5H, m), 7.49∼7.54(10H, m), 7.74∼7.88(6H, m), 8.32(1H, s), 8.68(2H, m) | 1008.25 | 1007.42 |
| **821** | δ = 2.34(12H, s), 6.44(4H, m), 6.55∼6.63(12H, m), 6.69(2H, m), 6.81(2H, m), 7.08(6H, m), 7.2(4H, m), 7.32(2H, m), 7.41(2H, m), 7.51∼7.54(8H, m), 7.79(2H, m), 8.32(1H, s), 8.68(2H, m) | 1014.30 | 1013.47 |
| **822** | δ = 6.63(12H, m), 6.69(6H, m), 6.81(6H, m), 7.2(12H, m), 7.41(2H, m), 7.51∼7.54(12H, m), 7.79(2H, m), 8.1(2H, m), 8.32(1H, s), 8.34(2H, m), 8.99(2H, m) | 1110.39 | 1109.47 |
| **823** | δ = 6.63(12H, m), 6.69(4H, m), 6.81(6H, m), 7.08(1H, m), 7.2(12H, m), 7.32(1H, m), 7.41(2H, m), 7.51∼7.54(10H, m), 7.79(2H, m), 8.1(1H, m), 8.32(1H, s), 8.34(1H, m), 8.68(1H. m), 8.99(1H, m) | 1034.29 | 1033.44 |
| **824** | δ = 6.63(10H, m), 6.69(4H, m), 6.81(5H, m), 7.08(1H, m), 7.2(10H, m), 7.32∼7.41(4H, m), 7.49∼7.54(12H, m), 7.74∼7.88(6H, m), 8.1(1H, m), 8.32(1H, s), 8.34(1H, m), 8.68(1H, m), 8.99(1H, m) | 1084.35 | 1083.46 |
| **825** | δ = 6.63(12H, m), 6.69(4H, m), 6.81(6H, m), 7.08(1H, m), 7.2(12H, m), 7.32(1H, m), 7.41(2H, m), 7.51∼7.54(10H, m), 7.79(2H, m), 8.1(1H, m), 8.32(1H, s), 8.34(1H, m), 8.68(1H, m), 8.99(1H, m) | 1034.29 | 1033.44 |
| **826** | δ = 6.63(4H, m), 6.69(2H, m), 6.81(2H, m), 7.17∼7.2(6H, m), 7.4∼7.41(4H, m), 7.51∼7.54(8H, m), 7.69(2H, m), 7.79(2H, m), 8.1(2H, m), 8.32(1H, s), 8.34(2H, m), 8.99(2H, m) | 788.03 | 787.24 |
| **827** | δ = 6.63(2H, m), 6.69(2H, m), 6.81(1H, m), 7(2H, m), 7.2∼7.26(4H, m), 7.36∼7.41(3H, m), 7.49∼7.54(12H, m), 7.74∼7.88(6H, m), 8.32(1H, s), 8.5(2H, m), 8.61(2H, m), 8.85(2H, m), 9.02(2H, m) | 828.01 | 827.33 |
| **828** | δ = 3.83(6H, s), 6.63(4H, m), 6.69(2H, m), 6.81(2H, m), 7.2(4H, m), 7.39∼7.41(4H, m), 7.51∼7.54(8H, m), 7.63(2H, m), 7.79(2H, m), 8.32(1H, s), 8.82(2H, m) | 683.83 | 683.28 |
| **829** | δ = 6.63(4H, m), 6.69(2H, m), 6.81(2H, m), 7.14∼7.2(10H, m), 7.41(6H, m), 7.51∼7.54(10H, m), 7.78∼7.79(4H, m), 8.32(1H, s), 8.89(2H, m) | 807.97 | 807.31 |
| **830** | δ = 6.63(4H, m), 6.69(2H, m), 6.81(2H, m), 7.19∼7.25(10H, m), 7.41(6H, m), 7.51∼7.54(8H, m), 7.79∼7.82(4H, m), 8.06(2H, m), 8.32(1H, s), 8.73(2H, m) | 840.10 | 839.27 |
| **831** | δ = 1.35(18H, s), 6.63(4H, m), 6.69(2H, m), 6.81(2H, m), 7.2(4H, m), 7.41(2H, m), 7.51∼7.54(8H, m), 7.79∼7.8(4H, m), 8.04(2H, m), 8.32(1H, s), 8.83(2H, m) | 800.12 | 799.33 |
| **832** | δ = 0.91(12H, m), 1.82(2H, m), 2.54(4H, m), 6.63(4H, m), 6.69(2H, m), 6.81(2H, m), 7.2(4H, m), 7.41(2H, m), 7.51∼7.54(8H, m), 7.74∼7.79(4H, m), 7.98(2H, m), 8.32(1H, s), 8.88(2H, m) | 735.99 | 735.39 |

[Example 1] Electroluminescent properties of OLED employing organic electroluminescent compound according to the invention (I)

An OLED device was manufactured by using electroluminescent material according to the present invention.

First, a transparent electrode ITO thin film (15 Ω/□) (2) prepared from glass for OLED (1) (manufactured by Samsung-Corning) was subjected to ultrasonic washing with trichloroethylene, acetone, ethanol and distilled water, sequentially, and stored in isopropanol before use.

Then, an ITO substrate was equipped in a substrate folder of a vacuum vapor-deposit device, and 4,4',4"-tris(N,N-(2-naphthyl)-phenylamino)triphenylamine (2-TNATA) (of which the structure is shown below) was placed in a cell of the vacuum vapor-deposit device, which was then ventilated up to 10⁻⁶ torr of vacuum in the chamber. Electric current was applied to the cell to evaporate 2-TNATA, thereby providing vapor-deposit of a hole injecting layer (3) having 60 nm thickness on the ITO substrate.

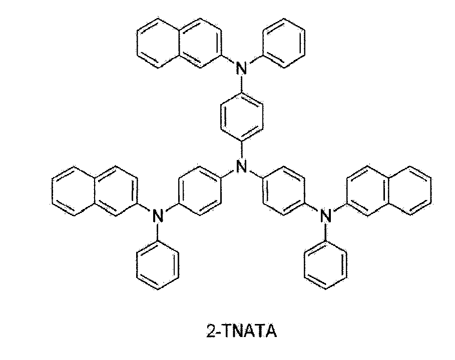

Then, to another cell of the vacuum vapor-deposit device, charged was N,N'-bis(a-naphthyl)-N,N'-diphenyl-4,4'-diamine (NPB) (of which the structure is shown below), and electric current was applied to the cell to evaporate NPB, thereby providing vapor-deposit of a hole transport layer (4) with 20 nm thickness on the hole injecting layer.

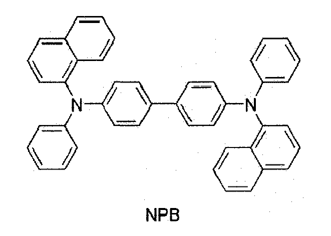

After forming the hole injecting layer and the hole transport layer, an electroluminescent layer was vapor-deposited as follows. To one cell of a vacuum vapor-deposit device, charged was H-33 (of which the structure is shown below) as host, while Compound (83) according to the present invention was charged to another cell as dopant. The two substances were evaporated at different rates to carry out doping at concentration of 2 to 5% by weight on the basis of the host, thereby providing vapor-deposit of an electroluminescent layer (5) with a thickness of 30 nm on the hole transport layer.

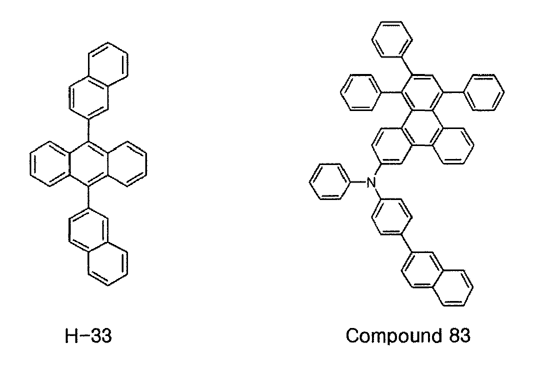

Then, tris(8-hydroxyquinoline)aluminum (III) (Alq) (of which the structure is shown below) was vapor-deposited as an electron transport layer (6) with a thickness of 20 nm, and lithium quinolate (Liq) (of which the structure shown below) was vapor-deposited as an electron injecting layer (7) with a thickness of 1 to 2 nm. Thereafter, an Al cathode (8) was vapor-deposited with a thickness of 150 nm by using another vacuum vapor-deposit device to manufacture an OLED.

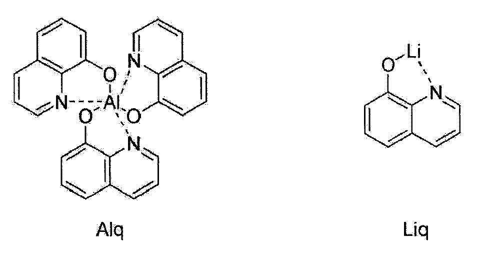

Each material employed for manufacturing an OLED was used as the electroluminescent material after purifying via vacuum sublimation at 10⁻⁶ torr.

### [Comparative Example 1] Electroluminescent properties of OLED employing conventional electroluminescent material

After forming a hole injecting layer and a hole transport layer according to the same procedure as described in Example 1, dinaphthylanthracene (DNA) was charged to another cell of said vacuum vapor-deposit device as electroluminescent host material, while Compound (A) (of which the structure is shown below) was charged to still another cell as blue electroluminescent material. An electroluminescent layer was vapor-deposited with a thickness of 30 nm on the hole transport layer, at the vapor-deposition rate of 100:1.

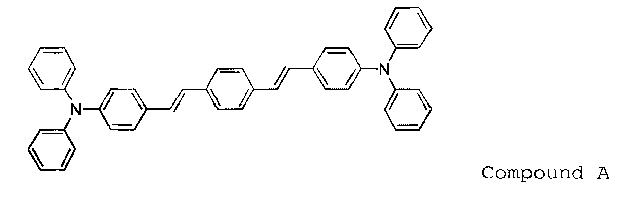

Then, an electron transport layer and an electron injecting layer were vapor-deposited according to the same procedures as in Example 1, and an Al cathode was vapor-deposited with a thickness of 150 nm by using another vacuum vapor-deposit device to manufacture an OLED.

The luminous efficiencies of the OLED's comprising the organic electroluminescent compounds according to the present invention (Example 1) or conventional electroluminescent compound (Comparative Example 1) were measured at 1,000 cd/m², respectively, and the results are shown in Table 2.

**Table 2**

| No. | Host | Dopant | Doping concentration | Luminous efficiency (cd/A) | Color | |
|---|---|---|---|---|---|---|
| | | | (wt%) | @1000 cd/m² | | |
| Ex.1 | 1 | H-30 | Compound **1** | 3.0 | 7.4 | Blue |
| | 2 | H-33 | Compound **83** | 3.0 | 7.5 | Blue |
| | 3 | H-35 | Compound **190** | 3.0 | 7.6 | Blue |
| | 4 | H-50 | Compound **398** | 3.0 | 7.3 | Blue |
| | 5 | H-90 | Compound **433** | 3.0 | 7.7 | Blue |
| | 6 | H-105 | Compound **484** | 3.0 | 7.5 | Blue |
| | 7 | H-114 | Compound **590** | 3.0 | 8.0 | Blue |
| | 8 | H-126 | Compound **679** | 3.0 | 7.9 | Blue |
| | 9 | H-128 | Compound **745** | 3.0 | 7.7 | Blue |
| | 10 | H-136 | Compound **805** | 3.0 | 7.9 | Blue |
| Comp.1 | | DNA | Compound A | 3.0 | 7.3 | Jade green |

As can be seen from Table 2, the blue electroluminescent devices to which the material of the present invention was applied showed significantly enhanced color purity (from jade green electroluminescence into light blue to blue electroluminescence), while maintaining at least comparable luminous efficiency, as compared to the device employing conventional electroluminescent material (Comparative Example 1).

### [Example 2] Electroluminescent properties of OLED employing organic electroluminescent compound according to the invention (II)

After forming a hole injecting layer (3) and a hole transport layer (4) according to the same procedure as described in Example 1, H-33 (of which the structure is shown below) was charged to one cell of a vacuum vapor-deposit device as host, while Compound (7) according to the present invention was charged to another cell as dopant. The two substances were evaporated at different rates to carry out doping at a concentration of 2 to 5% by weight on the basis of the host, thereby providing vapor-deposit of an electroluminescent layer (5) with a thickness of 30 nm on the hole transport layer.

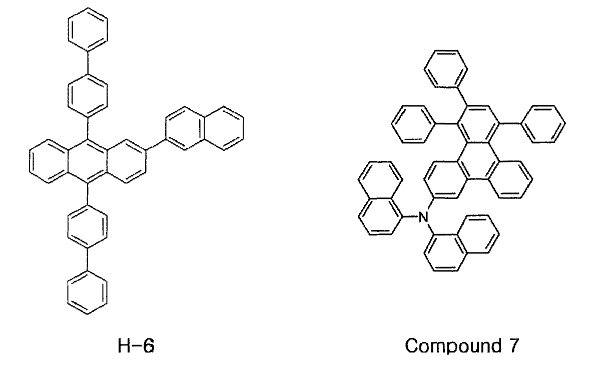

Then, an electron transport layer (6) and an electron injecting layer (7) were vapor-deposited according to the same procedure as in Example 1, and an Al cathode was vapor-deposited thereon with a thickness of 150 nm by using another vacuum vapor-deposit device to manufacture an OLED.

### [Comparative Example 2] Electroluminescent properties of OLED employing conventional electroluminescent material

After forming a hole injecting layer and a hole transport layer according to the same procedure as described in Example 1, tris(8-hydroxyquinoline)aluminum (III) (Alq) was charged to another cell of said vacuum vapor-deposit device as electroluminescent host material, while Coumarin 545T (C545T) (of which the structure is shown below) was charged to still another cell. The two substances were evaporated at different rates to carry out doping, thereby providing an electroluminescent layer with a thickness of 30 nm on the hole transport layer. The doping concentration preferably is from 1 to 3 mol% on the basis of Alq.

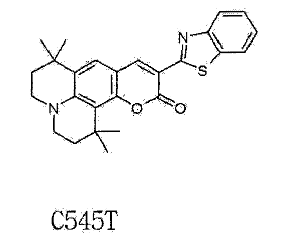

Then, an electron transport layer and an electron injecting layer were vapor-deposited according to the same procedure as in Example 1, and an Al cathode was vapor-deposited thereon with a thickness of 150 nm by using another vacuum vapor-deposit device to manufacture an OLED.

### [Comparative Example 3] Electroluminescent properties of OLED employing conventional electroluminescent material

After forming a hole injecting layer (3) and a hole transport layer (4) according to the same procedure as described in Example 1, H-6 was charged to another cell of a vacuum vapor-deposit device as electroluminescent host material, while Compound (G) was charged to still another cell. The two substances were evaporated at different rates to carry out doping at a concentration of 2 to 5% by weight on the basis of the host, thereby providing vapor-deposit of an electroluminescent layer with a thickness of 30 nm on the hole transport layer.

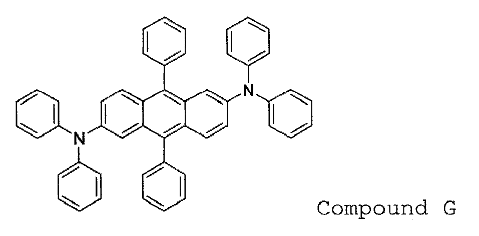

Then, an electron transport layer (6) and an electron injecting layer (7) were vapor-deposited according to the same procedure as in Example 1, and an Al cathode (8) was vapor-deposited thereon with a thickness of 150 nm by using another vacuum vapor-deposit device to manufacture an OLED.

The luminous efficiencies of the OLED's comprising the organic electroluminescent compound according to the present invention (Example 2) or conventional electroluminescent compounds (Comparative Examples 2 and 3) were measured at 5,000 cd/m², respectively, and the results are shown in Table 3.

**Table 3**

| No. | | Host | Dopant | Doping concentration (wt%) | Luminous efficiency (cd/A) | Color |
|---|---|---|---|---|---|---|
| | | | | | @1000cd/m² | |
| Ex. 2 | 1 | H-6 | Compound **7** | 3 | 19.7 | Green |
| | 2 | H-9 | Compound **33** | 3 | 18.1 | Green |
| | 3 | H-22 | Compound **92** | 3 | 16.6 | Green |
| | 4 | H-53 | Compound **391** | 3 | 19.7 | Green |
| | 5 | H-63 | Compound **428** | 3 | 19.0 | Green |
| | 6 | H-67 | Compound **594** | 3 | 19.0 | Green |
| | 7 | H-73 | Compound **617** | 3 | 20.0 | Green |
| | 8 | H-75 | Compound **688** | 3 | 21.0 | Green |
| | 9 | H-78 | Compound **727** | 3 | 18.5 | Green |
| | 10 | H-88 | Compound **820** | 3 | 17.9 | Green |
| Comp.2 | | Alq | C545T | 1 | 10.3 | Green |
| Comp.3 | | H-6 | Compound G | 3.0 | 16.3 | Green |

As can be seen from Table 3, the green electroluminescent device to which the inventive material was applied showed significantly improved luminous efficiency, while maintaining at least comparable color purity as compared to the devices according to Comparative Example 2 or 3.

### [Example 3] Electroluminescent properties of OLED employing organic electroluminescent compound according to the invention (III)

After forming a hole injecting layer (3) according to the same procedure as in Example 1, Compound (673) (of which the structure is shown below) was charged to another cell of the vacuum vapor-deposit device, and electric current was applied to the cell to evaporate the material to vapor-deposit a hole transport layer (4) with a thickness of 20 nm on the hole injecting layer.

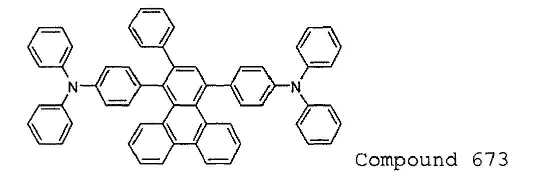

Then, an electroluminescent layer was vapor-deposited thereon as follows. Dinaphthylanthracene (DNA) was charged to one cell of said vacuum vapor-deposit device as electroluminescent material, and perylene (of which the structure is shown below) was charged to another cell. Then the two cells were simultaneously heated to carry out vapor-deposition at a vapor-deposit rate of 2 to 5% by weight, thus providing an electroluminescent layer (5) having 30 nm thickness vapor-deposited on the hole transport layer.

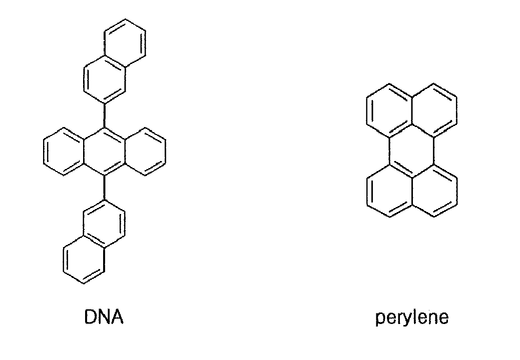

Then, an electron transport layer (6) and an electron injecting layer (7) were vapor-deposited according to the same procedure as in Example 1, and an Al cathode (8) was vapor-deposited thereon with a thickness of 150 nm by using another vacuum vapor-deposit device to manufacture an OLED.

### [Comparative Example 4] Electroluminescent properties of OLED employing conventional electroluminescent material

After forming a hole injecting layer (3) according to the same procedure as in Example 1, charged was N,N'-bis(α-naphthyl)-N,N'-diphenyl-4,4'-diamine (NPB) (of which the structure is shown below) to another cell of the vacuum vapor-deposit device, and electric current was applied to the cell to evaporate NPB, thereby providing vapor-deposit of a hole transport layer (4) with 20 nm thickness on the hole injecting layer.

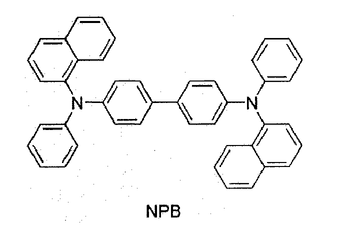

An electroluminescent layer was then vapor-deposited as follows. To one cell of a vacuum vapor-deposit device, charged was dinaphthylanthracene (DNA) as electroluminescent material, while perylene (of which the structure is shown below) was charged to another cell. The two cells were simultaneously heated to carry out vapor-deposit of perylene at a vapor-deposit rate of 2 to 5% by weight, thereby providing vapor-deposit of an electroluminescent layer (5) with a thickness of 30 nm on the hole transport layer.

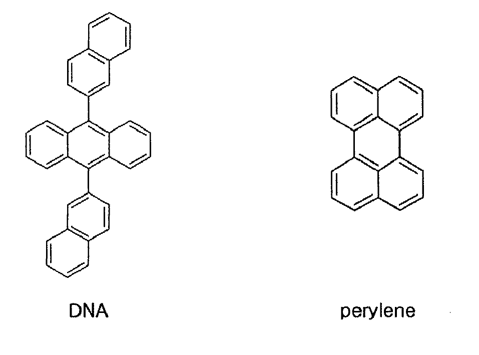

Then, an electron transport layer (6) and an electron injecting layer (7) were vapor-deposited according to the same procedure as in Example 1, and an Al cathode (8) was vapor-deposited thereon with a thickness of 150 nm by using another vacuum vapor-deposit device to manufacture an OLED.

The luminous efficiencies of the OLED's comprising the organic electroluminescent compound according to the present invention (Example 3) or conventional electroluminescent compound (Comparative Example 4) were measured at 1,000 cd/m², respectively, and the results are shown in Table 4.

**Table 4**

| No. | | Material for hole transport layer | Operation voltage (V) @1,000 cd/m² | Luminous efficiency (cd/A) @1,000 cd/m² | Color |
|---|---|---|---|---|---|
| Ex.4 | 1 | Compound **673** | 5 | 5.4 | Blue |
| | 2 | Compound **691** | 4,8 | 5.6 | Blue |
| Comp.4 | | NPB | 6 | 4.5 | Blue |

It was confirmed that the compounds developed by the present invention showed better properties as compared to the conventional materials in view of performances.

## Claims

1. An organic electroluminescent compound represented by Chemical Formula (1): 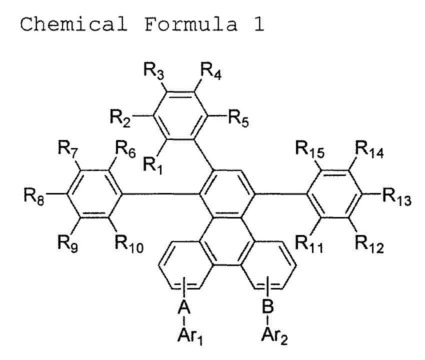 wherein, A and B independently represent a chemical bond, (C6-C60)arylene, (C3-C60)heteroarylene, (C6-C60)arylenoxy, (C1-C60)alkylenoxy, (C6-C60)arylenethio, (C1-C60)alkylenethio or (C1-C60)alkylene;
Ar₁ and Ar₂ independently represent hydrogen or deuterium, or a substituent selected from the following structures: 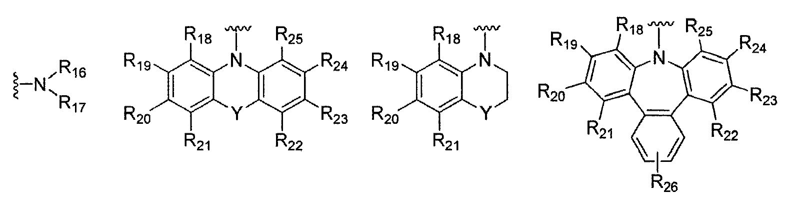 R₁ through R₅ independently represent hydrogen, deuterium, halogen, (C1-C60)alkyl, (C6-C60)aryl, (C3-C60)heteroaryl containing one or more heteroatom(s) selected from N, O and S, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkyl(C6-C60)aryl, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or each of them may be linked to an adjacent substituent via (C3-C60)alkylene or (C3-C60)alkenylene with or without a fused ring to form an alicyclic ring, or a monocyclic or polycyclic aromatic ring;
R₆ through R₁₅ independently represent hydrogen, deuterium, halogen, (C1-C60)alkyl, (C6-C60)aryl, (C3-C60)heteroaryl containing one or more heteroatom(s) selected from N, O and S, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkyl(C6-C60)aryl, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro, hydroxyl, 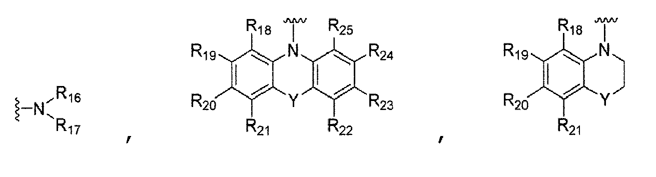 or 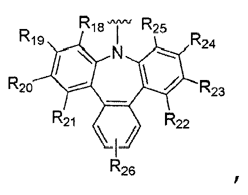 or each of them may be linked to an adjacent substituent via (C3-C60)alkylene or (C3-C60)alkenylene with or without a fused ring to form an alicyclic ring, or a monocyclic or polycyclic aromatic ring;
R₁₆ and R₁₇ independently represent (C6-C60)aryl, (C3-C60)heteroaryl containing one or more heteroatom(s) selected
from N, O and S, 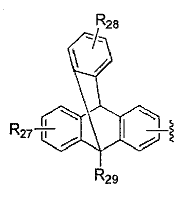 or 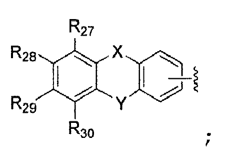 R₁₈ through R₂₆ and R₂₇ to R₃₀ independently represent hydrogen, deuterium, halogen, (C1-C60)alkyl, (C6-C60)aryl, (C3-C60)heteroaryl containing one or more heteroatom(s) selected from N, O and S, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkyl(C6-C60)aryl, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or each of them may be linked to an adjacent substituent via (C3-C60)alkylene or (C3-C60)alkenylene with or without a fused ring to form an alicyclic ring, or a monocyclic or polycyclic aromatic ring;
X and Y independently represent a chemical bond, - (CR₃₁R₃₂)ₘ-, -N(R₃₃)-, -S-, -O-, -Si(R₃₄)(R₃₅)-, -P(R₃₆)-, -C(=O)-, -B(R₃₇)-, -In(R₃₈)-, -Se-, -Ge(R₃₉)(R₄₀)-, -Sn(R₄₁)(R₄₂)-, - Ga(R₄₃)- or -(R₄₄)C=C(R₄₅)-; excluding that both X and Y represent chemical bonds;
R₃₁ through R₄₅ independently represent hydrogen, deuterium, halogen, (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C1-C60)alkyl(C6-C60)aryl, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or R₃₁ and R₃₂, R₃₄ and R₃₅, R₃₉ and R₄₀, R₄₁ and R₄₂, or R₄₄ and R₄₅ may be linked via (C3-C60)alkylene or (C3-C60)alkenylene with or without a fused ring to form an alicyclic ring, or a monocyclic or polycyclic aromatic ring;
the arylene, heteroarylene, arylenethio, arylenoxy, alkylenoxy or alkylenethio of A and B; the alkyl, aryl, heteroaryl, heterocycloalkyl, cycloalkyl, alkylsilyl, arylsilyl, alkenyl, alkynyl, alkylamino or arylamino of R₁ through R₄₅ may be further substituted by one or more substituent(s) selected from a group consisting of halogen, (C1-C60)alkyl, halo(C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, carbazolyl, (C1-C60)alkylamino, (C6-C60)arylamino, (C1-C60)alkyl(C6-C60)aryl, (C6-C60)ar(C1-C60)alkyl, (C1-C60)alkyl(C6-C60)aryl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro and hydroxyl;
m is an integer from 1 to 4; provided that, at least one substituent(s) among Ar₁, Ar₂, R₆, R₇, R₈, R₉, R₁₀, R₁₁, R₁₂, R₁₃, R₁₄ and R₁₅ represent (s)

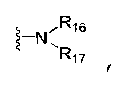

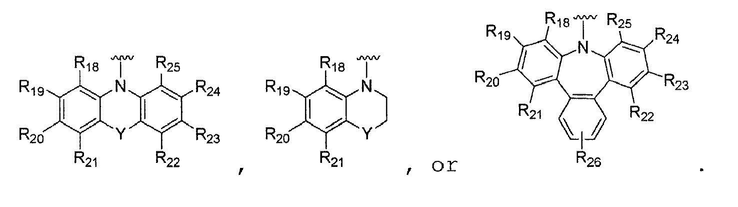

2. The organic electroluminescent compound according to claim 1, wherein, Ar₁ and Ar₂ independently represent hydrogen, deuterium, or a substituent represented by one of the following structural formulas: 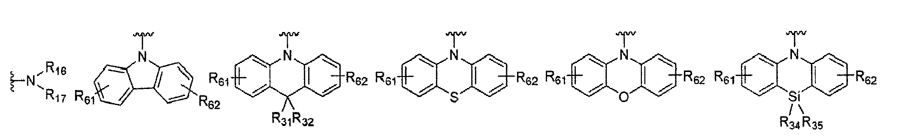 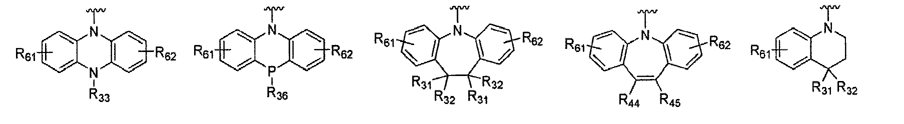 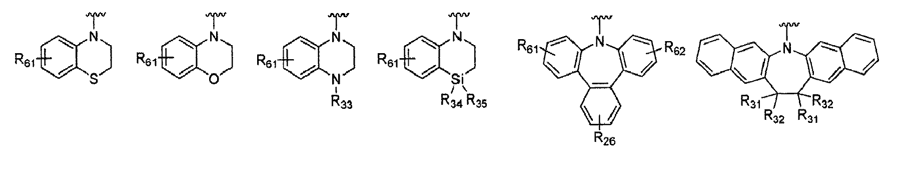 wherein, R₁₆ and R₁₇ are defined as in claim 1;
R₂₆, R₃₁ through R₃₆, R₄₄ and R₄₅ independently represent hydrogen, deuterium, halogen, (C1-C60)alkyl, (C6-C60) aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C1-C60)alkyl(C6-C60)aryl, (C6-C60)ar(C1-C60)alkyl, (C1-C60)alkyloxy, (C1-C60)alkylthio, (C6-C60)aryloxy, (C1-C60)arylthio, (C1-C60)alkoxycarbonyl, (C1-C60)alkylcarbonyl, (C6-C60)arylcarbonyl, carboxyl, nitro or hydroxyl, or R₃₁ and R₃₂, or R₃₄ and R₃₅ may be linked via (C3-C60)alkylene or (C3-C60)alkenylene with or without a fused ring to form an alicyclic ring, or a monocyclic or polycyclic aromatic ring; and
R₆₁ and R₆₂ independently represent hydrogen, deuterium, (C1-C60)alkyl or (C6-C60)aryl.

3. An organic electroluminescent device which is comprised of a first electrode; a second electrode; and at least one organic layer(s) interposed between the first electrode and the second electrode; wherein the organic layer comprises an organic electroluminescent compound represented by Chemical Formula (1): 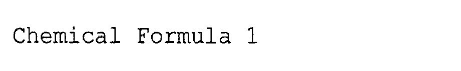 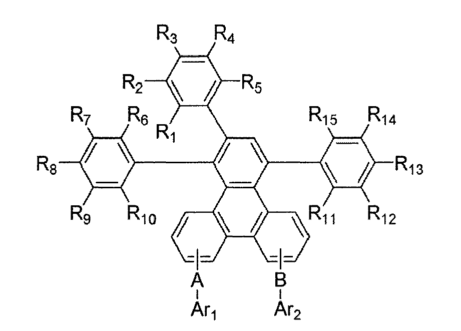 wherein, A and B independently represent a chemical bond, (C6-C60)arylene, (C3-C60)heteroarylene, (C6-C60)arylenoxy, (C1-C60)alkylenoxy, (C6-C60)arylenethio, (C1-C60)alkylenethio or (C1-C60)alkylene;
Ar₁ and Ar₂ independently represent hydrogen or deuterium, or a substituent selected from the following structures: 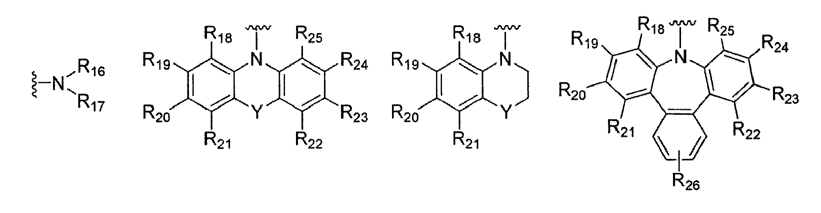 R₁ through R₅ independently represent hydrogen, deuterium, halogen, (C1-C60)alkyl, (C6-C60)aryl, (C3-C60)heteroaryl containing one or more heteroatom(s) selected from N, O and S, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkyl(C6-C60)aryl, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or each of them may be linked to an adjacent substituent via (C3-C60)alkylene or (C3-C60)alkenylene with or without a fused ring to form an alicyclic ring, or a monocyclic or polycyclic aromatic ring;
R₆ through R₁₅ independently represent hydrogen, deuterium, halogen, (C1-C60)alkyl, (C6-C60)aryl, (C3-C60)heteroaryl containing one or more heteroatom(s) selected from N, O and S, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkyl(C6-C60)aryl, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro, hydroxyl, 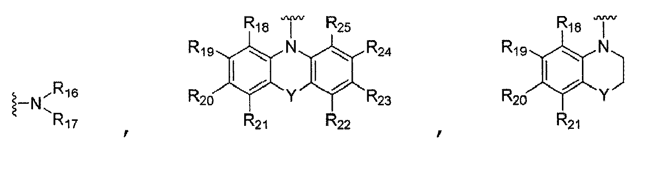 or 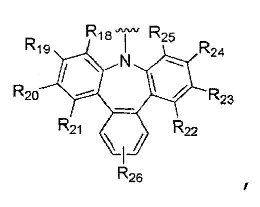 or each of them may be linked to an adjacent substituent via (C3-C60)alkylene or (C3-C60)alkenylene with or without a fused ring to form an alicyclic ring, or a monocyclic or polycyclic aromatic ring;
R₁₆ and R₁₇ independently represent (C6-C60)aryl, (C3-C60)heteroaryl containing one or more heteroatom(s) selected
from N, O and S, 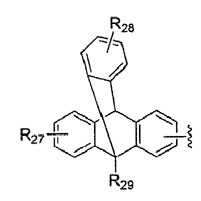 or 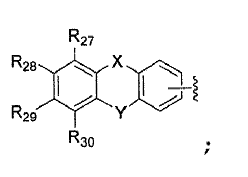 R₁₈ through R₂₆ and R₂₇ to R₃₀ independently represent hydrogen, deuterium, halogen, (C1-C60)alkyl, (C6-C60)aryl, (C3-C60)heteroaryl containing one or more heteroatom(s) selected from N, O and S, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkyl(C6-C60)aryl, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or each of them may be linked to an adjacent substituent via (C3-C60)alkylene or (C3-C60)alkenylene with or without a fused ring to form an alicyclic ring, or a monocyclic or polycyclic aromatic ring; X and Y independently represent a chemical bond, - (CR₃₁R₃₂)ₘ-, -N(R₃₃)-, -S-, -O-, -Si(R₃₄)(R₃₅)-, -P(R₃₆)-, -C(=O)-, -B(R₃₇)-, -In(R₃₈)-, -Se-, -Ge(R₃₉)(R₄₀)-, -Sn(R₄₁)(R₄₂)-, - Ga(R₄₃)- or (R₄₄)C=C(R₄₅)-; excluding that both X and Y represent chemical bonds;
R₃₁ through R₄₅ independently represent hydrogen, deuterium, halogen, (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C1-C60)alkyl(C6-C60)aryl, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or R₃₁ and R₃₂, R₃₄ and R₃₅, R₃₉ and R₄₀, R₄₁ and R₄₂, or R₄₄ and R₄₅ may be linked via (C3-C60)alkylene or (C3-C60)alkenylene with or without a fused ring to form an alicyclic ring, or a monocyclic or polycyclic aromatic ring;
the arylene, heteroarylene, arylenethio, arylenoxy, alkylenoxy or alkylenethio of A and B; the alkyl, aryl, heteroaryl, heterocycloalkyl, cycloalkyl, alkylsilyl, arylsilyl, alkenyl, alkynyl, alkylamino or arylamino of R₁ through R₄₅ may be further substituted by one or more substituent(s) selected from a group consisting of halogen, (C1-C60)alkyl, halo(C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, carbazolyl, (C1-C60)alkylamino, (C6-C60)arylamino, (C1-C60)alkyl(C6-C60)aryl, (C6-C60)ar(C1-C60)alkyl, (C1-C60)alkyl(C6-C60)aryl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro and hydroxyl;
m is an integer from 1 to 4;
provided that, at least one substituent(s) among Ar1, Ar2, R6, R7, R8, R9, R10, R11, R12, R13, R14 and R15 represent(s) 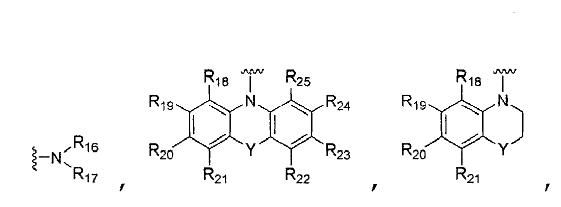 or 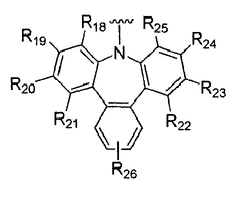 and
one or more host(s) selected from the compounds represented by Chemical Formula (2) or (3):
Chemical Formula 2
(Ar₁₁)_{b}-L₁-(Ar₁₂)_{c}
Chemical Formula 3
(Ar₁₃)_{d}-L₂-(Ar₁₄)ₑ
wherein, L₁ represents (C6-C60)arylene or (C4-C60)heteroarylene;
L₂ represents anthracenylene;
Ar₁₁ through Ar₁₄ are independently selected from hydrogen, deuterium, (C1-C60)alkyl, (C1-C60)alkoxy, halogen, (C4-C60)heteroaryl, (C5-C60)cycloalkyl or (C6-C60)aryl; the cycloalkyl, aryl or heteroaryl of Ar₁₁ through Ar₁₄ may be further substituted by one or more substituent(s) selected from a group consisting of (C6-C60)aryl or (C4-C60)heteroaryl with or without one or more substituent(s) selected from a group consisting of (C1-C60)alkyl with or without halogen substituent(s), (C1-C60)alkoxy, (C3-C60)cycloalkyl, halogen, cyano, tri(C1-C60)alkylsilyl, di(C1-C60)alkyl(C6-C60)arylsilyl and tri(C6-C60)arylsilyl; (C1-C60)alkyl with or without halogen substituent(s), (C1-C60)alkoxy, (C3-C60)cycloalkyl, halogen, cyano, tri(C1-C60)alkylsilyl, di(C1-C60)alkyl(C6-C60)arylsilyl and tri(C6-C60)arylsilyl, and
b, c, d and e independently represent an integer from 0 to 4.

4. The organic electroluminescent device according to claim 3, wherein the host is selected from the compounds represented by one of Chemical Formulas (4) to (7) : 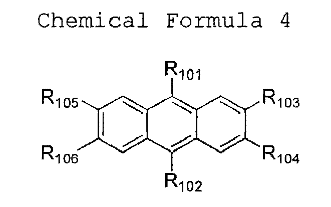 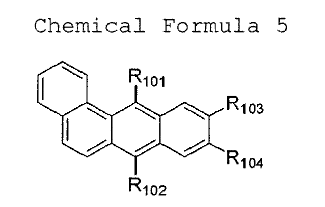 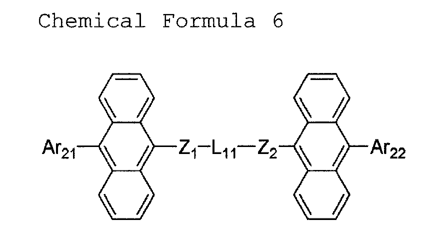 wherein, R₁₀₁ and R₁₀₂ independently represent hydrogen, deuterium, (C1-C60)alkyl, halogen, (C6-C60)aryl, (C3-C60)heteroaryl, 5- or 6-membered heterocycloalkyl containing one or more heteroatom(s) selected from N, O and S, or (C3-C60)cycloalkyl; the aryl or heteroaryl of R₁₀₁ and R₁₀₂ may be further substituted by one or more substituent(s) selected from a group consisting of (C1-C60)alkyl, halo(C1-C60)alkyl, (C1-C60)alkyloxy, (C3-C60)cycloalkyl, (C6-C60)aryl, (C4-C60)heteroaryl, halogen, cyano, tri(C1-C60)alkylsilyl, di(C1-C60)alkyl(C6-C60)arylsilyl and tri(C6-C60)arylsilyl;
R₁₀₃ through R₁₀₆ independently represent hydrogen, deuterium, (C1-C60)alkyl, (C1-C60)alkyloxy, halogen, (C4-C60)heteroaryl, (C5-C60)cycloalkyl or (C6-C60)aryl; the heteroaryl, cycloalkyl or aryl of R₁₀₃ through R₁₀₆ may be further substituted by one or more substituent(s) selected from a group consisting of (C1-C60)alkyl with or without halogen substituent(s), (C1-C60)alkyloxy, (C3-C60)cycloalkyl, halogen, cyano, tri(C1-C60)alkylsilyl, di(C1-C60)alkyl(C6-C60)arylsilyl and tri(C6-C60)arylsilyl;
Z₁ and Z₂ independently represent a chemical bond or (C6-C60)arylene with or without one or more substituent(s) selected from (C1-C60)alkyl, (C1-C60)alkyloxy, (C6-C60)aryl, (C4-C60)heteroaryl and halogen;
Ar₂₁ and Ar₂₂ independently represent aryl selected from the following structures, or (C4-C60)heteroaryl: 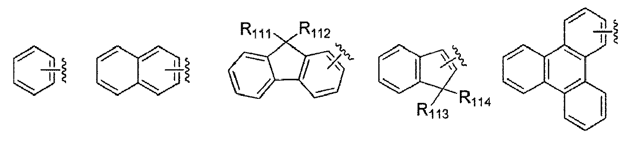 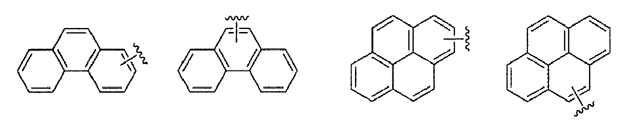 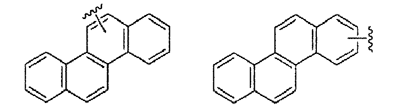 the aryl or heteroaryl of Ar₂₁ and Ar₂₂ may be further substituted by one or more substituent(s) selected from (C1-C60)alkyl, (C1-C60)alkyloxy, (C6-C60)aryl and (C4-C60)heteroaryl;
L₁₁ represents (C6-C60)arylene, (C4-C60)heteroarylene or a group having the following structure: 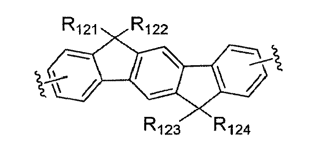 the arylene or heteroarylene of L₁₁ may be further substituted by one or more substituent(s) selected from (C1-C60)alkyl, (C1-C60)alkyloxy, (C6-C60)aryl, (C4-C60)heteroaryl and halogen;
R₁₁₁ through R₁₁₄ independently represent hydrogen, deuterium, (C1-C60)alkyl or (C6-C60)aryl, or each of them may be linked to an adjacent substituent via (C3-C60)alkylene or (C3-C60)alkenylene with or without a fused ring to form an alicyclic ring, or a monocyclic or polycyclic aromatic ring;
R₁₂₁ through R₁₂₄ independently represent hydrogen, deuterium, (C1-C60)alkyl, (C1-C60)alkyloxy, (C6-C60)aryl, (C4-C60)heteroaryl or halogen, or each of them may be linked to an adjacent substituent via (C3-C60)alkylene or (C3-C60)alkenylene with or without a fused ring to form an alicyclic ring, or a monocyclic or polycyclic aromatic ring; 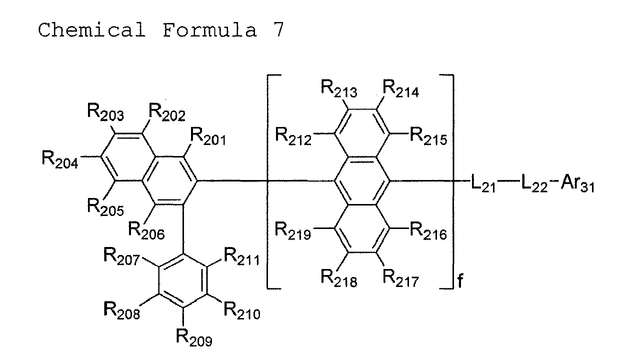 wherein, L₂₁ and L₂₂ independently represent a chemical bond, (C6-C60)arylene or (C3-C60)heteroarylene; the arylene or heteroarylene of L₂₁ and L₂₂ may be further substituted by one or more substituent(s) selected from a group consisting of (C1-C60)alkyl, halogen, cyano, (C1-C60)alkyloxy, (C3-C60)cycloalkyl, (C6-C60)aryl, (C3-C60)heteroaryl, tri(C1-C60)alkylsilyl, di(C1-C60)alkyl(C6-C60)arylsilyl or tri(C6-C60)arylsilyl;
R₂₀₁ through R₂₁₉ independently represent hydrogen, deuterium, halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkyloxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of R₂₀₁ through R₂₁₉ may be linked to an adjacent substituent via (C3-C60)alkylene or (C3-C60)alkenylene with or without a fused ring to form an alicyclic ring, or a monocyclic or polycyclic aromatic ring;
Ar₃₁ represents (C6-C60)aryl, (C4-C60)heteroaryl, 5- or 6-membered heterocycloalkyl containing one or more heteroatom(s) selected from N, O and S, (C3-C60)cycloalkyl, adamantyl, (C7-C60)bicycloalkyl, or a substituent selected from the following structures: 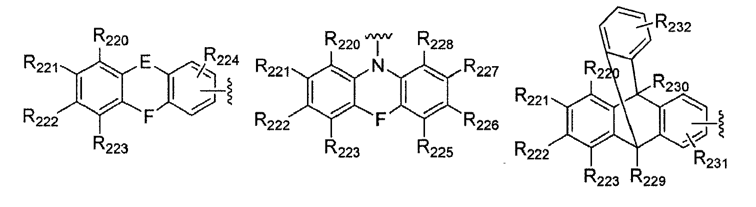 wherein, R₂₂₀ through R₂₃₂ independently represent hydrogen, deuterium, halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl;
E and F independently represent a chemical bond, - (CR₂₃₃R₂₃₄)_{g}-, -N(R2₃₅)-, -S-, -O-, -Si(R₂₃₆) (R₂₃₇)-_{'} -P(R₂₃₈)-, - C(=O)-, -B(R₂₃₉)- , -In(R₂₄₀)-, -Se-, -Ge(R₂₄₁) (R₂₄₂) -, Sn (R₂₄₃) (R₂₄₄)-, -Ga(R₂₄₅)- or - (R₂₄₆) C=C (R₂₄₇) -;
R₂₃₃ through R₂₄₇ independently represent hydrogen, deuterium, halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkyloxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of R₂₃₃ through R₂₄₇ may be linked to an adjacent substituent via (C3-C60)alkylene or (C3-C60)alkenylene with or without a fused ring to form an alicyclic ring, or a monocyclic or polycyclic aromatic ring;
the aryl, heteroaryl, heterocycloalkyl, adamantyl or bicycloalkyl of Ar₃₁; the alkyl, alkenyl, alkynyl, cycloalkyl, heterocycloalkyl, aryl, heteroaryl, arylsilyl, alkylsilyl, alkylamino or arylamino of R₂₀₁ though R₂₃₂ may be further substituted by one or more substituent(s) selected from halogen, (C1-C60)alkyl, (C6-C60)aryl, (C4-C60)heteroaryl, 5-or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkyloxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl;
f is an integer from 1 to 4; and
g is an integer from 0 to 4.

5. The organic electroluminescent device according to claim 3, wherein the organic layer comprises one or more compound(s) selected from a group consisting of arylamine compounds and styrylarylamine compounds.

6. The organic electroluminescent device according to claim 3, wherein the organic layer comprises one or more metal(s) selected from a group consisting of organometals of Group 1, Group 2, 4^{th} period and 5^{th} period transition metals, lanthanide metals and d-transition elements in the Periodic Table of Elements.

7. The organic electroluminescent device according to claim 3, wherein the organic layer comprises an electroluminescent layer and a charge generating layer.

8. A white electroluminescent device which comprises an organic electroluminescent compound represented by Chemical Formula (1) : 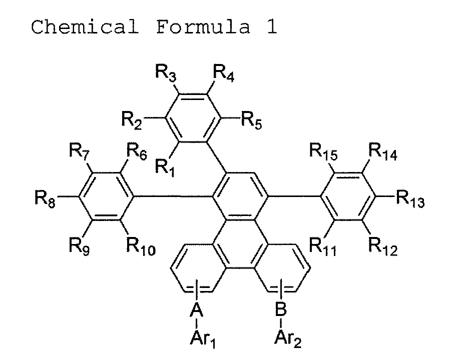 wherein, A and B independently represent a chemical bond, (C6-C60)arylene, (C3-C60)heteroarylene, (C6-C60)arylenoxy, (C1-C60)alkylenoxy, (C6-C60)arylenethio, (C1-C60)alkylenethio or (C1-C60)alkylene;
Ar₁ and Ar₂ independently represent hydrogen or deuterium, or a substituent selected from the following structures: 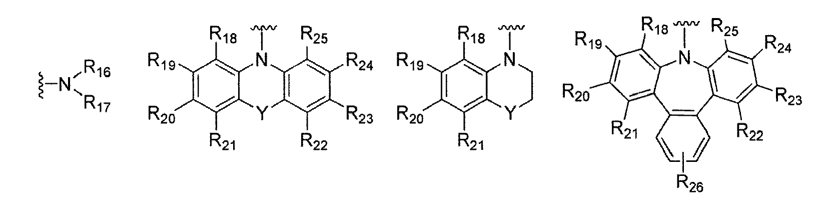 R₁ through R₅ independently represent hydrogen, deuterium, halogen, (C1-C60)alkyl, (C6-C60)aryl, (C3-C60)heteroaryl containing one or more heteroatom(s) selected from N, O and S, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkyl(C6-C60)aryl, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or each of them may be linked to an adjacent substituent via (C3-C60)alkylene or (C3-C60)alkenylene with or without a fused ring to form an alicyclic ring, or a monocyclic or polycyclic aromatic ring;
R₆ through R₁₅ independently represent hydrogen, deuterium, halogen, (C1-C60)alkyl, (C6-C60)aryl, (C3-C60)heteroaryl containing one or more heteroatom(s) selected from N, O and S, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkyl(C6-C60)aryl, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro, hydroxyl, 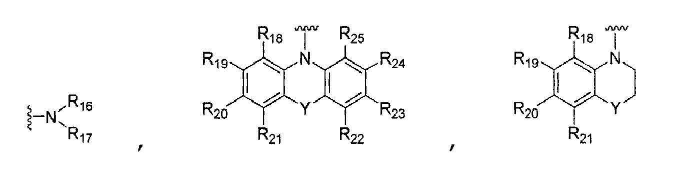 or 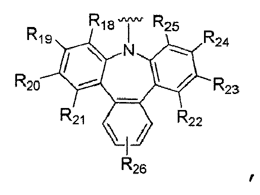 or each of them may be linked to an adjacent substituent via (C3-C60)alkylene or (C3-C60)alkenylene with or without a fused ring to form an alicyclic ring, or a monocyclic or polycyclic aromatic ring;
R₁₆ and R₁₇ independently represent (C6-C60)aryl, (C3-C60)heteroaryl containing one or more heteroatom(s) selected
from N, O and S, 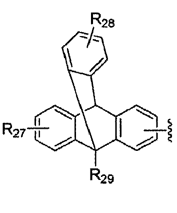 or 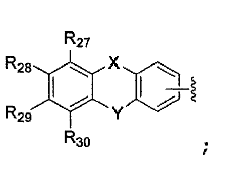 R₁₈ through R₂₆ and R₂₇ to R₃₀ independently represent hydrogen, deuterium, halogen, (C1-C60)alkyl, (C6-C60)aryl, (C3-C60)heteroaryl containing one or more heteroatom(s) selected from N, O and S, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkyl(C6-C60)aryl, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or each of them may be linked to an adjacent substituent via (C3-C60)alkylene or (C3-C60)alkenylene with or without a fused ring to form an alicyclic ring, or a monocyclic or polycyclic aromatic ring;
X and Y independently represent a chemical bond, - (CR₃₁R₃₂)ₘ-, -N(R₃₃)-, -S-, -O-, -Si(R₃₄) (R₃₅) *-,* -P (R₃₆)-, -C(=O)-, -B (R₃₇)-, -In(R₃₈)-, -Se-, -Ge(R₃₉) (R₄₀)*-,* -Sn (R₄₁) (R₄₂)-, Ga(R₄₃)-or-(R₄₄)C=C(R₄₅)-, excluding that both X and Y represent chemical bonds;
R₃₁ through R₄₅ independently represent hydrogen, deuterium, halogen, (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C1-C60)alkyl(C6-C60)aryl, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or R₃₁ and R₃₂, R₃₄ and R₃₅, R₃₉ and R₄₀, R₄₁ and R₄₂, or R₄₄ and R₄₅ may be linked via (C3-C60)alkylene or (C3-C60)alkenylene with or without a fused ring to form an alicyclic ring, or a monocyclic or polycyclic aromatic ring;
the arylene, heteroarylene, arylenethio, arylenoxy, alkylenoxy or alkylenethio of A and B; the alkyl, aryl, heteroaryl, heterocycloalkyl, cycloalkyl, alkylsilyl, arylsilyl, alkenyl, alkynyl, alkylamino or arylamino of R₁ through R₄₅ may be further substituted by one or more substituent(s) selected from a group consisting of halogen, (C1-C60)alkyl, halo(C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, carbazolyl, (C1-C60)alkylamino, (C6-C60)arylamino, (C1-C60)alkyl(C6-C60)aryl, (C6-C60)ar(C1-C60)alkyl, (C1-C60)alkyl(C6-C60)aryl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro and hydroxyl;
m is an integer from 1 to 4; provided that, at least one substituent(s) among Ar₁, Ar₂, R₆,
R₇, R₈, R₉, R₁₀, R₁₁, R₁₂, R₁₃, R₁₄ and R₁₅ represent (s)

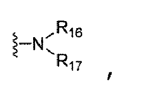

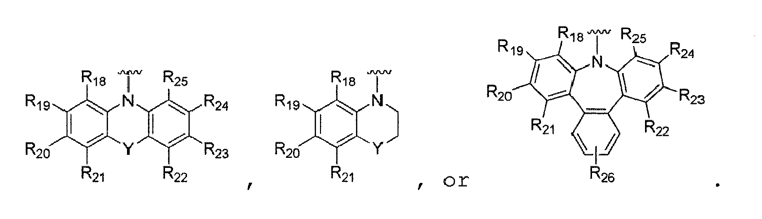

9. An organic solar cell which comprises an organic electroluminescent compound represented by Chemical Formula (1) : 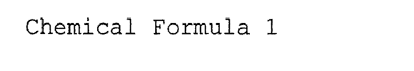 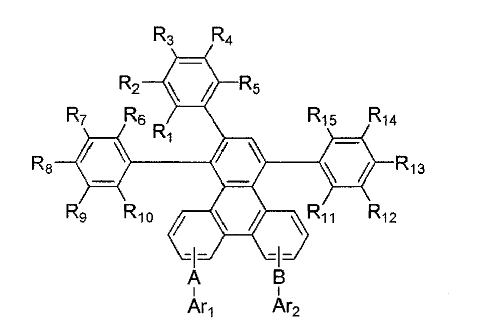 wherein, A and B independently represent a chemical bond, (C6-C60)arylene, (C3-C60)heteroarylene, (C6-C60)arylenoxy, (C1-C60)alkylenoxy, (C6-C60)arylenethio, (C1-C60)alkylenethio or (C1-C60)alkylene;
Ar₁ and Ar₂ independently represent hydrogen or deuterium, or a substituent selected from the following structures: 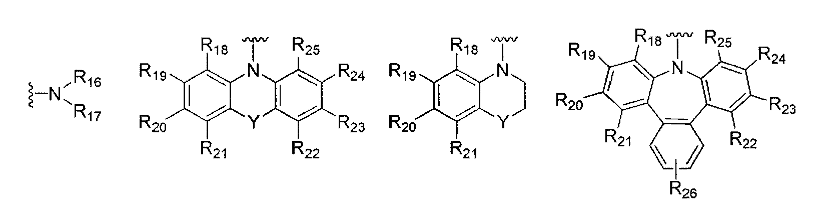 R₁ through R₅ independently represent hydrogen, deuterium, halogen, (C1-C60)alkyl, (C6-C60)aryl, (C3-C60)heteroaryl containing one or more heteroatom(s) selected from N, O and S, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkyl(C6-C60)aryl, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or each of them may be linked to an adjacent substituent via (C3-C60)alkylene or (C3-C60)alkenylene with or without a fused ring to form an alicyclic ring, or a monocyclic or polycyclic aromatic ring;
R₆ through R₁₅ independently represent hydrogen, deuterium, halogen, (C1-C60)alkyl, (C6-C60)aryl, (C3-C60)heteroaryl containing one or more heteroatom(s) selected from N, O and S, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkyl(C6-C60)aryl, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro, hydroxyl, 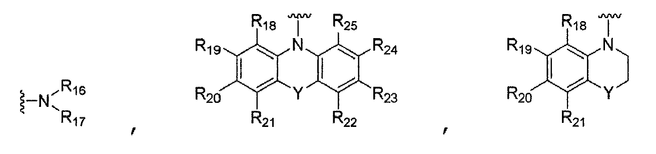 or 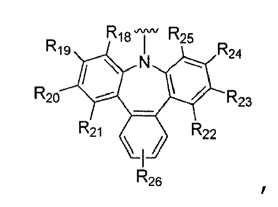 or each of them may be linked to an adjacent substituent via (C3-C60)alkylene or (C3-C60)alkenylene with or without a fused ring to form an alicyclic ring, or a monocyclic or polycyclic aromatic ring;
R₁₆ and R₁₇ independently represent (C6-C60)aryl, (C3-C60)heteroaryl containing one or more heteroatom(s) selected
from N, O and S, 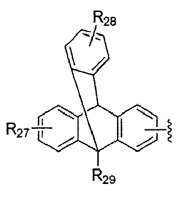 or 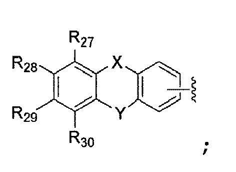 R₁₈ through R₂₆ and R₂₇ to R₃₀ independently represent hydrogen, deuterium, halogen, (C1-C60)alkyl, (C6-C60) aryl, (C3-C60)heteroaryl containing one or more heteroatom(s) selected from N, O and S, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkyl(C6-C60)aryl, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or each of them may be linked to an adjacent substituent via (C3-C60)alkylene or (C3-C60)alkenylene with or without a fused ring to form an alicyclic ring, or a monocyclic or polycyclic aromatic ring;
X and Y independently represent a chemical bond, - (CR₃₁R₃₂)ₘ-, -N(R₃₃)-, -S-, -O-, -Si(R₃₄)(R₃₅)-, -P(R₃₆)-, -C(=O)-, -B(R₃₇)-, -In(R₃₈)-, -Se-, -Ge (R₃₉) (R₄₀)-, -Sn(R₄₁) (R₄₂)-, - Ga(R₄₃)- or -(R44)C=C(R₄₅)-; excluding that both X and Y represent chemical bonds;
R₃₁ through R₄₅ independently represent hydrogen, deuterium, halogen, (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C1-C60)alkyl(C6-C60)aryl, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or R₃₁ and R₃₂, R₃₄ and R₃₅, R₃₉ and R₄₀, R₄₁ and R₄₂, or R₄₄ and R₄₅ may be linked via (C3-C60)alkylene or (C3-C60)alkenylene with or without a fused ring to form an alicyclic ring, or a monocyclic or polycyclic aromatic ring;
the arylene, heteroarylene, arylenethio, arylenoxy, alkylenoxy or alkylenethio of A and B; the alkyl, aryl, heteroaryl, heterocycloalkyl, cycloalkyl, alkylsilyl, arylsilyl, alkenyl, alkynyl, alkylamino or arylamino of R₁ through R₄₅ may be further substituted by one or more substituent(s) selected from a group consisting of halogen, (C1-C60)alkyl, halo(C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, carbazolyl, (C1-C60)alkylamino, (C6-C60)arylamino, (C1-C60)alkyl(C6-C60)aryl, (C6-C60)ar(C1-C60)alkyl, (C1-C60)alkyl(C6-C60)aryl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro and hydroxyl;
m is an integer from 1 to 4; provided that, at least one substituent(s) among Ar₁, Ar₂, R₆,
R₇, R_{8,} R₉ R₁₀, R₁₁, R₁₂, R₁₃, R₁₄ and R₁₅ represent (s) 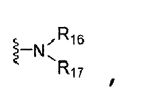 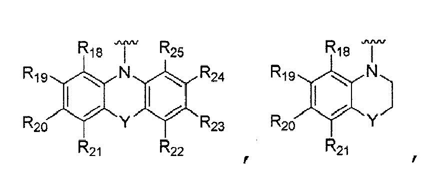 or
